# EUROPEAN PATENT SPECIFICATION

(11) **EP 4 376 874 B1**
(45) Date of publication and mention of the grant of the patent: **04.03.2026**
(21) Application number: 22758100.6
(22) Date of filing: 28.07.2022
(51) Int. Cl.: A61K 31/519, C07K 14/47, C07K 14/725, C07K 14/705, A61K 40/11, A61K 40/24, A61K 40/31, A61K 40/32, A61K 40/42, A61K 40/46, A61K 31/506, A61K 45/06

(54) **CHIMERIC TIM4 RECEPTORS AND USES THEREOF**
CHIMÄRE TIM4-REZEPTOREN UND VERWENDUNGEN DAVON
RÉCEPTEURS TIM4 CHIMÉRIQUES ET LEURS UTILISATIONS

(30) Priority: 28.07.2021 US 202163226643 P; 28.07.2021 US 202163226712 P; 28.07.2021 US 202163226736 P; 13.08.2021 WO PCT/US2021/046014; 13.08.2021 WO PCT/US2021/046041; 13.08.2021 WO PCT/US2021/046043; 16.02.2022 US 202263311016 P; 16.02.2022 US 202263311042 P; 16.02.2022 US 202263311043 P; 16.02.2022 US 202263311045 P; 29.04.2022 US 202263336972 P; 29.04.2022 US 202263336980 P; 13.05.2022 US 202263341999 P; 13.05.2022 US 202263342031 P
(43) Date of publication of application: 05.06.2024
(73) Proprietor: Cero Therapeutics Holdings, Inc., South San Francisco, CA 94080 (US)
(72) Inventor: COREY, Daniel Mark, Menlo Park, California 94025 (US); CIENIEWICZ, Brandon, South San Francisco, California 94080 (US); THOMAS, Sunil, San Bruno, California 94066 (US)
(74) Representative: Hoffmann Eitle
(86) International application number: PCT/US2022/074281
(87) International publication number: WO 2023/010097

(56) References cited:
- WO-A1-2019/191340
- WO-A1-2021/067875
- WO-A1-2022/036265
- WO-A1-2022/036285
- CN-A- 106 749 675

## Description

### REFERENCE TO AN ELECTRONIC SEQUENCE LISTING

The contents of the electronic sequence listing (200265_41801WO_SequenceListing.xml; Size: 266,847 bytes; and Date of Creation: July 28, 2022).

### BACKGROUND

Upon exposure to antigen, naive antigen-specific T cells undergo activation that promotes their clonal expansion, differentiation, and development into functional, effector T cells that can kill cells expressing the cognate antigen (e.g., tumor cells). Following antigen clearance, the majority of effector T cells undergo apoptosis, and a subset of the surviving effector T cells differentiate into memory T cells that can confer long-term protection against antigen re-exposure. However, prolonged antigen exposure may result in T cell exhaustion, enabling the persistence of tumor cells. T cell exhaustion refers to a dysfunctional state acquired by T cells experiencing persistent TCR stimulation characterized by upregulated expression of immune checkpoint molecules (e.g., PD-1, CTLA-4, Tim-3), impaired effector function, poor proliferation, and metabolic defects. Engineered T cells expressing chimeric antigen receptors (CARs) can also develop exhaustion.

WO 2019/191340 A1 relates to cellular immunotherapy compositions comprising a combination of immune cells or cellular subsets modified with chimeric engulfment receptors and chimeric antigen receptors/or T cell receptor binding proteins, and methods of using such cellular immunotherapy compositions.

WO 2021/067875 A1 relates to chimeric Tim4 receptors as well as cells modified to include chimeric Tim4 receptor molecules.

CN 106 749 675 A relates to recombinant lentiviruses encoding a chimeric antigen receptor.

### BRIEF SUMMARY

The invention is set out in the appended set of claims.
In some embodiments, the extracellular domain of the chimeric Tim4 receptors described herein optionally includes an extracellular spacer domain positioned between and connecting the binding domain and transmembrane domain. In some embodiments, the TLR2 TIR signaling domain consists of the amino acid sequence set forth in SEQ ID NO: 17.

In some embodiments, the chimeric Tim4 receptor comprises the amino acid sequence of SEQ ID NO: 18 or SEQ ID NO: 18 absent amino acids 1-24.

In some embodiments, the chimeric Tim4 receptor comprises the amino acid sequence of SEQ ID NO: 19 or SEQ ID NO: 19 absent amino acids 1-24.

### BRIEF DESCRIPTION OF THE SEVERAL VIEWS OF THE DRAWINGS

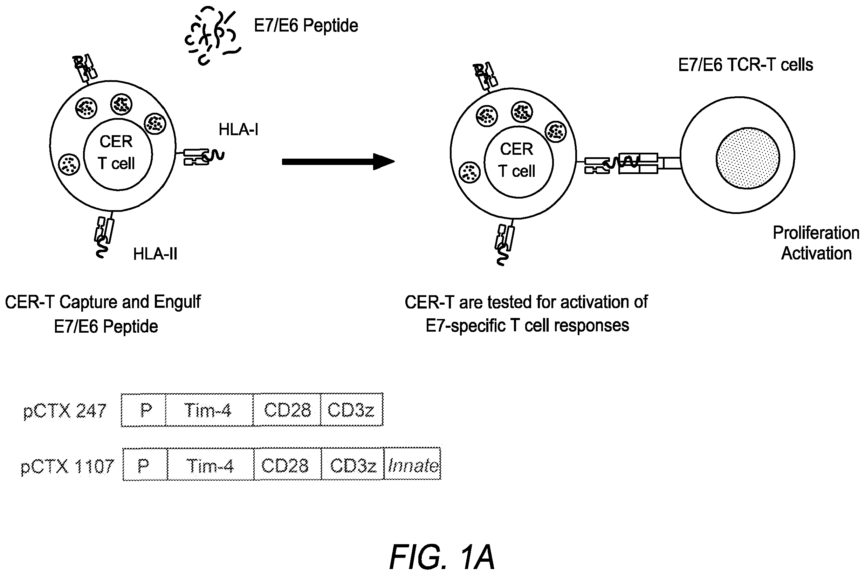

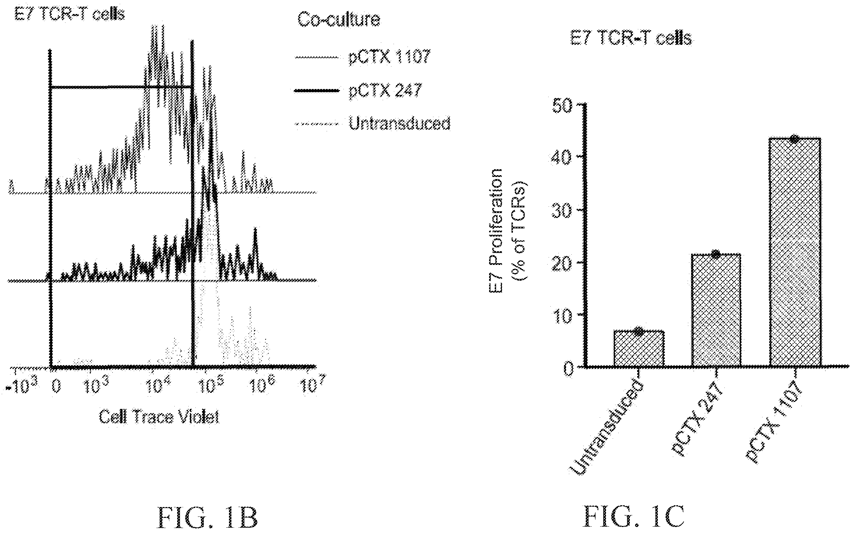

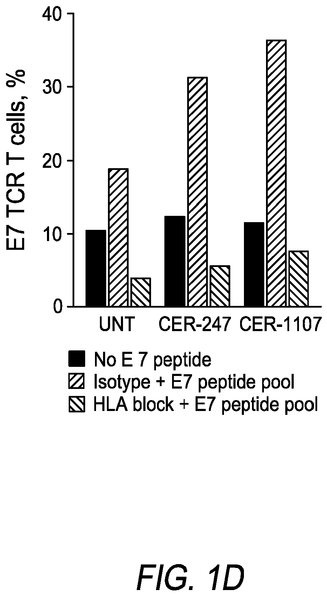

**FIGS. 1A-1D**: an in vitro co-culture system to evaluate T cell antigen presentation function shows that addition of T cells containing chimeric Tim4 receptors with a TLR intracellular signaling domain added to the CD3ζ signaling domain (and optionally a CD28 signaling domain) show enhanced capacity to serve as an antigen presenting cell (APC). **FIG. 1A**: a schematic of pCTX247 (also referred to herein as CER247) and pCTX1107 (also referred to herein as CER1107 or CER1236) chimeric Tim4 receptors and pCTX1107 T cells pulsed with E7 peptides and co-cultured with E7-specific T cells to evaluate their antigen presentation capacity. **FIG. 1B**: E7-specific proliferation responses were measured by CT Violet dye dilution after 6 days in the presence of autologous CER-T (TLR containing or non-containing CERs) pulsed with E7 peptides. **FIG. 1C**: The addition of a TLR-2 ICD (pCTX1107) triggered proliferation responses of E7-specific TCRs whereas non-TLR containing CERs were less stimulatory. All data collected by FACS. Cell tracing show E7 TCR-T cells using an anti-mouse TCRb. E7 TCR-T cells were labeled with CT Violet at the time of co-culture. **FIG. 1D**: Percentage of proliferated E7 TCR cells in culture among live CD3+ cells determined by flow cytometry based on staining of cell trace violet low E7 TCRb+ cells.

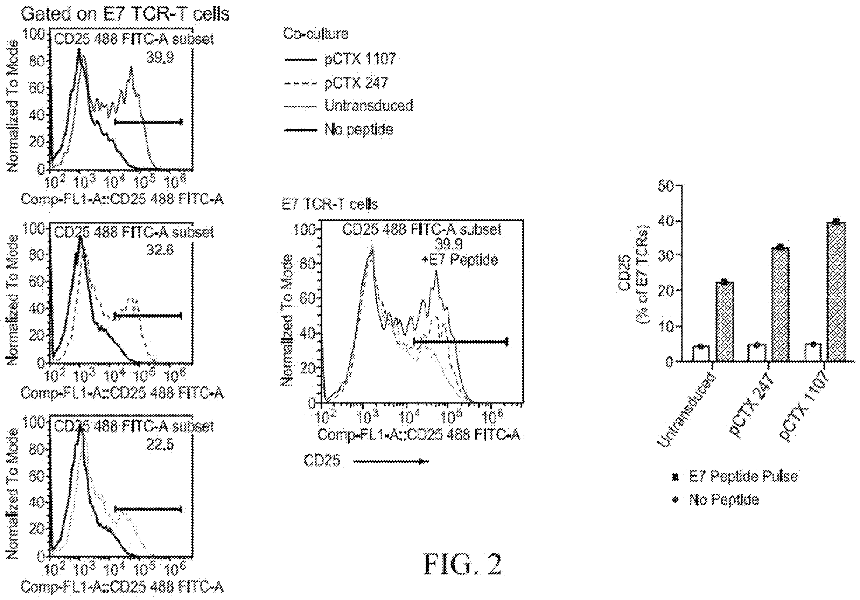

**FIG. 2**: T cells containing chimeric Tim4 receptors with a TLR intracellular signaling domain added to the CD3ζ signaling domain (and optionally a CD28 signaling domain) are strong stimulators of autologous E7-specific T cell responses. CER-T cells were pulsed with E7 peptide and tested for their capacity to trigger an autologous E7-specific T cell response. CD25, a T cell activation marker, was evaluated on E7 TCR-T cells, 24 hours after CER-T cells were pulsed with E7 peptides. CER-T pCTX1107 contains a TLR-2 intracellular domain (ICD) and is a strong stimulator of E7-specific activation.

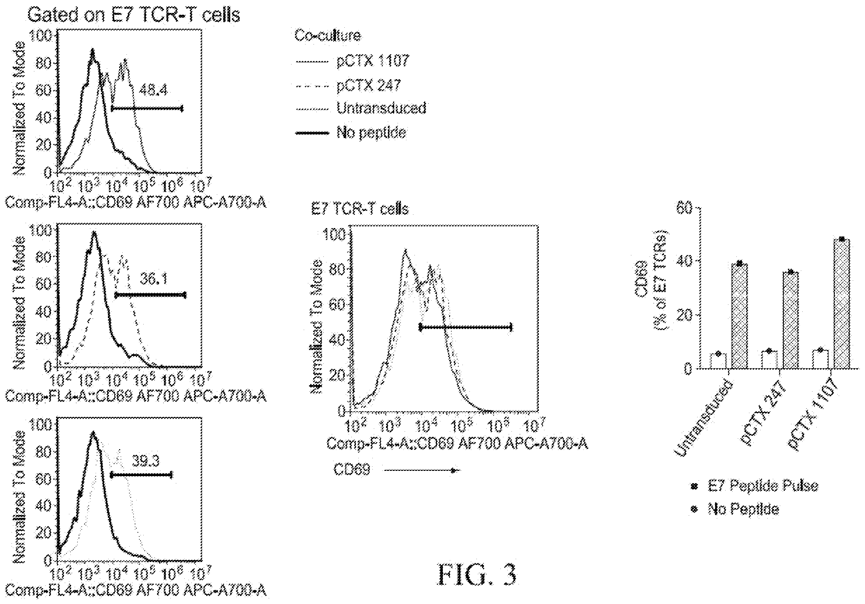

**FIG. 3****:** T cells containing chimeric Tim4 receptors with a TLR intracellular signaling domain added to the CD3ζ signaling domain (and optionally a CD28 signaling domain) are strong stimulators of autologous E7-specific T cell responses. CER-T cells were pulsed with E7 peptide and tested for their capacity to trigger an autologous E7-specific T cell response. CD69, an early marker for T cell activation, was evaluated on E7 TCR-T cells, 24 hours after CER-T cells were pulsed with E7 peptides. CER-T pCTX1107 contains a TLR-2 intracellular domain and is a strong stimulator of E7-specific activation.
**FIG. 4****:** Robust Cell Surface Chimeric Tim4 Receptor Expression and Detection Using anti-Tim-4 antibodies. Chimeric Tim4 Receptor cell surface staining was evaluated using an anti-Tim-4 antibody (9F4) on day 5 post-transduction. pCTX1107 contains a TLR-2 intracellular sequence. The lentiviral cassette contains a p2A fragment followed by a truncated EGFRt polypeptide.

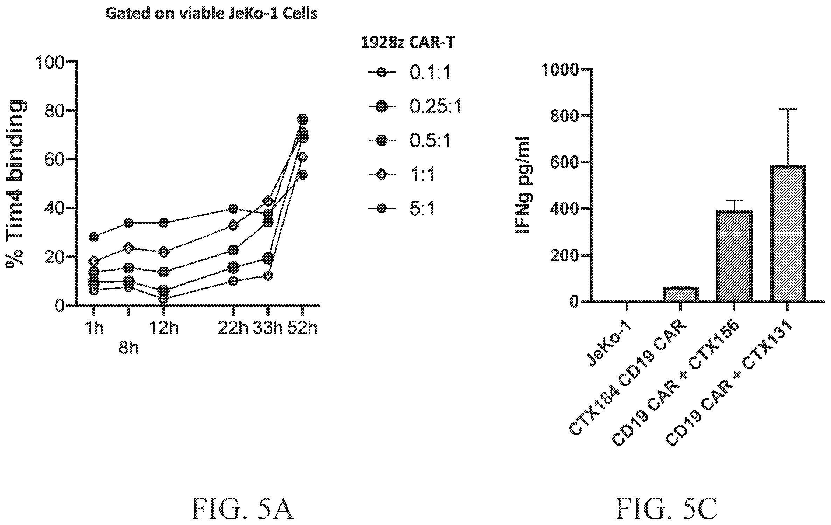

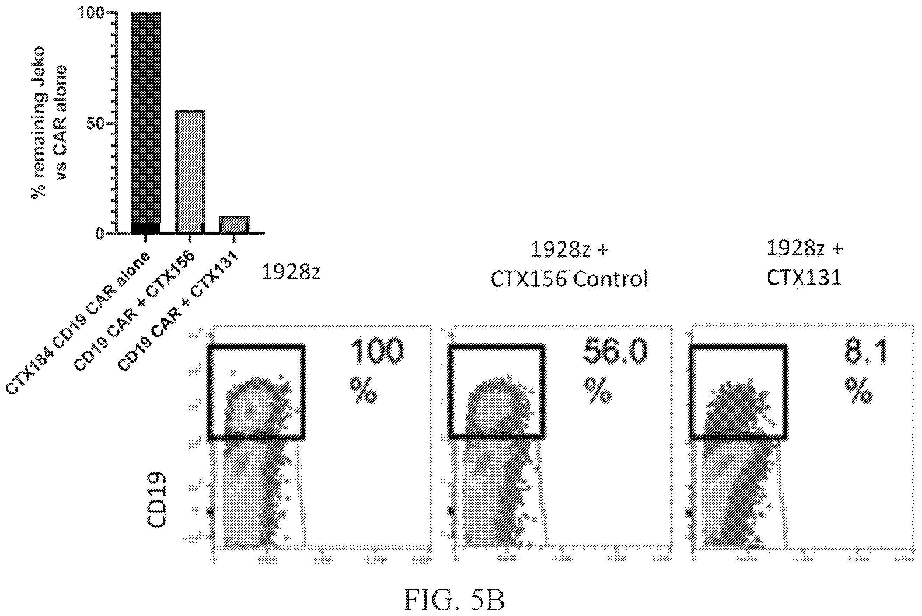

**FIGS. 5A-5C****:** pCTX131 CER-T cells enhance the potency CD1928z CAR-T cell. **FIG. 5A****:** Kinetics of Ptd-Ser induction on JeKo-1 MCL cells in response to CD1928z CAR-T cells. JeKo-1 MCL cells were co-cultured at increasing effector:target ratios and evaluated for Ptd-Ser induction over time. Kinetic curves represent the percentage of viable JeKo-1 targets that bind to rTim-4, a Ptd-Ser binding protein. **FIG. 5B** **top:** JeKo-1 cells were co-cultured with pCTX184 (1928z) + pCTX131, pCTX184 + CTX156 Control T cells or pCTX184 cells alone for 48hrs at a 1:1 T cell: JeKo-1 ratio. Samples treated with CTX184 + CER 131 exhibited substantially fewer tumor cells in culture ~2 days later when compared to samples treated with CTX184 alone, or CTX184 + CTX156 Control T cells. All data were collected via FACs. **FIG. 5B** **bottom:** Representative flow plots for enumeration of remaining JeKo-1 cells after 48h co-culture. **FIG. 5B** **top:** Raw flow data from FIG. 5B bottom used to calculate bar graph of remaining JeKo-1 cells. **FIG. 5C****:** JeKo-1 cells were co-cultured with pCTX184 (1928z) + pCTX131, pCTX184 + CTX156 Control T cells or pCTX184 cells alone for 48hrs at a 0.5:1 T cell: JeKo-1 ratio. Samples treated with CTX184 + CER 131 exhibited increased IFN-γ secretion as compared to samples treated with CTX184 alone, or CTX184 + CTX156 Control T cells.
**FIGS. 6A-6B****:** pCTX131 Chimeric Tim4 Receptor-T cells enhance the potency CD1928z CAR-T cells. Cytotoxic responses evaluating mixtures of CD1928z CAR-T (pCTX184, also called CAR184) + pCTX131 Chimeric Tim4 Receptor-T cells. **FIG. 6A****:** pCTX131 Chimeric Tim4 Receptor-T cells were combined with CD1928z CAR-T cells (pCTX184) at varying ratios and JekO-1 cell counts were quantified over time. **FIG. 6B****:** Caspase 3/7 responses. All data were collected via incucyte.

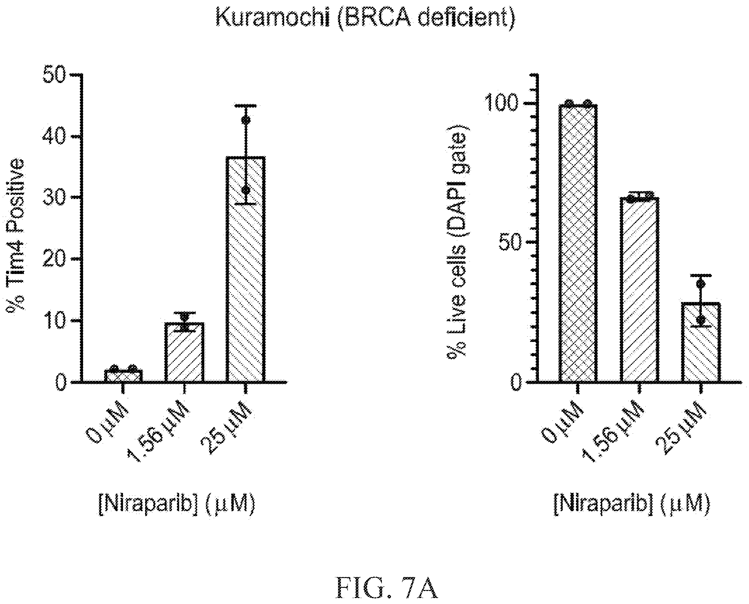

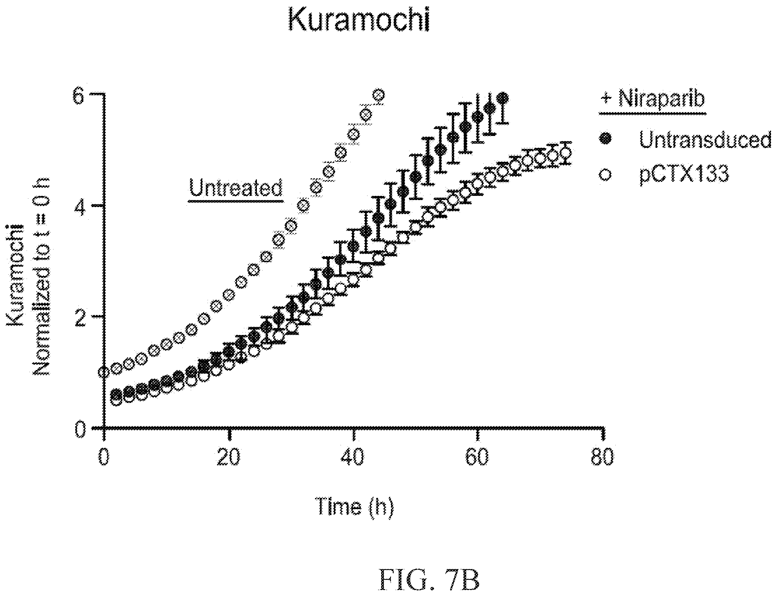

**FIGS. 7A-7B****:** pCTX133, a TLR-2 containing Chimeric Tim4 Receptor Enhances Potency of Niraparib in an Ovarian Cancer Model. **FIG. 7A**: Flow cytometry measurement of surface PtdSer. Kuramochi cells were treated with 1.56 or 25 µM Niraparib or with equivalent volume of DMSO (control). 48 hours later samples were trypsinized and stained using a Tim4-Fc followed by a fluorescently-labeled secondary antibody to the Tim4-Fc. **FIG. 7B****:** Kuramochi cells pre-treated for ~20 hours with 1.56 µM Niraparib were co-cultured with pCTX133 and Untransduced CD4 T cells from donor 32 at a 2:1 T cell:Kuramochi ratio and a final Niraparib concentration of 1.56 µM. Samples treated with Niraparib + pCTX133 exhibited substantially fewer tumor cells in culture ~3 days later when compared to samples treated with Niraparib alone, or Niraparib + untransduced T cells. All data were collected via IncuCyte.
**FIGS. 8A-8B****:** Chimeric Tim4 Receptor-T Cell + BTK inhibitor (ibrutinib) combination for hematologic malignancy. **FIG. 8A****:** Ibrutinib induces expression of phosphatidylserine on target cells. **FIG. 8B****:** Synergistic Chimeric Tim4 Receptor-T cell mediated cell killing in combination with BTK inhibitor small molecule. CTX136 (Tim4-CD28-CD3z) T cells co-cultured at a 3:1, 2:1, and 1:1 E:T in presence of Ibrutinib showed a substantial increase in killing as compared to empty vector transduced cells or Ibrutinib treatment alone.
**FIGS. 9A-9B** show transfection of Jurkat cells with various chimeric Tim4 receptor constructs, pCTX1183, pCTX1161, pCTX1189, pCTX1184, pCTX1163, pCTX1162, pCTX1190, pCTX1186, pCTX1187, pCTX1164, pCTX1185, and pCTX1165. FIG. 9B is normalized to untransfected cells.
**FIG. 10** is a bar graph showing activation of HPV E7 TCR T cells mediated by antigen presentation by chimeric Tim4-T cells is blocked by anti-HLA-I antibodies.
**FIG. 11** depicts various chimeric Tim4 receptors (also referred to herein as chimeric engulfment receptors (CERs)) that were designed. SP = signal peptide; TMD = transmembrane domain.
**FIGS. 12A-12B** depict induction of phosphatidylserine exposure in REC-1 and JEKO-1 Cells with ibrutinic treatment at 24 hours (FIG. 12A) and 48 hours (FIG. 12B).

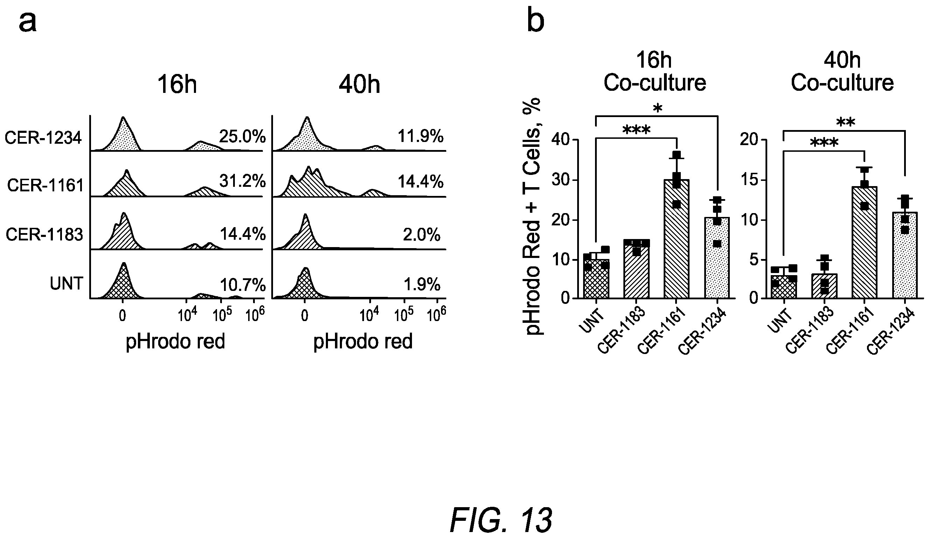

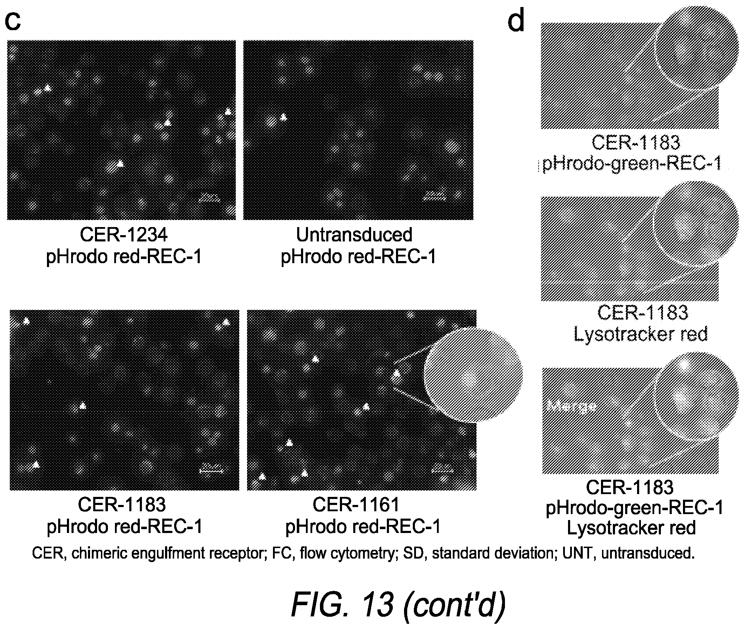

**FIGS. 13A-13D** depict phagocytic activity of REC-1 cells by CER T cells after ibrutinib treatment. **FIG. 13A****:** Representative flow cytometry histograms showing engulfment of pHrodo red-labeled REC-1 target cells by CER1234, CER1161, and CER1183 T cells. **FIG. 13B****:** Graphical summary of flow cytometry data showing engulfment of pHrodo red-labeled REC-1 target cells by CER1234, CER1161, and CER1183 T cells. *P<0.01, **P<0.001, ***P<0.0001; mean + SD, n = 4. **FIG. 13C****:** Representative fluorescence microscopy images (at 40x) showing engulfment of pHrodo red-labeled REC-1 target cells by CER1234, CER1161, and CER1183 T cells. **FIG. 13D****:** Engulfed pHrodo-green-labeled REC-1 tumor fragments co-localize into Lyso-tracker red-labeled lysosomes.
**FIG. 14A-14B****:** Cytotoxic activity of CER1183 and CER1234 T Cells with Ibrutinib Against REC-1 Cells. **FIG. 14A****:** Vehicle pre-treatment followed by +/-0.5 µM ibrutinib. **FIG 14B****:** µM ibrutinib pre-treatment followed by +/- 0.5 µM ibrutinib.
**FIGS. 15A-15B****:** Induction of Cell-Surface Markers of T-Cell Activation in Ibrutinib-Treated Co-Cultures of CER1183 and CER1234 T cells and REC-1 Target Cells. Samples depicted in bar graphs from left to right, untransduced, CER-1183, and CER-1234. **FIG. 15A****:** PD-1 expression in co-cultures. **FIG. 15B****:** 4-1BB expression in co-cultures.
**FIGS. 16A-16H****:** Induction of Cytokines in Ibrutinib-Treated Co-Cultures of CER1183 and CER1234 T cells and REC-1 Target Cells. Samples depicted in bar graphs from left to right, untransduced, CER-1183, and CER-1234. **FIG. 16A****:** TNFα induction. **FIG. 16B****:** IL-2 induction. **FIG. 16C****:** IFNγ. **FIG. 16D****:** IL-5 induction. **FIG. 16E****:** IL-6 induction. **FIG. 16F****:** IL-10 induction. **FIG. 16G****:** IL-4 induction. **FIG. 16H****:** granzyme B induction.
**FIGS. 17A-17B****:** Expression of CER1236 T cells. CER1236 T cells readily express Tim4 on transduced T cells 6 days post-transducion.
**FIG. 18****:** CER T-cell characteristics. Top graph: memory T cell subsets from top to bottom: Temra, Tem, Tcm, Naive.

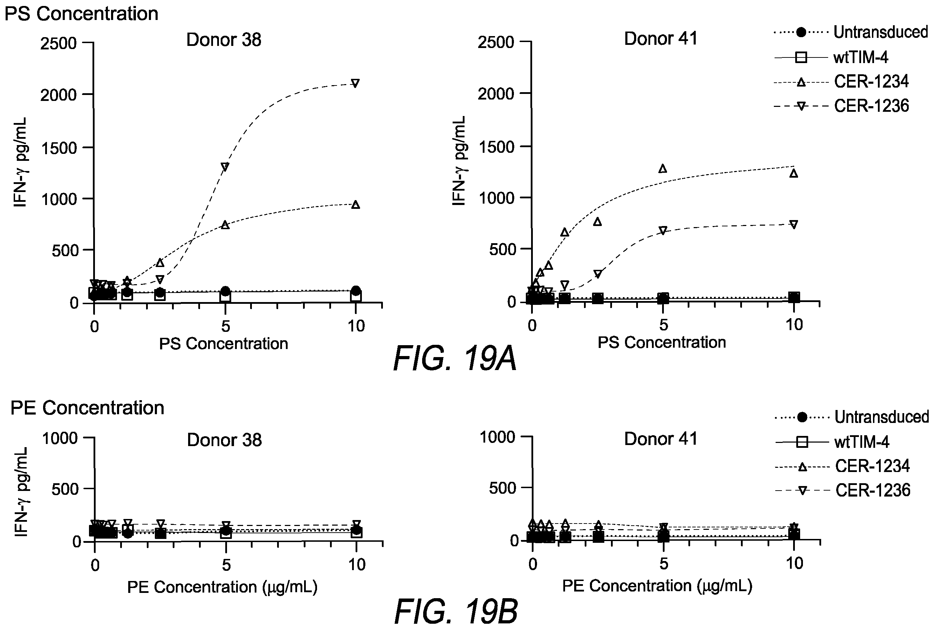

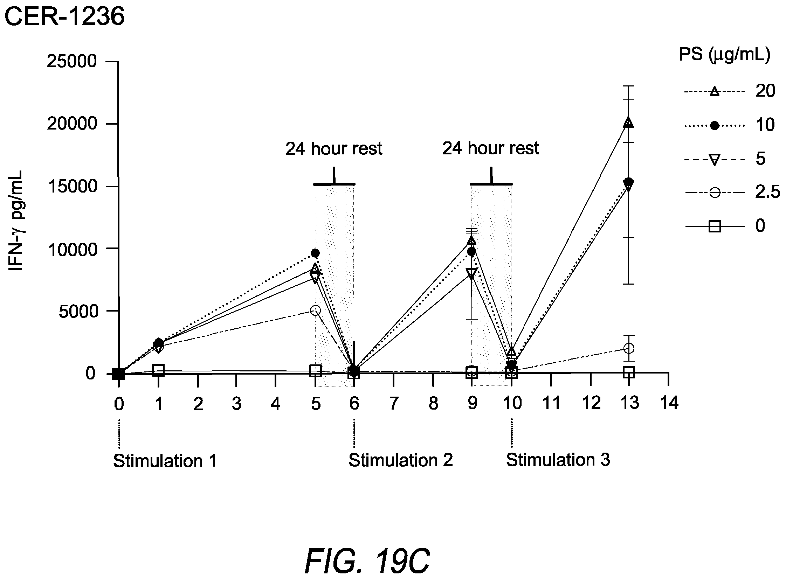

**FIGS. 19A-19C****:** CER T cells are activated and produce IFN-γ in response to phosphatidylserine engagement in a cell-free in vitro system. **FIG. 19A****:** IFN-γ production in response phosphatidylserine (PS). IFN-γ production was induced in CER-1236 and CER-1234 T cells stimulated with PS (**FIG. 19A**), but not with phosphatidylethanolamine (PE) (**FIG. 19B**). PS EC50 values for CER-1234 and CER-1236 from Donors 38 and 41 were as follows: CER-1234, 3.52 µg/mL (SE ± 0.13) and 1.88 µg/mL (SE ± 0.48), respectively; CER-1236, 4.74 µg/mL (SE ± 0.02), and 3.16 µg/mL (SE ± 0.09), respectively. **FIG. 19C****:** CER1236 T cells are capable of recursive stimulation in vitro. Recursive IFN-γ induction with 3 serial stimulations at the indicated time points following a 24-hour rest after the first stimulation.
**FIG. 20****:** FACs and imaging-based detection of pHrodo dye-labeled tumor cell phagocytosis and endocytosis.
**FIG. 21****:** CER1236 T cells exhibit increased engulfment of Jeko1 TMEM30A^{-/-} cells (left panel - fluorescent microscopy image; right panel phagocytic index values).
**FIG. 22****:** CER1234 or CER1236 T cells display cytotoxic function against Jeko1 TMEM30A^{-/-} MCL cells as measured by incucyte assay.
**FIG. 23****:** CER1236 T cells synergize with ibrutinib to enhance killing of REC-1 MCL cells.
**FIG. 24****:** Ibrutinib-induced phosphatidylserine exposure enhances CER1234 T cell cytokine response (IFN-γ - left graph; granzyme B - right graph; untransduced left sample, CER-1234 right sample).

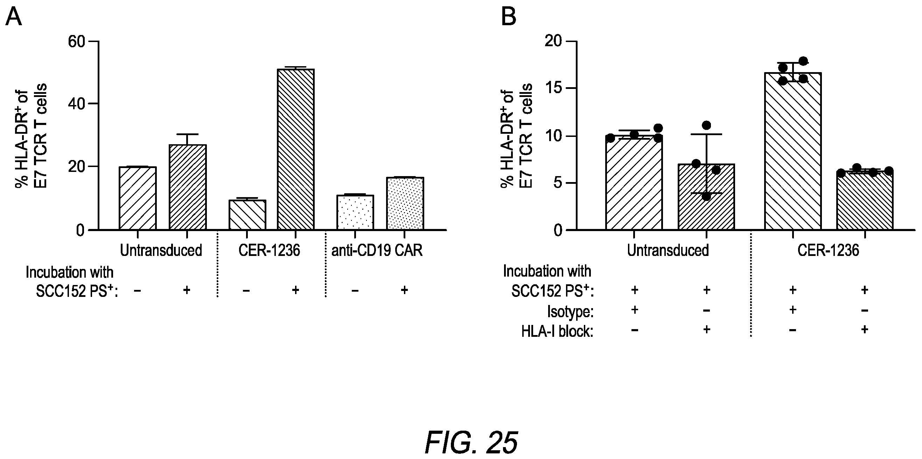

**FIGS. 25A-25B****:** CER1236 T cells activate E7 TCR T cells via presentation of tumor cell-derived E7 antigen in an HLA-I-dependent manner. **FIG. 25A****:** Following co-culture with E7 oncoprotein positive/PS-positive SCC152 target cells, CER-1236, but not untransduced T cells or anti-CD19 CAR T cells, induced HLA-DR on E7 TCR cells. **FIG. 25B****:** In a separate experiment, induction of HLA-DR on E7 TCR cells following co-culture of CER-1236 with E7 oncoprotein positive/PS-positive SCC152 cells was blocked by HLA-I blocking antibody, but not a control isotype antibody. Bars depict percentage of E7 TCR T cells that express the activation marker, HLA-DR. Error bars show SEM.
**FIG. 26****:** CER1236 T cells mediate anti-tumor effects in MCL xenografts (Jeko1 TMEM30A^{-/-} cells) (top left). Top right graph shows in vivo expansion data. Bottom graph shows cytotoxicity data.

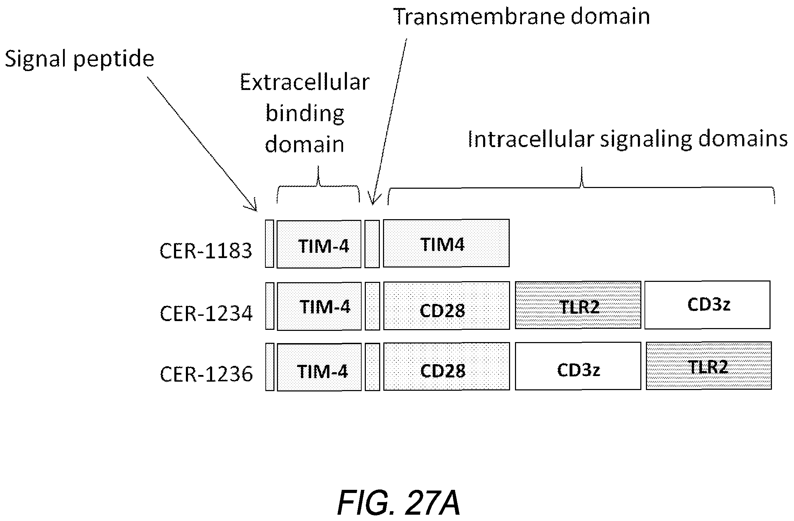

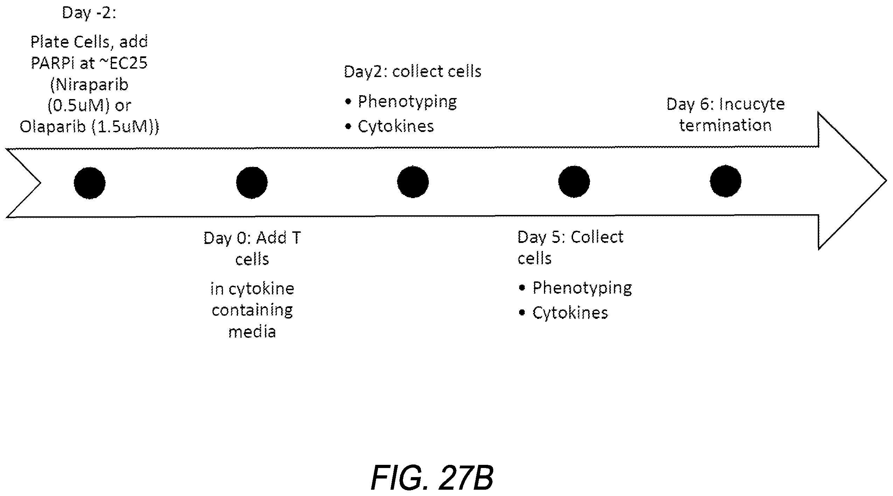

**FIGS. 27A-27B** show a (**FIG. 27A**) schematic of chimeric Tim4 receptors, CER-1234 and CER1236 of the present disclosure; and (**FIG. 27B**) a schematic of assay set up for assessing immune phenotype of the chimeric Tim4 receptors in co-culture with PARP inhibitor treated ovarian cancer cells. CER-1234 and CER1236 have an extracellular Tim4 binding domain, CD28 transmembrane domain, CD28 signaling domain, TLR2 signaling domain, and CD3ζ signaling domain, and showing the orientation of the signaling domains in the chimeric Tim4 receptor constructs. A control molecule, CER-1183, having a Tim4 extracellular binding domain, a Tim4 transmembrane domain, and Tim4 signaling domain is also depicted.
**FIG. 28** shows the various T cell products created from transduction of donor T cells with CER-1234, CER-1236, and control CER-1183, the multiplicity of infection (MOI), and % T cells that are CER+.

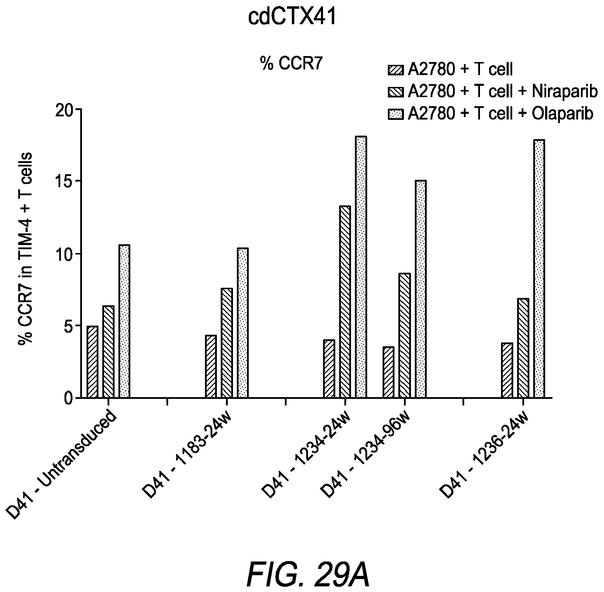

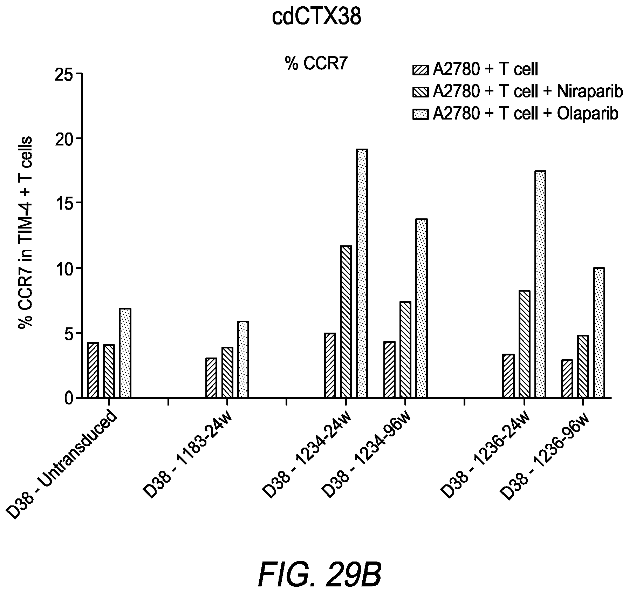

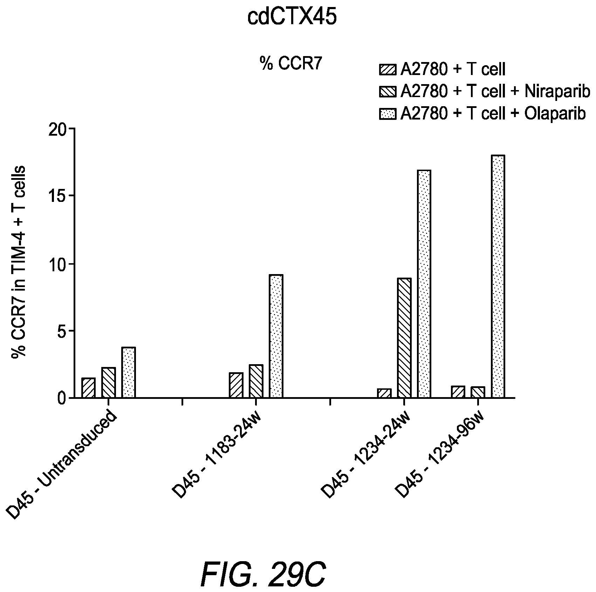

**FIGS. 29A-29C** show % of CCR7+ expressing cells in Tim4+ T cells (T cells transduced with CER-1234, CER-1236, or control CER-1183) at 120 hours of co-culture of chimeric Tim4 receptor transduced T cells, olaparib or niraparib, and target A2780 ovarian cancer cells. Samples on bar graph from left to right: A2780 + T cell, A2780 + T cell + niraparib, and A2780 + T cell + olaparib. FIG. 29A shows CCR7 expression on Tim4+ T cells from donor 41. FIG. 29B shows CCR7 expression on Tim4+ T cells from donor 38. FIG. 3C shows CCR7 expression on Tim4+ T cells from donor 45. For FIGS. 29A-29C, co-culture conditions A2780+T cell; A2780 + T cell + niraparib; A27080 + T cell + olaparib are shown from left to right in the bar graph for each donor T cell sample.

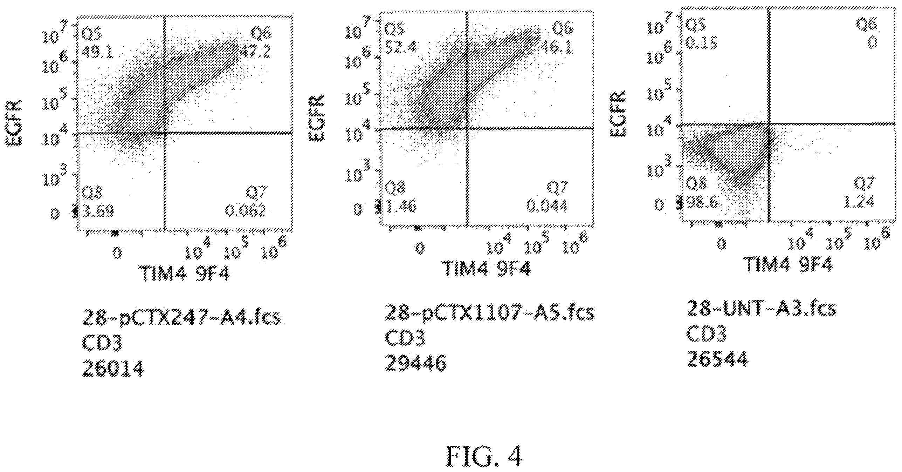

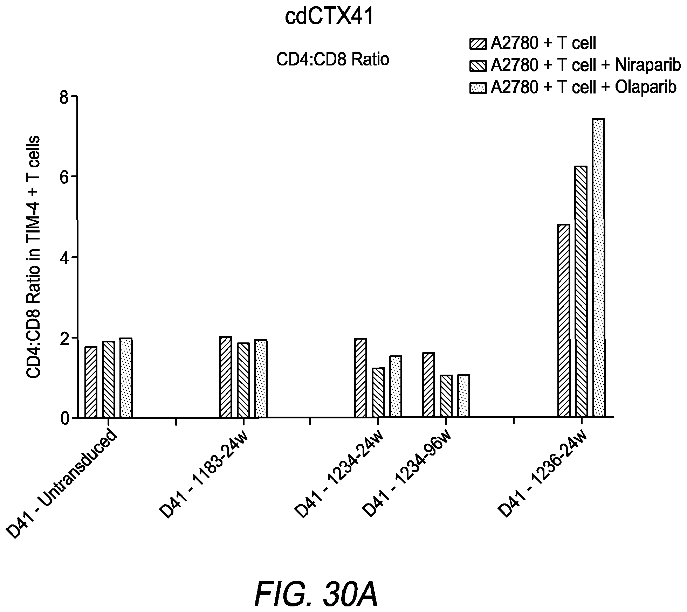

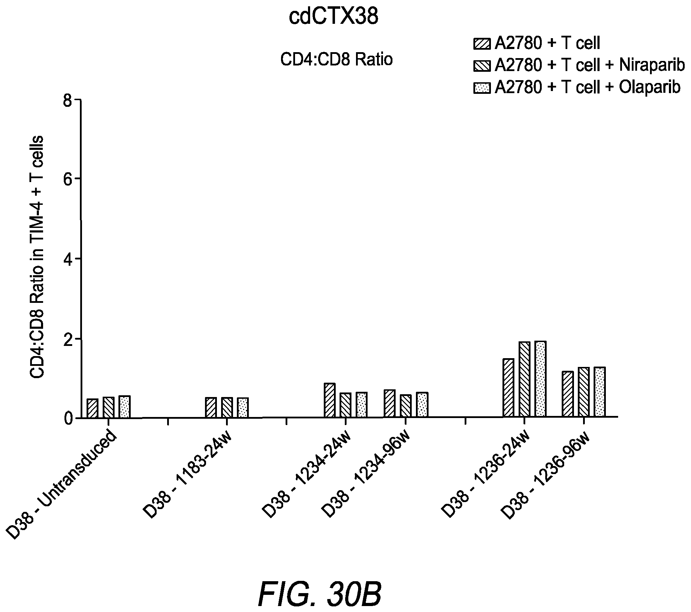

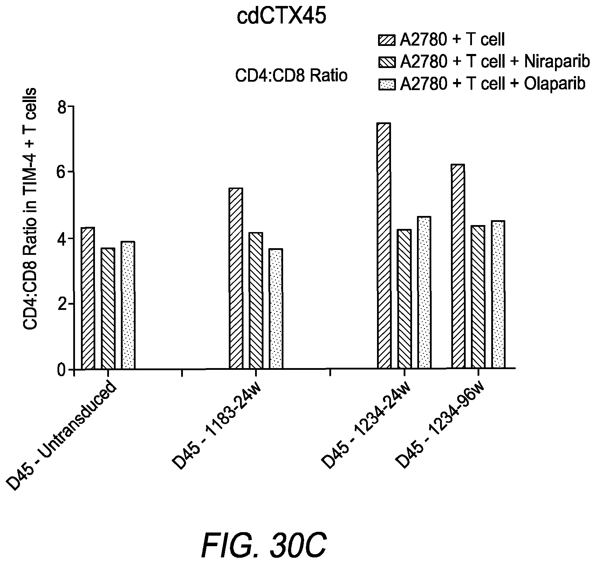

**FIGS. 30A-30C** show CD4/CD8 T cell ratio at 120 hours of co-culture of chimeric Tim4 receptor transduced T cells, olaparib or niraparib, and target A2780 ovarian cancer cells. Samples on bar graph from left to right: A2780 + T cell, A2780 + T cell + niraparib, and A2780 + T cell + olaparib. FIG. 30A shows CD4/CD8 T cell ratio in T cells from donor 41. FIG. 30B shows CD4/CD8 T cell ratio in T cells from donor 38. FIG. 30C shows CD4/CD8 T cell ratio in T cells from donor 45. For FIGS. 4A-4C, co-culture conditions A2780+T cell; A2780 + T cell + niraparib; A27080 + T cell + olaparib are shown from left to right in the bar graph for each donor T cell sample.
**FIGS. 31A-31B****:** PARP inhibitors induce phosphatidylserine on A2780 ovarian cancer cells. Viability (**FIG. 31A**) and phosphatidylserine (PS) exposure (**FIG. 31B**) of A2780 cells treated with niraparib or olaparib (both at 0.25 to 6 µM) at 96 hours.
**FIG. 32****:** CER T-cell characteristics.

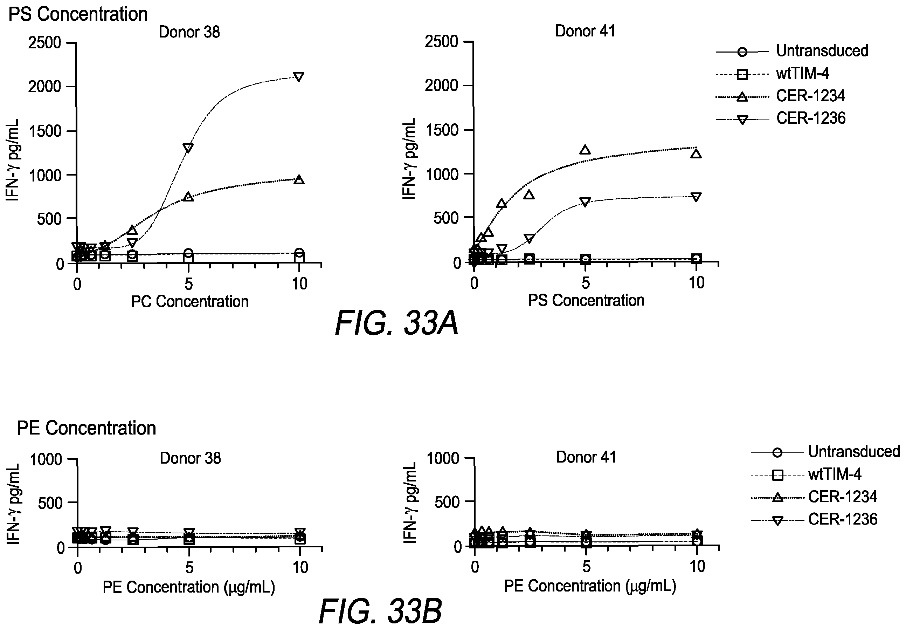

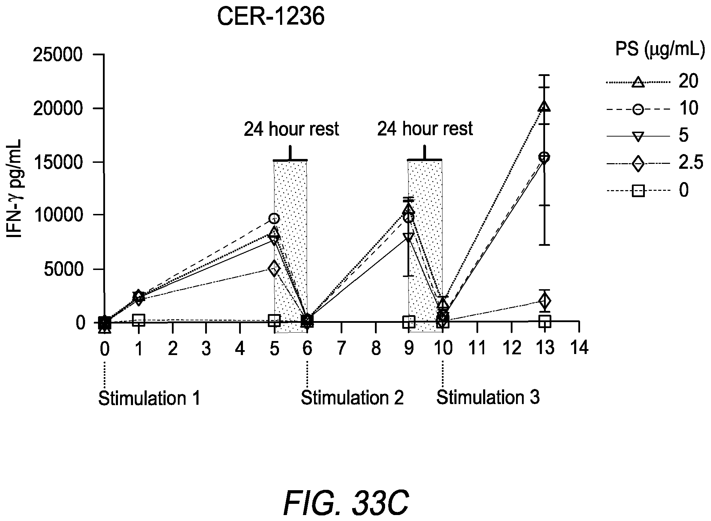

**FIG. 33A-33C****:** CER T cells are activated and produce IFN-γ in response to phosphatidylserine engagement in a cell-free in vitro system. **FIG. 33A****:** IFN-γ production in response phosphatidylserine (PS). IFN-γ production was induced in CER-1236 and CER-1234 T cells stimulated with PS (**FIG. 33A**), but not with phosphatidylethanolamine (PE) (**FIG. 33B**). PS EC50 values for CER-1234 and CER-1236 from Donors 38 and 41 were as follows: CER-1234, 3.52 µg/mL (SE ± 0.13) and 1.88 µg/mL (SE ± 0.48), respectively; CER-1236, 4.74 µg/mL (SE ± 0.02), and 3.16 µg/mL (SE ± 0.09), respectively. **FIG. 33C****:** CER1236 T cells are capable of recursive stimulation in vitro. Recursive IFN-γ induction with 3 serial stimulations at the indicated time points following a 24-hour rest after the first stimulation.
**FIG. 34A-34C****:** CER T cells in Combination with PARP Inhibitors Are Cytotoxic Against A2780 Cells. **FIG. 34A****:** Reduced proliferation of A2780 cells is observed 120 hours after co-culture with CERs and PARPi measured by incucyte for 2 donors. **FIG. 34B****:** At 120 hours, the ratio of cells remaining in treated samples compared to untreated A2780 samples was quantified using the incucyte data from **FIG. 34A****.** **FIG. 34C****:** Proliferation of CER T cells in co-culture assays.
**FIGS. 35A-35C****:** Cytokine production by CER T cells in response to co-culture with PARP inhibitor-treated A2780 ovarian cancer cells. Samples from left to right: no drug, niraparib treated, and olaparib treated. **FIG. 35A** - IFN-γ. **FIG. 35B** - TNF-α. **FIG. 35C** - granzyme B.

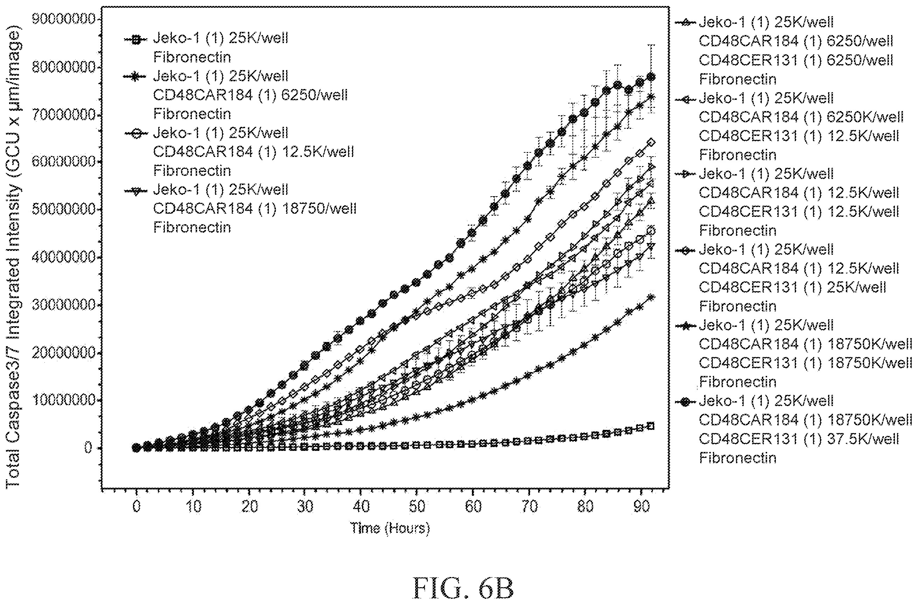

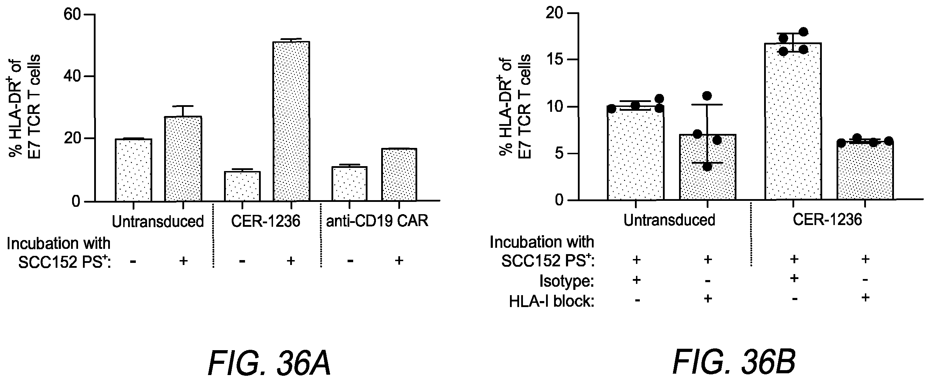

**FIGS. 36A-36B****:** CER1236 T cells activate E7 TCR T cells via presentation of tumor cell-derived E7 antigen in an HLA-I-dependent manner. **FIG. 36A****:** Following co-culture with E7 oncoprotein positive/PS-positive SCC152 target cells, CER-1236, but not untransduced T cells or anti-CD19 CAR T cells, induced HLA-DR on E7 TCR cells. **FIG. 6B****:** In a separate experiment, induction of HLA-DR on E7 TCR cells following co-culture of CER-1236 with E7 oncoprotein positive/PS-positive SCC152 cells was blocked by HLA-I blocking antibody, but not a control isotype antibody. Bars depict percentage of E7 TCR T cells that express the activation marker, HLA-DR. Error bars show SEM.
**FIG. 37**: Ibrutinib-induced phosphatidylserine exposure enhances CER1236 T cell IFN-γ response. No response was seen for untransduced cells. JeKo-1 MCL mCherry+ cells were pretreated with 20µM Ibrutinib or vehicle for 48h. Jeko-1 MCL cells were then co-cultured with CER1236 T cells in serum-free media + 200IU/ml IL-2 with 0.5 µM Ibrutinib or vehicle. After 120h, IFN-γ secretion was measured using ELLA automated ELISA.
**FIG. 38**: CER1236 T cells have enhanced and more complete cytotoxic effect against Ibrutinib-treated REC-1 MCL cells but not untreated REC-1 MCL cells.
**FIG. 39**: Graph showing NSCLC H1975 cell growth for cells pretreated with 100 nM osimertinib or vehicle and then co-cultured with CER1236 T cells.
**FIG. 40**: Graphs showing cytokine production by CER1236 T cells co-cultured with NSCLC H1975 cells with 100 nM osimertinib or vehicle (left to right: granzyme B, TNFα, IL-6, and IFNγ).
**FIG. 41**: Bar graphs showing fold expansion of total CD3+ T cells (untransduced + vehicle, untransduced + osimertinib, CER1236 + vehicle, CER1236 + osimertinib) determined by quantitative flow cytometry using quantitation beads over a time course (48hrs, 96hrs, 120hrs, and 144hrs).
**FIG. 42**: Flow cytometry plots measuring CD3+ cells (y-axis) and H1975 cell (x-axis) in co-culture experiments with H1975 cells pretreated with vehicle or osimertinib (4m88 nM, 19.53 nM) and untransduced T cells (top row) or CER1236 T cells (bottom row).
**FIG. 43** is a schematic depicting assay design for measuring CER1236 reactivation in response to stimulus by repeated phosphatidylserine stimulation of CER1236 T cells and measurement of IFNγ production.
**FIG. 44** are graphs showing IFNγ production in CER1234 T cells (left) or CER1236 T cells (right) from three donors in response to titrating doses of plate-bound phosphatidylserine.
**FIG. 45** are graphs showing IFNγ production in CER1234 T cells or CER1236 T cells by donor in response to increasing doses of plate-bound phosphatidylserine.
**FIG. 46** are graphs showing IFNγ production in CER1234 T cells or CER1236 T cells by donor in response to increasing doses of plate-bound phosphatidylserine at day 14 post-thaw.
**FIG. 47** are graphs showing T cell expansion (left), IFNγ production (center), and cell viability (right) in CER1236 T cells in response to increasing doses of plate-bound phosphatidylserine.
**FIG. 48** are graphs showing T cell expansion (left), IFNγ production (center), and cell viability (right) in CER1234 T cells in response to increasing doses of plate-bound phosphatidylserine.
**FIG. 49** is a schematic depicting design of exemplary chimeric Tim4 constructs used in the antigen presentation experiments.
**FIG. 50** shows bar graphs measuring T cell activation markers CD25 (left) and HLA-DR (right) as measured by flow cytometry. CER1183, CER1161, CER1234 or CER1236 T cells were cultured alone or co-cultured with phosphatidylserine-positive HPV E7 oncoprotein-positive cells (TMEM30A-/- SCC152) for 48 hours, followed by positive selection of T cells and co-culture for a 4 days with cell trace violet labeled-HPV E7 TCR T cells. Error bars shows SEM.
**FIG. 51** shows bar graphs measuring T cell activation markers HLA-DR as measured by flow cytometry. CER1183, CER1161, CER1234 or CER1236 T cells were cultured alone or co-cultured with phosphatidylserine-positive HPV E7 oncoprotein-positive cells (TMEM30A-/- SCC152) for 48 hours, followed by positive selection of T cells and co-culture for 4 days (**FIG. 51**, left graph) or 6 days (**FIG. 51**, right graph) with cell trace violet labeled-HPV E7 TCR T cells. Error bars shows SEM.
**FIG. 52** shows bar graphs measuring T cell activation markers CD25 (left graph) and HLA-DR (right graph) as measured by flow cytometry. CER1183, CER1161, CER1234 or CER1236 T cells were cultured alone or co-cultured with phosphatidylserine-positive HPV E7 oncoprotein-positive cells (TMEM30A-/- SCC152) for 48 hours, followed by positive selection of T cells and co-culture for 4 days with cell trace violet labeled-HPV E7 TCR T cells. Error bars shows SEM.
**FIG. 53** shows bar graph measuring T cell activation markers HLA-DR as measured by flow cytometry. CER1183, CER1161, CER1234 or CER1236 T cells were cultured alone or co-cultured with phosphatidylserine-positive HPV E7 oncoprotein-positive cells (TMEM30A-/- SCC152) for 48 hours, followed by positive selection of T cells and co-culture for 6 days with cell trace violet labeled-HPV E7 TCR T cells. Error bars shows SEM.
**FIG. 54** shows bar graphs measuring T cell activation markers CD25 (left graph) and HLA-DR (right graph) as measured by flow cytometry. CER1183, CER1161, CER1234 or CER1236 T cells were cultured alone or co-cultured with phosphatidylserine-positive HPV E7 oncoprotein-positive cells (TMEM30A-/- SCC152) for 48 hours, followed by positive selection of T cells and co-culture for 4 days with cell trace violet labeled-HPV E7 TCR T cells. Error bars shows SEM.

### DETAILED DESCRIPTION

In one aspect, the present disclosure provides chimeric T-cell immunoglobulin mucin protein 4 (Tim4) receptors according to claim 1, also referred to as chimeric engulfment receptors (CERs). Chimeric Tim4 receptors of the present disclosure confer engulfment and/or cytotoxic activity to chimeric Tim4 receptor-modified host cells (e.g., T cells), with the cytotoxic activity being induced upon binding of the chimeric Tim4 receptor to its target antigen, phosphatidylserine. In some embodiments, chimeric Tim4 receptors confer engulfment, cytotoxicity, and enhanced antigen capture, antigen processing, and antigen presentation activity to modified host cells (e.g., T cells).

In some embodiments, the extracellular domain of the chimeric Tim4 receptors described herein optionally includes an extracellular spacer domain positioned between and connecting the binding domain and transmembrane domain.

In certain embodiments, the TLR2 TIR signaling domain consists of the amino acid sequence set forth in SEQ ID NO: 17. In some embodiments, the Tim4 receptor comprises or consists of the amino acid sequence set forth in SEQ ID NO: 19. In some embodiments, the chimeric Tim4 receptor comprises or consists of the amino acid sequence set forth in SEQ ID NO: 18.

In some embodiments, when expressed in a host cell, such as a T cell, the chimeric Tim4 receptors of the present disclosure also confer engulfment activity to the host cell. For example, in certain such embodiments, binding of the chimeric Tim4 receptor expressed in a host cell to a phosphatidylserine target may induce both cytolytic and engulfment responses by the host cell. In particular embodiments of the modified host cells described herein, the host cell does not naturally exhibit an engulfment phenotype prior to modification with the chimeric Tim4 receptor.

In another aspect, host cells modified with chimeric Tim4 receptors of the present disclosure can be used to eliminate target cells bearing surface exposed phosphatidylserine, *e.g.*, for the treatment of cancer. In normal, healthy cells phosphatidylserine is located in the inner leaflet of the plasma membrane. However, certain cellular events, such as damage, apoptosis, necrosis, and stress, activates a "scramblase" that quickly exposes phosphatidylserine on the cell surface, where it can bind to receptors such as Tim4. Endogenous tumor-specific effector T cells can induce exposure of phosphatidylserine on the outer membrane of targeted tumor cells during cytolysis. Furthermore, certain cancer therapies (e.g., chemotherapy, molecularly targeted therapy, radiotherapy, CAR-T cells, etc.) can induce exposure of phosphatidylserine on targeted tumor cells or cells in the tumor microenvironment by inducing apoptosis, cellular stress, cellular damage, etc. Engineered host cells expressing the presently disclosed chimeric Tim4 receptors may clear damaged, stressed, apoptotic, or necrotic tumor cells bearing surface exposed phosphatidylserine by inducing apoptosis in the tumor cells bearing surface exposed phosphatidylserine. In certain embodiments, host cells expressing chimeric Tim4 receptors disclosed herein clear damaged, stressed, apoptotic, or necrotic tumor cells bearing surface exposed phosphatidylserine by inducing apoptosis and by engulfment. Engineered host cells comprising chimeric Tim4 receptors according to the present description may be administered to a subject alone, or in combination with one or more additional therapeutic agents, including for example CAR-T cells, TCRs, antibodies, radiation therapy, chemotherapies, molecularly targeted therapy, small molecules, oncolytic viruses, electropulse therapy, etc.

In another aspect, host cells modified with chimeric Tim4 receptors of the present disclosure can be used to enhance an effector response (*e.g.*, a tumor specific immune response). In embodiments, host cells modified with chimeric Tim4 receptors of the present disclosure can be used to enhance anti-tumor efficacy (*e*.*g.,* tumor trafficking, expansion, and persistence). Embodiments of the chimeric Tim4 receptors of the present disclosure are capable of costimulating T cells via at least one costimulatory signaling pathway upon binding phosphatidylserine. In certain embodiments, the chimeric Tim4 receptors described herein provide costimulatory signals via at least two distinct signaling pathways. In certain embodiments, the enhanced effector response is enhanced T cell proliferation, cytokine production, cytotoxic activity, persistence, or any combination thereof. Host cells expressing chimeric Tim4 receptors according to the present description may be administered to a subject alone, or in combination with one or more additional therapeutic agents, including for example CAR-T cells, TCRs, antibodies, radiation therapy, chemotherapies, small molecules, oncolytic viruses, electropulse therapy, etc.

In another aspect, host cells modified with chimeric Tim4 receptors of the present disclosure can be used to inhibit or reduce immune cell exhaustion. In certain embodiments, immune cell exhaustion refers to T cell exhaustion, NK cell exhaustion, or both. Tumor cells may provide continuous antigen stimulation to immune cells, often in the absence of costimulatory ligands, which may result in immune cell exhaustion (*e.g.*, reduced proliferative capacity, reduced effector function, and upregulation of immunosuppressive molecules). Cancer therapies, such as chemotherapy, molecularly targeted therapy, radiotherapy, CAR-T cell therapy, etc., can also provide prolonged antigen stimulation in the absence of costimulatory signals or when the strength or duration of costimulatory signals is limited. Chimeric Tim4 receptors of the present disclosure are capable of costimulating immune cells via at least one costimulatory signaling domain upon binding phosphatidylserine. In certain embodiments, chimeric Tim4 receptors provide costimulatory signals via at least two distinct signaling pathways. Host cells expressing chimeric Tim4 receptors may be administered to a subject alone, or in combination with one or more additional therapeutic agents, including for example CAR-T cells, TCRs, antibodies, radiation therapy, chemotherapies, small molecules, oncolytic viruses, electropulse therapy, etc.

In some embodiments, host cells (e.g., T cells) modified with chimeric Tim4 receptors of the present disclosure exhibit enhanced antigen capture, antigen processing, and/or antigen presentation activity. Ligand-binding to the phagocytic receptor portion of the chimeric Tim4 receptor mediates a cascade of events including: T cell activation, signal transduction, cytolytic function, production of cytokines and chemokines, partial engulfment of target cells, and downstream transcriptional programs that lead to presentation of target cell antigens. Expression of chimeric Tim4 receptors in non- or weakly phagocytic immune cells such as mature polyclonal T cells can enable and enhance antigen-specific capture through engulfment of target cell fragments. In some embodiments, the added functionality of chimeric Tim4 receptor-mediated antigen capture supports enhanced presentation of non-targeted antigens while also eliciting direct cytolytic activity against primed tumor cell targets.

For combination therapy compositions comprising a chimeric Tim4 receptor according to the present description and cellular immunotherapy, e.g., CARs or TCRs, the chimeric Tim4 receptor and cellular immunotherapy agent (e.g., CAR or TCR) can be expressed on separate engineered cells or expressed on the same engineered cell to produce a bispecific, multifunctional engineered cell. Chimeric Tim4 receptor and a cellular immunotherapy agent expressed on the same engineered cell can be expressed from separate vectors, or on the same vector as a multicistronic construct.

In another aspect, host cells modified with chimeric Tim4 receptors of the present disclosure can be used to enhance the effect of a therapeutic agent that induces cellular stress, damage, necrosis, or apoptosis. For example, certain therapeutic agents, such as chemotherapy, specific inhibitors of driver mutations associated with cancer (e.g., molecularly targeted therapy such as BRAF inhibitors, EGFR inhibitors, ALK/ROS1 kinase inhibitors, BTK inhibitors, PARP inhibitors), radiation therapy, UV light therapy, electropulse therapy, adoptive cellular immunotherapy (e.g., CAR-T cells, TCRs) and oncolytic viral therapy, can induce cell damage or death in tumor cells or diseased cells. Cells expressing a chimeric Tim4 receptor as presently described can bind to the phosphatidylserine moieties exposed on the outer leaflet of damaged or dying cells resulting from any one or more of such therapeutic agents and induce cytolysis or both cytolysis and engulfment of the targeted cells.

In another aspect, the present disclosure provides the chimeric Tim4 receptor for use in methods for enhancing CCR7+ expressing T cells in a subject having cancer, wherein the cancer is breast cancer, ovarian cancer, colorectal cancer, fallopian cancer, peritoneal cancer, prostate cancer, lung cancer, or melanoma, the method comprising administering to the subject an effective amount of the chimeric Tim4 receptor according to the present description and (ii) a Poly ADP-ribose polymerase (PARP) inhibitor. Chemokine receptor CCR7 is expressed on T cells of early differentiation status, e.g., naïve, stem cell memory (T_{SCM}) and central memory (T_{CM}) T cells. Early pre-clinical studies with therapeutic T cells have shown that naive and early-differentiated T cells possess T cells possess an enhanced capacity for long term persistence and can elicit potent anti-tumor responses. Thus, enhancing CCR7 chimeric Tim4 receptor T cells is likely to also enhance engraftment and persistence *in vivo.*

In another aspect, the present disclosure provides the chimeric Tim4 receptor for use in methods for enhancing CD4/CD8 T cell ratio in a subject having cancer, wherein the cancer is breast cancer, ovarian cancer, colorectal cancer, fallopian cancer, peritoneal cancer, prostate cancer, lung cancer, or melanoma, the method comprising administering to the subject an effective amount of the chimeric Tim4 receptor according to the present description and optionally, a Poly ADP-ribose polymerase (PARP) inhibitor. CD4 T cells are known for their helper function and evoke cytolytic activities by enhancing CD8 T cells activity through cytokine production. However, adoptive cell therapy using CD4 T cells (chimeric antigen receptor (CAR)) demonstrated comparable effectiveness in directly killing target tumor cells both in vitro and in vivo. While adoptive cell therapy with CD4 T cells (CAR) show initial slower granzyme B secretion and tumor killing, but are less prone to activation induced cell death and exhaustion compared to CD8 counterparts, which confers CD4 CAR T cells a relative better persistence following antigen exposure. Thus, enhancing CD4 T cell subsets for chimeric Tim4 receptor adoptive cell therapy is likely to confer improved persistence and less exhaustion.

In another aspect, host cells expressing chimeric Tim receptors disclosed herein clear damaged, stressed, apoptotic, or necrotic tumor cells bearing surface exposed phosphatidylserine by inducing apoptosis and by engulfment. In another aspect, host cells modified with chimeric Tim receptors of the present disclosure can be used to enhance the effect of a PARP inhibitor that induces cellular stress, damage, necrosis, or apoptosis. For example, administration of a PARP inhibitor may increase levels of surface phosphatidylserine, thus resulting in a synergistic combination. Cells expressing a chimeric Tim receptor as presently described can bind to the phosphatidylserine moieties exposed on the outer leaflet of damaged or dying cells resulting from administration of the PARP inhibitor and induce cytolysis or both cytolysis and engulfment of the targeted cells.

In another aspect, the present disclosure provides the chimeric Tim4 receptor for use in methods for the treatment of cancer in a subject comprising administering the chimeric Tim4 receptor in combination with an inhibitor of Poly (ADP-ribose) polymerase (PARP). The chimeric Tim4 receptor may be administred as a composition comprising the chimeric Tim receptor, a polynucleotide encoding the chimeric Tim4 receptor, a vector comprising the polynucleotide encoding the chimeric Tim4 receptor, or an engineered host cell comprising the chimeric Tim4 receptor, the polynucleotide encoding the chimeric Tim4 receptor of the vector comprising the polynucleotide encoding the chimeric Tim4 receptor, wherein optionally the composition further comprises a pharmaceutically acceptable excipient. The PARP inhibitor may be administered at a subtherapeutic dosage. In another aspect, the present disclosure provides a pharmaceutical compositions or combination comprising a chimeric Tim4 receptor and a PARP inhibitor. Chimeric Tim4 receptors useful in compositions of the present disclosure confer engulfment, cytotoxicity, and/or antigen presentation activity to chimeric Tim4 receptor-modified host cells (e.g., T cells), with the cytotoxic activity being induced upon binding of the chimeric Tim4 receptor to its target antigen, phosphatidylserine. DNA damaging agents such as PARP inhibitors may synergize with chimeric Tim4 receptors by promoting cell damage and externalization of phosphatidylserine, which induces effector function of chimeric Tim4 receptors, such as engulfment, cytotoxicity, costimulatory activity, antigen presentation, or a combination thereof.

Prior to setting forth this disclosure in more detail, it may be helpful to an understanding thereof to provide definitions of certain terms to be used herein.

In the present description, any concentration range, percentage range, ratio range, or integer range is to be understood to include the value of any integer within the recited range and, when appropriate, fractions thereof (such as one tenth and one hundredth of an integer), unless otherwise indicated. Also, any number range recited herein relating to any physical feature, such as polymer subunits, size or thickness, are to be understood to include any integer within the recited range, unless otherwise indicated. As used herein, the term "about" means ± 20% of the indicated range, value, or structure, unless otherwise indicated. It should be understood that the terms "a" and "an" as used herein refer to "one or more" of the enumerated components. The use of the alternative (e.g., "or") should be understood to mean either one, both, or any combination thereof of the alternatives. As used herein, the terms "include," "have" and "comprise" are used synonymously, which terms and variants thereof are intended to be construed as non-limiting.

Terms understood by those in the art of antibody technology are each given the meaning acquired in the art, unless expressly defined differently herein. The term "antibody" is used in the broadest sense and includes polyclonal and monoclonal antibodies. An "antibody" may refer to an intact antibody comprising at least two heavy (H) chains and two light (L) chains inter-connected by disulfide bonds, as well as an antigen-binding portion (or antigen-binding domain) of an intact antibody that has or retains the capacity to bind a target molecule. An antibody may be naturally occurring, recombinantly produced, genetically engineered, or modified forms of immunoglobulins, for example intrabodies, peptibodies, nanobodies, single domain antibodies, SMIPs, multispecific antibodies (e.g., bispecific antibodies, diabodies, triabodies, tetrabodies, tandem di-scFV, tandem tri-scFv, ADAPTIR). A monoclonal antibody or antigen-binding portion thereof may be non-human, chimeric, humanized, or human, preferably humanized or human. Immunoglobulin structure and function are reviewed, for example, in Harlow et al., Eds., Antibodies: A Laboratory Manual, Chapter 14 (Cold Spring Harbor Laboratory, Cold Spring Harbor, 1988). "Antigen-binding portion" or "antigen-binding domain" of an intact antibody is meant to encompass an "antibody fragment," which indicates a portion of an intact antibody and refers to the antigenic determining variable regions or complementary determining regions of an intact antibody. Examples of antibody fragments include, but are not limited to, Fab, Fab', F(ab')₂, and Fv fragments, Fab' -SH, F(ab')₂, diabodies, linear antibodies, scFv antibodies, VH, and multispecific antibodies formed from antibody fragments. A "Fab" (fragment antigen binding) is a portion of an antibody that binds to antigens and includes the variable region and CH1 of the heavy chain linked to the light chain via an inter-chain disulfide bond. An antibody may be of any class or subclass, including IgG and subclasses thereof (IgG₁, IgG₂, IgG₃, IgG₄), IgM, IgE, IgA, and IgD.

The term "variable region" or "variable domain" refers to the domain of an antibody heavy or light chain that is involved in binding of the antibody to antigen. The variable domains of the heavy chain and light chain (VH and VL, respectively) of a native antibody generally have similar structures, with each domain comprising four conserved framework regions (FRs) and three CDRs. (*See, e.g.,* Kindt et al. Kuby Immunology, 6th ed., W.H. Freeman and Co., page 91 (2007)). A single VH or VL domain may be sufficient to confer antigen-binding specificity. Furthermore, antibodies that bind a particular antigen may be isolated using a VH or VL domain from an antibody that binds the antigen to screen a library of complementary VL or VH domains, respectively. *See, e.g.,* Portolano et al., J. Immunol. 150:880-887 (1993); Clarkson et al., Nature 352:624-628 (1991).

The terms "complementarity determining region" and "CDR," which are synonymous with "hypervariable region" or "HVR," are known in the art to refer to non-contiguous sequences of amino acids within antibody variable regions, which confer antigen specificity and/or binding affinity. In general, there are three CDRs in each heavy chain variable region (HCDR1, HCDR2, HCDR3) and three CDRs in each light chain variable region (LCDR1, LCDR2, LCDR3).

As used herein, the terms "binding domain", "binding region", and "binding moiety" refer to a molecule, such as a peptide, oligopeptide, polypeptide, or protein that possesses the ability to specifically and non-covalently bind, associate, unite, recognize, or combine with a target molecule (e.g., phosphatidylserine). A binding domain includes any naturally occurring, synthetic, semi-synthetic, or recombinantly produced binding partner for a biological molecule or other target of interest. Exemplary binding domains include single chain antibody variable regions *(e.g.,* domain antibodies, sFv, scFv, Fab), receptor ectodomains *(e.g.,* Tim4), ligands (e.g., cytokines, chemokines), or synthetic polypeptides selected for the specific ability to bind to a biological molecule.

"T cell receptor" (TCR) refers to a molecule found on the surface of T cells (also referred to as T lymphocytes) that is generally responsible for recognizing antigens bound to major histocompatibility complex (MHC) molecules. The TCR is generally composed of a disulfide-linked heterodimer of the highly variable α and β chains (also known as TCRα and TCRβ, respectively) in most T cells. In a small subset of T cells, the TCR is made up of a heterodimer of γ and δ chains (also known as TCRγ and TCRδ, respectively). Each chain of the TCR is a member of the immunoglobulin superfamily and possesses one N-terminal immunoglobulin variable domain, one immunoglobulin constant domain, a transmembrane region, and a short cytoplasmic tail at the C-terminal end (*see* Janeway et al., Immunobiology: The Immune System in Health and Disease, 3rd Ed., Current Biology Publications, p. 4:33, 1997). TCRs of the present disclosure may be from various animal species, including human, mouse, rat, cat, dog, goat, horse, or other mammals. TCRs may be cell-bound (*i.e.,* have a transmembrane region or domain) or in soluble form. TCRs include recombinantly produced, genetically engineered, fusion, or modified forms of TCRs, including for example, scTCRs, soluble TCRs, TCR fusion constructs (TRuC^{™}; *see,* U.S. Patent Publication No. 2017/0166622).

The term "variable region" or "variable domain" of a TCR α-chain (Vα) and β-chain (Vβ), or Vγ and Vδ for γδ TCRs, are involved in binding of the TCR to antigen. The V_{α} and V_{β} of a native TCR generally have similar structures, with each variable domain comprising four conserved FRs and three CDRs. The V_{α} domain is encoded by two separate DNA segments, the variable gene segment (V gene) and the joining gene segment (J gene); the V_{β} domain is encoded by three separate DNA segments, the variable gene segment (V gene), the diversity gene segment (D gene), and the joining gene segment (J gene). A single V_{α} or V_{β} domain may be sufficient to confer antigen-binding specificity. "Major histocompatibility complex molecule" (MHC molecule) refers to a glycoprotein that delivers a peptide antigen to a cell surface. MHC class I molecules are heterodimers composed of a membrane spanning α chain (with three α domains) and a non-covalently associated β2 microglobulin. MHC class II molecules are composed of two transmembrane glycoproteins, α and β, both of which span the membrane. Each chain has two domains. MHC class I molecules deliver peptides originating in the cytosol to the cell surface, where peptide:MHC complex is recognized by CD8⁺ T cells. MHC class II molecules deliver peptides originating in the vesicular system to the cell surface, where they are recognized by CD4⁺ T cells. An MHC molecule may be from various animal species, including human, mouse, rat, or other mammals.

"Chimeric antigen receptor" (CAR) refers to a chimeric protein comprising two or more distinct domains and can function as a receptor when expressed on the surface of a cell. CARs are generally composed of an extracellular domain comprising a binding domain that binds a target antigen, an optional extracellular spacer domain, a transmembrane domain, and an intracellular signaling domain (*e.g.*, an immunoreceptor tyrosine-based activation motif (ITAM)-containing T cell activating motif, and optionally an intracellular costimulatory domain). In certain embodiments, an intracellular signaling domain of a CAR has an ITAM-containing T cell activating domain (*e.g.,* CD3ζ) and an intracellular costimulatory domain (*e.g.,* CD28). In certain embodiments, a CAR is synthesized as a single polypeptide chain or is encoded by a nucleic acid molecule as a single chain polypeptide.

A variety of assays are known for identifying binding domains of the present disclosure that specifically bind a particular target, as well as determining binding domain affinities, such as Western blot, ELISA, and BIACORE^{®} analysis (*see also, e.g.,* Scatchard et al., Ann. N.Y. Acad. Sci. 51:660, 1949; and U.S. Patent Nos. 5,283,173, 5,468,614, or the equivalent). As used herein, "specifically binds" refers to an association or union of a binding domain, or a fusion protein thereof, to a target molecule with an affinity or Kₐ *(i.e.,* an equilibrium association constant of a particular binding interaction with units of 1/M) equal to or greater than 10⁵ M⁻¹, while not significantly associating or uniting with any other molecules or components in a sample.

The terms "antigen" and "Ag" refer to a molecule that is capable of inducing an immune response. The immune response that is induced may involve antibody production, the activation of specific immunologically-competent cells, or both. Macromolecules, including proteins, glycoproteins, and glycolipids, can serve as an antigen. Antigens can be derived from recombinant or genomic DNA. As contemplated herein, an antigen need not be encoded (i) solely by a full length nucleotide sequence of a gene or (ii) by a "gene" at all. An antigen can be generated or synthesized, or an antigen can be derived from a biological sample. Such a biological sample can include, but is not limited, to a tissue sample, a tumor sample, a cell, or a biological fluid.

The term "epitope" or "antigenic epitope" includes any molecule, structure, amino acid sequence or protein determinant within an antigen that is specifically bound by a cognate immune binding molecule, such as an antibody or fragment thereof (*e.g.*, scFv), T cell receptor (TCR), chimeric Tim4 receptor, or other binding molecule, domain or protein. Epitopic determinants generally contain chemically active surface groupings of molecules, such as amino acids or sugar side chains, and can have specific three dimensional structural characteristics, as well as specific charge characteristics. An epitope may be a linear epitope or a conformational epitope.

As used herein, the term "Tim4" (T-cell immunoglobulin and mucin domain containing protein 4), also known as "TimD4", refers to a phosphatidylserine receptor that is typically expressed on antigen presenting cells, such as macrophages and dendritic cells. Tim4 mediates the phagocytosis of apoptotic, necrotic, damaged, injured, or stressed cells, which present phosphatidylserine (PtdSer) on the exofacial (outer) leaflet of the cell membrane. Tim4 is also capable of binding to Tim1 expressed on the surface of T cells and inducing proliferation and survival. In certain embodiments, Tim4 refers to human Tim4. An exemplary human Tim4 protein comprises an amino acid sequence of SEQ ID NO: 1.

As used herein, the term "Tim4 binding domain" refers to the N-terminal immunoglobulin-fold domain of Tim4 that possesses a metal ion-dependent pocket that selectively binds PtdSer. An exemplary human Tim4 binding domain comprises an amino acid sequence of SEQ ID NO:5, and an exemplary mouse Tim4 binding domain comprises an amino acid sequence of SEQ ID NO:2.

A Tim4 binding domain includes a variable immunoglobulin (IgV) like domain (referred to herein as an "IgV domain") and a Mucin like domain ("referred to herein as a "mucin domain"). An exemplary human Tim4 IgV domain comprises an amino acid sequence of SEQ ID NO:3, and an exemplary human Tim4 mucin domain comprises an amino acid sequence of SEQ ID NO:4. In certain embodiments, the Tim4 binding domain does not include a signal peptide. An exemplary human Tim4 signal peptide has the amino acid sequences of SEQ ID NO:7. An exemplary mouse Tim4 signal peptide has the amino acid sequences of SEQ ID NO:8.

As used herein, an "effector domain" is an intracellular portion of a fusion protein or receptor that can directly or indirectly promote a biological or physiological response in a cell expressing the effector domain when receiving the appropriate signal. In certain embodiments, an effector domain is part of a protein or protein complex that receives a signal when bound, or it binds directly to a target molecule, which triggers a signal from the effector domain. An effector domain may directly promote a cellular response when it contains one or more signaling domains or motifs, such as an immunoreceptor tyrosine-based activation motif (ITAM). In other embodiments, an effector domain will indirectly promote a cellular response by associating with one or more other proteins that directly promote a cellular response.

As used herein, a "costimulatory signaling domain" refers to an intracellular signaling domain, or functional portion thereof, of a costimulatory molecule, which, when activated in conjunction with a primary or classic (e.g., ITAM-driven) activation signal (provided by, for example, a CD3ζ intracellular signaling domain), promotes or enhances a T cell response, such as T cell activation, cytokine production, proliferation, differentiation, survival, effector function, or combinations thereof. Costimulatory signaling domains include, for example, CD27, CD28, CD40L, GITR, NKG2C, CARD1, CD2, CD7, CD27, CD30, CD40, CD54 (ICAM), CD83, CD134 (OX-40), CD137 (4-1BB), CD150 (SLAMF1), CD152 (CTLA4), CD223 (LAG3), CD226, CD270 (HVEM), CD273 (PD-L2), CD274 (PD-L1), CD278 (ICOS), DAP10, LAT, LFA-1, LIGHT, NKG2C, SLP76, TRIM, or any combination thereof.

As used herein, an "immunoreceptor tyrosine-based activation motif (ITAM) activating domain" refers to an intracellular signaling domain or functional portion thereof which is naturally or endogenously present on an immune cell receptor or a cell surface marker and contains at least one immunoreceptor tyrosine-based activation motif (ITAM). ITAM refers to a conserved motif of YXXL/I-X₆₋₈-YXXL/I. In certain embodiments an ITAM signaling domain contains one, two, three, four, or more ITAMs. An ITAM signaling domain may initiate T cell activation signaling following antigen binding or ligand engagement. ITAM-signaling domains include, for example, intracellular signaling domains of CD3γ, CD3δ, CD3ε, CD3ζ, CD79a, and CD66d.

"Junction amino acids" or "junction amino acid residues" refer to one or more (*e.g.*, about 2-20) amino acid residues between two adjacent motifs, regions or domains of a polypeptide. Junction amino acids may result from the construct design of a chimeric protein (*e.g.*, amino acid residues resulting from the use of a restriction enzyme site during the construction of a nucleic acid molecule encoding a chimeric protein).

"Nucleic acid molecule" and "polynucleotide" can be in the form of RNA or DNA, which includes cDNA, genomic DNA, and synthetic DNA. A nucleic acid molecule may be composed of naturally occurring nucleotides (such as deoxyribonucleotides and ribonucleotides), analogs of naturally occurring nucleotides (*e.g.*, α-enantiomeric forms of naturally occurring nucleotides), or a combination of both. Modified nucleotides can have modifications in or replacement of sugar moieties, or pyrimidine or purine base moieties. Nucleic acid monomers can be linked by phosphodiester bonds or analogs of such linkages. Analogs of phosphodiester linkages include phosphorothioate, phosphorodithioate, phosphoroselenoate, phosphorodiselenoate, phosphoroanilothioate, phosphoranilidate, phosphoramidate, and the like. A nucleic acid molecule may be double stranded or single stranded, and if single stranded, may be the coding strand or non-coding (anti-sense strand). A coding molecule may have a coding sequence identical to a coding sequence known in the art or may have a different coding sequence, which, as the result of the redundancy or degeneracy of the genetic code, or by splicing, can encode the same polypeptide.

"Encoding" refers to the inherent property of specific polynucleotide sequences, such as DNA, cDNA, and mRNA sequences, to serve as templates for synthesis of other polymers and macromolecules in biological processes having either a defined sequence of nucleotides (*i.e.*, rRNA, tRNA and mRNA) or a defined sequence of amino acids and the biological properties resulting therefrom. Thus, a polynucleotide encodes a protein if transcription and translation of mRNA corresponding to that polynucleotide produces the protein in a cell or other biological system. Both a coding strand and a non-coding strand can be referred to as encoding a protein or other product of the polynucleotide. Unless otherwise specified, a "nucleotide sequence encoding an amino acid sequence" includes all nucleotide sequences that are degenerate versions of each other and that encode the same amino acid sequence.

As used herein, the terms "peptide," "polypeptide," and "protein" are used interchangeably, and refer to a compound comprised of amino acid residues covalently linked by peptide bonds. A protein or peptide must contain at least two amino acids, and no limitation is placed on the maximum number of amino acids that can comprise a protein's or peptide's sequence. Polypeptides include any peptide or protein comprising two or more amino acids joined to each other by peptide bonds. As used herein, the term refers to both short chains, which also commonly are referred to in the art as peptides, oligopeptides and oligomers, for example, and to longer chains, which generally are referred to in the art as proteins, of which there are many types. "Polypeptides" include, for example, biologically active fragments, substantially homologous polypeptides, oligopeptides, homodimers, heterodimers, variants of polypeptides, modified polypeptides, derivatives, analogs, fusion proteins, among others. The polypeptides include natural peptides, recombinant peptides, synthetic peptides, or a combination thereof.

As used herein, the term "mature polypeptide" or "mature protein" refers to a protein or polypeptide that is secreted or localized in the cell membrane or inside certain cell organelles (*e.g.*, the endoplasmic reticulum, golgi, or endosome) and does not include an N-terminal signal peptide.

A "signal peptide", also referred to as "signal sequence", "leader sequence", "leader peptide", "localization signal" or "localization sequence", is a short peptide (usually 15-30 amino acids in length) present at the N-terminus of newly synthesized proteins that are destined for the secretory pathway. A signal peptide typically comprises a short stretch of hydrophilic, positively charged amino acids at the N-terminus, a central hydrophobic domain of 5-15 residues, and a C-terminal region with a cleavage site for a signal peptidase. In eukaryotes, a signal peptide prompts translocation of the newly synthesized protein to the endoplasmic reticulum where it is cleaved by the signal peptidase, creating a mature protein that then proceeds to its appropriate destination.

The term "chimeric" refers to any nucleic acid molecule or protein that is not endogenous and comprises sequences joined or linked together that are not normally found joined or linked together in nature. For example, a chimeric nucleic acid molecule may comprise regulatory sequences and coding sequences that are derived from different sources, or regulatory sequences and coding sequences that are derived from the same source but arranged in a manner different than that found in nature.

As used herein, the term "endogenous" or "native" refers to a gene, protein, compound, molecule, or activity that is normally present in a host or host cell, including naturally occurring variants of the gene, protein, compound, molecule, or activity.

As used herein, "homologous" or "homolog" refers to a molecule or activity from a host cell that is related by ancestry to a second gene or activity, *e.g.*, from the same host cell, from a different host cell, from a different organism, from a different strain, from a different species. For example, a heterologous molecule or heterologous gene encoding the molecule may be homologous to a native host cell molecule or gene that encodes the molecule, respectively, and may optionally have an altered structure, sequence, expression level or any combination thereof.

As used herein, "heterologous" nucleic acid molecule, construct or sequence refers to a nucleic acid molecule or portion of a nucleic acid molecule that is not native to a host cell, but can be homologous to a nucleic acid molecule or portion of a nucleic acid molecule from the host cell. The source of the heterologous nucleic acid molecule, construct or sequence can be from a different genus or species. In some embodiments, the heterologous nucleic acid molecules are not naturally occurring. In certain embodiments, a heterologous nucleic acid molecule is added (*i.e.,* not endogenous or native) into a host cell or host genome by, for example, conjugation, transformation, transfection, transduction, electroporation, or the like, wherein the added molecule can integrate into the host cell genome or exist as extra-chromosomal genetic material (*e.g.,* as a plasmid or other form of self-replicating vector), and can be present in multiple copies. In addition, "heterologous" refers to a non-native enzyme, protein or other activity encoded by a non-endogenous nucleic acid molecule introduced into the host cell, even if the host cell encodes a homologous protein or activity.

As used herein, the term "engineered," "recombinant," "modified" or "non-natural" refers to an organism, microorganism, cell, nucleic acid molecule, or vector that has been modified by introduction of a heterologous nucleic acid molecule, or refers to a cell or microorganism that has been genetically engineered by human intervention-that is, modified by introduction of a heterologous nucleic acid molecule, or refers to a cell or microorganism that has been altered such that expression of an endogenous nucleic acid molecule or gene is controlled, deregulated or constitutive, where such alterations or modifications can be introduced by genetic engineering. Human-generated genetic alterations can include, for example, modifications introducing nucleic acid molecules (which may include an expression control element, such as a promoter) encoding one or more proteins, chimeric receptors, or enzymes, or other nucleic acid molecule additions, deletions, substitutions, or other functional disruption of or addition to a cell's genetic material. Exemplary modifications include those in coding regions or functional fragments thereof heterologous or homologous polypeptides from a reference or parent molecule. Additional exemplary modifications include, for example, modifications in non-coding regulatory regions in which the modifications alter expression of a gene or operon.

As used herein, the term "transgene" refers to a gene or polynucleotide encoding a protein of interest (e.g., chimeric Tim4 receptor) whose expression is desired in a host cell and that has been transferred by genetic engineering techniques into a cell. A transgene may encode proteins of therapeutic interest as well as proteins that are reporters, tags, markers, suicide proteins, etc. A transgene may be from a natural source, modification of a natural gene, or a recombinant or synthetic molecule. In certain embodiments, a transgene is a component of a vector.

The term "overexpressed" or "overexpression" of an antigen refers to an abnormally high level of antigen expression in a cell. Overexpressed antigen or overexpression of antigen is often associated with a disease state, such as in hematological malignancies and cells forming a solid tumor within a specific tissue or organ of a subject. Solid tumors or hematological malignancies characterized by overexpression of a tumor antigen can be determined by standard assays known in the art.

The "percent identity" between two or more nucleic acid or amino acid sequences is a function of the number of identical positions shared by the sequences (*i.e.,* % identity = number of identical positions/total number of positions x 100), taking into account the number of gaps, and the length of each gap that needs to be introduced to optimize alignment of two or more sequences. The comparison of sequences and determination of percent identity between two or more sequences can be accomplished using a mathematical algorithm, such as BLAST and Gapped BLAST programs at their default parameters (*e.g.,* Altschul et al., J. Mol. Biol. 215:403, 1990; see also BLASTN at www.ncbi.nlm.nih.gov/BLAST).

A "conservative substitution" is recognized in the art as a substitution of one amino acid for another amino acid that has similar properties. Exemplary conservative substitutions are well known in the art (*see, e.g.,* WO 97/09433, page 10, published March 13, 1997; Lehninger, Biochemistry, Second Edition; Worth Publishers, Inc. NY:NY (1975), pp.71-77; Lewin, Genes IV, Oxford University Press, NY and Cell Press, Cambridge, MA (1990), p. 8).

The term "promoter" as used herein is defined as a DNA sequence recognized by the synthetic machinery of the cell, or introduced synthetic machinery, required to initiate the specific transcription of a polynucleotide sequence.

As used herein, the term "promoter/regulatory sequence" means a nucleic acid sequence which is required for expression of a gene product operably linked to the promoter/regulatory sequence. In some instances, this sequence may be the core promoter sequence and in other instances, this sequence may also include an enhancer sequence and other regulatory elements that are required for expression of the gene product. The promoter/regulatory sequence may, for example, be one that expresses the gene product in a tissue specific manner.

A "constitutive" promoter is a nucleotide sequence which, when operably linked with a polynucleotide which encodes or specifies a gene product, causes the gene product to be produced in a cell under most or all physiological conditions of the cell.

An "inducible" promoter is a nucleotide sequence which, when operably linked with a polynucleotide which encodes or specifies a gene product, causes the gene product to be produced in a cell substantially only when an inducer which corresponds to the promoter is present in the cell.

A "tissue-specific" promoter is a nucleotide sequence which, when operably linked with a polynucleotide encodes or specified by a gene, causes the gene product to be produced in a cell substantially only if the cell is a cell of the tissue type corresponding to the promoter.

The phrase "under transcriptional control" or "operatively linked" as used herein means that a promoter is in the correct location and orientation in relation to a polynucleotide to control the initiation of transcription by RNA polymerase and expression of the polynucleotide.

A "vector" is a nucleic acid molecule that is capable of transporting another nucleic acid. Vectors may be, for example, plasmids, cosmids, viruses, or phage. The term should also be construed to include non-plasmid and non-viral compounds which facilitate transfer of nucleic acid into cells. An "expression vector" is a vector that is capable of directing the expression of a protein encoded by one or more genes carried by the vector when it is present in the appropriate environment.

In certain embodiments, the vector is a viral vector. Examples of viral vectors include, but are not limited to, adenovirus vectors, adeno-associated virus vectors, retrovirus vectors, gamma retrovirus vectors, and lentivirus vectors. "Retroviruses" are viruses having an RNA genome. "Gamma retrovirus" refers to a genus of the retroviridae family. Examples of gamma retroviruses include mouse stem cell virus, murine leukemia virus, feline leukemia virus, feline sarcoma virus, and avian reticuloendotheliosis viruses. "Lentivirus" refers to a genus of retroviruses that are capable of infecting dividing and non-dividing cells. Examples of lentiviruses include, but are not limited to HIV (human immunodeficiency virus, including HIV type 1 and HIV type 2, equine infectious anemia virus, feline immunodeficiency virus (FIV), bovine immune deficiency virus (BIV), and simian immunodeficiency virus (SIV).

In other embodiments, the vector is a non-viral vector. Examples of non-viral vectors include lipid-based DNA vectors, modified mRNA (modRNA), self-amplifying mRNA, closed-ended linear duplex (CELiD) DNA, and transposon-mediated gene transfer (PiggyBac, Sleeping Beauty). Where a non-viral delivery system is used, the delivery vehicle can be a liposome. Lipid formulations can be used to introduce nucleic acids into a host cell *in vitro, ex vivo,* or *in vivo.* The nucleic acid may be encapsulated in the interior of a liposome, interspersed within the lipid bilayer of a liposome, attached to a liposome via a linking molecule that is associated with both the liposome and the nucleic acid, contained or complexed with a micelle, or otherwise associated with a lipid.

As used herein, the term "engulfment" refers to a receptor-mediated process wherein endogenous or exogenous cells or particles greater than 100 nm in diameter are internalized by a phagocyte or host cell of the present disclosure. Engulfment is typically composed of multiple steps: (1) tethering of the target cell or particle via binding of an engulfment receptor to a pro-engulfment marker or antigenic marker directly or indirectly (via a bridging molecule) on a target cell or particle; and (2) internalization or engulfment of the whole target cell or particle, or a portion thereof. In certain embodiments, internalization may occur via cytoskeletal rearrangement of a phagocyte or host cell to form a phagosome, a membrane-bound compartment containing the internalized target. Engulfment may further include maturation of the phagosome, wherein the phagosome becomes increasingly acidic and fuses with lysosomes (to form a phagolysosome), whereupon the engulfed target is degraded (*e.g.*, "phagocytosis"). Alternatively, phagosome-lysosome fusion may not be observed in engulfment. In yet another embodiment, a phagosome may regurgitate or discharge its contents to the extracellular environment before complete degradation. In some embodiments, engulfment refers to phagocytosis. In some embodiments, engulfment includes tethering of the target cell or particle by the phagocyte of host cell of the present disclosure, but not internalization. In some embodiments, engulfment includes tethering of the target cell or particle by the phagocyte of host cell of the present disclosure and internalization of part of the target cell or particle.

As used herein, the term "phagocytosis" refers to an engulfment process of cells or large particles (≥ 0.5 µm) wherein tethering of a target cell or particle, engulfment of the target cell or particle, and degradation of the internalized target cell or particle occurs. In certain embodiments, phagocytosis comprises formation of a phagosome that encompasses the internalized target cell or particle and phagosome fusion with a lysosome to form a phagolysosome, wherein the contents therein are degraded. In certain embodiments, during phagocytosis, following binding of a chimeric Tim4 receptor expressed on a host cell of the present disclosure to a phosphatidylserine expressed by a target cell or particle, a phagocytic synapse is formed; an actin-rich phagocytic cup is generated at the phagocytic synapse; phagocytic arms are extended around the target cell or particle through cytoskeletal rearrangements; and ultimately, the target cell or particle is pulled into the phagocyte or host cell through force generated by motor proteins. As used herein, "phagocytosis" includes the process of "efferocytosis", which specifically refers to the phagocytosis of apoptotic or necrotic cells in a non-inflammatory manner.

The term "immune system cell" or "immune cell" means any cell of the immune system that originates from a hematopoietic stem cell in the bone marrow. Hematopoietic stem cells give rise to two major lineages, a myeloid progenitor cell (which give rise to myeloid cells such as monocytes, macrophages, dendritic cells, megakaryocytes and granulocytes) and a lymphoid progenitor cell (which give rise to lymphoid cells such as T cells, B cells and natural killer (NK) cells). Exemplary immune system cells include a CD4+ T cell, a CD8+ T cell, a CD4- CD8- double negative T cell, a γδ T cell, a regulatory T cell, a natural killer cell, and a dendritic cell. Macrophages and dendritic cells may also be referred to as "antigen presenting cells" or "APCs," which are specialized cells that can activate T cells when a major histocompatibility complex (MHC) receptor on the surface of the APC complexed with a peptide interacts with a TCR on the surface of a T cell.

The term "T cells" refers to cells of T cell lineage. "Cells of T cell lineage" refer to cells that show at least one phenotypic characteristic of a T cell or a precursor or progenitor thereof that distinguishes the cells from other lymphoid cells, and cells of the erythroid or myeloid lineages. Such phenotypic characteristics can include expression of one or more proteins specific for T cells (*e.g.* , CD3⁺, CD4⁺, CD8⁺), or a physiological, morphological, functional, or immunological feature specific for a T cell. For example, cells of the T cell lineage may be progenitor or precursor cells committed to the T cell lineage; CD25⁺ immature and inactivated T cells; cells that have undergone CD4 or CD8 linage commitment; thymocyte progenitor cells that are CD4⁺CD8⁺ double positive; single positive CD4⁺ or CD8⁺; TCRαβ or TCR γδ; or mature and functional or activated T cells. The term "T cells" encompasses naive T cells (CD45 RA+, CCR7+, CD62L+, CD27+, CD45RO-), central memory T cells (CD45RO⁺, CD62L⁺, CD8⁺), effector memory T cells (CD45RA+, CD45RO-, CCR7-, CD62L-, CD27-), mucosal-associated invariant T (MAIT) cells, Tregs, natural killer T cells, and tissue resident T cells.

The term "B cells" refers to cells of the B cell lineage. "Cells of B cell lineage" refer to cells that show at least one phenotypic characteristic of a B cell or a precursor or progenitor thereof that distinguishes the cells from other lymphoid cells, and cells of the erythroid or myeloid lineages. Such phenotypic characteristics can include expression of one or more proteins specific for B cells (*e.g.* , CD19⁺, CD72+, CD24+, CD20⁺), or a physiological, morphological, functional, or immunological feature specific for a B cell. For example, cells of the B cell lineage may be progenitor or precursor cells committed to the B cell lineage (e.g., pre-pro-B cells, pro-B cells, and pre-B cells); immature and inactivated B cells or mature and functional or activated B cells. Thus, "B cells" encompass naive B cells, plasma cells, regulatory B cells, marginal zone B cells, follicular B cells, lymphoplasmacytoid cells, plasmablast cells, and memory B cells (*e.g.,* CD27⁺, IgD⁻).

The term "cytotoxic activity," also referred to as "cytolytic activity," with respect to a cell (*e.g.,* a T cell or NK cell) expressing an immune receptor (*e.g., a* TCR) or a chimeric Tim4 receptor according to the present disclosure on its surface, means that upon antigen-specific signaling (e.g., via the TCR, chimeric Tim4 receptor), the cell induces a target cell to undergo apoptosis. In some embodiments, a cytotoxic cell may induce apoptosis in a target cell via the release of cytotoxins, such as perforin, granzyme, and granulysin, from granules. Perforins insert into the target cell membrane and form pores that allow water and salts to rapidly enter the target cell. Granzymes are serine proteases that induce apoptosis in the target cell. Granulysin is also capable of forming pores in the target cell membrane and is a proinflammatory molecule. In some embodiments, a cytotoxic cell may induce apoptosis in a target cell via interaction of Fas ligand, which is upregulated on T cell following antigen-specific signaling, with Fas molecules expressed on the target cell. Fas is an apoptosis-signaling receptor molecule on the surface of a number of different cells.

The term "exhaustion" with respect to immune cells refers to a state of immune cell dysfunction defined by poor effector function (e.g., reduced cytokine production, reduced cytotoxic activity), reduced proliferative capacity, increased expression of immune checkpoint molecules, and a transcriptional state distinct from that of functional effector or memory cells. In certain embodiments, an exhausted immune cell becomes unresponsive to the presence of its target antigen. Immune cell exhaustion may result from chronic exposure to a target antigen (e.g., as may result from chronic infection) or when it enters an immunosuppressive environment (e.g., a tumor microenvironment). In certain embodiments, immune cell exhaustion refers to T cell exhaustion, NK cell exhaustion, or both. In certain embodiments, exhausted T cells exhibit; (a) increased expression of PD-1, TIGIT, LAG3, TIM3, or any combination thereof; (b) decreased production of IFN-γ, IL-2, TNF-α, or any combination thereof; or both (a) and (b). In certain embodiments, exhausted NK cells exhibit; (a) increased expression of PD-1, NKG2A, TIM3, or any combination thereof; (b) decreased production of IFN-γ, TNF-α, or both; or both (a) and (b).

A "disease" is a state of health of a subject wherein the subject cannot maintain homeostasis, and wherein, if the disease is not ameliorated, then the subject's health continues to deteriorate. In contrast, a "disorder" or "undesirable condition" in a subject is a state of health in which the subject is able to maintain homeostasis, but in which the subject's state of health is less favorable than it would be in the absence of the disorder or undesirable condition. Left untreated, a disorder or undesirable condition does not necessarily result in a further decrease in the subject's state of health.

The term "cancer" as used herein is defined as disease characterized by the rapid and uncontrolled growth of aberrant cells. The aberrant cells may form solid tumors or constitute a hematological malignancy. Cancer cells can spread locally or through the bloodstream and lymphatic system to other parts of the body. Examples of various cancers include, but are not limited to, breast cancer, prostate cancer, ovarian cancer, cervical cancer, skin cancer, pancreatic cancer, colorectal cancer, renal cancer, liver cancer, brain cancer, lymphoma, leukemia, lung cancer and the like.

The term "subject," "patient" and "individual" are used interchangeably herein and are intended to include living organisms in which an immune response can be elicited (e.g., mammals). Examples of subjects include humans, primates, cows, horses, sheep, dogs, cats, mice, rats, rabbits, guinea pigs, pigs, and non-human transgenic species thereof.

"Adoptive cellular immunotherapy" or "adoptive immunotherapy" refers to the administration of naturally occurring or genetically engineered disease antigen-specific immune cells (*e.g.,* T cells). Adoptive cellular immunotherapy may be autologous (immune cells are from the recipient), allogeneic (immune cells are from a donor of the same species) or syngeneic (immune cells are from a donor genetically identical to the recipient).

"Autologous" refers to any material (e.g., a graft of organ, tissue, cells) derived from the same subject to which it is later to be re-introduced.

"Allogeneic" refers to a graft derived from a different subject of the same species.

A "therapeutically effective amount" or "effective amount" of a chimeric protein or cell expressing a chimeric protein of this disclosure (e.g., a chimeric Tim4 receptor or a cell expressing a chimeric Tim4 receptor) refers to that amount of protein or cells sufficient to result in amelioration of one or more symptoms of the disease, disorder, or undesired condition being treated. When referring to an individual active ingredient or a cell expressing a single active ingredient, administered alone, a therapeutically effective dose refers to the effects of that ingredient or cell expressing that ingredient alone. When referring to a combination, a therapeutically effective dose refers to the combined amounts of active ingredients or combined adjunctive active ingredient with a cell expressing an active ingredient that results in a therapeutic effect, whether administered serially or simultaneously.

"Treat" or "treatment" or "ameliorate" refers to medical management of a disease, disorder, or undesired condition of a subject. In general, an appropriate dose or treatment regimen comprising a host cell expressing a chimeric protein of this disclosure is administered in an amount sufficient to elicit a therapeutic or prophylactic benefit. Therapeutic or prophylactic/preventive benefit includes improved clinical outcome; lessening or alleviation of symptoms associated with a disease, disorder, or undesired condition; decreased occurrence of symptoms; improved quality of life; longer disease-free status; diminishment of extent of disease, disorder, or undesired condition; stabilization of disease state; delay of disease progression; remission; survival; prolonged survival; or any combination thereof.

The term "anti-tumor effect" refers to a biological effect which can be manifested by a decrease in tumor volume, a decrease in the number of tumor cells, a decrease in the number of metastases, an increase in life expectancy, or amelioration of various physiological symptoms associated with a cancerous condition. An "anti-tumor effect" can also be manifested by prevention of a hematological malignancy or tumor formation.

"Autoimmune disease" refers to a disorder that results from an autoimmune response. An autoimmune disease is the result of an inappropriately excessive response to a self-antigen. An autoimmune response may involve self-reactive B-cells that produce autoantibodies, self-reactive T-cells, or both. An "autoantibody" as used herein is an antibody produced by a subject that binds to a self-antigen also produced by the subject.

Additional definitions are provided throughout the present disclosure.

### Chimeric Tim4 Receptors

The present disclosure provides chimeric Tim4 receptors according to claim 1.

In certain embodiments, the extracellular domain of the chimeric Tim4 receptors described herein optionally includes an extracellular spacer domain positioned between and connecting the binding domain and transmembrane domain.

When expressed in a host cell, chimeric Tim4 receptors of the present disclosure can confer a phosphatidylserine-specific, T-cell cytotoxicity to the modified host cell, such as a T-cell, *(e.g.,* the host cell becomes cytotoxic to a stressed, damaged, injured, apoptotic, or necrotic cell expressing phosphatidylserine on its surface) with innate functions of phagocytosis and antigen processing and presentation. The intracellular CD28 signaling domain and CD3ζ signaling domain serve as T-cell activators. The intracellular TLR2 signaling domain enhances the ability of host cells to present an exogenous soluble antigen and activate class I-restricted T cells. In some embodiments, the chimeric Tim4 receptors induce apoptosis in targeted cells via release of granzymes, perforin, granulysin, or any combination thereof. In some embodiments, cells expressing a chimeric Tim4 receptor according to the present description exhibit an engulfment phenotype specific to phosphatidylserine presenting cells. In some embodiments, cells, e.g., T cells, expressing a chimeric Tim4 receptor according to the present disclosure exhibit enhanced antigen presenting activity. Without wishing to be bound by theory, the combination of T-cell antigen capture and presentation capabilities with inducible and target-specific cytotoxic function in single T cells suggests the potential for secondary immune responses via activation and enhancement of endogenous antitumor immunity.

T cells comprising a chimeric Tim4 receptor comprising from N-terminus to C-terminus: (a) an extracellular domain comprising a Tim4 binding domain; (b) a CD28 transmembrane domain; (b) an intracellular signaling domain comprising a CD28 signaling domain, (c) a CD3ζ signaling domain, and (d) a TLR2 TIR signaling domain, such as for example a chimeric Tim4 receptor comprising the amino acid sequence set forth in SEQ ID NO:19, demonstrates the ability to become reactivated upon additional antigen exposure in vitro, suggesting a durable effect in vivo. Furthermore, a chimeric Tim4 receptor comprising from N-terminus to C-terminus: (a) an extracellular domain comprising a Tim4 binding domain; (b) a CD28 transmembrane domain; (b) an intracellular signaling domain comprising a CD28 signaling domain, (c) a CD3ζ signaling domain, and (d) a TLR2 TIR signaling domain, such as for example a chimeric Tim4 receptor comprising the amino acid sequence set forth in SEQ ID NO: 19, exhibited more consistent antigen-presentation function compared to a chimeric Tim4 receptor comprising from N-terminus to C-terminus: (a) an extracellular domain comprising a Tim4 binding domain; (b) a CD28 transmembrane domain; (b) an intracellular signaling domain comprising a CD28 signaling domain, (c) a TLR2 TIR signaling domain, and (d) a CD3ζ signaling domain, such as for example a chimeric Tim4 receptor comprising the amino acid sequence set forth in SEQ ID NO: 18.

Signaling by the intracellular signaling domain(s) is triggered by binding of the extracellular domain to phosphatidylserine. The signals transduced by the intracellular signaling domain promote effector function of the chimeric Tim4 receptor containing cell. Examples of effector function include cytotoxic activity, secretion of cytokines, proliferation, anti-apoptotic signaling, persistence, expansion, engulfment of a target cell or particle expressing phosphatidylserine on its surface, antigen presentation, or any combination thereof.

Chimeric Tim4 receptors according to the present disclosure can be used in a variety of therapies where clearance of apoptotic, necrotic, damaged, or stressed cells is beneficial, while providing costimulation that enhances cellular immune response, reduces immune cell exhaustion, or both.

Component parts of the fusion proteins of the present disclosure are further described in detail herein.

### Extracellular Domain

As described herein, a chimeric Tim4 receptor comprises an extracellular domain comprising a Tim4 binding domain. The Tim4 binding domain confers specificity to phosphatidylserine (PtdSer), which is a phospholipid with a negatively charged head-group and a component of the cell membrane. In healthy cells, phosphatidylserine is preferentially found in the inner leaflet of the cell membrane. However, when cells are stressed, damaged or undergo apoptosis or necrosis, phosphatidylserine is exposed on the outer leaflet of the cell membrane. Thus, phosphatidylserine may be used as a marker to distinguish stressed, damaged, apoptotic, necrotic, pyroptotic, or oncotic cells. Binding of phosphatidylserine by the Tim binding domain may block the interaction between the phosphatidylserine and another molecule and, for example, interfere with, reduce or eliminate certain functions of the phosphatidylserine (e.g., signal transduction). In some embodiments, the binding of a phosphatidylserine may induce certain biological pathways or identify the phosphatidylserine molecule or a cell expressing phosphatidylserine for elimination.

Phosphatidylserine binding is generally regulated by the IgV domain. The core phosphatidylserine binding domain is a four amino acid sequence in the IgV domain (e.g., amino acids 95-98 of SEQ ID NO:3). A Tim4 binding domain binds minimally to cells with low phosphatidylserine density.

In certain embodiments, the Tim4 binding domain consists of an amino acid sequence having 100% identity to SEQ ID NO:5 or SEQ ID NO:6. In some embodiments, the Tim4 binding domain further comprises the Tim4 signal sequence of SEQ ID NO:7.

In some embodiments, the extracellular domain optionally comprises an extracellular, non-signaling spacer or linker domain. Where included, such a spacer or linker domain may position the binding domain away from the host cell surface to further enable proper cell/cell contact, binding, and activation. When included in a chimeric receptor as described herein, an extracellular spacer domain is generally located between the extracellular binding domain and the transmembrane domain of the chimeric Tim4 receptor. The length of the extracellular spacer may be varied to optimize target molecule binding based on the selected target molecule, selected binding epitope, binding domain size and affinity (*see, e.g.,* Guest et al., J. Immunother. 28:203-11, 2005; PCT Publication No. WO 2014/031687). In some embodiments, an extracellular spacer domain is an immunoglobulin hinge region (*e.g.,* IgG1, IgG2, IgG3, IgG4, IgA, IgD). An immunoglobulin hinge region may be a wild type immunoglobulin hinge region or an altered wild type immunoglobulin hinge region. An altered IgG₄ hinge region is described in PCT Publication No. WO 2014/031687. In some embodiments, an extracellular spacer domain comprises a modified IgG₄ hinge region having an amino acid sequence of ESKYGPPCPPCP (SEQ ID NO:9). Other examples of hinge regions that may be used in the chimeric Tim4 receptors described herein include the hinge region from the extracellular regions of type 1 membrane proteins, such as CD8a, CD4, CD28 and CD7, which may be wild-type or variants thereof. In some embodiments, an extracellular spacer domain comprises a CD28 hinge region having an amino acid sequence of SEQ ID NO:10. In some embodiments, an extracellular spacer domain comprises all or a portion of an immunoglobulin Fc domain selected from: a CH1 domain, a CH2 domain, a CH3 domain, or combinations thereof (*see, e.g.,* PCT Publication WO2014/031687). In some embodiments, an extracellular spacer domain may comprise a stalk region of a type II C-lectin (the extracellular domain located between the C-type lectin domain and the transmembrane domain). Type II C-lectins include CD23, CD69, CD72, CD94, NKG2A, and NKG2D.

In some embodiments, an extracellular domain comprises amino acid sequences derived from any mammalian species, including humans, primates, cows, horses, goats, sheep, dogs, cats, mice, rats, rabbits, guinea pigs, pigs, non-human transgenic species thereof, or any combination thereof. In certain embodiments, an extracellular domain is murine, human, or chimeric.

### Intracellular Signaling Domain

The intracellular signaling domain of a chimeric Tim4 receptor as described herein is an intracellular effector domain and is capable of transmitting functional signals to a cell in response to binding of the extracellular domain of the chimeric Tim4 receptor and phosphatidylserine. The signals transduced by the intracellular signaling domain promote effector function of the chimeric Tim4 receptor containing cell. Examples of effector function include cytotoxic activity, secretion of cytokines, proliferation, anti-apoptotic signaling, persistence, expansion, engulfment of a target cell or particle expressing phosphatidylserine on its surface, antigen capture, antigen processing, antigen presentation, or any combination thereof.

In some embodiments, a full length signaling molecule or full length intracellular component of a signaling molecule is used. In some embodiments, a truncated portion of a signaling molecule or intracellular component of a signaling molecule is used, provided that the truncated portion retains sufficient signal transduction activity.

In some embodiments, a TLR2 TIR signaling domain is a TLR2 signaling domain having the peptide sequence of HRFHGLWYMKMMWAWLQAKRKPRKAPSRN removed from the N-terminus of the TLR2 signaling domain.

As used herein, the designation of primary, secondary, and tertiary intracellular signaling domains includes but is not limited to arrangements of the primary intracellular signaling domain at the N-terminus, secondary intracellular signaling domain in the middle, and tertiary intracellular signaling domain at the C-terminus of the intracellular portion of the chimeric Tim4 receptor. Thus, designation of the primary intracellular signaling domain does not limit the use of the selected intracellular signaling domain at the N-terminus of the intracellular portion of the chimeric Tim4 receptor. Designation of the secondary intracellular signaling domain does not limit the use of the selected intracellular signaling domain in the middle (or at the C-terminus for those chimeric Tim4 receptors only having two intracellular signaling domains) of the intracellular portion of the chimeric Tim4 receptor. Designation of the tertiary intracellular signaling domain does not limit the use of the selected intracellular signaling domain at the C-terminus of the intracellular portion of the chimeric Tim4 receptor. Thus, different arrangements of the primary, secondary, and/or tertiary intracellular signaling domains within the intracellular portion of the chimeric Tim4 receptor are contemplated.

In some embodiments, the CD28 signaling domain consists of the amino acid sequence set forth in SEQ ID NO: 12. In some embodiments, the CD3ζ signaling domain consists of the amino acid sequence set forth in SEQ ID NO:14. In some embodiments, the TLR2 signaling domain is a TLR2 TIR signaling domain consisting of the amino acid sequence of SEQ ID NO:17.

In some embodiments, an intracellular signaling domain comprises from N-terminus to C-terminus: a CD28 primary intracellular signaling domain, a TLR2 secondary intracellular signaling domain, and a CD3ζ tertiary intracellular signaling domain. In some embodiments, an intracellular signaling domain comprises from N-terminus to C-terminus: a CD28 primary intracellular signaling domain, a CD3ζ secondary intracellular signaling domain, and a TLR2 tertiary intracellular signaling domain.

In some embodiments, an intracellular signaling domain comprises a combination of primary, secondary, and tertiary intracellular signaling domain as set forth in SEQ ID NO:39 or SEQ ID NO:40.

### Transmembrane Domain

The transmembrane domain of a chimeric Tim4 receptor connects and is positioned between the extracellular domain and the intracellular signaling domain. The transmembrane domain is a hydrophobic alpha helix that transverses the host cell membrane. The transmembrane domain may be directly fused to the binding domain or to the extracellular spacer domain if present. In certain embodiments, the transmembrane domain consists of an amino acid sequence having 100% identity SEQ ID NO:11.

It is understood that direct fusion of one domain to another domain of a chimeric Tim4 receptor described herein does not preclude the presence of intervening junction amino acids. Junction amino acids may be natural or non-natural (e.g., resulting from the construct design of a chimeric protein). For example, junction amino acids may result from restriction enzyme sites used for joining one domain to another domain or cloning polynucleotides encoding chimeric Tim4 receptors into vectors.

### Chimeric Tim4 receptors

Chimeric Tim4 receptors and component sequences are described in Tables 1 and 2. In some embodiments, a chimeric Tim 4 receptor comprises the amino acid sequence of SEQ ID NO:18 or the amino acid sequence of SEQ ID NO:18 absent the signal sequence (amino acids 1-24). In some embodiments, a chimeric Tim 4 receptor comprises the amino acid sequence of SEQ ID NO:19 or the amino acid sequence of SEQ ID NO:19 absent the signal sequence (amino acids 1-24).

**Table 1: Chimeric Tim4 Receptors and Components**

| **Name** | **Amino Acid Sequence** | **SEQ ID NO:#** |
|---|---|---|
| Human Tim4 (signal peptide=amino acids 1-24, underlined) | MSKEPLILWLMIEFWWLYLTPVTSETVVTEVLGHR VTLPCLYSSWSHNSNSMCWGKDQCPYSGCKEALIR TDGMRVTSRKSAKYRLQGTIPRGDVSLTILNPSESDS GVYCCRIEVPGWFNDVKINVRLNLQRASTTTHRTAT TTTRRTTTTSPTTTRQMTTTPAALPTTVVTTPDLTTG TPLQMTTIAVFTTANTCLSLTPSTLPEEATGLLTPEPS KEGPILTAESETVLPSDSWSSVESTSADTVLLTSKES KVWDLPSTSHVSMWKTSDSVSSPQPGASDTAVPEQ NKTTKTGQMDGIPMSMKNEMPISQLLMIIAPSLGFV LFALFVAFLLRGKLMETYCSQKHTRLDYIGDSKNVL NDVQHGREDEDGLFTL | 1 |
| Mouse Tim4 binding domain (signal peptide=amino acids 122, underlined) | MSKGLLLLWLVTELWWLYLTPAASEDTIIGFLGQPV TLPCHYLSWSQSRNSMCWGKGSCPNSKCNAELLRT DGTRIISRKSTKYTLLGKVQFGEVSLTISNTNRGDSG VYCCRIEVPGWFNDVKKNVRLELRRATTTKKPTTT TRPTTTPYVTTTTPELLPTTVMTTSVLPTTTPPQTLA TTAFSTAVTTCPSTTPGSFSQETTKGSAFTTESETLPA SNHSQRSMMTISTDIAVLRPTGSNPGILPSTSQLTTQ KTTLTTSESLQKTTKSHQINSRQT | 2 |
| Human Tim4 IgV domain | | 3 |
| Human Tim4 Mucin domain | | 4 |
| Human Tim4 binding domain (signal peptide=amino acids 1-24, underlined) | MSKEPLILWLMIEFWWLYLTPVTSETVVTEVLGHR VTLPCLYSSWSHNSNSMCWGKDQCPYSGCKEALIR TDGMRVTSRKSAKYRLQGTIPRGDVSLTILNPSESDS GVYCCRIEVPGWFNDVKINVRLNLQRASTTTHRTAT TTTRRTTTTSPTTTRQMTTTPAALPTTVVTTPDLTTG TPLQMTTIAVFTTANTCLSLTPSTLPEEATGLLTPEPS KEGPILTAESETVLPSDSWSSVESTSADTVLLTSKES KVWDLPSTSHVSMWKTSDSVSSPQPGASDTAVPEQ NKTTKTGQMDGIPMSMKNEMPISQ | 5 |
| Human Tim4 binding domain (without signal peptide) | | 6 |
| Human Tim4 signal peptide | MSKEPLILWLMIEFWWLYLTPVTS | 7 |
| Mouse Tim4 signal peptide | MSKGLLLLWLVTELWWLYLTPA | 8 |
| Modified IgG4 hinge | ESKYGPPCPPCP | 9 |
| CD28 hinge | IEVMYPPPYLDNEKSNGTIIHVKGKHLCPSPLFPGPS KP | 10 |
| CD28 transmembrane domain | FWVLVVVGGVLACYSLLVTVAFIIFWV | 11 |
| Wildtype CD28 costimulatory signaling domain | RSKRSRLLHSDYMNMTPRRPGPTRKHYQPYAPPRD FAAYRS | 12 |
| CD28 costimulatory signaling domain with L186G/L187G substitutions (positions reference to full length protein) | RSKRSRGGHSDYMNMTPRRPGPTRKHYQPYAPPRD FAAYRS | 13 |
| wildtype CD3ζ activating domain | RVKFSRSADAPAYQQGQNQLYNELNLGRREEYDVL DKRRGRDPEMGGKPQRRKNPQEGLYNELQKDKMA EAYSEIGMKGERRRGKGHDGLYQGLSTATKDTYDA LHMQALPPR | 14 |
| mutated human CD3ζ ITAM-containing activating domain | RVKFSRSADAPAYQQGQNQLYNELNLGRREEYDVL DKRRGRDPEMGGKPRRKNPQEGLYNELQKDKMAE AYSEIGMKGERRRGKGHDGLYQGLSTATKDTYDAL HMQALPPR | 15 |
| TLR2 signaling domain | HRFHGLWYMKMMWAWLQAKRKPRKAPSRNICYD AFVSYSERDAYWVENLMVQELENFNPPFKLCLHKR DFIPGKWIIDNIIDSIEKSHKTVFVLSENFVKSEWCKY ELDFSHFRLFDENNDAAILILLEPIEKKAIPQRFCKLR KIMNTKTYLEWPMDEAQREGFWVNLRAAIKS | 16 |
| TLR2 TIR signaling domain | ICYDAFVSYSERDAYWVENLMVQELENFNPPFKLC LHKRDFIPGKWIIDNIIDSIEKSHKTVFVLSENFVKSE WCKYELDFSHFRLFDENNDAAILILLEPIEKKAIPQR FCKLRKIMNTKTYLEWPMDEAQREGFWVNLRAAIK S | 17 |
| CER-1234 (signal peptide=amino acids 1-24 underlined) | MSKEPLILWLMIEFWWLYLTPVTSETVVTEVLGHR VTLPCLYSSWSHNSNSMCWGKDQCPYSGCKEALIR TDGMRVTSRKSAKYRLQGTIPRGDVSLTILNPSESDS GVYCCRIEVPGWFNDVKINVRLNLQRASTTTHRTAT TTTRRTTTTSPTTTRQMTTTPAALPTTVVTTPDLTTG TPLQMTTIAVFTTANTCLSLTPSTLPEEATGLLTPEPS KEGPILTAESETVLPSDSWSSVESTSADTVLLTSKES KVWDLPSTSHVSMWKTSDSVSSPQPGASDTAVPEQ NKTTKTGQMDGIPMSMKNEMPISQFWVLVVVGGV LACYSLLVTVAFIIFWVRSKRSRLLHSDYMNMTPRR PGPTRKHYQPYAPPRDFAAYRSICYDAFVSYSERDA YWVENLMVQELENFNPPFKLCLHKRDFIPGKWIIDN IIDSIEKSHKTVFVLSENFVKSEWCKYELDFSHFRLF DENNDAAILILLEPIEKKAIPQRFCKLRKIMNTKTYL EWPMDEAQREGFWVNLRAAIKSRVKFSRSADAPAY QQGQNQLYNELNLGRREEYDVLDKRRGRDPEMGG KPQRRKNPQEGLYNELQKDKMAEAYSEIGMKGERR RGKGHDGLYQGLSTATKDTYDALHMQALPPR | 18 |
| CER-1236 (also referred to herein as CER1107) (signal peptide=amino acids 1-24 underlined) | MSKEPLILWLMIEFWWLYLTPVTSETVVTEVLGHR VTLPCLYSSWSHNSNSMCWGKDQCPYSGCKEALIR TDGMRVTSRKSAKYRLQGTIPRGDVSLTILNPSESDS GVYCCRIEVPGWFNDVKINVRLNLQRASTTTHRTAT TTTRRTTTTSPTTTRQMTTTPAALPTTVVTTPDLTTG TPLQMTTIAVFTTANTCLSLTPSTLPEEATGLLTPEPS KEGPILTAESETVLPSDSWSSVESTSADTVLLTSKES KVWDLPSTSHVSMWKTSDSVSSPQPGASDTAVPEQ NKTTKTGQMDGIPMSMKNEMPISQFWVLVVVGGV LACYSLLVTVAFIIFWVRSKRSRLLHSDYMNMTPRR PGPTRKHYQPYAPPRDFAAYRSRVKFSRSADAPAY QQGQNQLYNELNLGRREEYDVLDKRRGRDPEMGG KPQRRKNPQEGLYNELQKDKMAEAYSEIGMKGERR RGKGHDGLYQGLSTATKDTYDALHMQALPPRICYD AFVSYSERDAYWVENLMVQELENFNPPFKLCLHKR DFIPGKWIIDNIIDSIEKSHKTVFVLSENFVKSEWCKY ELDFSHFRLFDENNDAAILILLEPIEKKAIPQRFCKLR KIMNTKTYLEWPMDEAQREGFWVNLRAAIKS | 19 |
| CER 1183 (wildtype human Tim4) | MSKEPLILWLMIEFWWLYLTPVTSETVVTEVLGHR VTLPCLYSSWSHNSNSMCWGKDQCPYSGCKEALIR TDGMRVTSRKSAKYRLQGTIPRGDVSLTILNPSESDS GVYCCRIEVPGWFNDVKINVRLNLQRASTTTHRTAT TTTRRTTTTSPTTTRQMTTTPAALPTTVVTTPDLTTG TPLQMTTIAVFTTANTCLSLTPSTLPEEATGLLTPEPS KEGPILTAESETVLPSDSWSSVESTSADTVLLTSKES KVWDLPSTSHVSMWKTSDSVSSPQPGASDTAVPEQ NKTTKTGQMDGIPMSMKNEMPISQLLMIIAPSLGFV LFALFVAFLRGKLMETYCSQKHTRLDYIGDSKNVL NDVQHGREDEDGLFTL | 20 |
| CER1161 (also referred to herein as CER247) (signal peptide=amino acids 1-24 underlined) | MSKEPLILWLMIEFWWLYLTPVTSETVVTEVLGHR VTLPCLYSSWSHNSNSMCWGKDQCPYSGCKEALIR TDGMRVTSRKSAKYRLQGTIPRGDVSLTILNPSESDS GVYCCRIEVPGWFNDVKINVRLNLQRASTTTHRTAT TTTRRTTTTSPTTTRQMTTTPAALPTTVVTTPDLTTG TPLQMTTIAVFTTANTCLSLTPSTLPEEATGLLTPEPS KEGPILTAESETVLPSDSWSSVESTSADTVLLTSKES KVWDLPSTSHVSMWKTSDSVSSPQPGASDTAVPEQ NKTTKTGQMDGIPMSMKNEMPISQFWVLVVVGGV LACYSLLVTVAFIIFWVRSKRSRLLHSDYMNMTPRR PGPTRKHYQPYAPPRDFAAYRSRVKFSRSADAPAY QQGQNQLYNELNLGRREEYDVLDKRRGRDPEMGG KPQRRKNPQEGLYNELQKDKMAEAYSEIGMKGERR RGKGHDGLYQGLSTATKDTYDALHMQALPPR | 21 |
| pCTX1162 (also referred to herein as CER1162) (signal peptide=amino acids 1-24 underlined) | MSKEPLILWLMIEFWWLYLTPVTSETVVTEVLGHR VTLPCLYSSWSHNSNSMCWGKDQCPYSGCKEALIR TDGMRVTSRKSAKYRLQGTIPRGDVSLTILNPSESDS GVYCCRIEVPGWFNDVKINVRLNLQRASTTTHRTAT TTTRRTTTTSPTTTRQMTTTPAALPTTVVTTPDLTTG TPLQMTTIAVFTTANTCLSLTPSTLPEEATGLLTPEPS KEGPILTAESETVLPSDSWSSVESTSADTVLLTSKES KVWDLPSTSHVSMWKTSDSVSSPQPGASDTAVPEQ NKTTKTGQMDGIPMSMKNEMPISQFWVLVVVGGV LACYSLLVTVAFIIFWVRSKRSRLLHSDYMNMTPRR PGPTRKHYQPYAPPRDFAAYRSHRFHGLWYMKMM WAWLQAKRKPRKAPSRNICYDAFVSYSERDAYWV ENLMVQELENFNPPFKLCLHKRDFIPGKWIIDNIIDSI EKSHKTVFVLSENFVKSEWCKYELDFSHFRLFDENN DAAILILLEPIEKKAIPQRFCKLRKIMNTKTYLEWPM DEAQREGFWVNLRAAIKSRVKFSRSADAPAYQQGQ NQLYNELNLGRREEYDVLDKRRGRDPEMGGKPQR RKNPQEGLYNELQKDKMAEAYSEIGMKGERRRGK GHDGLYQGLSTATKDTYDALHMQALPPR | 22 |
| pCTX1163 (also referred to herein as CER1163) (signal peptide=amino acids 1-24 underlined) | MSKEPLILWLMIEFWWLYLTPVTSETVVTEVLGHR VTLPCLYSSWSHNSNSMCWGKDQCPYSGCKEALIR TDGMRVTSRKSAKYRLQGTIPRGDVSLTILNPSESDS GVYCCRIEVPGWFNDVKINVRLNLQRASTTTHRTAT TTTRRTTTTSPTTTRQMTTTPAALPTTVVTTPDLTTG TPLQMTTIAVFTTANTCLSLTPSTLPEEATGLLTPEPS KEGPILTAESETVLPSDSWSSVESTSADTVLLTSKES KVWDLPSTSHVSMWKTSDSVSSPQPGASDTAVPEQ NKTTKTGQMDGIPMSMKNEMPISQFWVLVVVGGV LACYSLLVTVAFIIFWVRSKRSRLLHSDYMNMTPRR PGPTRKHYQPYAPPRDFAAYRSRVKFSRSADAPAY QQGQNQLYNELNLGRREEYDVLDKRRGRDPEMGG KPQRRKNPQEGLYNELQKDKMAEAYSEIGMKGERR RGKGHDGLYQGLSTATKDTYDALHMQALPPRHRF HGLWYMKMMWAWLQAKRKPRKAPSRNICYDAFV SYSERDAYWVENLMVQELENFNPPFKLCLHKRDFIP GKWIIDNIIDSIEKSHKTVFVLSENFVKSEWCKYELD FSHFRLFDENNDAAILILLEPIEKKAIPQRFCKLRKIM NTKTYLEWPMDEAQREGFWVNLRAAIKS | 23 |
| CER-1250 (signal peptide=amino acids 1-24 underlined) | MSKEPLILWLMIEFWWLYLTPVTSETVVTEVLGHR VTLPCLYSSWSHNSNSMCWGKDQCPYSGCKEALIR TDGMRVTSRKSAKYRLQGTIPRGDVSLTILNPSESDS GVYCCRIEVPGAAAAVKINVRLNLQRASTTTHRTAT TTTRRTTTTSPTTTRQMTTTPAALPTTVVTTPDLTTG TPLQMTTIAVFTTANTCLSLTPSTLPEEATGLLTPEPS KEGPILTAESETVLPSDSWSSVESTSADTVLLTSKES KVWDLPSTSHVSMWKTSDSVSSPQPGASDTAVPEQ NKTTKTGQMDGIPMSMKNEMPISQFWVLVVVGGV LACYSLLVTVAFIIFWVRSKRSRLLHSDYMNMTPRR PGPTRKHYQPYAPPRDFAAYRSICYDAFVSYSERDA YWVENLMVQELENFNPPFKLCLHKRDFIPGKWIIDN IIDSIEKSHKTVFVLSENFVKSEWCKYELDFSHFRLF DENNDAAILILLEPIEKKAIPQRFCKLRKIMNTKTYL EWPMDEAQREGFWVNLRAAIKSRVKFSRSADAPAY QQGQNQLYNELNLGRREEYDVLDKRRGRDPEMGG KPQRRKNPQEGLYNELQKDKMAEAYSEIGMKGERR RGKGHDGLYQGLSTATKDTYDALHMQALPPR | 24 |
| CER-1251 (signal peptide=amino acids 1-24 underlined) | MSKEPLILWLMIEFWWLYLTPVTSETVVTEVLGHR VTLPCLYSSWSHNSNSMCWGKDQCPYSGCKEALIR TDGMRVTSRKSAKYRLQGTIPRGDVSLTILNPSESDS GVYCCRIEVPGAAAAVKINVRLNLQRASTTTHRTAT TTTRRTTTTSPTTTRQMTTTPAALPTTVVTTPDLTTG TPLQMTTIAVFTTANTCLSLTPSTLPEEATGLLTPEPS KEGPILTAESETVLPSDSWSSVESTSADTVLLTSKES KVWDLPSTSHVSMWKTSDSVSSPQPGASDTAVPEQ NKTTKTGQMDGIPMSMKNEMPISQFWVLVVVGGV LACYSLLVTVAFIIFWV RSKRSRLLHSDYMNMTPRRPGPTRKHYQPYAPPRD FAAYRSRVKFSRSADAPAYQQGQNQLYNELNLGRR EEYDVLDKRRGRDPEMGGKPQRRKNPQEGLYNEL QKDKMAEAYSEIGMKGERRRGKGHDGLYQGLSTA TKDTYDALHMQALPPRICYDAFVSYSERDAYWVEN LMVQELENFNPPFKLCLHKRDFIPGKWIIDNIIDSIEK SHKTVFVLSENFVKSEWCKYELDFSHFRLFDENNDA AILILLEPIEKKAIPQRFCKLRKIMNTKTYLEWPMDE AQREGFWVNLRAAIKS | 25 |
| CER1253 (CD8a signal peptide=amino acids 1-24 underlined, anti-CD19scFv-CD28TM-CD28 ICD-CD3z ICD) | MALPVTALLLPLALLLHADIQMTQTTSSLSASLGDR VTISCRASQDISKYLNWYQQKPDGTVKLLIYHTSRL HSGVPSRFSGSGSGTDYSLTISNLEQEDIATYFCQQG NTLPYTFGGGTKLEITGSTSGSGKPGSGEGSTKGEV KLQESGPGLVAPSQSLSVTCTVSGVSLPDYGVSWIR QPPRKGLEWLGVIWGSETTYYNSALKSRLTIIKDNS KSQVFLKMNSLQTDDTAIYYCAKHYYYGGSYAMD YWGQGTSVTVSS FWVLVVVGGVLACYSLLVTVAFIIFWVRSKRSRLLH SDYMNMTPRRPGPTRKHYQPYAPPRDFAAYRSRVK FSRSADAPAYQQGQNQLYNELNLGRREEYDVLDKR RGRDPEMGGKPQRRKNPQEGLYNELQKDKMAEAY SEIGMKGERRRGKGHDGLYQGLSTATKDTYDALH MQALPPR | 26 |
| CD28-TLR2 TIR-CD3z intracellular signaling | RSKRSRLLHSDYMNMTPRRPGPTRKHYQPYAPPRD FAAYRSICYDAFVSYSERDAYWVENLMVQELENFN PPFKLCLHKRDFIPGKWIIDNIIDSIEKSHKTVFVLSE NFVKSEWCKYELDFSHFRLFDENNDAAILILLEPIEK KAIPQRFCKLRKIMNTKTYLEWPMDEAQREGFWVN LRAAIKSRVKFSRSADAPAYQQGQNQLYNELNLGR REEYDVLDKRRGRDPEMGGKPQRRKNPQEGLYNEL QKDKMAEAYSEIGMKGERRRGKGHDGLYQGLSTA TKDTYDALHMQALPPR | 39 |
| CD28-CD3z-TLR2 TIR intracellular signaling | RSKRSRLLHSDYMNMTPRRPGPTRKHYQPYAPPRD FAAYRSRVKFSRSADAPAYQQGQNQLYNELNLGRR EEYDVLDKRRGRDPEMGGKPQRRKNPQEGLYNEL QKDKMAEAYSEIGMKGERRRGKGHDGLYQGLSTA TKDTYDALHMQALPPRICYDAFVSYSERDAYWVEN LMVQELENFNPPFKLCLHKRDFIPGKWIIDNIIDSIEK SHKTVFVLSENFVKSEWCKYELDFSHFRLFDENNDA AILILLEPIEKKAIPQRFCKLRKIMNTKTYLEWPMDE AQREGFWVNLRAAIKS | 40 |

In some embodiments, the chimeric Tim4 receptor further comprises an N-terminal signal peptide of SEQ ID NO:7. In some embodiments, the chimeric Tim4 receptor comprises or consists of SEQ ID NO: 18 or SEQ ID NO: 18 absent the signal peptide (amino acids 1-24). In some embodiments, the chimeric Tim4 receptor comprises or consists of SEQ ID NO: 19 or SEQ ID NO: 19 absent the signal peptide (amino acids 1-24).

Chimeric Tim4 receptor having the specific domains and arrangement of from N-terminus to C-terminus: a Tim4 binding domain, a CD28 transmembrane domain, a CD28 primary intracellular signaling domain, a CD3ζ secondary intracellular signaling domain, and a TLR2 TIR tertiary intracellular signaling domain, such as that provided by the amino acid sequence set forth in SEQ ID NO: 19 or SEQ ID NO:19 absent the signal peptide (amino acids 1-24), may have improved properties compared with a chimeric Tim4 receptor with the same domains but the positions of the TLR2 TIR and CD3ζ signaling domains switched, such as that provided by the amino acid sequence set forth in SEQ ID NO:18 or SEQ ID NO:18 absent the signal peptide (amino acids 1-24) (also referred to as CER1234). T cells transduced with a chimeric engulfment receptor according to SEQ ID NO:19 or SEQ ID NO: 19 absent the signal peptide (amino acids 1-24), also referred to as CER1236 T cells, display any combination of the following: (i) more consistent and higer inducibility in response to phosphatidylserine stimulation across multiple donor T cell samples; (ii) more consistent antigen presentation function; (iii) higher CD4:CD8 T cell ratios across multiple donors compared to CER1234 T cells. Furthermore, T cells transduced with a chimeric engulfment receptor according to SEQ ID NO: 19 or SEQ ID NO: 19 absent the signal peptide (amino acids 1-24) displayed a predominantly central memory and effector memory phenotype, repeated activation in response to serial phosphatidylserine exposure, and synergistic activity against target cells when combined with a PARP inhibitor, BTK inhibitor, or EGFR inhibitor.

**Table 2: Chimeric Engulfment Receptors**

| **Construct Number** | **Construct Description** | **Signal Peptide** | **Extracellular Domain** | **Transmembrane Domain** | **Intracellular Domain #1** | **Intracellular Domain #2** | **Intracellular Domain #3** |
|---|---|---|---|---|---|---|---|
| pCTX1161 [SEQ ID NO:21] | hTIM4-TIM4-CD28tm-CD28icd-CD3zICD | hTIM4 [SEQ ID NO:7] | TIM4 [SEQ ID NO:6] | CD28 [SEQ ID NO:11] | CD28 [SEQ ID NO:12] | CD3z [SEQ ID NO:14] | |
| pCTX1162 [SEQ ID NO:22] | hTIM4-TIM4-CD28tm-CD28icd-TLR2ICD-CD3zICD | hTIM4 [SEQ ID NO:7] | TIM4 [SEQ ID NO:6] | CD28 [SEQ ID NO:11] | CD28 [SEQ ID NO:12] | TLR2 [SEQ ID NO:16] | CD3z [SEQ ID NO:14] |
| pCTX1163 [SEQ ID NO:23] | hTIM4-TIM4-CD28tm-CD28icd-CD3zICD-TLR2ICD | hTIM4 [SEQ ID NO:7] | TIM4 [SEQ ID NO:6] | CD28 [SEQ ID NO:11] | CD28 [SEQ ID NO:12] | CD3z [SEQ ID NO:14] | TLR2 [SEQ ID NO:16] |
| CER1234 [SEQ ID NO:18] | hTIM4-TIM4-CD28tm-CD28icd-TLR2 TIR ICD-CD3zICD | hTIM4 [SEQ ID NO:7] | TIM4 [SEQ ID NO:6] | CD28 [SEQ ID NO:11] | CD28 [SEQ ID NO:12] | TLR2 TIR [SEQ ID NO:17] | CD3z [SEQ ID NO:14] |
| CER1236 [SEQ ID NO:19] | hTIM4-TIM4-CD28tm-CD28icd-CD3zICD-TLR2 TIR ICD | hTIM4 [SEQ ID NO:7] | TIM4 [SEQ ID NO:6] | CD28 [SEQ ID NO:11] | CD28 [SEQ ID NO:12] | CD3z [SEQ ID NO:14] | TLR2 TIR [SEQ ID NO:17] |

### Polynucleotides, Vectors, and Host Cells

The present disclosure provides nucleic acid molecules that encode any one or more of the chimeric Tim4 receptors described herein. A nucleic acid may refer to a single- or double-stranded DNA, cDNA, or RNA, and may include a positive and a negative strand of the nucleic acid which complement one another, including antisense DNA, cDNA, and RNA. A nucleic acid may be naturally occurring or synthetic forms of DNA or RNA. The nucleic acid sequences encoding a desired chimeric Tim4 receptor can be obtained or produced using recombinant methods known in the art using standard techniques, such as by screening libraries from cells expressing the desired sequence or a portion thereof, by deriving the sequence from a vector known to include the same, or by isolating the sequence or a portion thereof directly from cells or tissues containing the same as described in, for example, Sambrook et al. (1989 and 2001 editions; Molecular Cloning: A Laboratory Manual, Cold Spring Harbor Laboratory Press, NY) and Ausubel et al. (Current Protocols in Molecular Biology, 2003). Alternatively, the sequence of interest can be produced synthetically, rather than being cloned.

The polynucleotides encoding chimeric Tim4 receptors of the present disclosure may be operatively linked to expression control sequences. Expression control sequences may include appropriate transcription initiation, termination, promoter and enhancer sequences; efficient RNA processing signals such as splicing and polyadenylation signals; sequences that stabilize cytoplasmic mRNA; sequences that enhance translation efficiency (*i.e.,* Kozak consensus sequences); sequences that enhance protein stability; and possibly sequences that enhance protein secretion.

In certain embodiments, a polynucleotide encoding a chimeric Tim4 receptor comprises a sequence encoding a signal peptide (also referred to as leader peptide or signal sequence) at the 5'-end for targeting of the precursor protein to the secretory pathway. The signal peptide is optionally cleaved from the N-terminus of the extracellular domain during cellular processing and localization of the chimeric Tim4 receptor to the host cell membrane. A polypeptide from which a signal peptide sequence has been cleaved or removed may also be called a mature polypeptide. Examples of signal peptides that may be used in the chimeric Tim4 receptors of the present disclosure include signal peptides derived from endogenous secreted proteins, including, *e.g*., GM-CSF (amino acid sequence of SEQ ID NO:27), Tim1 (amino acid sequence of SEQ ID NO:28), or Tim4 (amino acid sequence of SEQ ID NO:7, or 8). In certain embodiments, a polynucleotide sequence encodes a mature chimeric Tim4 receptor polypeptide, or a polypeptide sequence comprises a mature chimeric Tim4 receptor polypeptide. It is understood by persons of skill in the art that for sequences disclosed herein that include a signal peptide sequence, the signal peptide sequence may be replaced with another signal peptide that is capable of trafficking the encoded protein to the extracellular membrane.

In certain embodiments, a chimeric Tim4 receptor encoding polynucleotide of the present disclosure is codon optimized for efficient expression in a target host cell comprising the polynucleotide (*see, e.g,* Scholten et al., Clin. Immunol. 119:135-145 (2006)). As used herein, a "codon optimized" polynucleotide comprises a heterologous polynucleotide having codons modified with silent mutations corresponding to the abundances of tRNA in a host cell of interest.

A single polynucleotide molecule may encode one, two, or more chimeric Tim4 receptors according to any of the embodiments disclosed herein. A polynucleotide encoding more than one transgene may comprise a sequence (*e.g.,* IRES, viral 2A peptide) disposed between each gene for multicistronic expression.

Polynucleotides encoding at least two transgenes (*e.g.,* a chimeric Tim4 receptor and CAR) provided in the present disclosure may be used to compose tandem expression cassettes. A tandem expression cassette refers to a component of a vector nucleic acid comprising at least two transgenes under the control of, or operatively linked to, the same set of regulatory sequences for tandem or co-expression of the at least two transgenes. Regulatory sequences that may be used in tandem expression cassettes of the present disclosure include appropriate transcription initiation, termination, promoter and enhancer sequences; efficient RNA processing signals such as splicing and polyadenylation signals; sequences that stabilize cytoplasmic mRNA; sequences that enhance translation efficiency *(i.e.,* Kozak consensus sequences); sequences that enhance protein stability; sequences that enhance protein secretion, or any combination thereof.

In one aspect, the present disclosure provides a tandem expression cassette comprising a polynucleotide encoding a chimeric Tim4 receptor of the present disclosure and a polynucleotide encoding a cellular immunotherapy agent (e.g., a CAR, TCR, etc.).

In certain embodiments, a tandem expression cassette can be constructed to optimize spatial and temporal control. For example, a tandem expression cassette can include promoter elements to optimize spatial and temporal control. In some embodiments, a tandem expression cassette includes tissue specific promoters or enhancers that enable specific induction of a tandem expression cassette to an organ, a cell type (*e.g.,* immune cell), or a pathologic microenvironment, such as a tumor or infected tissue. An "enhancer" is an additional promoter element that can function either cooperatively or independently to activate transcription. In certain embodiments, a tandem expression cassette includes a constitutive promoter. An exemplary constitutive promoter for use in tandem expression cassettes of the present disclosure is an EF-1α promoter. In certain embodiments, a tandem expression cassette includes an inducible promoter. In certain embodiments, a tandem expression cassette includes a tissue specific promoter.

The at least two transgenes contained within the tandem expression cassettes may be in any order. For example, a tandem expression cassette comprising a polynucleotide encoding a chimeric Tim4 receptor and a polynucleotide encoding a CAR may be arranged from 5' to 3': chimeric Tim4 receptor-CAR, or CAR- chimeric Tim4 receptor.

In certain embodiments, receptors that comprise two or more polypeptide chains that associate to form a multimer or complex may be encoded by two or more polynucleotide molecules within a tandem expression construct. Exemplary multimeric receptors contemplated for expression in tandem expression constructs of the present disclosure include multichain CARs, TCRs, TCR-CARs, and TRuC^{™} constructs. Accordingly, exemplary tandem expression cassette embodiments encoding a chimeric Tim4 receptor and a TCR may comprise a polynucleotide encoding a chimeric Tim4 receptor, a polynucleotide encoding a TCRα chain polypeptide, and a polynucleotide encoding a TCRβ chain polypeptide.

In certain embodiments, tandem expression cassettes of the present disclosure may comprise an internal ribosome entry site (IRES) or peptide cleavage site such as a furin cleavage site or viral 2A peptide, disposed between each polynucleotide contained within the tandem expression cassette to allow for co-expression of multiple proteins from a single mRNA. For example, an IRES, furin cleavage site, or viral 2A peptide may be disposed between a polynucleotide encoding a chimeric Tim4 receptor and a polynucleotide encoding a CAR within a tandem expression cassette. In another example, an IRES, furin cleavage site, or viral 2A peptide may be disposed between each of a polynucleotide encoding a chimeric Tim4 receptor, a polynucleotide encoding a TCRα chain polypeptide, and a polynucleotide encoding a TCRβ chain polypeptide. In certain embodiments, a viral 2A peptide is a porcine teschovirus-1 (P2A), *Thosea asigna* virus (T2A), equine rhinitis A virus (E2A), foot-and-mouth disease virus (F2A), or variant thereof. An exemplary T2A peptide comprises an amino acid sequence of any one of SEQ ID NOs:29-33. An exemplary P2A peptide comprises an amino acid sequence of SEQ ID NO: 34 or 35. An exemplary E2A peptide sequence comprises an amino acid sequence of SEQ ID NO:36. An exemplary F2A peptide sequence comprises an amino acid sequence of SEQ ID NO:37.

Certain embodiments of tandem expression cassettes of the present disclosure comprise a polynucleotide encoding a CAR/or TCR specific for a target antigen (*e.g.,* tumor antigen) and a polynucleotide encoding a chimeric Tim4 receptor of the present disclosure. Upon binding a target cell expressing the target antigen by the CAR/or TCR, a cell modified to express such a tandem expression cassette induces apoptosis of the target cell. Apoptosis induces exposure of pro-engulfment markers on the target cell, such as phosphatidylserine, which may then target the damaged or apoptotic cells for engulfment by the chimeric Tim4 receptor.

A polynucleotide encoding a desired chimeric Tim4 receptor can be inserted into an appropriate vector, *e.g.,* a viral vector, non-viral plasmid vector, and non-viral vectors, such as lipid-based DNA vectors, modified mRNA (modRNA), self-amplifying mRNA, CELiD, and transposon-mediated gene transfer (PiggyBac, Sleeping Beauty), for introduction into a host cell of interest (*e.g.,* an immune cell). Polynucleotides encoding a chimeric Tim4 receptor of the present disclosure can be cloned into any suitable vector, such as an expression vector, a replication vector, a probe generation vector, or a sequencing vector. In certain embodiments, a polynucleotide encoding the extracellular domain, a polynucleotide encoding the transmembrane domain, and a polynucleotide encoding the intracellular signaling domain are joined together into a single polynucleotide and then inserted into a vector. In other embodiments, a polynucleotide encoding the extracellular domain, a polynucleotide encoding the transmembrane domain, and a polynucleotide encoding the intracellular signaling domain may be inserted separately into a vector such that the expressed amino acid sequence produces a functional chimeric Tim4 receptor. A vector that encodes a chimeric Tim4 receptor is referred to herein as a "chimeric Tim4 receptor vector."

In certain embodiments, a vector comprises a polynucleotide encoding one chimeric Tim4 receptor. In certain embodiments, a vector comprises one polynucleotide encoding two or more chimeric Tim4 receptors. In certain embodiments, a single polynucleotide encoding two or more chimeric Tim4 receptors is cloned into a cloning site and expressed from a single promoter, with each chimeric Tim4 receptor sequence separated from each other by an internal ribosomal entry site (IRES), furin cleavage site, or viral 2A peptide to allow for co-expression of multiple genes from a single open reading frame (*e.g.,* a multicistronic vector). In certain embodiments, a viral 2A peptide is a porcine teschovirus-1 (P2A), *Thosea asigna* virus (T2A), equine rhinitis A virus (E2A), foot-and-mouth disease virus (F2A), or variant thereof. An exemplary T2A peptide comprises an amino acid sequence of any one of SEQ ID NOs:29-33. An exemplary P2A peptide comprises an amino acid sequence of SEQ ID NO:34 or 35. An exemplary E2A peptide sequence comprises an amino acid sequence of SEQ ID NO:36. An exemplary F2A peptide sequence comprises an amino acid sequence of SEQ ID NO:37.

In certain embodiments, a vector comprises two or more polynucleotides, each polynucleotide encoding a chimeric Tim4 receptor. The two or more polynucleotides encoding chimeric Tim4 receptors may be cloned sequentially into a vector at different cloning sites, with each chimeric Tim4 receptor expressed under the regulation of different promoters. In certain embodiments, vectors that allow long-term integration of a transgene and propagation to daughter cells are utilized. Examples include viral vectors such as, adenovirus, adeno-associated virus, vaccinia virus, herpes viruses, cytomegalovirus, pox virus, or retroviral vectors, such as lentiviral vectors. Vectors derived from lentivirus can be used to achieve long-term gene transfer and have added advantages over vectors including the ability to transduce non-proliferating cells, such as hepatocytes, and low immunogenicity.

In certain embodiments, a vector comprises a polynucleotide encoding a chimeric Tim4 receptor and a polynucleotide encoding a cellular immunotherapy agent (e.g., chimeric antigen receptor, recombinant TCR, etc.). In certain embodiments, a single polynucleotide encoding the chimeric Tim4 receptor and cellular immunotherapy agent (e.g., CAR) is cloned into a cloning site and expressed from a single promoter, with the chimeric Tim4 receptor sequence and cellular immunotherapy agent (e.g., CAR) sequence separated from each other by an internal ribosomal entry site (IRES), furin cleavage site, or viral 2A peptide to allow for co-expression of multiple genes from a single open reading frame *(e.g.,* a multicistronic vector). In certain embodiments, a viral 2A peptide is a porcine teschovirus-1 (P2A), *Thosea asigna* virus (T2A), equine rhinitis A virus (E2A), foot-and-mouth disease virus (F2A), or variant thereof. An exemplary T2A peptide comprises an amino acid sequence of SEQ ID NO:29-33. An exemplary P2A peptide comprises an amino acid sequence of SEQ ID NO:34 or 35. An exemplary E2A peptide sequence comprises an amino acid sequence of SEQ ID NO:36. An exemplary F2A peptide sequence comprises an amino acid sequence of SEQ ID NO:37.

In certain embodiments, a polynucleotide encoding the chimeric Tim4 receptor and a polynucleotide encoding the cellular immunotherapy agent (e.g., CAR) binding protein are joined together into a single polynucleotide and then inserted into a vector. In other embodiments, a polynucleotide encoding the CER, and a polynucleotide encoding the CAR or TCR binding protein may be inserted separately into a vector in the same or different cloning sites, such that the expressed amino acid sequence produces a functional CER and CAR/or TCR. A vector that encodes a tandem expression cassette is referred to herein as a "tandem expression vector."

In certain embodiments, a vector comprises a polynucleotide encoding a chimeric Tim4 receptor and a polynucleotide encoding a cellular immunotherapy agent (e.g., CAR). The polynucleotides encoding the chimeric Tim4 receptor and cellular immunotherapy agent (e.g., CAR) may be cloned sequentially into a vector at different cloning sites, with the chimeric Tim4 receptor and cellular immunotherapy agent (e.g., CAR) expressed under the regulation of different promoters.

A vector that encodes a core virus is referred to herein as a "viral vector." There are a large number of available viral vectors suitable for use with the compositions of the instant disclosure, including those identified for human gene therapy applications (*see* Pfeifer and Verme, Ann. Rev. Genomics Hum. Genet. 2:177, 2001). Suitable viral vectors include vectors based on RNA viruses, such as retrovirus-derived vectors, *e.g.*, Maloney murine leukemia virus (MLV)-derived vectors, and include more complex retrovirus-derived vectors, *e.g*., lentivirus-derived vectors. HIV-1-derived vectors belong to this category. Other examples include lentivirus vectors derived from HIV-2, FIV, equine infectious anemia virus, SIV, and Maedi-Visna virus (ovine lentivirus). Methods of using retroviral and lentiviral viral vectors and packaging cells for transducing mammalian host cells with viral particles containing chimeric receptor transgenes are known in the art and have been previous described, for example, in U.S. Patent 8,119,772; Walchli et al., PLoS One 6:327930, 2011; Zhao et al., J. Immunol. 174:4415, 2005; Engels et al., Hum. Gene Ther. 14:1155, 2003; Frecha et al., Mol. Ther. 18:1748, 2010; Verhoeyen et al., Methods Mol. Biol. 506:97, 2009. Retroviral and lentiviral vector constructs and expression systems are also commercially available.

In certain embodiments, a viral vector is used to introduce a non-endogenous polynucleotide encoding a chimeric Tim4 receptor to a host cell. A viral vector may be a retroviral vector or a lentiviral vector. A viral vector may also include a nucleic acid sequence encoding a marker for transduction. Transduction markers for viral vectors are known in the art and include selection markers, which may confer drug resistance, or detectable markers, such as fluorescent markers or cell surface proteins that can be detected by methods such as flow cytometry. In particular embodiments, a viral vector further comprises a gene marker for transduction comprising a fluorescent protein (*e.g.,* green, yellow), an extracellular domain of human CD2, or a truncated human EGFR (EGFRt or tEGFR; *see* Wang et al., Blood 118:1255, 2011). An exemplary tEGFR comprises an amino acid sequence of SEQ ID NO:38. When a viral vector genome comprises a plurality of genes to be expressed in a host cell as separate proteins from a single transcript, the viral vector may also comprise additional sequences between the two (or more) genes allowing for multicistronic expression. Examples of such sequences used in viral vectors include internal ribosome entry sites (IRES), furin cleavage sites, viral 2A peptides (*e.g.,* T2A, P2A, E2A, F2A), or any combination thereof.

Other viral vectors also can be used for polynucleotide delivery including DNA viral vectors, including, for example adenovirus-based vectors and adeno-associated virus (AAV)-based vectors; vectors derived from herpes simplex viruses (HSVs), including amplicon vectors, replication-defective HSV and attenuated HSV (Krisky et al., Gene Ther. 5: 1517, 1998).

Other viral vectors recently developed for gene therapy uses can also be used with the compositions and methods of this disclosure. Such vectors include those derived from baculoviruses and α-viruses. (Jolly, D J. 1999. Emerging Viral Vectors. pp 209-40 in Friedmann T. ed. The Development of Human Gene Therapy. New York: Cold Spring Harbor Lab), or plasmid vectors (such as sleeping beauty or other transposon vectors).

In certain embodiments, a chimeric Tim4 receptor vector can be constructed to optimize spatial and temporal control. For example, a chimeric Tim4 receptor vector can include promoter elements to optimize spatial and temporal control. In some embodiments, a chimeric Tim4 receptor vector includes tissue specific promoters or enhancers that enable specific induction of a chimeric Tim4 receptor to an organ, a cell type (*e.g.,* immune cell), or a pathologic microenvironment, such as a tumor or infected tissue. An "enhancer" is an additional promoter element that can function either cooperatively or independently to activate transcription. In certain embodiments, a chimeric Tim4 receptor vector includes a constitutive promoter. In certain embodiments, a chimeric Tim4 receptor vector includes an inducible promoter. In certain embodiments, a chimeric Tim4 receptor vector includes a tissue specific promoter.

In certain embodiments, a chimeric Tim4 receptor vector can include a gene encoding a homing receptor, such as CCR4 or CXCR4, to improve homing and antitumor activity *in vivo.*

Where temporal control is desired, a chimeric Tim4 receptor vector may include an element that allows for inducible depletion of transduced cells. For example, such a vector may include an inducible suicide gene. A suicide gene may be an apoptotic gene or a gene that confers sensitivity to an agent (*e.g.,* a drug). Exemplary suicide genes include chemically inducible caspase 9 (iCASP9) (U.S. Patent Publication No. 2013/0071414), chemically inducible Fas, or Herpes simplex virus thymidine kinase (HSV-TK), which confers sensitivity to ganciclovir. In further embodiments, a chimeric Tim4 receptor vector can be designed to express a known cell surface antigen that, upon infusion of an associated antibody, enables depletion of transduced cells. Examples of cell surface antigens and their associated antibodies that may be used for depletion of transduced cells include CD20 and Rituximab, RQR8 (combined CD34 and CD20 epitopes, allowing CD34 selection and anti-CD20 deletion) and Rituximab, and EGFR and Cetuximab.

Inducible vector systems, such as the tetracycline (Tet)-On vector system which activates transgene expression with doxycycline (Heinz et al., Hum. Gene Ther. 2011, 22:166-76) may also be used for inducible chimeric Tim4 receptor expression. Inducible chimeric Tim4 receptor expression may be also accomplished via retention using a selective hook (RUSH) system based on streptavidin anchored to the membrane of the endoplasmic reticulum through a hook and a streptavidin binding protein introduced into the chimeric Tim4 receptor structure, where addition of biotin to the system leads to the release of the chimeric Tim4 receptor from the endoplasmic reticulum (Agaugue et al., 2015, Mol. Ther. 23(Suppl. 1):S88).

In certain embodiments, a chimeric Tim4 receptor modified host cell may also be modified to co-express one or more small GTPases. Rho GTPases, a family of small (~21 k Da) signaling G proteins and also a subfamily of the Ras superfamily, regulate actin cytoskeleton organization in various cell types and promote pseudopod extension and phagosome closure during phagocytosis (*see, e.g.,* Castellano et al., 2000, J. Cell Sci. 113:2955-2961). Engulfment requires F-actin recruitment beneath tethered cells or particles, and F-actin rearrangement to allow membrane extension resulting in cell or particle internalization. RhoGTPases include RhoA, Rac1, Rac2, RhoG, and CDC42. Other small GTPases, such as Rap1, is involved in regulation of complement mediated phagocytosis. Co-expression of a small GTPase with the chimeric Tim4 receptor may promote target cell or particle internalization and/or phagosome formation by the host cell. In some embodiments, a recombinant nucleic acid molecule encoding a GTPase is encoded on a separate vector than the chimeric Tim4 receptor-containing vector. In other embodiments, a recombinant nucleic acid molecule encoding a GTPase is encoded on the same vector as the chimeric Tim4 receptor . The GTPase and chimeric Tim4 receptor may be expressed under the regulation of different promoters on the same vector (*e.g.,* at different multiple cloning sites). Alternatively, the chimeric Tim4 receptor and GTPase may be expressed under the regulation of one promoter in a multicistronic vector. The polynucleotide sequence encoding the chimeric Tim4 receptor and the polynucleotide sequence encoding the small GTPase(s) may be separated from each other by an IRES or viral 2A peptide in a multicistronic vector. Exemplary 2A peptides include T2A (SEQ ID NO:29-33), P2A (SEQ ID NO:34 or 35), E2A (SEQ ID NO:36), F2A (SEQ ID NO:37). Examples of GTPases that may be co-expressed with a chimeric Tim4 receptor include Rac1, Rac2, Rab5 (also referred to as Rab5a), Rab7, Rap1, RhoA, RhoG, CDC42, or any combination thereof. In specific embodiments, the GTPase comprises or is a sequence that is at least 90%, at least 91%, at least 92%, at least 93%, at least 94%, at least 95%, at least 96%, at least 97%, at least 98%, at least 99%, at least 99.5%, or 100% identical to a Rac1 amino acid sequence of SEQ ID NO:41, a Rab5 amino acid sequence of SEQ ID NO:42, a Rab7 amino acid sequence of SEQ ID NO:43, a Rap1 amino acid sequence of SEQ ID NO:44, a RhoA amino acid sequence of SEQ ID NO:45, a CDC42 amino acid sequence of SEQ ID NO:46, or any combination thereof.

In certain embodiments, a cell, such as an immune cell, obtained from a subject may be engineered into a non-natural or recombinant cell (*e.g.,* a non-natural or recombinant immune cell) by introducing a polynucleotide that encodes a chimeric Tim4 receptor as described herein, whereby the cell expresses a cell surface localized chimeric Tim4 receptor. In certain embodiments, a host cell is an immune cell, such as a myeloid progenitor cell or a lymphoid progenitor cell. Exemplary immune cells that may be modified to comprise a polynucleotide encoding a chimeric Tim4 receptor or a vector comprising a polynucleotide encoding a chimeric Tim4 receptor include a T cell, a natural killer cell, a B cell, a lymphoid precursor cell, an antigen presenting cell, a dendritic cell, a Langerhans cell, a myeloid precursor cell, a mature myeloid cell, a monocyte, or a macrophage.

In certain embodiments, a B cell is genetically modified to express one or more chimeric Tim4 receptors. B cells possess certain properties that may be advantageous as host cells, including: trafficking to sites of inflammation, capable of internalizing and presenting antigen, capable of costimulating T cells, highly proliferative, and self-renewing (persist for life). In certain embodiments, a chimeric Tim4 receptor modified B cell is capable of digesting an engulfed target cell or engulfed target particle into smaller peptides and presenting them to T cells via an MHC molecule. Antigen presentation by a chimeric Tim4 receptor modified B cell may contribute to antigen spreading of the immune response to non-targeted antigens. B cells include progenitor or precursor cells committed to the B cell lineage (*e.g.,* pre-pro-B cells, pro-B cells, and pre-B cells); immature and inactivated B cells; or mature and functional or activated B cells. In certain embodiments, B cells may be naive B cells, plasma cells, regulatory B cells, marginal zone B cells, follicular B cells, lymphoplasmacytoid cell, plasmablast cell, memory B cells, or any combination thereof. Memory B cells may be distinguished from naive B cells by expression of CD27, which is absent on naive B cells. In certain embodiments, the B cells can be primary cells or cell lines derived from human, mouse, rat, or other mammals. B cell lines are well known in the art. If obtained from a mammal, a B cell can be obtained from numerous sources, including blood, bone marrow, spleen, lymph node, or other tissues or fluids. A B cell composition may be enriched or purified.

In certain embodiments, a T cell is genetically modified to express one or more chimeric Tim4 receptors. Exemplary T cells include CD4⁺ helper, CD8⁺ effector (cytotoxic), naive (CD45 RA+, CCR7+, CD62L+, CD27+, CD45RO-), central memory (CD45RO⁺, CD62L⁺, CD8⁺), effector memory (CD45RA+, CD45RO-, CCR7-, CD62L-, CD27-), T memory stem, regulatory, mucosal-associated invariant (MAIT), γδ (gd), tissue resident T cells, natural killer T cells, or any combination thereof. In certain embodiments, the T cells can be primary cells or cell lines derived from human, mouse, rat, or other mammals. If obtained from a mammal, a T cell can be obtained from numerous sources, including blood, bone marrow, lymph node, thymus, or other tissues or fluids. A T cell composition may be enriched or purified. T cell lines are well known in the art, some of which are described in Sandberg et al., Leukemia 21:230, 2000. In certain embodiments, the T cells lack endogenous expression of a TCRα gene, TCRβ gene, or both. Such T cells may naturally lack endogenous expression of TCRα and β chains, or may have been modified to block expression (*e.g.,* T cells from a transgenic mouse that does not express TCR α and β chains or cells that have been manipulated to inhibit expression of TCR α and β chains) or to knockout a TCRα chain, a TCRβ chain, or both genes.

In certain embodiments, host cells expressing a chimeric Tim protein of this disclosure on the cell surface are not T cells or cells of a T cell lineage, but cells that are progenitor cells, stem cells or cells that have been modified to express cell surface anti-CD3.

In certain embodiments, a chimeric Tim4 receptor modified host cell may also be modified to co-express a cellular immunotherapy agent (e.g., CAR, TCR, etc.). In some embodiments, the cellular immunotherapy agent comprises a chimeric antigen receptor (CAR). CARs are recombinant receptors that generally comprise: an extracellular domain comprising a binding domain that binds to a target antigen; an intracellular signaling domain (e.g., comprising an ITAM containing intracellular signaling domain and optionally an intracellular costimulatory domain), and a transmembrane domain positioned between and connecting the extracellular domain and the intracellular signaling domain.

Binding domains suitable for use in CARs of the present disclosure include any antigen-binding polypeptide. A binding domain may comprise an antibody or antigen binding fragment thereof, including for example, a full length heavy chain, Fab fragment, Fab', F(ab')₂, sFv, VH domain, VL domain, dAb, VHH, CDR, and scFv. In certain embodiments, a CAR binding domain is murine, chimeric, human, or humanized.

In certain embodiments, the binding domain of the CAR targets a cancer or tumor antigen. Exemplary antigens that a CAR may target include CD138, CD38, CD33, CD123, CD72, CD79a, CD79b, mesothelin, PSMA, BCMA, ROR1, MUC-16, L1CAM, CD22, CD19, CD20, CD23, CD24, CD37, CD30, CA125, CD56, c-Met, EGFR, GD-3, HPV E6, HPV E7, MUC-1, HER2, folate receptor α, CD97, CD171, CD179a, CD44v6, WT1, VEGF-α, VEGFR1, IL-13Rα1, IL-13Rα2, IL-11Rα, PSA, FcRH5, NKG2D ligand, NY-ESO-1, TAG-72, CEA, ephrin A2, ephrin B2, Lewis A antigen, Lewis Y antigen, MAGE, MAGE-A1, RAGE-1, folate receptor β, EGFRviii, VEGFR-2, LGR5, SSX2, AKAP-4, FLT3, fucosyl GM1, GM3, o-acetyl-GD2, and GD2.

In certain embodiments, the extracellular domain of CARs provided in the present disclosure optionally comprises an extracellular, non-signaling spacer or linker domain. Where included, such a spacer or linker domain may position the binding domain away from the host cell surface to further enable proper cell to cell contact, binding, and activation. An extracellular spacer domain is generally located between the extracellular binding domain and the transmembrane domain of the CAR. The length of the extracellular spacer may be varied to optimize target molecule binding based on the selected target molecule, selected binding epitope, binding domain size and affinity *(see, e.g.,* Guest et al., J. Immunother. 28:203-11, 2005; PCT Publication No. WO 2014/031687). In certain embodiments, an extracellular spacer domain is an immunoglobulin hinge region (*e.g.,* IgG1, IgG2, IgG3, IgG4, IgA, IgD). An immunoglobulin hinge region may be a wild type immunoglobulin hinge region or an altered wild type immunoglobulin hinge region. An altered IgG₄ hinge region is described in PCT Publication No. WO 2014/031687. In a particular embodiment, an extracellular spacer domain comprises a modified IgG₄ hinge region having an amino acid sequence of SEQ ID NO:9.

Other examples of hinge regions that may be used in the CARs described herein include the hinge region from the extracellular regions of type 1 membrane proteins, such as CD8a, CD4, CD28 and CD7, which may be wild-type or variants thereof. In a particular embodiment, an extracellular spacer domain comprises a CD8a hinge region having an amino acid sequence of SEQ ID NO:172. In another particular embodiment, an extracellular spacer domain comprises a CD28 hinge region having an amino acid sequence of SEQ ID NO:10. In further embodiments, an extracellular spacer domain comprises all or a portion of an immunoglobulin Fc domain selected from: a CH1 domain, a CH2 domain, a CH3 domain, or combinations thereof (*see, e.g.,* PCT Publication WO2014/031687). In yet further embodiments, an extracellular spacer domain may comprise a stalk region of a type II C-lectin (the extracellular domain located between the C-type lectin domain and the transmembrane domain). Type II C-lectins include CD23, CD69, CD72, CD94, NKG2A, and NKG2D.

CARs of the present disclosure comprise a transmembrane domain that connects and is positioned between the extracellular domain and the intracellular signaling domain. The transmembrane domain ranges in length from about 15 amino acids to about 30 amino acids. The transmembrane domain is a hydrophobic alpha helix that transverses the host cell membrane and anchors the CAR in the host cell membrane. The transmembrane domain may be directly fused to the binding domain or to the extracellular spacer domain if present. In certain embodiments, the transmembrane domain is derived from an integral membrane protein (*e.g.,* receptor, cluster of differentiation (CD) molecule, enzyme, transporter, cell adhesion molecule, or the like). The transmembrane domain can be selected from the same molecule as the extracellular domain or the intracellular signaling domain (*e.g.,* a CAR comprises a CD28 costimulatory signaling domain and a CD28 transmembrane domain). In certain embodiments, the transmembrane domain and the extracellular domain are each selected from different molecules. In other embodiments, the transmembrane domain and the intracellular signaling domain are each selected from different molecules. In yet other embodiments, the transmembrane domain, the extracellular domain, and the intracellular signaling domain are each selected from different molecules.

Exemplary transmembrane domains for use in CARs of the present disclosure include a CD28, CD2, CD4, CD8a, CD5, CD3ε, CD3δ, CD3ζ, CD9, CD16, CD22, CD25, CD27, CD33, CD37, CD40, CD45, CD64, CD79A, CD79B, CD80, CD86, CD95 (Fas), CD134 (OX40), CD137 (4-1BB), CD150 (SLAMF1), CD152 (CTLA4), CD154 (CD40L), CD200R, CD223 (LAG3), CD270 (HVEM), CD272 (BTLA), CD273 (PD-L2), CD274 (PD-L1), CD278 (ICOS), CD279 (PD-1), CD300, CD357 (GITR), A2aR, DAP10, FcRα, FcRβ, FcRγ, Fyn, GAL9, KIR, Lck, LAT, LRP, NKG2D, NOTCH1, NOTCH2, NOTCH3, NOTCH4, PTCH2, ROR2, Ryk, Slp76, SIRPα, pTα, TCRα, TCRβ, TIM3, TRIM, LPA5, and Zap70 transmembrane domain. An exemplary CD28 transmembrane domain comprises an amino acid sequence of SEQ ID NO:11. In a particular embodiment, a transmembrane domain comprises a CD8a transmembrane domain having an amino acid sequence of SEQ ID NO:174.

The intracellular signaling domain of a CAR is an intracellular effector domain and is capable of transmitting functional signals to a cell in response to binding of the extracellular domain of the CAR to a target molecule (e.g., cancer antigen) and activates at least one of the normal effector functions or responses of the immune cell, e.g., T cell engineered to express the CAR. In some embodiments, the CAR induces a function of a T cell such as cytolytic activity or T helper activity, such as secretion of cytokines or other factors. The intracellular signaling domain may be any portion of an intracellular signaling molecule that retains sufficient signaling activity. In some embodiments, the intracellular signaling domain is obtained from an antigen receptor component (e.g., TCR) or costimulatory molecule. In some embodiments, a full length intracellular signaling domain of an antigen receptor or costimulatory molecule is used. In some embodiments, a truncated portion of an intracellular signaling domain of an antigen receptor or costimulatory molecule is used, provided that the truncated portion retains sufficient signal transduction activity. In further embodiments, an intracellular signaling domain is a variant of a full length or truncated portion of an intracellular signaling domain of an antigen receptor co stimulatory molecule, provided that the variant retains sufficient signal transduction activity (*i.e.,* is a functional variant).

In some embodiments, the intracellular signaling domain of a CAR comprises an immunoreceptor tyrosine-based activation motif (ITAM) containing signaling domain. An ITAM containing signaling domain generally contains at least one (one, two, three, four, or more) ITAMs, which refer to a conserved motif of YXXL/I-X₆₋₈-YXXL/I. An ITAM containing signaling domain may initiate T cell activation signaling following antigen binding or ligand engagement. ITAM-signaling domains include, for example, intracellular signaling domains of CD3γ, CD3δ, CD3ε, CD3ζ, CD5, CD22, CD79a, CD278 (ICOS), DAP12, FcRγ, and CD66d. Exemplary CD3ζ signaling domains that may be used in CARs of the present disclosure comprise the amino acid sequence of SEQ ID NO: 177 or SEQ ID NO:178.

CAR intracellular signaling domains optionally comprise a costimulatory signaling domain, which, when activated in conjunction with a primary or classic (e.g., ITAM-driven) activation signal, promotes or enhances T cell response, such as T cell activation, cytokine production, proliferation, differentiation, survival, effector function, or combinations thereof. Costimulatory signaling domains for use in CARs include, for example, CD27, CD28, CD40L, GITR, NKG2C, CARD1, CD2, CD7, CD27, CD30, CD40, CD54 (ICAM), CD83, CD134 (OX-40), CD137 (4-1BB), CD150 (SLAMF1), CD152 (CTLA4), CD223 (LAG3), CD226, CD270 (HVEM), CD273 (PD-L2), CD274 (PD-L1), CD278 (ICOS), DAP10, LAT, LFA-1, LIGHT, NKG2C, SLP76, TRIM, ZAP70, or any combination thereof. In some embodiments, the costimulatory signaling domain comprises a OX40, CD2, CD27, CD28, ICAM-1, LFA-1 (CD11a/CD18), ICOS (CD278), or 4-1BB (CD137) signaling domain. Exemplary CD28 costimulatory signaling domains that may be used in CARs of the present disclosure comprise an amino acid sequence of SEQ ID NO:179 or 180. An exemplary 4-1BB costimulatory signaling domain comprises an amino acid sequence of SEQ ID NO: 181. In certain embodiments, a CAR comprises one, two, or more costimulatory signaling domains.

In some embodiments, CARs are recombinant receptors composed of an scFv binding domain derived from an antibody, a transmembrane domain, and an intracellular signaling domain(s). In some embodiments, the intracellular signaling domain(s) are derived from a TCR.

In certain embodiments, a chimeric antigen receptor comprises an amino acid sequences derived from any mammalian species, including humans, primates, cows, horses, goats, sheep, dogs, cats, mice, rats, rabbits, guinea pigs, pigs, non-human transgenic species thereof, or any combination thereof. In certain embodiments, chimeric antigen receptor is murine, chimeric, human, or humanized.

In certain embodiments, a CAR is a first generation CAR, a second generation CAR, or a third generation CAR. A first generation CAR generally has an intracellular signaling domain comprising an intracellular signaling domain of CD3ζ, FcγRI, or other ITAM-containing activating domain to provide a T cell activation signal. Second generation CARs further comprise a costimulatory signaling domain *(e.g.,* a costimulatory signaling domain from an endogenous T cell costimulatory receptor, such as CD28, 4-1BB, or ICOS). Third generation CARs comprise an ITAM-containing activating domain, a first costimulatory signaling domain and a second costimulatory signaling domain.

In some embodiments, one or more of the extracellular domain, the binding domain, the linker, the transmembrane domain, the intracellular signaling domain, or the costimulatory domain comprises junction amino acids. "Junction amino acids" or "junction amino acid residues" refer to one or more (e.g., about 2-20) amino acid residues between two adjacent domains, motifs, regions, modules, or fragments of a protein, such as between a binding domain and an adjacent linker, between a transmembrane domain and an adjacent extracellular or intracellular domain, or on one or both ends of a linker that links two domains, motifs, regions, modules, or fragments (e.g., between a linker and an adjacent binding domain or between a linker and an adjacent hinge). Junction amino acids may result from the construct design of a fusion protein (e.g., amino acid residues resulting from the use of a restriction enzyme site or self-cleaving peptide sequences during the construction of a polynucleotide encoding a fusion protein). For example, a transmembrane domain of a fusion protein may have one or more junction amino acids at the amino-terminal end, carboxy -terminal end, or both.

In certain embodiments, an engineered host cell co-expresses a chimeric Tim4 receptor and an anti-CD72 CAR.

In certain embodiments, a chimeric Tim4 receptor modified host cell co-expresses a recombinant TCR. Recombinant TCR proteins include "traditional" TCRs composed of a heterodimer of α chain polypeptide and β chain polypeptide or a heterodimer of a γ chain polypeptide and a δ chain polypeptide, binding fragments and fusion proteins thereof, including for example, single chain TCRs, single domain TCRs, soluble TCR fusion TCR proteins, and TCR fusion constructs (TRuC^{™}). In certain embodiments, a tandem expression cassette comprises a polynucleotide encoding a recombinant TCR beta chain comprising a TCR beta variable region and a TCR beta constant region, and a polynucleotide encoding a recombinant TCR alpha chain comprising a TCR alpha variable region and a TCR alpha constant region. In certain embodiments, a recombinant TCR is an enhanced affinity TCR. In one embodiment, a recombinant TCR is an enhanced affinity TCR.

In certain embodiments, a recombinant TCR binding protein is a single chain TCR (scTCR) comprising a Vα joined to a Vβ by a flexible linker. In some embodiments, a scTCR comprises a Vα-linker-Vβ polypeptide. In other embodiments, a scTCR comprises a Vβ-linker-Vα polypeptide.

In certain embodiments, a chimeric Tim4 receptor modified host cell may also be modified to co-express a single chain TCR (scTCR) fusion protein. A scTCR fusion protein comprises a binding domain comprising a scTCR (a TCR Vα domain linked to a TCR Vβ domain), an optional extracellular spacer, a transmembrane domain, and an intracellular component comprising a single intracellular signaling domain providing an T cell activation signal (*e.g.,* a CD3ζ ITAM-containing activating domain) and optionally a costimulatory signaling domain (*see,* Aggen et al., 2012, Gene Ther. 19:365-374; Stone et al., Cancer Immunol. Immunother. 2014, 63:1163-76).

In certain embodiments, a chimeric Tim4 receptor modified host cell may also be modified to co-express a T cell receptor-based chimeric antigen receptor (TCR-CAR). A TCR-CAR is a heterodimeric fusion protein generally comprising a soluble TCR (a polypeptide chain comprising a Vα domain and Cα domain and a polypeptide chain comprising a Vβ domain and a Cβ domain) wherein the VβCβ polypeptide chain is linked to a transmembrane domain and an intracellular signaling component (*e.g.,* an ITAM-containing activating domain and optionally a costimulatory signaling domain) (*see, e.g.,* Walseng et al., 2017 Scientific Reports 7:10713).

In certain embodiments, an engineered host cell that co-expresses a chimeric Tim4 receptor and a cellular immunotherapy agent (e.g., CAR, TCR, etc.) comprises a recombinant nucleic acid encoding the chimeric Tim4 receptor and a recombinant nucleic acid molecule encoding the cellular immunotherapy agent on separate vectors within the engineered host cell.

In some embodiments, an engineered host cell that co-expresses a chimeric Tim4 receptor and a cellular immunotherapy agent (e.g., CAR, TCR, etc.) comprises a recombinant nucleic acid encoding the chimeric Tim4 receptor and a recombinant nucleic acid molecule encoding the cellular immunotherapy agent on the same vector as the chimeric Tim4 receptor within an engineered host cell. The chimeric Tim4 receptor and cellular immunotherapy agent may be expressed under the regulation of different promoters on the same vector (*e.g.,* at different multiple cloning sites). Alternatively, the chimeric Tim4 receptor and cellular immunotherapy agent may be expressed under the regulation of one promoter in a multicistronic vector (e.g., tandem expression vector). The polynucleotide sequence encoding the chimeric Tim4 receptor and the polynucleotide sequence encoding the cellular immunotherapy agent may be separated by an IRES or viral 2A peptide in a multicistronic vector.

Tandem expression cassettes, tandem expression vectors, and engineered host cells comprising the same are described in International Application Publication No. WO2019/191339.

In certain embodiments, gene editing methods are used to modify the host cell genome to comprise a polynucleotide encoding a chimeric Tim4 receptor of the present disclosure. Gene editing, or genome editing, is a method of genetic engineering wherein DNA is inserted, replaced, or removed from a host cell's genome using genetically engineered endonucleases. The nucleases create specific double-stranded breaks at targeted loci in the genome. The host cell's endogenous DNA repair pathways then repair the induced break(s), *e.g*., by non-homologous ending joining (NHEJ) and homologous recombination. Exemplary endonucleases useful for gene editing include a zinc finger nuclease (ZFN), a transcription activator-like effector (TALE) nuclease, a clustered regularly interspaced short palindromic repeats (CRISPR)/Cas nuclease system (*e.g.,* CRISPR-Cas9), a meganuclease, or combinations thereof. Methods of disrupting or knocking out genes or gene expression in immune cells including B cells and T cells, using gene editing endonucleases are known in the art and described, for example, in International Application Publication Nos. WO 2015/066262; WO 2013/074916; WO 2014/059173; Cheong et al., Nat. Comm. 2016 7:10934; Chu et al., Proc. Natl. Acad. Sci. USA 2016 113:12514-12519.

In certain embodiments, expression of an endogenous gene of the host cell is inhibited, knocked down, or knocked out. Examples of endogenous genes that may be inhibited, knocked down, or knocked out in a B cell include IGH, IGκ, IGλ, or any combination thereof. Examples of endogenous genes that may be inhibited, knocked down, or knocked out in a T cell include a TCR gene (TRA or TRB), an HLA gene (HLA class I gene or HLA class II gene), an immune checkpoint molecule (PD-L1, PD-L2, CD80, CD86, B7-H3, B7-H4, HVEM, adenosine, GAL9, VISTA, CEACAM-1, CEACAM-3, CEACAM-5, PVRL2, PD-1, CTLA-4, BTLA, KIR, LAG3, TIM3, A2aR, CD244/2B4, CD160, TIGIT, LAIR-1, or PVRIG/CD 112R), or any combination thereof. Expression of an endogenous gene may be inhibited, knocked down, or knocked out at the gene level, transcriptional level, translational level, or a combination thereof. Methods of inhibiting, knocking down, or knocking out an endogenous gene may be accomplished, for example, by an RNA interference agent (*e.g.,* siRNA, shRNA, miRNA, etc.) or an engineered endonuclease (*e.g.,* CRISPR/Cas nuclease system, a zinc finger nuclease (ZFN), a Transcription Activator Like Effector nuclease (TALEN), a meganuclease), or any combination thereof. In certain embodiments, an endogenous B cell gene (*e.g.,* IGH, IGκ, or IGλ) is knocked out by insertion of a polynucleotide encoding a chimeric Tim4 receptor of the present disclosure into the locus of the endogenous B cell gene, such as via an engineered endonuclease. In certain embodiments, an endogenous T cell gene (*e.g.,* a TCR gene, an HLA gene, or an immune checkpoint molecule gene) is knocked out by insertion of a polynucleotide encoding a chimeric Tim4 receptor of the present disclosure into the locus of the endogenous T cell gene, such as via an engineered endonuclease.

In certain embodiments, a host cell may be genetically modified to express one type of chimeric Tim4 receptor. In other embodiments, a host cell may express at least two or more different chimeric Tim4 receptors.

The present disclosure also provides a composition comprising a population of chimeric Tim4 receptor modified host cells. In certain embodiments, the population of chimeric Tim4 receptor modified host cells may be a population of B cells, a population of T cells, a population of natural killer cells, a population of lymphoid precursor cells, a population of antigen presenting cells, a population of dendritic cells, a population of Langerhans cells, a population of myeloid precursor cells, a population of mature myeloid cells, or any combination thereof. Furthermore, a population of chimeric Tim4 receptor modified host cells of a particular cell type may be composed of one or more subtypes. For example, a population of B cells may be composed of chimeric Tim4 receptor modified naive B cells, plasma cells, regulatory B cells, marginal zone B cells, follicular B cells, lymphoplasmacytoid cells, plasmablast cells, memory B cells, or any combination thereof. In another example, a population of T cells may be composed of chimeric Tim4 receptor modified CD4⁺ helper T cells, CD8⁺ effector (cytotoxic) T cells, naive (CD45 RA+, CCR7+, CD62L+, CD27+, CD45RO-) T cells, central memory (CD45RO⁺, CD62L⁺, CD8⁺) T cells, effector memory (CD45RA+, CD45RO-, CCR7-, CD62L-, CD27-) T cells, T memory stem cells, regulatory T cells, mucosal-associated invariant T cells (MAIT), γδ (gd) cells, tissue resident T cells, natural killer T cells, or any combination thereof.

In certain embodiments, a population of host cells is composed of cells that each expresses the same chimeric Tim4 receptor(s). In other embodiments, a population of host cells is composed of a mixture of two or more subpopulation of host cells, wherein each subpopulation expresses a different chimeric Tim4 receptor or set of chimeric Tim4 receptors.

In certain embodiments, when preparing chimeric Tim4 receptor modified host cells, *e.g.,* B cells or T cells, one or more growth factor cytokines that promotes proliferation of the host cells, *e.g.,* B cells or T cells, may be added to the cell culture. The cytokines may be human or non-human. Exemplary growth factor cytokines that may be used to promote T cell proliferation include IL-2, IL-15, or the like. Exemplary growth factor cytokines that may be used to promote B cell proliferation include CD40L, IL-2, IL-4, IL-15, IL-21, BAFF, or the like.

Prior to genetic modification of the host cells with a chimeric Tim4 receptor vector, a source of host cells (*e.g.,* T cells, B cells, natural killer cells, etc.) is obtained from a subject (*e.g.,* whole blood, peripheral blood mononuclear cells, bone marrow, lymph node tissue, cord blood, thymus tissue, tissue from a site of infection, ascites, pleural effusion, spleen tissue), from which host cells are isolated using methods known in the art. Specific host cell subsets can be collected in accordance with known techniques and enriched or depleted by known techniques, such as affinity binding to antibodies, flow cytometry and/or immunomagnetic selection. After enrichment and/or depletion steps and introduction of a chimeric Tim4 receptor, *in vitro* expansion of the desired modified host cells can be carried out in accordance with known techniques, or variations thereof that will be apparent those skilled in the art.

Chimeric Tim4 receptors of the present disclosure confer cytotoxic activity to host cells expressing the chimeric Tim4 receptors that is specific for phosphatidylserine. Thus, upon binding phosphatidylserine exposed on the surface of a target cell, a host cell expressing a chimeric Tim4 receptor is capable of inducing apoptosis of the target cell. In certain embodiments, the host cell expressing the chimeric Tim4 receptor induces apoptosis of the target cell via: release of granzymes, perforins, granulysin, or any combination thereof; Fas ligand-Fas interaction; or both. In further embodiments, the chimeric Tim4 receptor further confers phosphatidylserine specific engulfment activity to host cells expressing the chimeric Tim4 receptor. In yet further embodiments, the host cell does not naturally exhibit an engulfment phenotype prior to modification with the chimeric Tim4 receptor.

Chimeric Tim4 receptors of the present disclosure may also be capable of costimulating T cells via at least one signaling pathway. In certain embodiments, chimeric Tim4 receptors provide costimulatory signals to T cells via at least two distinct signaling pathways (*e.g.,* via the selected costimulatory signaling domain(s) in the chimeric Tim4 receptor). For example, a chimeric Tim4 receptor comprising a CD28 costimulatory signaling domain may be capable of providing a costimulatory signal via CD28 and Tim1. In certain embodiments, host immune cells expressing the chimeric Tim4 receptors exhibit reduction or inhibition of immune cell exhaustion. In certain embodiments, the host immune cell is a T cell or NK cells. In certain embodiments, exhausted T cells exhibit; (a) increased expression of PD-1, TIGIT, LAG3, TIM3, or any combination thereof; (b) decreased production of IFN-γ, IL-2, TNF-α, or any combination thereof; or both (a) and (b). In certain embodiments, exhausted NK cells exhibit; (a) increased expression of PD-1, NKG2A, TIM3, or any combination thereof; (b) decreased production of IFN-γ, TNF-α, or both; or both (a) and (b).

In certain embodiments, host cells expressing the chimeric Tim4 receptors exhibit an enhanced effector response (*e.g.,* tumor specific). In certain embodiments, the effector response is enhanced T cell proliferation, cytokine production (*e.g.,* IFN-γ, IL-2, TNF-α), cytotoxic activity, persistence, or any combination thereof.

Host cells expressing chimeric Tim4 receptors may be administered to a subject alone, or in combination with other therapeutic agents, including for example CAR-T cells, TCRs, antibodies, radiation therapy, chemotherapies, small molecules, oncolytic viruses, electropulse therapy, etc.

In certain embodiments host cells expressing the chimeric Tim4 receptors exhibit a reduced immunosuppressive response to phosphatidylserine. Phosphatidylserine is one of the primary apoptotic cell ligands that signal "eat me" to phagocytes. The removal of apoptotic cells by phagocytes generally reduces or prevents an inflammatory response via secretion of anti-inflammatory cytokines IL-10 and TGF-β and the decrease of secretion of inflammatory cytokines TNF-α, IL,-1*β*, and IL-12. Thus, phosphatidylserine may act as an immunosuppressive signal during the clearance of apoptotic cells. In certain embodiments, upon binding phosphatidylserine, a chimeric Tim4 receptor modified host cell exhibits increased antigen-specific cytokine production (*e.g.,* IFN-γ, IL-2, TNF-α), thereby reducing the immunosuppressive response to phosphatidylserine.

In some embodiments, T cells expressing the chimeric Tim4 receptors exhibit increased or enhanced antigen capture, antigen processing, and/or antigen presentation activity. Methods of measuring the ability of chimeric Tim4 receptor T cells to present present target peptide antigens and induce target peptide specific activation to target peptide specific T cells are described in Examples 2 and 4.

The expression of chimeric Tim4 receptors on host cells may be functionally characterized according to any of a large number of art-accepted methodologies for assaying host cell (*e.g.,* T cell) activity, including determination of T cell binding, activation or induction and also including determination of T cell responses that are antigen-specific. Examples include determination of T cell proliferation, T cell cytokine release, antigen-specific T cell stimulation, CTL activity (*e.g.,* by detecting ⁵¹Cr or Europium release from pre-loaded target cells), changes in T cell phenotypic marker expression, and other measures of T cell functions. Procedures for performing these and similar assays are may be found, for example, in Lefkovits (Immunology Methods Manual: The Comprehensive Sourcebook of Techniques, 1998). *See, also,* Current Protocols in Immunology; Weir, Handbook of Experimental Immunology, Blackwell Scientific, Boston, MA (1986); Mishell and Shigii (eds.) Selected Methods in Cellular Immunology, Freeman Publishing, San Francisco, CA (1979); Green and Reed, Science 281:1309 (1998) and references cited therein. Cytokine levels may be determined according to methods known in the art, including for example, ELISA, ELISPOT, intracellular cytokine staining, flow cytometry, and any combination thereof (*e.g.,* intracellular cytokine staining and flow cytometry). Immune cell proliferation and clonal expansion resulting from an antigen-specific elicitation or stimulation of an immune response may be determined by isolating lymphocytes, such as circulating lymphocytes in samples of peripheral blood cells or cells from lymph nodes, stimulating the cells with antigen, and measuring cytokine production, cell proliferation and/or cell viability, such as by incorporation of tritiated thymidine or non-radioactive assays, such as MTT assays and the like.

In certain embodiments, a chimeric Tim4 receptor modified host cell has a phagocytic index of about 20 to about 1,500 for a target cell. A "phagocytic index" is a measure of phagocytic activity of the transduced host cell as determined by counting the number of target cells or particles ingested per chimeric Tim4 receptor modified host cell during a set period of incubation of a suspension of target cells or particles and chimeric Tim4 receptor modified host cells in media. Phagocytic index may be calculated by multiplying [total number of engulfed target cells/total number of counted chimeric Tim4 receptor modified cells (*e.g.,* phagocytic frequency)] x [average area of target cell or particle staining per chimeric Tim4 receptor ⁺ host cell x 100 (*e.g.,* hybrid capture)] or [total number of engulfed particles/total number of counted chimeric Tim4 receptor modified host cells] x [number of chimeric Tim4 receptor modified host cells containing engulfed particles/ total number of counted chimeric Tim4 receptor cells] x 100. In certain embodiments, a chimeric Tim4 receptor modified cell has a phagocytic index of about 30 to about 1,500; about 40 to about 1,500; about 50 to about 1,500; about 75 to about 1,500; about 100 to about 1,500; about 200 to about 1,500; about 300 to about 1,500; about 400 to about 1,500; about 500 to about 1,500; about 20 to about 1,400; about 30 to about 1,400; about 40 to about 1,400; about 50 to about 1,400; about 100 to about 1,400; about 200 to about 1,400; about 300 to about 1,400; about 400 to about 1,400; about 500 to about 1,400; about 20 to about 1,300; about 30 to about 1,300; about 40 to about 1,300; about 50 to about 1,300; about 100 to about 1,300; about 200 to about 1,300; about 300 to about 1,300; about 400 to about 1,300; about 500 to about 1,300; about 20 to about 1,200; about 30 to about 1,200; about 40 to about 1,200; about 50 to about 1,200; about 100 to about 1,200; about 200 to about 1,200; about 300 to about 1,200; about 400 to about 1,200; about 500 to about 1,200; about 20 to about 1,100; about 30 to about 1,100; about 40 to about 1,100; about 50 to about 1,100; about 100 to about 1,100; about 200 to about 1,100; about 300 to about 1,100; about 400 to about 1,100; or about 500 to about 1,100; about 20 to about 1,000; about 30 to about 1,000; about 40 to about 1,000; about 50 to about 1,000; about 100 to about 1,000; about 200 to about 1,000; about 300 to about 1,000; about 400 to about 1,000; or about 500 to about 1,000; about 20 to about 750; about 30 to about 750; about 40 to about 750; about 50 to about 750; about 100 to about 750; about 200 to about 750; about 300 to about 750; about 400 to about 750; or about 500 to about 750; about 20 to about 500; about 30 to about 500; about 40 to about 500; about 50 to about 500; about 100 to about 500; about 200 to about 500; or about 300 to about 500. In further embodiments, the incubation time is from about 2 hours to about 4 hours, about 2 hours, about 3 hours, or about 4 hours. In yet further embodiments, a chimeric Tim4 receptor modified cell exhibits phagocytic index that is statistically significantly higher than a cell transduced with truncated EGFR control. Phagocytic index may be calculated using methods known in the art and as further described in the Examples and PCT Application No. PCT/US2017/053553, including quantification by flow cytometry or fluorescence microscopy.

Host cells may be from an animal, such as a human, primate, cow, horse, sheep, dog, cat, mouse, rat, rabbit, guinea pig, pig, or a combination thereof. In a preferred embodiment, the animal is a human. Host cells may be obtained from a healthy subject or a subject having a disease associated with expression or overexpression of an antigen.

### Methods of Use

In one aspect, the present disclosure provides methods for conferring or enhancing phosphatidylserine-specific cytotoxic activity of a cell comprising introducing into a host cell a nucleic acid molecule encoding at least one chimeric Tim4 receptor or a chimeric Tim4 receptor vector according to any of the embodiments described herein; and expressing the at least one chimeric Tim4 receptor in the host cell, wherein the at least one chimeric Tim4 receptor enhances the phosphatidylserine-specific cytotoxic activity of the host cell as compared to a the host cell prior to modification to express a chimeric Tim4 receptor. In certain embodiments, the cytotoxic activity of the host cell is increased at least about 10%, 15%, 20%, 25%, 30%, 35%, 40%, 50%, 55%, 60%, 65%, 70%, 75%, 80% , 85%, 90%, 95%, 100%, 110%, 120%, 130%, 140%, 150%, 160%, 170%, 180%, 190%, 200% or more as compared to the host cell prior to modification with a nucleic acid molecule encoding a chimeric Tim4 receptor or a chimeric Tim4 receptor vector. In some embodiments, the host cell is an immune cell. In some embodiments, the host cell is a T cell or an NK cell. Methods of measuring cytotoxic activity of host cells, particularly immune cells such as T cells and NK cells, include a chromium (⁵¹Cr)-release assay, a β-gal or firefly luciferase release assay, flow cytometric methods of mediating target cell death and effector cell activity *(see, e.g.,* Expert Rev. Vaccines, 2010, 9:601-616).

In certain embodiments, methods for conferring or enhancing phosphatidylserine-specific cytotoxic activity of a cell further comprise conferring or enhancing phosphatidylserine-specific engulfment activity of the host cell expressing the at least one chimeric Tim4 receptor. In certain such embodiments, the host cell does not naturally exhibit an engulfment phenotype prior to modification with the chimeric Tim4 receptor. For example in certain such embodiments, the engulfment activity of the host cell is increased at least about 10%, 15%, 20%, 25%, 30%, 35%, 40%, 50%, 55%, 60%, 65%, 70%, 75%, 80% , 85%, 90%, 95%, 100%, 110%, 120%, 130%, 140%, 150%, 160%, 170%, 180%, 190%, 200% or more as compared to the host cell prior to modification to express the chimeric Tim4 receptor vector. In certain embodiments, the host cell does not naturally possess engulfment activity. In some embodiments, the host cell is an immune cell. In some embodiments, the host cell is a T cell or an NK cell. Methods of measuring engulfment activity of host cells include methods as described in International Application Publication No. WO2018/064076.

In another aspect, a chimeric Tim4 receptor, a polynucleotide encoding a chimeric Tim4 receptor, a chimeric Tim4 receptor vector, or a host cell that expresses a chimeric Tim4 receptor according to any of the embodiments provided herein may be used to enhance effector function of the host cell. In certain embodiments, enhanced effector function comprises increased cytotoxic activity, increased antigen specific cytokine production (*e.g.,* IFN-γ, IL-2, TNF-α, or any combination thereof), increased anti-apoptotic signaling, increased persistence, increased expansion, increased proliferation, or any combination thereof. In certain embodiments, the effector function of the host cell is enhanced at least about 10%, 15%, 20%, 25%, 30%, 35%, 40%, 50%, 55%, 60%, 65%, 70%, 75%, 80% , 85%, 90%, 95%, 100%, 110%, 120%, 130%, 140%, 150%, 160%, 170%, 180%, 190%, 200% or more as compared to a host cell that is not modified with a nucleic acid molecule encoding a chimeric Tim4 receptor or a chimeric Tim4 receptor vector. In some embodiments, the host cell is an immune cell. In certain embodiments, the host cell is a T cell or an NK cell.

In another aspect, host cells modified with chimeric Tim4 receptors of the present disclosure can be used to inhibit or reduce immune cell exhaustion. In some embodiments, the immune cell is a T cell or NK cell. In certain embodiments, reduced exhaustion in T cells comprises; (a) decreased expression of PD-1, TIGIT, LAG3, TIM3, or any combination thereof in T cells; (b) increased production of IFN-γ, IL-2, TNF-α, or any combination thereof in T cells; or both (a) and (b). In certain embodiments, reduced exhaustion in NK cells comprises; (a) decreased expression of PD-1, NKG2A, TIM3, or any combination thereof in NK cells; (b) increased production of IFN-γ, TNF-α, or both in NK cells; or both (a) and (b). In certain embodiments, the expression of an immune checkpoint molecule is decreased at least about 5%, 10%, 15%, 20%, 25%, 30%, 35%, 40%, 50%, 55%, 60%, 65%, 70%, 75%, 80% , 85%, 90%, 95%, or 100% in a host immune cell expressing the chimeric Tim4 receptor as compared to a host immune cell that is not modified with a nucleic acid molecule encoding a chimeric Tim4 receptor or a chimeric Tim4 receptor vector. In certain embodiments, the expression of the cytokine is increased at least about 5%, 10%, 15%, 20%, 25%, 30%, 35%, 40%, 50%, 55%, 60%, 65%, 70%, 75%, 80% , 85%, 90%, 95%, 100%, 110%, 120%, 130%, 140%, 150%, 160%, 170%, 180%, 190%, 200% or more in a host immune cell expressing the chimeric Tim4 receptor as compared to a host immune cell that is not modified with a nucleic acid molecule encoding a chimeric Tim4 receptor or a chimeric Tim4 receptor vector.

In another aspect, a chimeric Tim4 receptor, a polynucleotide encoding a chimeric Tim4 receptor, a chimeric Tim4 receptor vector, or a host cell that expresses a chimeric Tim4 receptor according to any of the embodiments provided herein may be used to reduce an immunosuppressive response to phosphatidylserine in a host cell. In certain embodiments, the immunosuppressive response comprises secretion of anti-inflammatory cytokines (*e.g.,* IL-10, TGF-β, or both), the decrease in secretion of inflammatory cytokines (*e.g.,* TNF-α, IL-1*β*, and IL-12), or both. In certain embodiments, the immunosuppressive response of the host cell to phosphatidylserine is decreased at least about 5%, 10%, 15%, 20%, 25%, 30%, 35%, 40%, 50%, 55%, 60%, 65%, 70%, 75%, 80%, 85%, 90%, 95%, 100% as compared to a host cell that is not modified with a nucleic acid molecule encoding a chimeric Tim4 receptor or a chimeric Tim4 receptor vector. In some embodiments, the host cell is an immune cell. In certain embodiments, the host cell is a T cell or an NK cell.

In yet another aspect, a chimeric Tim4 receptor, a polynucleotide encoding a chimeric Tim4 receptor, a chimeric Tim4 receptor vector, or a host cell that expresses a chimeric Tim4 receptor according to any of the embodiments provided herein may be used to eliminate target cells bearing surface exposed phosphatidylserine, *e.g.,* for the elimination of cancer cells bearing surface presented phosphatidylserine. In certain embodiments, the target cells are damaged, stressed, apoptotic, necrotic cells (*e.g.,* tumor cells) bearing surface exposed phosphatidylserine. In certain embodiments, host cells expressing chimeric Tim4 receptors clear damaged, stressed, apoptotic, or necrotic target cells bearing surface exposed phosphatidylserine via inducing apoptosis, or both inducing apoptosis and engulfment. Host cells expressing chimeric Tim4 receptors may be administered to a subject alone, or in combination with other therapeutic agents, including for example CAR-T cells, TCRs, antibodies, radiation therapy, chemotherapy, small molecules, oncolytic viruses, electropulse therapy, etc.

In another aspect, a chimeric Tim4 receptor, a polynucleotides encoding a chimeric Tim4 receptor, a chimeric Tim4 receptor vector, or a host cell that expresses a chimeric Tim4 receptor according to any of the embodiments provided herein may be used to enhance the effect of a therapeutic agent that induces cellular stress, damage, necrosis, or apoptosis. Certain therapies, such as chemotherapy, radiation therapy, UV light therapy, electropulse therapy, adoptive cellular immunotherapy (*e.g.,* CAR-T cells, TCRs) and oncolytic viral therapy, can induce cell damage or death to tumor cells, diseased cells, and cells in their surrounding environment. Cells expressing chimeric Tim4 receptors can be administered in combination with the cell damaging/cytotoxic therapy to bind to the phosphatidylserine moieties exposed on the outer leaflet of targeted cells and clear stressed, damaged, diseased, apoptotic, necrotic cells.

In another aspect, the present disclosure provides strategies for conferring or enhancing antigen capture, antigen processing, and/or antigen presentation activity of a cell comprising introducing into a host cell a nucleic acid molecule encoding at least one chimeric Tim4 receptor or a chimeric Tim4 receptor vector according to any of the embodiments described herein; and expressing the at least one chimeric Tim4 receptor in the host cell, wherein the at least one chimeric Tim4 receptor enhances the antigen capture, antigen processing, and/or antigen presentation activity of the host cell as compared to a the host cell prior to modification to express a chimeric Tim4 receptor.

A chimeric Tim4 receptor, a polynucleotides encoding a chimeric Tim receptor, a chimeric Tim receptor vector, a host cell that expresses a chimeric Tim receptor, or a pharmaceutical composition thereof, according to any of the embodiments provided herein may enhance CCR7+ expressing T cells in a subject having cancer, optionally in combination with a PARP inhibitor. In some embodiments, the cancer is breast cancer, ovarian cancer, colorectal cancer, fallopian cancer, peritoneal cancer, prostate cancer, lung cancer, or melanoma. In some embodiments, the chimeric Tim4 receptor is a single chain chimeric protein comprising: (a) an extracellular domain comprising a Tim4 binding domain; (b) an intracellular signaling domain comprising a CD28 signaling domain, a CD3ζ signaling domain, and a TLR2 TIR signaling domain; and (c) a CD28 transmembrane domain positioned between and connecting the extracellular domain and the intracellular signaling domain.

Enhancement of CCR7 expression on the chimeric Tim4 expressing T cells may be at least 10%, 20%, 25%, 30%, 35%, 40%, 45%, 50%, 55%, 60%, 65%, 70%, 75%, 80%, 85%, 90%, 95%, 100%, 125%, 150%, 175%, 200%, 225%, 250%, 275%, 300%, 325%, 350%, 375%, 400% or more CCR7 expresion as compared to expression on chimeric Tim4 expressing T cells that are not administered with a PARP inhibitor.

A chimeric Tim4 receptor, a polynucleotides encoding a chimeric Tim receptor, a chimeric Tim receptor vector, a host cell that expresses a chimeric Tim receptor, or a pharmaceutical composition thereof, according to any of the embodiments provided herein may enhance CD4/CD8 T cell ratio in a subject having cancer, optionally in combination with a PARP inhibitor. In some embodimetns, the cancer is breast cancer, ovarian cancer, colorectal cancer, fallopian cancer, peritoneal cancer, prostate cancer, lung cancer, or melanoma. In some embodiments, the chimeric Tim4 receptor is a single chain chimeric protein comprising: (a) an extracellular domain comprising a Tim4 binding domain; (b) an intracellular signaling domain comprising a CD28 signaling domain, a CD3ζ signaling domain, and a TLR2 TIR signaling domain; and (c) a CD28 transmembrane domain positioned between and connecting the extracellular domain and the intracellular signaling domain.

Enhancing CD4/CD8 T cell ratio in chimeric Tim4 expressing T cells may be at least 10%, 20%, 25%, 30%, 35%, 40%, 45%, 50%, 55%, 60%, 65%, 70%, 75%, 80%, 85%, 90%, 95%, 100%, or greater CD4/CD8 T cell ratio as compared to expression on chimeric Tim4 expressing T cells that are not administered with a PARP inhibitor.

A chimeric Tim receptor, a polynucleotides encoding a chimeric Tim receptor, a chimeric Tim receptor vector, or a host cell that expresses a chimeric Tim receptor, in combination with a PARP inhibitor according to any of the embodiments provided herein may be for use in a method of treating a subject suffering from a disease, disorder or undesired condition. Embodiments of these methods include administering to a subject (i) a therapeutically effective amount of a pharmaceutical composition including one or more chimeric Tim receptors, polynucleotides encoding one or more chimeric Tim receptors, vectors comprising polynucleotides encoding one or more chimeric Tim receptors, or a population of host cells genetically modified to express one or more chimeric Tim receptors according to the present description; and (ii) a therapeutically effective amount of a pharmaceutical composition comprising a PARP inhibitor.

The chimeric Tim4 receptor compositions as described herein may be administered before PARP inhibitor therapy (*e.g.,* 1 day to 30 days or more before the PARP inhibitor therapy), concurrently with PARP inhibitor therapy (on the same day), or after PARP inhibitor therapy (*e.g.,* 1 day - 30 days or more after the PARP inhibitor therapy). In certain embodiments, the chimeric Tim4 receptor modified cells are administered after administration of the PARP inhibitor. In further embodiments, the chimeric Tim4 receptor modified cells are administered 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30 days after administration of the PARP inhibitor. In still further embodiments, the chimeric Tim4 receptor modified cells are administered within 4 weeks, within 3 weeks, within 2 weeks, or within 1 week after administration of the PARP inhibitor therapy. Where the PARP inhibitor therapy involves multiple doses, the chimeric Tim receptor modified cells may be administered after the initial dose of the PARP inhibitor, after the final dose of the PARP inhibitor, or in between multiple doses of the PARP inhibitor.

In another aspect, a chimeric Tim4 receptor, a polynucleotides encoding a chimeric Tim4 receptor, a chimeric Tim4 receptor vector, or a host cell that expresses a chimeric Tim4 receptor according to any of the embodiments provided herein may be for use in a method of treating a subject suffering from a disease, disorder or undesired condition. Embodiments of these methods include administering to a subject a therapeutically effective amount of a pharmaceutical composition including one or more chimeric Tim4 receptors, polynucleotides encoding one or more chimeric Tim4 receptors, vectors comprising polynucleotides encoding one or more chimeric Tim4 receptors, or a population of host cells genetically modified to express one or more chimeric Tim4 receptors according to the present description.

Diseases that may be treated with cells expressing a chimeric Tim4 receptor as described in the present disclosure include cancer and infectious diseases (viral, bacterial, fungal, protozoan infections). Adoptive immune and gene therapies are promising treatments for various types of cancer (Morgan et al., Science 314:126, 2006; Schmitt et al., Hum. Gene Ther. 20:1240, 2009; June, J. Clin. Invest. 117:1466, 2007) and infectious disease (Kitchen et al., PLoS One 4:38208, 2009; Rossi et al., Nat. Biotechnol. 25:1444, 2007; Zhang et al., PLoS Pathog. 6:e1001018, 2010; Luo et al., J. Mol. Med. 89:903, 2011).

A wide variety of cancers, including solid tumors and leukemias are amenable to the compositions disclosed herein. Exemplary cancers that may be treated using the receptors, modified host cells, and composition described herein include adenocarcinoma of the breast, prostate, and colon; all forms of bronchogenic carcinoma of the lung; myeloid leukemia; melanoma; hepatoma; neuroblastoma; papilloma; apudoma; choristoma; branchioma; malignant carcinoid syndrome; carcinoid heart disease; and carcinoma (*e.g.,* Walker, basal cell, basosquamous, Brown-Pearce, ductal, Ehrlich tumor, Krebs 2, Merkel cell, mucinous, non-small cell lung, oat cell, papillary, scirrhous, bronchiolar, bronchogenic, squamous cell, and transitional cell). Additional types of cancers that may be treated using the receptors, modified host cells, and composition described herein include histiocytic disorders; malignant histiocytosis; leukemia; Hodgkin's disease; immunoproliferative small; non-Hodgkin's lymphoma; plasmacytoma; multiple myeloma; chronic myeloid leukemia (CML); acute myeloid leukemia (AML); plasmacytoma; reticuloendotheliosis; melanoma; chondroblastoma; chondroma; chondrosarcoma; fibroma; fibrosarcoma; giant cell tumors; histiocytoma; lipoma; liposarcoma; mesothelioma; myxoma; myxosarcoma; osteoma; osteosarcoma; chordoma; craniopharyngioma; dysgerminoma; hamartoma; mesenchymoma; mesonephroma; myosarcoma; ameloblastoma; cementoma; odontoma; teratoma; thymoma; trophoblastic tumor. Further, the following types of cancers are also contemplated as amenable to treatment using the receptors, modified host cells, and composition described herein: adenoma; cholangioma; cholesteatoma; cyclindroma; cystadenocarcinoma; cystadenoma; granulosa cell tumor; gynandroblastoma; hepatoma; hidradenoma; islet cell tumor; Leydig cell tumor; papilloma; sertoli cell tumor; theca cell tumor; leimyoma; leiomyosarcoma; myoblastoma; myomma; myosarcoma; rhabdomyoma; rhabdomyosarcoma; ependymoma; ganglioneuroma; glioma; medulloblastoma; meningioma; neurilemmoma; neuroblastoma; neuroepithelioma; neurofibroma; neuroma; paraganglioma; paraganglioma nonchromaffin. The types of cancers that may be treated also include angiokeratoma; angiolymphoid hyperplasia with eosinophilia; angioma sclerosing; angiomatosis; glomangioma; hemangioendothelioma; hemangioma; hemangiopericytoma; hemangiosarcoma; lymphangioma; lymphangiomyoma; lymphangiosarcoma; pinealoma; carcinosarcoma; chondrosarcoma; cystosarcoma phyllodes; fibrosarcoma; hemangiosarcoma; leiomyosarcoma; leukosarcoma; liposarcoma; lymphangiosarcoma; myosarcoma; myxosarcoma; ovarian carcinoma; rhabdomyosarcoma; sarcoma; neoplasms; nerofibromatosis; cervical dysplasia, and peritoneal cancer.

Examples of hyperproliferative disorders amenable to therapy using the receptors, modified host cells, and composition described herein include B-cell cancers (B-cell malignancies), including B-cell lymphomas (such as various forms of Hodgkin's disease, non-Hodgkin's lymphoma (NHL) or central nervous system lymphomas), leukemias (such as acute lymphoblastic leukemia (ALL), chronic lymphocytic leukemia (CLL), Hairy cell leukemia, B cell blast transformation of chronic myeloid leukemia, acute myeloid leukemia (AML), chronic myeloid leukemia, and myelomas (such as multiple myeloma). Additional B cell cancers that may be treated using the receptors, modified host cells, and composition described herein include small lymphocytic lymphoma, small lymphocytic leukemia, Waldenström macroglobulinemia, B-cell prolymphocytic leukemia, lymphoplasmacytic lymphoma, splenic marginal zone lymphoma, plasma cell myeloma, solitary plasmacytoma of bone, extraosseous plasmacytoma, extra-nodal marginal zone B-cell lymphoma of mucosa-associated (MALT) lymphoid tissue, nodal marginal zone B-cell lymphoma, follicular lymphoma, mantle cell lymphoma, diffuse large B-cell lymphoma, mediastinal (thymic) large B-cell lymphoma, intravascular large B-cell lymphoma, primary effusion lymphoma, Burkitt's lymphoma/leukemia, B-cell proliferations of uncertain malignant potential, lymphomatoid granulomatosis, and post-transplant lymphoproliferative disorder.

In some embodiments, combination therapy comprising a chimeric Tim4 receptor, a polynucleotide encoding a chimeric Tim4 receptor, a chimeric Tim4 receptor vector, or a host cell that expresses a chimeric Tim4 receptor according to any of the embodiments provided herein and a PARP inhibitor is useful to treat solid tumors. In some embodiments, the solid tumor cancer is breast cancer, ovarian cancer, colorectal cancer, fallopian cancer, peritoneal cancer, or prostate cancer. In some embodiments, the breast cancer is triple negative breast cancer. In some embodiments, the ovarian cancer is advanced ovarian cancer. In some embodiments, the prostate cancer is advanced prostate cancer. In some embodiments, the solid tumor cancer is melanoma. In some embodiments, the solid tumor cancer is lung cancer. In some embodiments, the lung cancer is non-small cell lung cancer. In some embodiments, the solid tumor cancer is a Breast cancer (BRCA) mutated cancer. In someembodiments, the cancer is a BRCA1 mutated cancer, a BRCA2 mutated cancer, or both.

Infectious diseases include those associated with infectious agents and include any of a variety of bacteria *(e.g.,* pathogenic *E. coli, S. typhimurium, P. aeruginosa, B. anthracis, C. botulinum, C. difficile, C. perfringens, H. pylori, V. cholerae, Listeria spp., Rickettsia spp., Chlamydia spp.,* and the like), mycobacteria, and parasites (including any known parasitic member of the Protozoa). Infectious viruses include eukaryotic viruses, such as adenovirus, bunyavirus, herpesvirus, papovavirus, papillomavirus (*e.g.,* HPV), paramyxovirus, picornavirus, rhabdovirus (*e.g.,* Rabies), orthomyxovirus (*e.g.,* influenza), poxvirus *(e.g.,* Vaccinia), reovirus, retrovirus, lentivirus (*e.g.,* HIV), flavivirus (*e.g.,* HCV, HBV) or the like. In certain embodiments, a composition comprising a chimeric Tim4 receptor according to the present disclosure is used for treating infection with a microbe capable of establishing a persistent infection in a subject.

A chimeric Tim4 receptor of the present disclosure may be administered to a subject in cell-bound form (*e.g.,* gene therapy of target cell population). Thus, for example, a chimeric Tim4 receptor of the present disclosure may be administered to a subject expressed on the surface of T cells, Natural Killer Cells, Natural Killer T cells, B cells, lymphoid precursor cells, antigen presenting cells, dendritic cells, Langerhans cells, myeloid precursor cells, mature myeloid cells, including subsets thereof, or any combination thereof. In certain embodiments, the subject to be treated is administered an effective amount of chimeric Tim4 receptor modified cells (*i.e.,* recombinant cells that express one or more chimeric Tim4 receptors). The chimeric Tim4 receptor modified cells may be xenogeneic, syngeneic, allogeneic, or autologous to the subject.

Pharmaceutical compositions including chimeric Tim4 receptor modified cells may be administered in a manner appropriate to the disease or condition to be treated (or prevented) as determined by persons skilled in the medical art. An appropriate dose, suitable duration, and frequency of administration of the compositions will be determined by such factors as the condition of the patient, size, weight, body surface area, age, sex, type and severity of the disease, particular therapy to be administered, particular form of the active ingredient, time and the method of administration, and other drugs being administered concurrently. The present disclosure provides pharmaceutical compositions comprising chimeric Tim4 receptor modified cells and a pharmaceutically acceptable carrier, diluent, or excipient. Suitable excipients include water, saline, dextrose, glycerol, or the like and combinations thereof. Other suitable infusion medium can be any isotonic medium formulation, including saline, Normosol R (Abbott), Plasma-Lyte A (Baxter), 5% dextrose in water, or Ringer's lactate.

A treatment effective amount of cells in a pharmaceutical composition is at least one cell (for example, one chimeric Tim4 receptor modified T cell) or is more typically greater than 10² cells, for example, up to 10⁶, up to 10⁷, up to 10⁸ cells, up to 10⁹ cells, up to 10¹⁰ cells, or up to 10¹¹ cells or more. In certain embodiments, the cells are administered in a range from about 10⁶ to about 10¹⁰ cells/m², preferably in a range of about 10⁷ to about 10⁹ cells/m². The number of cells will depend upon the ultimate use for which the composition is intended as well the type of cells included therein. For example, a composition comprising cells modified to contain a chimeric Tim4 receptor will comprise a cell population containing from about 5% to about 95% or more of such cells. In certain embodiments, a composition comprising chimeric Tim4 receptor modified cells comprises a cell population comprising at least 5%, 10%, 15%, 20%, 25%, 30%, 35%, 40%, 45%, 50%, 55%, 60%, 65%, 70%, 75%, 80%, 85%, 90%, 95% or more of such cells. For uses provided herein, the cells are generally in a volume of a liter or less, 500 mls or less, 250 mls or less, or 100 mls or less. Hence the density of the desired cells is typically greater than 10⁴ cells/ml and generally is greater than 10⁷ cells/ml, generally 10⁸ cells/ml or greater. The cells may be administered as a single infusion or in multiple infusions over a range of time. Repeated infusions of chimeric Tim4 receptor modified cells may be separated by days, weeks, months, or even years if relapses of disease or disease activity are present. A clinically relevant number of immune cells can be apportioned into multiple infusions that cumulatively equal or exceed 10⁶, 10⁷, 10⁸, 10⁹, 10¹⁰, or 10¹¹ cells. A preferred dose for administration of a host cell comprising a recombinant expression vector as described herein is about 10⁷ cells/m², about 5 x 10⁷ cells/m², about 10⁸ cells/m², about 5 x 10⁸ cells/m², about 10⁹ cells/m², about 5 x 10⁹ cells/m², about 10¹⁰ cells/m², about 5 x 10¹⁰ cells/m², or about 10¹¹ cells/m².

Chimeric Tim4 receptor compositions as described herein may be administered intravenously, intraperitoneally, intranasally, intratumorly, into the bone marrow, into the lymph node, and/or into cerebrospinal fluid.

Chimeric Tim4 receptor compositions may be administered to a subject in combination with one or more additional therapeutic agents. Examples of therapeutic agents that may be administered in combination with a chimeric Tim compositions according to the present description include radiation therapy, adoptive cellular immunotherapy agent (*e.g.,* recombinant TCR, enhanced affinity TCR, CAR, TCR-CAR, scTCR fusion protein, dendritic cell vaccine), antibody therapy, immune checkpoint molecule inhibitor therapy, UV light therapy, electric pulse therapy, high intensity focused ultrasound therapy, oncolytic virus therapy, or a pharmaceutical therapy, such as a chemotherapeutic agent, a therapeutic peptide, a hormone, an aptamer, antibiotic, anti-viral agent, anti-fungal agent, anti-inflammatory agent, a small molecule therapy, or any combination thereof. In certain embodiments, the chimeric Tim4 receptor modified host cells may clear stressed, damaged, apoptotic, necrotic, infected, dead cells displaying surface phosphatidylserine induced by the one or more additional therapeutic agents.

In certain embodiments, the chimeric Tim4 receptor and adoptive cellular immunotherapy agent (*e.g.,* a CAR, TCR-CAR, TCR, etc. described above) are administered to the subject in the same host cell or different host cells. In certain embodiments, the chimeric Tim4 receptor and adoptive cellular immunotherapy agent are expressed in the same host cell from the same vector or from separate vectors. In certain embodiments, the chimeric Tim4 receptor and adoptive cellular immunotherapy agent are expressed in the same host cell from a multicistronic vector. In certain embodiments, the chimeric Tim4 receptor is expressed in the same host cell type as the adoptive cellular immunotherapy agent (*e.g.,* the chimeric Tim4 receptor is expressed CD4 T cells and the CAR/or TCR is expressed in CD4 T cells, or the chimeric Tim4 receptor is expressed CD8 T cells and the CAR/or TCR is expressed in CD8 T cells). In other embodiments, the chimeric Tim4 receptor is expressed in a different host cell type as the adoptive immunotherapy agent (*e.g.,* the chimeric Tim4 receptor is expressed CD4 T cells and the CAR/or TCR is expressed in CD8 T cells). Cellular immunotherapy compositions comprising a combination of immune cells or cellular subsets engineered with chimeric Tim4 receptors and a cellular immunotherapy agent (e.g., CAR, TCR, etc.), methods of making, and methods of use are described in PCT International Publication No. WO2019/191340.

Exemplary antigens that a recombinant TCR, enhanced affinity TCR, CAR, TCR-CAR, or scTCR fusion protein may target include WT-1, mesothelin, MART-1, NY-ESO-1, MAGE-A3, HPV E7, survivin, α Fetoprotein, and a tumor-specific neoantigen.

CARs of the present disclosure may target a variety of antigens, including a viral antigen, bacterial antigen, fungal antigen, parasitic antigen, tumor antigen, autoimmune disease antigen. Exemplary antigens that a CAR may target include CD138, CD38, CD33, CD123, CD72, CD79a, CD79b, mesothelin, PSMA, BCMA, ROR1, MUC-16, L1CAM, CD22, CD19, CD20, CD23, CD24, CD37, CD30, CA125, CD56, c-Met, EGFR, GD-3, HPV E6, HPV E7, MUC-1, HER2, folate receptor α, CD97, CD171, CD179a, CD44v6, WT1, VEGF-α, VEGFR1, IL-13Rα1, IL-13Rα2, IL-11Rα, PSA, FcRH5, NKG2D ligand, NY-ESO-1, TAG-72, CEA, ephrin A2, ephrin B2, Lewis A antigen, Lewis Y antigen, MAGE, MAGE-A1, RAGE-1, folate receptor β, EGFRviii, VEGFR-2, LGR5, SSX2, AKAP-4, FLT3, fucosyl GM1, GM3, o-acetyl-GD2, and GD2.

In some embodiments, the chimeric Tim4 receptor of the present disclosure is administered to a subject in combination with a CD72-specific CAR. In some embodiments, the binding domain of a CD72-specific CAR comprises:
(i) a heavy chain variable (VH) region, wherein the VH region comprises a heavy chain complementarity determining region 1 (HCDR-1) comprising the amino acid sequence set forth in SEQ ID NO:101; a heavy chain complementarity determining region 2 (HCDR-2) comprising the amino acid sequence set forth in SEQ ID NO: 102; and a heavy chain complementarity determining region 3 (HCDR-3) comprising the amino acid sequence set forth in SEQ ID NO: 103; and a light chain variable (VL) region, wherein the VL region comprises a light chain complementarity determining region 1 (LCDR-1) comprising the amino acid sequence set forth in SEQ ID NO:104; a light chain complementarity determining region 2 (LCDR-2) comprising the amino acid sequence set forth in SEQ ID NO:105; and a light chain complementarity determining region 3 (LCDR-3) comprising the amino acid sequence set forth in SEQ ID NO:106;
(ii) a heavy chain variable (VH) region, wherein the VH region comprises a heavy chain complementarity determining region 1 (HCDR-1) comprising the amino acid sequence set forth in SEQ ID NO: 107; a heavy chain complementarity determining region 2 (HCDR-2) comprising the amino acid sequence set forth in SEQ ID NO: 108; and a heavy chain complementarity determining region 3 (HCDR-3) comprising the amino acid sequence set forth in SEQ ID NO: 109; and a light chain variable (VL) region, wherein the VL region comprises a light chain complementarity determining region 1 (LCDR-1) comprising the amino acid sequence set forth in SEQ ID NO:110; a light chain complementarity determining region 2 (LCDR-2) comprising the amino acid sequence set forth in SEQ ID NO:111; and a light chain complementarity determining region 3 (LCDR-3) comprising the amino acid sequence set forth in SEQ ID NO: 112;
(iii) a heavy chain variable (VH) region, wherein the VH region comprises a heavy chain complementarity determining region 1 (HCDR-1) comprising the amino acid sequence set forth in SEQ ID NO:302; a heavy chain complementarity determining region 2 (HCDR-2) comprising the amino acid sequence set forth in SEQ ID NO:303; and a heavy chain complementarity determining region 3 (HCDR-3) comprising the amino acid sequence set forth in SEQ ID NO:304; and a light chain variable (VL) region, wherein the VL region comprises a light chain complementarity determining region 1 (LCDR-1) comprising the amino acid sequence set forth in SEQ ID NO:305; a light chain complementarity determining region 2 (LCDR-2) comprising the amino acid sequence set forth in SEQ ID NO:306; and a light chain complementarity determining region 3 (LCDR-3) comprising the amino acid sequence set forth in SEQ ID NO:307;
(iv) a heavy chain variable (VH) region, wherein the VH region comprises a heavy chain complementarity determining region 1 (HCDR-1) comprising the amino acid sequence set forth in SEQ ID NO:308; a heavy chain complementarity determining region 2 (HCDR-2) comprising the amino acid sequence set forth in SEQ ID NO:309; and a heavy chain complementarity determining region 3 (HCDR-3) comprising the amino acid sequence set forth in SEQ ID NO:310; and a light chain variable (VL) region, wherein the VL region comprises a light chain complementarity determining region 1 (LCDR-1) comprising the amino acid sequence set forth in SEQ ID NO:311; a light chain complementarity determining region 2 (LCDR-2) comprising the amino acid sequence set forth in SEQ ID NO:312; and a light chain complementarity determining region 3 (LCDR-3) comprising the amino acid sequence set forth in SEQ ID NO:313;
(v) a heavy chain variable (VH) region, wherein the VH region comprises a heavy chain complementarity determining region 1 (HCDR-1) comprising the amino acid sequence set forth in SEQ ID NO:314; a heavy chain complementarity determining region 2 (HCDR-2) comprising the amino acid sequence set forth in SEQ ID NO:315; and a heavy chain complementarity determining region 3 (HCDR-3) comprising the amino acid sequence set forth in SEQ ID NO:316; and a light chain variable (VL) region, wherein the VL region comprises a light chain complementarity determining region 1 (LCDR-1) comprising the amino acid sequence set forth in SEQ ID NO:317; a light chain complementarity determining region 2 (LCDR-2) comprising the amino acid sequence set forth in SEQ ID NO:318; and a light chain complementarity determining region 3 (LCDR-3) comprising the amino acid sequence set forth in SEQ ID NO:319;
(vi) a heavy chain variable (VH) region, wherein the VH region comprises a heavy chain complementarity determining region 1 (HCDR-1) comprising the amino acid sequence set forth in SEQ ID NO:320; a heavy chain complementarity determining region 2 (HCDR-2) comprising the amino acid sequence set forth in SEQ ID NO:321; and a heavy chain complementarity determining region 3 (HCDR-3) comprising the amino acid sequence set forth in SEQ ID NO:322; and a light chain variable (VL) region, wherein the VL region comprises a light chain complementarity determining region 1 (LCDR-1) comprising the amino acid sequence set forth in SEQ ID NO:323; a light chain complementarity determining region 2 (LCDR-2) comprising the amino acid sequence set forth in SEQ ID NO:324; and a light chain complementarity determining region 3 (LCDR-3) comprising the amino acid sequence set forth in SEQ ID NO:325;
(vii) a heavy chain variable (VH) region, wherein the VH region comprises a heavy chain complementarity determining region 1 (HCDR-1) comprising the amino acid sequence set forth in SEQ ID NO:326; a heavy chain complementarity determining region 2 (HCDR-2) comprising the amino acid sequence set forth in SEQ ID NO:327; and a heavy chain complementarity determining region 3 (HCDR-3) comprising the amino acid sequence set forth in SEQ ID NO:328; and a light chain variable (VL) region, wherein the VL region comprises a light chain complementarity determining region 1 (LCDR-1) comprising the amino acid sequence set forth in SEQ ID NO:329; a light chain complementarity determining region 2 (LCDR-2) comprising the amino acid sequence set forth in SEQ ID NO:330; and a light chain complementarity determining region 3 (LCDR-3) comprising the amino acid sequence set forth in SEQ ID NO:331;
(viii) a heavy chain variable (VH) region, wherein the VH region comprises a heavy chain complementarity determining region 1 (HCDR-1) comprising the amino acid sequence set forth in SEQ ID NO:332; a heavy chain complementarity determining region 2 (HCDR-2) comprising the amino acid sequence set forth in SEQ ID NO:333; and a heavy chain complementarity determining region 3 (HCDR-3) comprising the amino acid sequence set forth in SEQ ID NO:334; and a light chain variable (VL) region, wherein the VL region comprises a light chain complementarity determining region 1 (LCDR-1) comprising the amino acid sequence set forth in SEQ ID NO:335; a light chain complementarity determining region 2 (LCDR-2) comprising the amino acid sequence set forth in SEQ ID NO:336; and a light chain complementarity determining region 3 (LCDR-3) comprising the amino acid sequence set forth in SEQ ID NO:337;
(ix) a heavy chain variable (VH) region, wherein the VH region comprises a heavy chain complementarity determining region 1 (HCDR-1) comprising the amino acid sequence set forth in SEQ ID NO:338; a heavy chain complementarity determining region 2 (HCDR-2) comprising the amino acid sequence set forth in SEQ ID NO:339; and a heavy chain complementarity determining region 3 (HCDR-3) comprising the amino acid sequence set forth in SEQ ID NO:340; and a light chain variable (VL) region, wherein the VL region comprises a light chain complementarity determining region 1 (LCDR-1) comprising the amino acid sequence set forth in SEQ ID NO:341; a light chain complementarity determining region 2 (LCDR-2) comprising the amino acid sequence set forth in SEQ ID NO:342; and a light chain complementarity determining region 3 (LCDR-3) comprising the amino acid sequence set forth in SEQ ID NO:343; or
(x) a heavy chain variable (VH) region, wherein the VH region comprises a heavy chain complementarity determining region 1 (HCDR-1) comprising the amino acid sequence set forth in SEQ ID NO:344; a heavy chain complementarity determining region 2 (HCDR-2) comprising the amino acid sequence set forth in SEQ ID NO:345; and a heavy chain complementarity determining region 3 (HCDR-3) comprising the amino acid sequence set forth in SEQ ID NO:346; and a light chain variable (VL) region, wherein the VL region comprises a light chain complementarity determining region 1 (LCDR-1) comprising the amino acid sequence set forth in SEQ ID NO:347; a light chain complementarity determining region 2 (LCDR-2) comprising the amino acid sequence set forth in SEQ ID NO:348; and a light chain complementarity determining region 3 (LCDR-3) comprising the amino acid sequence set forth in SEQ ID NO:349.

In some embodiments, the binding domain of the CAR comprises:
(i) a VH region comprising the amino acid sequence set forth in SEQ ID NO:113 or a sequence having at least 90%, 91%, 92%, 93%, 94%, 95%, 96%, 97%, 98%, or 99% sequence identity to SEQ ID NO:113, and a VL region comprising the amino acid sequence set forth in SEQ ID NO:114 or a sequence having at least 90%, 91%, 92%, 93%, 94%, 95%, 96%, 97%, 98%, or 99% sequence identity to SEQ ID NO: 114;
(ii) a VH region comprising the amino acid sequence set forth in SEQ ID NO:115 or a sequence having at least 90%, 91%, 92%, 93%, 94%, 95%, 96%, 97%, 98%, or 99% sequence identity to SEQ ID NO:115, and a VL region comprising the amino acid sequence set forth in SEQ ID NO:116 or a sequence having at least 90%, 91%, 92%, 93%, 94%, 95%, 96%, 97%, 98%, or 99% sequence identity to SEQ ID NO:116;
(iii) a VH region comprising the amino acid sequence set forth in SEQ ID NO:117 or a sequence having at least 90%, 91%, 92%, 93%, 94%, 95%, 96%, 97%, 98%, or 99% sequence identity to SEQ ID NO:117, and a VL region comprising the amino acid sequence set forth in SEQ ID NO:118 or a sequence having at least 90%, 91%, 92%, 93%, 94%, 95%, 96%, 97%, 98%, or 99% sequence identity to SEQ ID NO:118;
(iv) a VH region comprising the amino acid sequence set forth in SEQ ID NO:119 or a sequence having at least 90%, 91%, 92%, 93%, 94%, 95%, 96%, 97%, 98%, or 99% sequence identity to SEQ ID NO:119, and a VL region comprising the amino acid sequence set forth in SEQ ID NO:120 or a sequence having at least 90%, 91%, 92%, 93%, 94%, 95%, 96%, 97%, 98%, or 99% sequence identity to SEQ ID NO: 120;
(v) a VH region comprising the amino acid sequence set forth in SEQ ID NO:121 or a sequence having at least 90%, 91%, 92%, 93%, 94%, 95%, 96%, 97%, 98%, or 99% sequence identity to SEQ ID NO:121, and a VL region comprising the amino acid sequence set forth in SEQ ID NO:122 or a sequence having at least 90%, 91%, 92%, 93%, 94%, 95%, 96%, 97%, 98%, or 99% sequence identity to SEQ ID NO: 122;
(vi) a VH region comprising the amino acid sequence set forth in SEQ ID NO:123 or a sequence having at least 90%, 91%, 92%, 93%, 94%, 95%, 96%, 97%, 98%, or 99% sequence identity to SEQ ID NO:123, and a VL region comprising the amino acid sequence set forth in SEQ ID NO:124 or a sequence having at least 90%, 91%, 92%, 93%, 94%, 95%, 96%, 97%, 98%, or 99% sequence identity to SEQ ID NO: 124;
(vii) a VH region comprising the amino acid sequence set forth in SEQ ID NO: 125 or a sequence having at least 90%, 91%, 92%, 93%, 94%, 95%, 96%, 97%, 98%, or 99% sequence identity to SEQ ID NO: 125, and a VL region comprising the amino acid sequence set forth in SEQ ID NO: 126 or a sequence having at least 90%, 91%, 92%, 93%, 94%, 95%, 96%, 97%, 98%, or 99% sequence identity to SEQ ID NO: 126;
(viii) a VH region comprising the amino acid sequence set forth in SEQ ID NO: 127 or a sequence having at least 90%, 91%, 92%, 93%, 94%, 95%, 96%, 97%, 98%, or 99% sequence identity to SEQ ID NO: 127, and a VL region comprising the amino acid sequence set forth in SEQ ID NO: 128 or a sequence having at least 90%, 91%, 92%, 93%, 94%, 95%, 96%, 97%, 98%, or 99% sequence identity to SEQ ID NO: 128; or
(ix) a VH region comprising the amino acid sequence set forth in SEQ ID NO: 129 or a sequence having at least 90%, 91%, 92%, 93%, 94%, 95%, 96%, 97%, 98%, or 99% sequence identity to SEQ ID NO: 129, and a VL region comprising the amino acid sequence set forth in SEQ ID NO: 130 or a sequence having at least 90%, 91%, 92%, 93%, 94%, 95%, 96%, 97%, 98%, or 99% sequence identity to SEQ ID NO: 130.

In some embodiments, the VH region and VL region are joined by a flexible linker. In some embodiments, the binding domain comprises a scFv comprising a VH region, a VL region, and a flexible linker, which can be in a VH-linker-VL orientation or a VL-linker-VH orientiation. In some embodiments, the flexible linker has from about 5 to about 50 amino acids in length and comprises a sequence rich in glycine, serine, and/or threonine. Exemplary linkers include linkers having (GGGGS)ₓ or (GGGS)ₓ, where x=2-5. In some embodiments, the flexible linker comprises the amino acid sequence set forth in any one of SEQ ID NOS: 165-170.

In some embodiments, the binding domain comprises the amino acid sequence set forth in any one of SEQ ID NOS: 131-164 or a sequence having at least 90%, 91%, 92%, 93%, 94%, 95%, 96%, 97%, 98%, or 99% sequence identity to the amino acid sequence set forth in any one of SEQ ID NOS:131-164.

In certain embodiments, the extracellular domain of CARs provided in the present disclosure optionally comprises an extracellular, non-signaling spacer or linker domain between the binding domain and the transmembrane domain. Where included, such a spacer or linker domain may position the binding domain away from the host cell surface to further enable proper cell to cell contact, binding, and activation. An extracellular spacer domain is generally located between the extracellular binding domain and the transmembrane domain of the CAR. The length of the extracellular spacer may be varied to optimize target molecule binding based on the selected target molecule, selected binding epitope, binding domain size and affinity (*see, e.g.,* Guest et al., J. Immunother. 28:203-11, 2005; PCT Publication No. WO 2014/031687). In certain embodiments, an extracellular spacer domain is an immunoglobulin hinge region (*e.g.,* IgG1, IgG2, IgG3, IgG4, IgA, IgD). An immunoglobulin hinge region may be a wild type immunoglobulin hinge region or an altered wild type immunoglobulin hinge region. An altered IgG₄ hinge region is described in PCT Publication No. WO 2014/031687. In some embodiments, an extracellular spacer domain comprises a modified IgG₄ hinge region having an amino acid sequence of ESKYGPPCPPCP (SEQ ID NO:9).

Other examples of hinge regions that may be used in the CARs described herein include the hinge region from the extracellular regions of type 1 membrane proteins, such as CD8a, CD4, CD28 and CD7, which may be wild-type or variants thereof. An exemplary CD8a hinge region comprises the amino acid sequence set forth in SEQ ID NO:22. An exemplary CD28 hinge region comprises the amino acid sequence set forth in SEQ ID NO:10. In some embodiments, an extracellular spacer domain comprises all or a portion of an immunoglobulin Fc domain selected from: a CH1 domain, a CH2 domain, a CH3 domain, or combinations thereof (*see, e.g.,* PCT Publication WO2014/031687). In yet further embodiments, an extracellular spacer domain may comprise a stalk region of a type II C-lectin (the extracellular domain located between the C-type lectin domain and the transmembrane domain). Type II C-lectins include CD23, CD69, CD72, CD94, NKG2A, and NKG2D.

CARs of the present disclosure comprise a transmembrane domain that connects and is positioned between the extracellular domain and the intracellular signaling domain. The transmembrane domain ranges in length from about 15 amino acids to about 30 amino acids. The transmembrane domain is a hydrophobic alpha helix that transverses the host cell membrane and anchors the CAR in the host cell membrane. The transmembrane domain may be directly fused to the binding domain or to the extracellular spacer domain if present. In certain embodiments, the transmembrane domain is derived from an integral membrane protein (*e.g.,* receptor, cluster of differentiation (CD) molecule, enzyme, transporter, cell adhesion molecule, or the like). The transmembrane domain can be selected from the same molecule as the extracellular domain or the intracellular signaling domain (*e.g.,* a CAR that comprises a CD28 costimulatory signaling domain and a CD28 transmembrane domain). In some embodiments, the transmembrane domain and the extracellular domain are each selected from different molecules. In some embodiments, the transmembrane domain and the intracellular signaling domain are each selected from different molecules. In yet other embodiments, the transmembrane domain, the extracellular domain, and the intracellular signaling domain are each selected from different molecules.

Exemplary transmembrane domains for use in CARs of the present disclosure include a CD28, CD2, CD4, CD8a, CD5, CD3ε, CD3δ, CD3ζ, CD9, CD16, CD22, CD25, CD27, CD33, CD37, CD40, CD45, CD64, CD79A, CD79B, CD80, CD86, CD95 (Fas), CD134 (OX40), CD137 (4-1BB), CD150 (SLAMF1), CD152 (CTLA4), CD154 (CD40L), CD200R, CD223 (LAG3), CD270 (HVEM), CD272 (BTLA), CD273 (PD-L2), CD274 (PD-L1), CD278 (ICOS), CD279 (PD-1), CD300, CD357 (GITR), A2aR, DAP10, FcRa, FcRβ, FcRγ, Fyn, GAL9, KIR, Lck, LAT, LRP, NKG2D, NOTCH1, NOTCH2, NOTCH3, NOTCH4, PTCH2, ROR2, Ryk, Slp76, SIRPα, pTα, TCRα, TCRβ, TIM3, TRIM, LPA5, and Zap70 transmembrane domain. An exemplary CD8a transmembrane domain comprises an amino acid sequence of SEQ ID NO:174. An exemplary CD28 transmembrane domain comprises an amino acid sequence of SEQ ID NO:11.

The intracellular signaling domain of a CAR is an intracellular effector domain and is capable of transmitting functional signals to a cell in response to binding of the extracellular domain of the CAR to a target molecule (e.g., CD72) and activates at least one of the normal effector functions or responses of the immune cell, e.g., T cell engineered to express the CAR. In some embodiments, the CAR induces a function of a T cell such as cytolytic activity or T helper activity, such as secretion of cytokines or other factors. The intracellular signaling domain may be any portion of an intracellular signaling molecule that retains sufficient signaling activity. In some embodiments, the intracellular signaling domain is obtained from an antigen receptor component (e.g., TCR) or costimulatory molecule. In some embodiments, a full length intracellular signaling domain of an antigen receptor or costimulatory molecule is used. In some embodiments, a truncated portion of an intracellular signaling domain of an antigen receptor or costimulatory molecule is used, provided that the truncated portion retains sufficient signal transduction activity. In further embodiments, an intracellular signaling domain is a variant of a full length or truncated portion of an intracellular signaling domain of an antigen receptor co stimulatory molecule, provided that the variant retains sufficient signal transduction activity (*i.e.,* is a functional variant).

In certain embodiments, the intracellular signaling domain of a CAR comprises an immunoreceptor tyrosine-based activation motif (ITAM) containing signaling domain. An ITAM containing signaling domain generally contains at least one (one, two, three, four, or more) ITAMs, which refer to a conserved motif of YXXL/I-X₆₋₈-YXXL/I. An ITAM containing signaling domain may initiate T cell activation signaling following antigen binding or ligand engagement. ITAM-signaling domains include, for example, intracellular signaling domains of CD3γ, CD3δ, CD3ε, CD3ζ, CD5, CD22, CD79a, CD278 (ICOS), DAP10, DAP12, FcRγ, and CD66d. Exemplary CD3ζ signaling domains that may be used in CARs of the present disclosure comprise an amino acid sequence of SEQ ID NO: 177 or 178.

CAR intracellular signaling domains optionally comprise a costimulatory signaling domain, which, when activated in conjunction with a primary or classic (*e.g.,* ITAM-driven) activation signal, promotes or enhances T cell response, such as T cell activation, cytokine production, proliferation, differentiation, survival, effector function, or combinations thereof. Costimulatory signaling domains for use in CARs include, for example, CD27, CD28, CD40L, GITR, NKG2C, CARD1, CD2, CD7, CD27, CD30, CD40, CD54 (ICAM), CD83, CD134 (OX-40), CD137 (4-1BB), CD150 (SLAMF1), CD152 (CTLA4), CD223 (LAG3), CD226, CD270 (HVEM), CD273 (PD-L2), CD274 (PD-L1), CD278 (ICOS), DAP10, LAT, LFA-1, LIGHT, NKG2C, SLP76, TRIM, ZAP70, or any combination thereof. In a particular embodiment, the costimulatory signaling domain comprises a OX40, CD2, CD27, CD28, ICAM-1, LFA-1 (CD11a/CD18), ICOS (CD278), or 4-1BB (CD137) signaling domain. Exemplary CD28 costimulatory signaling domains that may be used in CARs of the present disclosure comprise an amino acid sequence set forth in SEQ ID NO:179 or SEQ ID NO:180 (variant containing L186G, L187G mutations of the native CD28 protein). An exemplary 4-1BB costimulatory signaling domain comprises an amino acid sequence of SEQ ID NO:181. In certain embodiments, a CAR comprises one, two, or more costimulatory signaling domains.

In some embodiments, a CAR of the present disclosure is a first generation CAR, a second generation CAR, or a third generation CAR. A first generation CAR generally has an intracellular signaling domain comprising an intracellular signaling domain of CD3ζ, FcγRI, or other ITAM-containing activating domain to provide a T cell activation signal. Second generation CARs further comprise a costimulatory signaling domain (*e.g.,* a costimulatory signaling domain from an endogenous T cell costimulatory receptor, such as CD28, 4-1BB, or ICOS). Third generation CARs comprise an ITAM-containing activating domain, a first costimulatory signaling domain and a second costimulatory signaling domain.

In some embodiments, one or more of the extracellular domain, the binding domain, the linker, the transmembrane domain, the intracellular signaling domain, or the costimulatory domain comprises junction amino acids. "Junction amino acids" or "junction amino acid residues" refer to one or more (e.g., about 2-20) amino acid residues between two adjacent domains, motifs, regions, modules, or fragments of a protein, such as between a binding domain and an adjacent linker, between a transmembrane domain and an adjacent extracellular or intracellular domain, or on one or both ends of a linker that links two domains, motifs, regions, modules, or fragments (e.g., between a linker and an adjacent binding domain or between a linker and an adjacent hinge). Junction amino acids may result from the construct design of a fusion protein (e.g., amino acid residues resulting from the use of a restriction enzyme site or self-cleaving peptide sequences during the construction of a polynucleotide encoding a fusion protein). For example, a transmembrane domain of a fusion protein may have one or more junction amino acids at the amino-terminal end, carboxy -terminal end, or both.

CARs of the present disclosure may comprise polynucleotide sequences derived from any mammalian species, including humans, primates, cows, horses, goats, sheep, dogs, cats, mice, rats, rabbits, guinea pigs, pigs, transgenic species thereof, or any combination thereof. In some embodiments, the chimeric antigen receptor is murine, chimeric, human, or humanized.

An exemplary CAR according to the present disclosure comprises: an extracellular domain comprising an scFv comprising the amino acid sequence set forth in SEQ ID NO:131 and an extracellular spacer domain comprising an IgG4 hinge, a CD28 transmembrane domain, a CD28 costimulatory signaling domain, and a CD3ζ signaling domain. In one embodiment, the CAR comprises the amino acid sequence set forth in SEQ ID NO:182 or SEQ ID NO:182 without amino acids 1-21.

An exemplary CAR according to the present disclosure comprises: an extracellular domain comprising an scFv comprising the amino acid sequence set forth in SEQ ID NO:132 and an extracellular spacer domain comprising an IgG4 hinge, a CD28 transmembrane domain, a CD28 costimulatory signaling domain, and a CD3ζ signaling domain. In one embodiment, the CAR comprises the amino acid sequence set forth in SEQ ID NO:183 or SEQ ID NO:183 without amino acids 1-21.

An exemplary CAR according to the present disclosure comprises: an extracellular domain comprising an scFv comprising the amino acid sequence set forth in SEQ ID NO:131 and an extracellular spacer domain comprising an IgG4 hinge, a CD28 transmembrane domain, a 4-1BB costimulatory signaling domain, and a CD3ζ signaling domain. In one embodiment, the CAR comprises the amino acid sequence set forth in SEQ ID NO:184 or SEQ ID NO:184 without amino acids 1-21.

An exemplary CAR according to the present disclosure comprises: an extracellular domain comprising an scFv comprising the amino acid sequence set forth in SEQ ID NO:132 and an extracellular spacer domain comprising an IgG4 hinge, a CD28 transmembrane domain, a 4-1BB costimulatory signaling domain, and a CD3ζ signaling domain. In one embodiment, the CAR comprises the amino acid sequence set forth in SEQ ID NO:185 or SEQ ID NO:185 without amino acids 1-21.

An exemplary CAR according to the present disclosure comprises: an extracellular domain comprising an scFv comprising the amino acid sequence set forth in SEQ ID NO:131 and an extracellular spacer domain comprising a CD28 hinge, a CD28 transmembrane domain, a CD28 costimulatory signaling domain, and a CD3ζ signaling domain. In one embodiment, the CAR comprises the amino acid sequence set forth in SEQ ID NO:186 or SEQ ID NO:186 without amino acids 1-21.

An exemplary CAR according to the present disclosure comprises: an extracellular domain comprising an scFv comprising the amino acid sequence set forth in SEQ ID NO:132 and an extracellular spacer domain comprising a CD28 hinge, a CD28 transmembrane domain, a CD28 costimulatory signaling domain, and a CD3ζ signaling domain. In one embodiment, the CAR comprises the amino acid sequence set forth in SEQ ID NO:187 or SEQ ID NO:187 without amino acids 1-21.

An exemplary CAR according to the present disclosure comprises: an extracellular domain comprising an scFv comprising the amino acid sequence set forth in SEQ ID NO:131 and an extracellular spacer domain comprising a CD28 hinge, a CD28 transmembrane domain, a 4-1BB costimulatory signaling domain, and a CD3ζ signaling domain. In one embodiment, the CAR comprises the amino acid sequence set forth in SEQ ID NO:188 or SEQ ID NO:188 without amino acids 1-21.

An exemplary CAR according to the present disclosure comprises: an extracellular domain comprising an scFv comprising the amino acid sequence set forth in SEQ ID NO:132 and an extracellular spacer domain comprising a CD28 hinge, a CD28 transmembrane domain, a 4-1BB costimulatory signaling domain, and a CD3ζ signaling domain. In one embodiment, the CAR comprises the amino acid sequence set forth in SEQ ID NO:189 or SEQ ID NO:189 without amino acids 1-21.

An exemplary CAR according to the present disclosure comprises: an extracellular domain comprising an scFv comprising the amino acid sequence set forth in SEQ ID NO:131 and an extracellular spacer domain comprising an IgG4 hinge, a CD8a transmembrane domain, a CD28 costimulatory signaling domain, and a CD3ζ signaling domain. In one embodiment, the CAR comprises the amino acid sequence set forth in SEQ ID NO:190 or SEQ ID NO:190 without amino acids 1-21.

An exemplary CAR according to the present disclosure comprises: an extracellular domain comprising an scFv comprising the amino acid sequence set forth in SEQ ID NO:132 and an extracellular spacer domain comprising an IgG4 hinge, a CD8a transmembrane domain, a CD28 costimulatory signaling domain, and a CD3ζ signaling domain. In one embodiment, the CAR comprises the amino acid sequence set forth in SEQ ID NO:191 or SEQ ID NO:191 without amino acids 1-21.

An exemplary CAR according to the present disclosure comprises: an extracellular domain comprising an scFv comprising the amino acid sequence set forth in SEQ ID NO:131 and an extracellular spacer domain comprising a CD8a hinge, a CD8a transmembrane domain, a CD28 costimulatory signaling domain, and a CD3ζ signaling domain. In one embodiment, the CAR comprises the amino acid sequence set forth in SEQ ID NO:192 or SEQ ID NO:192 without amino acids 1-21.

An exemplary CAR according to the present disclosure comprises: an extracellular domain comprising an scFv comprising the amino acid sequence set forth in SEQ ID NO:132 and an extracellular spacer domain comprising a CD8a hinge, a CD8a transmembrane domain, a CD28 costimulatory signaling domain, and a CD3ζ signaling domain. In one embodiment, the CAR comprises the amino acid sequence set forth in SEQ ID NO:193 or SEQ ID NO:193 without amino acids 1-21.

An exemplary CAR according to the present disclosure comprises: an extracellular domain comprising an scFv comprising the amino acid sequence set forth in SEQ ID NO:131 and an extracellular spacer domain comprising an IgG4 hinge, a CD8a transmembrane domain, a 4-1BB costimulatory signaling domain, and a CD3ζ signaling domain. In one embodiment, the CAR comprises the amino acid sequence set forth in SEQ ID NO:194 or SEQ ID NO:194 without amino acids 1-21.

An exemplary CAR according to the present disclosure comprises: an extracellular domain comprising an scFv comprising the amino acid sequence set forth in SEQ ID NO:132 and an extracellular spacer domain comprising an IgG4 hinge, a CD8a transmembrane domain, a 4-1BB costimulatory signaling domain, and a CD3ζ signaling domain. In one embodiment, the CAR comprises the amino acid sequence set forth in SEQ ID NO:195 or SEQ ID NO:195 without amino acids 1-21.

An exemplary CAR according to the present disclosure comprises: an extracellular domain comprising an scFv comprising the amino acid sequence set forth in SEQ ID NO:131 and an extracellular spacer domain comprising a CD8a hinge, a CD8a transmembrane domain, a 4-1BB costimulatory signaling domain, and a CD3ζ signaling domain. In one embodiment, the CAR comprises the amino acid sequence set forth in SEQ ID NO:196 or SEQ ID NO:196 without amino acids 1-21.

An exemplary CAR according to the present disclosure comprises: an extracellular domain comprising an scFv comprising the amino acid sequence set forth in SEQ ID NO:132 and an extracellular spacer domain comprising a CD8a hinge, a CD8a transmembrane domain, a 4-1BB costimulatory signaling domain, and a CD3ζ signaling domain. In one embodiment, the CAR comprises the amino acid sequence set forth in SEQ ID NO:197 or SEQ ID NO:197 without amino acids 1-21.

An exemplary CAR according to the present disclosure comprises: an extracellular domain comprising an scFv comprising the amino acid sequence set forth in SEQ ID NO:132 and an extracellular spacer domain comprising an IgG4 hinge, a CD28 transmembrane domain, a 4-1BB costimulatory signaling domain, and a CD3ζ signaling domain. In one embodiment, the CAR comprises the amino acid sequence set forth in SEQ ID NO:198 or SEQ ID NO:198 without amino acids 1-21.

An exemplary CAR according to the present disclosure comprises: an extracellular domain comprising an scFv comprising the amino acid sequence set forth in SEQ ID NO:132 and an extracellular spacer domain comprising an IgG4 hinge, a CD28 transmembrane domain, a 4-1BB costimulatory signaling domain, and a CD3ζ signaling domain. In one embodiment, the CAR comprises the amino acid sequence set forth in SEQ ID NO:199 or SEQ ID NO:199 without amino acids 1-20.

An exemplary CAR according to the present disclosure comprises: an extracellular domain comprising an scFv comprising the amino acid sequence set forth in SEQ ID NO:132 and an extracellular spacer domain comprising an IgG4 hinge, a CD28 transmembrane domain, a 4-1BB costimulatory signaling domain, and a CD3ζ signaling domain. In one embodiment, the CAR comprises the amino acid sequence set forth in SEQ ID NO:200 or SEQ ID NO:200 without amino acids 1-18.

An exemplary CAR according to the present disclosure comprises: an extracellular domain comprising an scFv comprising the amino acid sequence set forth in SEQ ID NO:132 and an extracellular spacer domain comprising an IgG4 hinge, a CD28 transmembrane domain, a 4-1BB costimulatory signaling domain, and a CD3ζ signaling domain. In one embodiment, the CAR comprises the amino acid sequence set forth in SEQ ID NO:201 or SEQ ID NO:201 without amino acids 1-20.

In some embodiments, the CAR comprises the amino acid sequence of a CAR set forth in Table 3, e.g., any one of SEQ ID NOS: 182-234, or the amino acid sequence set forth in any one of SEQ ID NOS: 182-234 absent the signal peptide.

Exemplary sequences for signal peptides, binding domains, extracellular spacers, transmembrane domains, and intracellular signaling domains for use in CARs of the present disclosure and exemplary CAR sequences are set forth in Table 3.

**Table 3: Embodiments of CD72 CARs and components.**

| **Name** | **Amino Acid Sequence** | **SEQ ID NO:** |
|---|---|---|
| Sc02-004 HCDR1 | GYLMS | 101 |
| Sc02-004 HCDR2 | VISYDGSNKYYADSVKG | 102 |
| Sc02-004 HCDR3 | ARRDTNLFDY | 103 |
| Sc02-004 LCDR1 | RASQSISSYLN | 104 |
| Sc02-004 LCDR2 | AASSLQS | 105 |
| Sc02-004 LCDR3 | QQSYSTPPT | 106 |
| Sc02-025 HCDR1 | SYYMH | 107 |
| Sc02-025 HCDR2 | IINPSGGGTSYAQKFQG | 108 |
| Sc02-025 HCDR3 | DYYVTYDSWFDS | 109 |
| Sc02-025 LCDR1 | QGDSLRSYYAS | 110 |
| Sc02-025 LCDR2 | GKNNRPS | 111 |
| Sc02-025 LCDR3 | NSRDSSGNHVV | 112 |
| Sc02-002 HCDR1 | GFTFSD | 302 |
| Sc02-002 HCDR2 | ISYDGSNK | 303 |
| Sc02-002 HCDR3 | AKDRGSAQGYPLDY | 304 |
| Sc02-002 LCDR1 | QSVSSTY | 305 |
| Sc02-002 LCDR2 | GAS | 306 |
| Sc02-002 LCDR3 | QQGSAFP | 307 |
| Sc02-003 HCDR1 | GFTFSNYV | 308 |
| Sc02-003 HCDR2 | ISYDGSNK | 309 |
| Sc02-003 HCDR3 | AKSHSNMSFDY | 310 |
| Sc02-003 LCDR1 | FTEHFQ | 311 |
| Sc02-003 LCDR2 | AAS | 312 |
| Sc02-003 LCDR3 | QQSYSTP | 313 |
| Sc02-004 HCDR1 | GFTFSGYL | 314 |
| Sc02-004 HCDR2 | ISYDGSNK | 315 |
| Sc02-004 HCDR3 | ARARRDTNLFDY | 316 |
| Sc02-004 LCDR1 | QSISSY | 317 |
| Sc02-004 LCDR2 | AAS | 318 |
| Sc02-004 LCDR3 | QQSYSTPPT | 319 |
| Sc02-024 HCDR1 | GYTFTSYY | 320 |
| Sc02-024 HCDR2 | INPSGGST | 321 |
| Sc02-024 HCDR3 | ARDYVRTSHSGFD | 322 |
| Sc02-024 LCDR1 | QSISSY | 323 |
| Sc02-024 LCDR2 | AAS | 324 |
| Sc02-024 LCDR3 | QQSYSTPPT | 325 |
| Sc02-025 HCDR1 | GYTFTSYY | 326 |
| Sc02-025 HCDR2 | INPSGGGT | 327 |
| Sc02-025 HCDR3 | ARDYYVTYDSWFDS | 328 |
| Sc02-025 LCDR1 | SLRSYY | 329 |
| Sc02-025 LCDR2 | GKN | 330 |
| Sc02-025 LCDR3 | NSRDSSGNHVV | 331 |
| Sc02-041 HCDR1 | GFTFSNYV | 332 |
| Sc02-041 HCDR2 | ISYDGSNK | 333 |
| Sc02-041 HCDR3 | AKSHSNMAFDY | 334 |
| Sc02-041 LCDR1 | QSISSY | 335 |
| Sc02-041 LCDR2 | AAS | 336 |
| Sc02-041 LCDR3 | QQSYSTPPT | 337 |
| Sc02-057 HCDR1 | GYTFTSYY | 338 |
| Sc02-057 HCDR2 | INPSGGST | 339 |
| Sc02-057 HCDR3 | ARDYQLSMTSGFDY | 340 |
| Sc02-057 LCDR1 | QSISSY | 341 |
| Sc02-057 LCDR2 | AAS | 342 |
| Sc02-057 LCDR3 | QQSYSTPPT | 343 |
| Sc02-132 HCDR1 | GFTFSNYV | 344 |
| Sc02-132 HCDR2 | ISYDGSNK | 345 |
| Sc02-132 HCDR3 | VKSHSNMAFDY | 346 |
| Sc02-132 LCDR1 | QSISSY | 347 |
| Sc02-132 LCDR2 | AAS | 348 |
| Sc02-132 LCDR3 | QQSYSTPPT | 349 |
| Sc02-004 VH | LVESGGGLVQPGGSLRLSCAASGFTFSGYLMSWVRQAP GKGLEWVAVISYDGSNKYYADSVKGRFTISRDNSKNTL YLQMDSLRAEDTAVYYCARARRDTNLFDYWGQGTLVT V | 113 |
| Sc02-004 VL | ELTQSPSSLSASVGDRVTITCRASQSISSYLNWYQQKPGK APKLLIYAASSLQSGVPSRFSGSGSGTDFTLTISSLQPEDF ATYYCQQSYSTPPTFGQGTKVEI | 114 |
| Sc02-025 VH | LVQSGAEVKKPGASVKVSCKASGYTFTSYYMHWVRQA PGQGLEWMGIINPSGGGTSYAQKFQGRVTMTRDTSTSTV YMELSSLRSEDTAVYYCARDYYVTYDSWFDSWGQGTL VTVS | 115 |
| Sc02-025 VL | VSVALGQTVRITCQGDSLRSYYASWYQQKPGQAPVLVI YGKNNRPSGIPDRFSGSSSGNTASLTITGAQAEDEADYYC NSRDSSGNHVVFGGGTKLTV | 116 |
| Sc02-002 VH | AEVQLVESGGGVVQPGRSLRLSCAASGFTFSDYTMSWV RQAPGKGLEWVAVISYDGSNKYYADSVKGRFTISRDNS KNTLYLQMNSLRAEDTAVYYCAKDRGSAQGYPLDYWG QGTLVTV | 117 |
| Sc02-002 VL | SELDIQMTQSPPTLSLSPGERATLSCRASQSVSSTYLTWY QQRPGQAPRLLIYGASSRATGIPDRFSGSGSGTDFTLTISR LEPEDVAVYYCQQGSAFPPTFGQGTKVEIKRAAA | 118 |
| Sc02-003 VH | AEVQLVESGGGLVQPGGSLRLPCAASGFTFSNYVMHWV RQAPGKGLEWVAVISYDGSNKYYADSVKGRFTISRDNS KNTLYLQMNSLRAEDTAVYYCAKSHSNMSFDYWGQGT LVTV | 119 |
| Sc02-003 VL | SELTQSPSSLSASVGDRVTITCRASQSISSYLNWYQQKPG KAPKLLIYAASSLQSGVPSRFSGSGSGTDFTLTISSLQPED FATYYCQQSYSTPPTFGQGTKVEIKRAAA | 120 |
| Sc02-024 VH | AQVQLVQSGPEVKKPGASVKVSCKASGYTFTSYYMHW VRQAPGQGLEWMGIINPSGGSTSYAQKFQGRVTMTRDT STSTVYMELSSLRSEDAAVYYCARDYVRTSHSGFDYWG QGTLVTV | 121 |
| Sc02-024 VL | SEIELTQSPSSLSASVGDRVTITCRASQSISSYLNWYQQKP GKAPKLLIYAASSLQSGVPSRFSGSGSGTDFTLTISSLQPE DFATYYCQQSYSTPPTFGQGTKVEIKRAAA | 122 |
| Sc02-025 VH | AQVQLVQSGAEVKKPGASVKVSCKASGYTFTSYYMHW VRQAPGQGLEWMGIINPSGGGTSYAQKFQGRVTMTRDT STSTVYMELSSLRSEDTAVYYCARDYYVTYDSWFDSWG QGTLVTVS | 123 |
| Sc02-025 VL | SELTQDPAVSVALGQTVRITCQGDSLRSYYASWYQQKP GQAPVLVIYGKNNRPSGIPDRFSGSSSGNTASLTITGAQA EDEADYYCNSRDSSGNHVVFGGGTKLTVLGAAA | 124 |
| Sc02-041 VH | AEVQLVESGGGLVQPGGSLRLPCAASGFTFSNYVMHWV RQAPGKGLEWVAVISYDGSNKYYADSVKGRFTISRDNS KNTLYLQMNSLRAEDTAVYYCAKSHSNMAFDYWGQGT LVTV | 125 |
| Sc02-041 VL | SELTQSPSSLSASVGDRVTITCRASQSISSYLNWYQQKPG KAPKLLIYAASSLQSGVPSRFSGSGSGTDFTLTISSLQPED FATYYCQQSYSTPPTFGQGTKVEIKRAAA | 126 |
| Sc02-057 VH | AQVQLVQSGAEVKKPGASVKVSCKASGYTFTSYYMHW VRQAPGQGLEWMGIINPSGGSTSYAQKFQGRVTMTRDT STSTVYMELSSLRSEDTAVYYCARDYQLSMTSGFDYWG QGTLVTV | 127 |
| Sc02-057 VL | SEIELTQSPSSLSASVGDRVTITCRASQSISSYLNWYQQKP GKAPKLLIYAASSLQSGVPSRFSGSGSGTDFTLTISSLQPE DFATYYCQQSYSTPPTFGQGTKVEIKRAAA | 128 |
| Sc02-132 VH | AEVQLVESGGGLVQPGGSLRLPCAASGFTFSNYVMHWV RQAPGKGLEWVAVISYDGSNKYYADSVKGRFTISRDNS KNTLYLQMNSLRAEDTAVYYCVKSHSNMAFDYWGQGT LVTV | 129 |
| Sc02-132 VL | SELTQSPSSLSASVGDRVTITCRASQSISSYLNWYQQKPG KAPKLLIYAASSLQSGVPSRFSGSGSGTDFTLTISSLQPED FATYYCQQSYSTPPTFGQGTKVEIKRAAA | 130 |
| Sc02-004 scFv | AEVQLVESGGGLVQPGGSLRLSCAASGFTFSGYLMSWV RQAPGKGLEWVAVISYDGSNKYYADSVKGRFTISRDNS KNTLYLQMDSLRAEDTAVYYCARARRDTNLFDYWGQG TLVTVLEGTGGSGGTGSGTGTSELTQSPSSLSASVGDRVT ITCRASQSISSYLNWYQQKPGKAPKLLIYAASSLQSGVPS RFSGSGSGTDFTLTISSLQPEDFATYYCQQSYSTPPTFGQG TKVEIKRAAA | 131 |
| Sc02-025 scFv | AQVQLVQSGAEVKKPGASVKVSCKASGYTFTSYYMHW VRQAPGQGLEWMGIINPSGGGTSYAQKFQGRVTMTRDT STSTVYMELSSLRSEDTAVYYCARDYYVTYDSWFDSWG QGTLVTVSRGGGGSGGGGSGGGGSSELTQDPAVSVALG QTVRITCQGDSLRSYYASWYQQKPGQAPVLVIYGKNNR PSGIPDRFSGSSSGNTASLTITGAQAEDEADYYCNSRDSS GNHVVFGGGTKLTVLGAAA | 132 |
| sc02-002 VL-VH W linker scFv | SELDIQMTQSPPTLSLSPGERATLSCRASQSVSSTYLTWY QQRPGQAPRLLIYGASSRATGIPDRFSGSGSGTDFTLTISR LEPEDVAVYYCQQGSAFPPTFGQGTKVEIKRAAAGSTSG SGKPGSGEGSTKGAEVQLVESGGGVVQPGRSLRLSCAAS GFTFSDYTMSWVRQAPGKGLEWVAVISYDGSNKYYAD SVKGRFTISRDNSKNTLYLQMNSLRAEDTAVYYCAKDR GSAQGYPLDYWGQGTLVTV | 133 |
| sc02-002 VH-VL W linker scFv | AEVQLVESGGGVVQPGRSLRLSCAASGFTFSDYTMSWV RQAPGKGLEWVAVISYDGSNKYYADSVKGRFTISRDNS KNTLYLQMNSLRAEDTAVYYCAKDRGSAQGYPLDYWG QGTLVTVGSTSGSGKPGSGEGSTKGSELDIQMTQSPPTLS LSPGERATLSCRASQSVSSTYLTWYQQRPGQAPRLLIYG ASSRATGIPDRFSGSGSGTDFTLTISRLEPEDVAVYYCQQ GSAFPPTFGQGTKVEIKRAAA | 134 |
| sc02-003 VL-VH W linker scFv | SELTQSPSSLSASVGDRVTITCRASQSISSYLNWYQQKPG KAPKLLIYAASSLQSGVPSRFSGSGSGTDFTLTISSLQPED FATYYCQQSYSTPPTFGQGTKVEIKRAAAGSTSGSGKPG SGEGSTKGAEVQLVESGGGLVQPGGSLRLPCAASGFTFS | 135 |
| | NYVMHWVRQAPGKGLEWVAVISYDGSNKYYADSVKG RFTISRDNSKNTLYLQMNSLRAEDTAVYYCAKSHSNMSF DYWGQGTLVTV | |
| sc02-003 VH-VL W linker scFv | AEVQLVESGGGLVQPGGSLRLPCAASGFTFSNYVMHWV RQAPGKGLEWVAVISYDGSNKYYADSVKGRFTISRDNS KNTLYLQMNSLRAEDTAVYYCAKSHSNMSFDYWGQGT LVTVGSTSGSGKPGSGEGSTKGSELTQSPSSLSASVGDRV TITCRASQSISSYLNWYQQKPGKAPKLLIYAASSLQSGVP SRFSGSGSGTDFTLTISSLQPEDFATYYCQQSYSTPPTFGQ GTKVEIKRAAA | 136 |
| sc02-004 VL-VH W linker scFv | SELTQSPSSLSASVGDRVTITCRASQSISSYLNWYQQKPG KAPKLLIYAASSLQSGVPSRFSGSGSGTDFTLTISSLQPED FATYYCQQSYSTPPTFGQGTKVEIKRAAAGSTSGSGKPG SGEGSTKGAEVQLVESGGGLVQPGGSLRLSCAASGFTFS GYLMSWVRQAPGKGLEWVAVISYDGSNKYYADSVKGR FTISRDNSKNTLYLQMDSLRAEDTAVYYCARARRDTNLF DYWGQGTLVTV | 137 |
| sc02-004 VH-VL W linker scFv | AEVQLVESGGGLVQPGGSLRLSCAASGFTFSGYLMSWV RQAPGKGLEWVAVISYDGSNKYYADSVKGRFTISRDNS KNTLYLQMDSLRAEDTAVYYCARARRDTNLFDYWGQG TLVTVGSTSGSGKPGSGEGSTKGSELTQSPSSLSASVGDR VTITCRASQSISSYLNWYQQKPGKAPKLLIYAASSLQSGV PSRFSGSGSGTDFTLTISSLQPEDFATYYCQQSYSTPPTFG QGTKVEIKRAAA | 138 |
| sc02-024 VL-VH W linker scFv | SEIELTQSPSSLSASVGDRVTITCRASQSISSYLNWYQQKP GKAPKLLIYAASSLQSGVPSRFSGSGSGTDFTLTISSLQPE DFATYYCQQSYSTPPTFGQGTKVEIKRAAAGSTSGSGKP GSGEGSTKGAQVQLVQSGPEVKKPGASVKVSCKASGYT FTSYYMHWVRQAPGQGLEWMGIINPSGGSTSYAQKFQG RVTMTRDTSTSTVYMELSSLRSEDAAVYYCARDYVRTS HSGFDYWGQGTLVTV | 139 |
| sc02-024 VH-VL W linker scFv | AQVQLVQSGPEVKKPGASVKVSCKASGYTFTSYYMHW VRQAPGQGLEWMGIINPSGGSTSYAQKFQGRVTMTRDT STSTVYMELSSLRSEDAAVYYCARDYVRTSHSGFDYWG QGTLVTVGSTSGSGKPGSGEGSTKGSEIELTQSPSSLSAS VGDRVTITCRASQSISSYLNWYQQKPGKAPKLLIYAASSL QSGVPSRFSGSGSGTDFTLTISSLQPEDFATYYCQQSYSTP PTFGQGTKVEIKRAAA | 140 |
| sc02-025 VL-VH W linker scFv | SELTQDPAVSVALGQTVRITCQGDSLRSYYASWYQQKP GQAPVLVIYGKNNRPSGIPDRFSGSSSGNTASLTITGAQA EDEADYYCNSRDSSGNHVVFGGGTKLTVLGAAAGSTSG SGKPGSGEGSTKGAQVQLVQSGAEVKKPGASVKVSCKA SGYTFTSYYMHWVRQAPGQGLEWMGIINPSGGGTSYAQ KFQGRVTMTRDTSTSTVYMELSSLRSEDTAVYYCARDY YVTYDSWFDSWGQGTLVTVS | 141 |
| sc02-025 VH-VL W linker scFv | AQVQLVQSGAEVKKPGASVKVSCKASGYTFTSYYMHW VRQAPGQGLEWMGIINPSGGGTSYAQKFQGRVTMTRDT STSTVYMELSSLRSEDTAVYYCARDYYVTYDSWFDSWG QGTLVTVSGSTSGSGKPGSGEGSTKGSELTQDPAVSVAL GQTVRITCQGDSLRSYYASWYQQKPGQAPVLVIYGKNN RPSGIPDRFSGSSSGNTASLTITGAQAEDEADYYCNSRDS SGNHVVFGGGTKLTVLGAAA | 142 |
| sc02-041 VL-VH W linker scFv | SELTQSPSSLSASVGDRVTITCRASQSISSYLNWYQQKPG KAPKLLIYAASSLQSGVPSRFSGSGSGTDFTLTISSLQPED FATYYCQQSYSTPPTFGQGTKVEIKRAAAGSTSGSGKPG SGEGSTKGAEVQLVESGGGLVQPGGSLRLPCAASGFTFS NYVMHWVRQAPGKGLEWVAVISYDGSNKYYADSVKG | 143 |
| | RFTISRDNSKNTLYLQMNSLRAEDTAVYYCAKSHSNMA FDYWGQGTLVTV | |
| sc02-041 VH-VL W linker scFv | AEVQLVESGGGLVQPGGSLRLPCAASGFTFSNYVMHWV RQAPGKGLEWVAVISYDGSNKYYADSVKGRFTISRDNS KNTLYLQMNSLRAEDTAVYYCAKSHSNMAFDYWGQGT LVTVGSTSGSGKPGSGEGSTKGSELTQSPSSLSASVGDRV TITCRASQSISSYLNWYQQKPGKAPKLLIYAASSLQSGVP SRFSGSGSGTDFTLTISSLQPEDFATYYCQQSYSTPPTFGQ GTKVEIKRAAA | 144 |
| sc02-057 VL-VH W linker scFv | SEIELTQSPSSLSASVGDRVTITCRASQSISSYLNWYQQKP GKAPKLLIYAASSLQSGVPSRFSGSGSGTDFTLTISSLQPE DFATYYCQQSYSTPPTFGQGTKVEIKRAAAGSTSGSGKP GSGEGSTKGAQVQLVQSGAEVKKPGASVKVSCKASGYT FTSYYMHWVRQAPGQGLEWMGIINPSGGSTSYAQKFQG RVTMTRDTSTSTVYMELSSLRSEDTAVYYCARDYQLSM TSGFDYWGQGTLVTV | 145 |
| sc02-057 VH-VL W linker scFv | AQVQLVQSGAEVKKPGASVKVSCKASGYTFTSYYMHW VRQAPGQGLEWMGIINPSGGSTSYAQKFQGRVTMTRDT STSTVYMELSSLRSEDTAVYYCARDYQLSMTSGFDYWG QGTLVTVGSTSGSGKPGSGEGSTKGSEIELTQSPSSLSAS VGDRVTITCRASQSISSYLNWYQQKPGKAPKLLIYAASSL QSGVPSRFSGSGSGTDFTLTISSLQPEDFATYYCQQSYSTP PTFGQGTKVEIKRAAA | 146 |
| sc02-132 VL-VH W linker scFv | SELTQSPSSLSASVGDRVTITCRASQSISSYLNWYQQKPG KAPKLLIYAASSLQSGVPSRFSGSGSGTDFTLTISSLQPED FATYYCQQSYSTPPTFGQGTKVEIKRAAAGSTSGSGKPG SGEGSTKGAEVQLVESGGGLVQPGGSLRLPCAASGFTFS NYVMHWVRQAPGKGLEWVAVISYDGSNKYYADSVKG RFTISRDNSKNTLYLQMNSLRAEDTAVYYCVKSHSNMA FDYWGQGTLVTV | 147 |
| sc02-132 VH-VL W linker scFv | AEVQLVESGGGLVQPGGSLRLPCAASGFTFSNYVMHWV RQAPGKGLEWVAVISYDGSNKYYADSVKGRFTISRDNS KNTLYLQMNSLRAEDTAVYYCVKSHSNMAFDYWGQGT LVTVGSTSGSGKPGSGEGSTKGSELTQSPSSLSASVGDRV TITCRASQSISSYLNWYQQKPGKAPKLLIYAASSLQSGVP SRFSGSGSGTDFTLTISSLQPEDFATYYCQQSYSTPPTFGQ GTKVEIKRAAA | 148 |
| sc02-002 VL-VH P 002 linker scFv | SELDIQMTQSPPTLSLSPGERATLSCRASQSVSSTYLTWY QQRPGQAPRLLIYGASSRATGIPDRFSGSGSGTDFTLTISR LEPEDVAVYYCQQGSAFPPTFGQGTKVEIKRAAALEGTG GSGGTGSGTGT AEVQLVESGGGVVQPGRSLRLSCAASGFTFSDYTMSWV RQAPGKGLEWVAVISYDGSNKYYADSVKGRFTISRDNS KNTLYLQMNSLRAEDTAVYYCAKDRGSAQGYPLDYWG QGTLVTV | 149 |
| sc02-002 VH-VL P 002 linker scFv | AEVQLVESGGGVVQPGRSLRLSCAASGFTFSDYTMSWV RQAPGKGLEWVAVISYDGSNKYYADSVKGRFTISRDNS KNTLYLQMNSLRAEDTAVYYCAKDRGSAQGYPLDYWG QGTLVTVLEGTGGSGGTGSGTGTSELDIQMTQSPPTLSLS PGERATLSCRASQSVSSTYLTWYQQRPGQAPRLLIYGAS SRATGIPDRFSGSGSGTDFTLTISRLEPEDVAVYYCQQGS AFPPTFGQGTKVEIKRAAA | 150 |
| sc02-003 VL-VH P 002 linker scFv | SELTQSPSSLSASVGDRVTITCRASQSISSYLNWYQQKPG KAPKLLIYAASSLQSGVPSRFSGSGSGTDFTLTISSLQPED FATYYCQQSYSTPPTFGQGTKVEIKRAAALEGTGGSGGT GSGTGTAEVQLVESGGGLVQPGGSLRLPCAASGFTFSNY | 151 |
| | VMHWVRQAPGKGLEWVAVISYDGSNKYYADSVKGRFT ISRDNSKNTLYLQMNSLRAEDTAVYYCAKSHSNMSFDY WGQGTLVTV | |
| sc02-003 VH-VL P 002 linker scFv | AEVQLVESGGGLVQPGGSLRLPCAASGFTFSNYVMHWV RQAPGKGLEWVAVISYDGSNKYYADSVKGRFTISRDNS KNTLYLQMNSLRAEDTAVYYCAKSHSNMSFDYWGQGT LVTVLEGTGGSGGTGSGTGTSELTQSPSSLSASVGDRVTI TCRASQSISSYLNWYQQKPGKAPKLLIYAASSLQSGVPSR FSGSGSGTDFTLTISSLQPEDFATYYCQQSYSTPPTFGQGT KVEIKRAAA | 152 |
| sc02-004 VL-VH P 002 linker scFv | SELTQSPSSLSASVGDRVTITCRASQSISSYLNWYQQKPG KAPKLLIYAASSLQSGVPSRFSGSGSGTDFTLTISSLQPED FATYYCQQSYSTPPTFGQGTKVEIKRAAALEGTGGSGGT GSGTGTAEVQLVESGGGLVQPGGSLRLSCAASGFTFSGY LMSWVRQAPGKGLEWVAVISYDGSNKYYADSVKGRFTI SRDNSKNTLYLQMDSLRAEDTAVYYCARARRDTNLFDY WGQGTLVTV | 153 |
| sc02-004 VH-VL P 002 linker scFv | AEVQLVESGGGLVQPGGSLRLSCAASGFTFSGYLMSWV RQAPGKGLEWVAVISYDGSNKYYADSVKGRFTISRDNS KNTLYLQMDSLRAEDTAVYYCARARRDTNLFDYWGQG TLVTVLEGTGGSGGTGSGTGTSELTQSPSSLSASVGDRVT ITCRASQSISSYLNWYQQKPGKAPKLLIYAASSLQSGVPS RFSGSGSGTDFTLTISSLQPEDFATYYCQQSYSTPPTFGQG TKVEIKRAAA | 154 |
| sc02-024 VL-VH P 024 linker scFv | SEIELTQSPSSLSASVGDRVTITCRASQSISSYLNWYQQKP GKAPKLLIYAASSLQSGVPSRFSGSGSGTDFTLTISSLQPE DFATYYCQQSYSTPPTFGQGTKVEIKRAAASSGGGGSGG GGSGGGGAQVQLVQSGPEVKKPGASVKVSCKASGYTFT SYYMHWVRQAPGQGLEWMGIINPSGGSTSYAQKFQGR VTMTRDTSTSTVYMELSSLRSEDAAVYYCARDYVRTSH SGFDYWGQGTLVTV | 155 |
| sc02-024 VH-VL P 024 linker scFv | AQVQLVQSGPEVKKPGASVKVSCKASGYTFTSYYMHW VRQAPGQGLEWMGIINPSGGSTSYAQKFQGRVTMTRDT STSTVYMELSSLRSEDAAVYYCARDYVRTSHSGFDYWG QGTLVTVSSGGGGSGGGGSGGGGSEIELTQSPSSLSASVG DRVTITCRASQSISSYLNWYQQKPGKAPKLLIYAASSLQS GVPSRFSGSGSGTDFTLTISSLQPEDFATYYCQQSYSTPPT FGQGTKVEIKRAAA | 156 |
| sc02-025 VL-VH P 025 linker scFv | SELTQDPAVSVALGQTVRITCQGDSLRSYYASWYQQKP GQAPVLVIYGKNNRPSGIPDRFSGSSSGNTASLTITGAQA EDEADYYCNSRDSSGNHVVFGGGTKLTVLGAAARGGG GSGGGGSGGGGS AQVQLVQSGAEVKKPGASVKVSCKASGYTFTSYYMHW VRQAPGQGLEWMGIINPSGGGTSYAQKFQGRVTMTRDT STSTVYMELSSLRSEDTAVYYCARDYYVTYDSWFDSWG QGTLVTVS | 157 |
| sc02-025 VH-VL P 025 linker scFv | AQVQLVQSGAEVKKPGASVKVSCKASGYTFTSYYMHW VRQAPGQGLEWMGIINPSGGGTSYAQKFQGRVTMTRDT STSTVYMELSSLRSEDTAVYYCARDYYVTYDSWFDSWG QGTLVTVSRGGGGSGGGGSGGGGSSELTQDPAVSVALG QTVRITCQGDSLRSYYASWYQQKPGQAPVLVIYGKNNR PSGIPDRFSGSSSGNTASLTITGAQAEDEADYYCNSRDSS GNHVVFGGGTKLTVLGAAA | 158 |
| sc02-041 VL-VH P 002 linker scFv | SELTQSPSSLSASVGDRVTITCRASQSISSYLNWYQQKPG KAPKLLIYAASSLQSGVPSRFSGSGSGTDFTLTISSLQPED FATYYCQQSYSTPPTFGQGTKVEIKRAAALEGTGGSGGT GSGTGTAEVQLVESGGGLVQPGGSLRLPCAASGFTFSNY | 159 |
| | VMHWVRQAPGKGLEWVAVISYDGSNKYYADSVKGRFT ISRDNSKNTLYLQMNSLRAEDTAVYYCAKSHSNMAFDY WGQGTLVTV | |
| sc02-041 VH-VL P 002 linker scFv | AEVQLVESGGGLVQPGGSLRLPCAASGFTFSNYVMHWV RQAPGKGLEWVAVISYDGSNKYYADSVKGRFTISRDNS KNTLYLQMNSLRAEDTAVYYCAKSHSNMAFDYWGQGT LVTVLEGTGGSGGTGSGTGTSELTQSPSSLSASVGDRVTI TCRASQSISSYLNWYQQKPGKAPKLLIYAASSLQSGVPSR FSGSGSGTDFTLTISSLQPEDFATYYCQQSYSTPPTFGQGT KVEIKRAAA | 160 |
| sc02-057 VL-VH P 024 linker scFv | SEIELTQSPSSLSASVGDRVTITCRASQSISSYLNWYQQKP GKAPKLLIYAASSLQSGVPSRFSGSGSGTDFTLTISSLQPE DFATYYCQQSYSTPPTFGQGTKVEIKRAAASSGGGGSGG GGSGGGGAQVQLVQSGAEVKKPGASVKVSCKASGYTFT SYYMHWVRQAPGQGLEWMGIINPSGGSTSYAQKFQGR VTMTRDTSTSTVYMELSSLRSEDTAVYYCARDYQLSMT SGFDYWGQGTLVTV | 161 |
| sc02-057 VH-VL P 024 linker scFv | AQVQLVQSGAEVKKPGASVKVSCKASGYTFTSYYMHW VRQAPGQGLEWMGIINPSGGSTSYAQKFQGRVTMTRDT STSTVYMELSSLRSEDTAVYYCARDYQLSMTSGFDYWG QGTLVTVSSGGGGSGGGGSGGGGSEIELTQSPSSLSASVG DRVTITCRASQSISSYLNWYQQKPGKAPKLLIYAASSLQS GVPSRFSGSGSGTDFTLTISSLQPEDFATYYCQQSYSTPPT FGQGTKVEIKRAAA | 162 |
| sc02-132 VL-VH P 002 linker scFv | SELTQSPSSLSASVGDRVTITCRASQSISSYLNWYQQKPG KAPKLLIYAASSLQSGVPSRFSGSGSGTDFTLTISSLQPED FATYYCQQSYSTPPTFGQGTKVEIKRAAALEGTGGSGGT GSGTGTAEVQLVESGGGLVQPGGSLRLPCAASGFTFSNY VMHWVRQAPGKGLEWVAVISYDGSNKYYADSVKGRFT ISRDNSKNTLYLQMNSLRAEDTAVYYCVKSHSNMAFDY WGQGTLVTV | 163 |
| sc02-132 VH-VL P 002 linker scFv | AEVQLVESGGGLVQPGGSLRLPCAASGFTFSNYVMHWV RQAPGKGLEWVAVISYDGSNKYYADSVKGRFTISRDNS KNTLYLQMNSLRAEDTAVYYCVKSHSNMAFDYWGQGT LVTVLEGTGGSGGTGSGTGTSELTQSPSSLSASVGDRVTI TCRASQSISSYLNWYQQKPGKAPKLLIYAASSLQSGVPSR FSGSGSGTDFTLTISSLQPEDFATYYCQQSYSTPPTFGQGT KVEIKRAAA | 164 |
| Flexible scFv linker | GGGGSGGGGSGGGGS | 165 |
| Flexible scFv linker | GTGGSGGTGSGTGTS | 166 |
| W scFv linker | GSTSGSGKPGSGEGSTKG | 167 |
| P 002 linker | LEGTGGSGGTGSGTGT | 168 |
| P 024 linker | SSGGGGSGGGGSGGGG | 169 |
| P 025 linker | RGGGGSGGGGSGGGGS | 170 |
| IgG4 hinge region (short) | ESKYGPPCPPCP | 171 |
| CD8a hinge region | TTTPAPRPPTPAPTIASQPLSLRPEACRPAAGGAVHTRGL DFACD | 172 |
| CD28 hinge region | IEVMYPPPYLDNEKSNGTIIHVKGKHLCPSPLFPGPSKP | 173 |
| CD8a transmembrane region | IYIWAPLAGTCGVLLL SLVITLYC | 174 |
| CD28 transmembrane region | FWVLVWGGVLACYSLLVTVAFIIFWV | 175 |
| CD28 transmembrane domain | MFWVLVVVGGVLACYSLLVTVAFIIFWV | 176 |
| wildtype CD3ζ signaling domain | RVKFSRSADAPAYQQGQNQLYNELNLGRREEYDVLDKR RGRDPEMGGKPQRRKNPQEGLYNELQKDKMAEAYSEIG MKGERRRGKGHDGLYQGLSTATKDTYDALHMQALPPR | 177 |
| variant CD3ζ signaling domain | RVKFSRSADAPAYQQGQNQLYNELNLGRREEYDVLDKR RGRDPEMGGKPRRKNPQEGLYNELQKDKMAEAYSEIG MKGERRRGKGHDGLYQGLSTATKDTYDALHMQALPPR | 178 |
| wildtype CD28 costimulatory signaling domain | RSKRSRLLHSDYMNMTPRRPGPTRKHYQPYAPPRDFAA YRS | 179 |
| variant CD28 costimulatory signaling domain with L 186G/L187G substitutions with positions in reference to full length protein | RSKRSRGGHSDYMNMTPRRPGPTRKHYQPYAPPRDFAA YRS | 180 |
| 4-1BB costimulatory signaling domain | KRGRKKLLYIFKQPFMRPVQTTQEEDGCSCRFPEEEEGG CEL | 181 |
| pCTX206 CAR: GMSCFR signal peptide (amino acids 1-21) - sc02-004 scFv - IgG4 Hinge - CD28 TM - CD28 ICD - CD3z | MLLVTSLLLCELPHPAFLLIPAEVQLVESGGGLVQPGGSL RLSCAASGFTFSGYLMSWVRQAPGKGLEWVAVISYDGS NKYYADSVKGRFTISRDNSKNTLYLQMDSLRAEDTAVY YCARARRDTNLFDYWGQGTLVTVLEGTGGSGGTGSGT GTSELTQSPSSLSASVGDRVTITCRASQSISSYLNWYQQK PGKAPKLLIYAASSLQSGVPSRFSGSGSGTDFTLTISSLQP EDFATYYCQQSYSTPPTFGQGTKVEIKRAAAESKYGPPC PPCPFWVLVVVGGVLACYSLLVTVAFIIFWVRSKRSRLL HSDYMNMTPRRPGPTRKHYQPYAPPRDFAAYRSRVKFS RSADAPAYQQGQNQLYNELNLGRREEYDVLDKRRGRD PEMGGKPQRRKNPQEGLYNELQKDKMAEAYSEIGMKG ERRRGKGHDGLYQGLSTATKDTYDALHMQALPPR | 182 |
| pCTX208 CAR: GMSCFR signal peptide (amino acids 1-21) - sc02-025 scFv - IgG4 Hinge - CD28 TM - CD28 ICD - CD3z CAR: GMSCFR signal peptide (amino acids 1-21) - sc02-004 scFv - IgG4 Hinge - CD28 TM - 4-1BB ICD - CD3z | MLLVTSLLLCELPHPAFLLIPAQVQLVQSGAEVKKPGAS VKVSCKASGYTFTSYYMHWVRQAPGQGLEWMGIINPSG GGTSYAQKFQGRVTMTRDTSTSTVYMELSSLRSEDTAV YYCARDYYVTYDSWFDSWGQGTLVTVSRGGGGSGGGG SGGGGSSELTQDPAVSVALGQTVRITCQGDSLRSYYASW YQQKPGQAPVLVIYGKNNRPSGIPDRFSGSSSGNTASLTI TGAQAEDEADYYCNSRDSSGNHVVFGGGTKLTVLGAA AESKYGPPCPPCPFWVLVVVGGVLACYSLLVTVAFIIFW VRSKRSRLLHSDYMNMTPRRPGPTRKHYQPYAPPRDFA AYRSRVKFSRSADAPAYQQGQNQLYNELNLGRREEYDV LDKRRGRDPEMGGKPQRRKNPQEGLYNELQKDKMAEA YSEIGMKGERRRGKGHDGLYQGLSTATKDTYDALHMQ ALPPR | 183 |
| | MLLVTSLLLCELPHPAFLLIPAEVQLVESGGGLVQPGGSL RLSCAASGFTFSGYLMSWVRQAPGKGLEWVAVISYDGS NKYYADSVKGRFTISRDNSKNTLYLQMDSLRAEDTAVY YCARARRDTNLFDYWGQGTLVTVLEGTGGSGGTGSGT GTSELTQSPSSLSASVGDRVTITCRASQSISSYLNWYQQK PGKAPKLLIYAASSLQSGVPSRFSGSGSGTDFTLTISSLQP EDFATYYCQQSYSTPPTFGQGTKVEIKRAAAESKYGPPC PPCPFWVLVVVGGVLACYSLLVTVAFIIFWVKRGRKKLL YIFKQPFMRPVQTTQEEDGCSCRFPEEEEGGCELRVKFSR SADAPAYQQGQNQLYNELNLGRREEYDVLDKRRGRDPE MGGKPQRRKNPQEGLYNELQKDKMAEAYSEIGMKGER RRGKGHDGLYQGLSTATKDTYDALHMQALPPR | 184 |
| CAR: GMSCFR signal peptide (amino acids 1-21) - sc02-025 scFv - IgG4 Hinge - CD28 TM - 4-1BB ICD - CD3z | MLLVTSLLLCELPHPAFLLIPAQVQLVQSGAEVKKPGAS VKVSCKASGYTFTSYYMHWVRQAPGQGLEWMGIINPSG GGTSYAQKFQGRVTMTRDTSTSTVYMELSSLRSEDTAV YYCARDYYVTYDSWFDSWGQGTLVTVSRGGGGSGGGG SGGGGSSELTQDPAVSVALGQTVRITCQGDSLRSYYASW YQQKPGQAPVLVIYGKNNRPSGIPDRFSGSSSGNTASLTI TGAQAEDEADYYCNSRDSSGNHVVFGGGTKLTVLGAA AESKYGPPCPPCPFWVLVVVGGVLACYSLLVTVAFIIFW VKRGRKKLLYIFKQPFMRPVQTTQEEDGCSCRFPEEEEGG CELRVKFSRSADAPAYQQGQNQLYNELNLGRREEYDVL DKRRGRDPEMGGKPQRRKNPQEGLYNELQKDKMAEAY SEIGMKGERRRGKGHDGLYQGLSTATKDTYDALHMQA LPPR | 185 |
| CAR: GMSCFR signal peptide (amino acids 1-21) - sc02-004 scFv - CD28 Hinge - CD28 TM - CD28 ICD - CD3z | MLLVTSLLLCELPHPAFLLIPAEVQLVESGGGLVQPGGSL RLSCAASGFTFSGYLMSWVRQAPGKGLEWVAVISYDGS NKYYADSVKGRFTISRDNSKNTLYLQMDSLRAEDTAVY YCARARRDTNLFDYWGQGTLVTVLEGTGGSGGTGSGT GTSELTQSPSSLSASVGDRVTITCRASQSISSYLNWYQQK PGKAPKLLIYAASSLQSGVPSRFSGSGSGTDFTLTISSLQP EDFATYYCQQSYSTPPTFGQGTKVEIKRAAAIEVMYPPP YLDNEKSNGTIIHVKGKHLCPSPLFPGPSKPFWVLVVVG GVLACYSLLVTVAFIIFWVRSKRSRLLHSDYMNMTPRRP GPTRKHYQPYAPPRDFAAYRSRVKFSRSADAPAYQQGQ NQLYNELNLGRREEYDVLDKRRGRDPEMGGKPQRRKN PQEGLYNELQKDKMAEAYSEIGMKGERRRGKGHDGLY QGLSTATKDTYDALHMQALPPR | 186 |
| CAR: GMSCFR signal peptide (amino acids 1-21) - sc02-025 scFv - CD28 Hinge - CD28 TM - CD28 ICD - CD3z | MLLVTSLLLCELPHPAFLLIPAQVQLVQSGAEVKKPGAS VKVSCKASGYTFTSYYMHWVRQAPGQGLEWMGIINPSG GGTSYAQKFQGRVTMTRDTSTSTVYMELSSLRSEDTAV YYCARDYYVTYDSWFDSWGQGTLVTVSRGGGGSGGGG SGGGGSSELTQDPAVSVALGQTVRITCQGDSLRSYYASW YQQKPGQAPVLVIYGKNNRPSGIPDRFSGSSSGNTASLTI TGAQAEDEADYYCNSRDSSGNHVVFGGGTKLTVLGAA AIEVMYPPPYLDNEKSNGTIIHVKGKHLCPSPLFPGPSKPF WVLVVVGGVLACYSLLVTVAFIIFWVRSKRSRLLHSDY MNMTPRRPGPTRKHYQPYAPPRDFAAYRSRVKFSRSAD APAYQQGQNQLYNELNLGRREEYDVLDKRRGRDPEMG GKPQRRKNPQEGLYNELQKDKMAEAYSEIGMKGERRR GKGHDGLYQGLSTATKDTYDALHMQALPPR | 187 |
| CAR: GMSCFR signal peptide (amino acids 1-21) - sc02-004 scFv - CD28 Hinge - CD28 TM - 4-1BB ICD - CD3z | MLLVTSLLLCELPHPAFLLIPAEVQLVESGGGLVQPGGSL RLSCAASGFTFSGYLMSWVRQAPGKGLEWVAVISYDGS | 188 |
| | NKYYADSVKGRFTISRDNSKNTLYLQMDSLRAEDTAVY YCARARRDTNLFDYWGQGTLVTVLEGTGGSGGTGSGT GTSELTQSPSSLSASVGDRVTITCRASQSISSYLNWYQQK PGKAPKLLIYAASSLQSGVPSRFSGSGSGTDFTLTISSLQP EDFATYYCQQSYSTPPTFGQGTKVEIKRAAAIEVMYPPP YLDNEKSNGTIIHVKGKHLCPSPLFPGPSKPFWVLVVVG GVLACYSLLVTVAFIIFWVKRGRKKLLYIFKQPFMRPVQT TQEEDGCSCRFPEEEEGGCELRVKFSRSADAPAYQQGQN QLYNELNLGRREEYDVLDKRRGRDPEMGGKPQRRKNP QEGLYNELQKDKMAEAYSEIGMKGERRRGKGHDGLYQ GLSTATKDTYDALHMQALPPR | |
| CAR: GMSCFR signal peptide - (amino acids 1-21) - sc02-025 scFv - CD28 Hinge - CD28 TM - 4-1BB ICD - CD3z | MLLVTSLLLCELPHPAFLLIPAQVQLVQSGAEVKKPGAS VKVSCKASGYTFTSYYMHWVRQAPGQGLEWMGIINPSG GGTSYAQKFQGRVTMTRDTSTSTVYMELSSLRSEDTAV YYCARDYYVTYDSWFDSWGQGTLVTVSRGGGGSGGGG SGGGGSSELTQDPAVSVALGQTVRITCQGDSLRSYYASW YQQKPGQAPVLVIYGKNNRPSGIPDRFSGSSSGNTASLTI TGAQAEDEADYYCNSRDSSGNHVVFGGGTKLTVLGAA AIEVMYPPPYLDNEKSNGTIIHVKGKHLCPSPLFPGPSKPF WVLVVVGGVLACYSLLVTVAFIIFWVKRGRKKLLYIFKQ PFMRPVQTTQEEDGCSCRFPEEEEGGCELRVKFSRSADAP AYQQGQNQLYNELNLGRREEYDVLDKRRGRDPEMGGK PQRRKNPQEGLYNELQKDKMAEAYSEIGMKGERRRGK GHDGLYQGLSTATKDTYDALHMQALPPR | 189 |
| CAR: GMSCFR signal peptide (amino acids 1-21) - sc02-004 scFv - IgG4 Hinge - CD8a TM - CD28 ICD - CD3z | MLLVTSLLLCELPHPAFLLIPAEVQLVESGGGLVQPGGSL RLSCAASGFTFSGYLMSWVRQAPGKGLEWVAVISYDGS NKYYADSVKGRFTISRDNSKNTLYLQMDSLRAEDTAVY YCARARRDTNLFDYWGQGTLVTVLEGTGGSGGTGSGT GTSELTQSPSSLSASVGDRVTITCRASQSISSYLNWYQQK PGKAPKLLIYAASSLQSGVPSRFSGSGSGTDFTLTISSLQP EDFATYYCQQSYSTPPTFGQGTKVEIKRAAAESKYGPPC PPCPIYIWAPLAGTCGVLLLSLVITLYCRSKRSRLLHSDYMN MTPRRPGPTRKHYQPYAPPRDFAAYRSRVKFSRSADAPA YQQGQNQLYNELNLGRREEYDVLDKRRGRDPEMGGKP QRRKNPQEGLYNELQKDKMAEAYSEIGMKGERRRGKG HDGLYQGLSTATKDTYDALHMQALPPR | 190 |
| CAR: GMSCFR signal peptide (amino acids 1-21) - sc02-025 scFv - IgG4 Hinge - CD8a TM - CD28 ICD - CD3z | MLLVTSLLLCELPHPAFLLIPAQVQLVQSGAEVKKPGAS VKVSCKASGYTFTSYYMHWVRQAPGQGLEWMGIINPSG GGTSYAQKFQGRVTMTRDTSTSTVYMELSSLRSEDTAV YYCARDYYVTYDSWFDSWGQGTLVTVSRGGGGSGGGG SGGGGSSELTQDPAVSVALGQTVRITCQGDSLRSYYASW YQQKPGQAPVLVIYGKNNRPSGIPDRFSGSSSGNTASLTI TGAQAEDEADYYCNSRDSSGNHVVFGGGTKLTVLGAA AESKYGPPCPPCPIYIWAPLAGTCGVLLLSLVITLYCRSKRSR LLHSDYMNMTPRRPGPTRKHYQPYAPPRDFAAYRSRVK FSRSADAPAYQQGQNQLYNELNLGRREEYDVLDKRRGR DPEMGGKPQRRKNPQEGLYNELQKDKMAEAYSEIGMK GERRRGKGHDGLYQGLSTATKDTYDALHMQALPPR | 191 |
| CAR: GMSCFR signal peptide (amino acids 1-21) - sc02-004 scFv - CD8a Hinge - | MLLVTSLLLCELPHPAFLLIPAEVQLVESGGGLVQPGGSL RLSCAASGFTFSGYLMSWVRQAPGKGLEWVAVISYDGS NKYYADSVKGRFTISRDNSKNTLYLQMDSLRAEDTAVY YCARARRDTNLFDYWGQGTLVTVLEGTGGSGGTGSGT GTSELTQSPSSLSASVGDRVTITCRASQSISSYLNWYQQK | 192 |
| CD8a TM - CD28 ICD - CD3z | PGKAPKLLIYAASSLQSGVPSRFSGSGSGTDFTLTISSLQP EDFATYYCQQSYSTPPTFGQGTKVEIKRAAATTTPAPRPP TPAPTIASQPLSLRPEACRPAAGGAVHTRGLDFACDIYIWAP LAGTCGVLLLSLVITLYCRSKRSRLLHSDYMNMTPRRPGPT RKHYQPYAPPRDFAAYRSRVKFSRSADAPAYQQGQNQL YNELNLGRREEYDVLDKRRGRDPEMGGKPQRRKNPQE GLYNELQKDKMAEAYSEIGMKGERRRGKGHDGLYQGL STATKDTYDALHMQALPPR | |
| CAR: GMSCFR signal peptide (amino acids 1-21) - sc02-025 scFv - CD8a Hinge - CD8a TM - CD28 ICD - CD3z | MLLVTSLLLCELPHPAFLLIPAQVQLVQSGAEVKKPGAS VKVSCKASGYTFTSYYMHWVRQAPGQGLEWMGIINPSG GGTSYAQKFQGRVTMTRDTSTSTVYMELSSLRSEDTAV YYCARDYYVTYDSWFDSWGQGTLVTVSRGGGGSGGGG SGGGGSSELTQDPAVSVALGQTVRITCQGDSLRSYYASW YQQKPGQAPVLVIYGKNNRPSGIPDRFSGSSSGNTASLTI TGAQAEDEADYYCNSRDSSGNHVVFGGGTKLTVLGAA ATTTPAPRPPTPAPTIASQPLSLRPEACRPAAGGAVHTRGLD FACDIYIWAPLAGTCGVLLLSLVITLYCRSKRSRLLHSDYMN MTPRRPGPTRKHYQPYAPPRDFAAYRSRVKFSRSADAPA YQQGQNQLYNELNLGRREEYDVLDKRRGRDPEMGGKP QRRKNPQEGLYNELQKDKMAEAYSEIGMKGERRRGKG HDGLYQGLSTATKDTYDALHMQALPPR | 193 |
| CAR: GMSCFR signal peptide (amino acids 1-21) - sc02-004 scFv - IgG4 Hinge - CD8a TM - 4-1BB ICD - CD3z | MLLVTSLLLCELPHPAFLLIPAEVQLVESGGGLVQPGGSL RLSCAASGFTFSGYLMSWVRQAPGKGLEWVAVISYDGS NKYYADSVKGRFTISRDNSKNTLYLQMDSLRAEDTAVY YCARARRDTNLFDYWGQGTLVTVLEGTGGSGGTGSGT GTSELTQSPSSLSASVGDRVTITCRASQSISSYLNWYQQK PGKAPKLLIYAASSLQSGVPSRFSGSGSGTDFTLTISSLQP EDFATYYCQQSYSTPPTFGQGTKVEIKRAAAESKYGPPC PPCPIYIWAPLAGTCGVLLLSLVITLYCKRGRKKLLYIFKQPF MRPVQTTQEEDGCSCRFPEEEEGGCELRVKFSRSADAPA YQQGQNQLYNELNLGRREEYDVLDKRRGRDPEMGGKP QRRKNPQEGLYNELQKDKMAEAYSEIGMKGERRRGKG HDGLYQGLSTATKDTYDALHMQALPPR | 194 |
| CAR: GMSCFR signal peptide (amino acids 1-21) - sc02-025 scFv - IgG4 Hinge - CD8a TM - 4-1BB ICD - CD3z | MLLVTSLLLCELPHPAFLLIPAQVQLVQSGAEVKKPGAS VKVSCKASGYTFTSYYMHWVRQAPGQGLEWMGIINPSG GGTSYAQKFQGRVTMTRDTSTSTVYMELSSLRSEDTAV YYCARDYYVTYDSWFDSWGQGTLVTVSRGGGGSGGGG SGGGGSSELTQDPAVSVALGQTVRITCQGDSLRSYYASW YQQKPGQAPVLVIYGKNNRPSGIPDRFSGSSSGNTASLTI TGAQAEDEADYYCNSRDSSGNHVVFGGGTKLTVLGAA AESKYGPPCPPCPIYIWAPLAGTCGVLLLSLVITLYCKRGRK KLLYIFKQPFMRPVQTTQEEDGCSCRFPEEEEGGCELRVK FSRSADAPAYQQGQNQLYNELNLGRREEYDVLDKRRGR DPEMGGKPQRRKNPQEGLYNELQKDKMAEAYSEIGMK GERRRGKGHDGLYQGLSTATKDTYDALHMQALPPR | 195 |
| CAR: GMSCFR signal peptide (amino acids 1-21) - sc02-004 scFv-CD8a Hinge - CD8a TM - 4-1BB ICD - CD3z | MLLVTSLLLCELPHPAFLLIPAEVQLVESGGGLVQPGGSL RLSCAASGFTFSGYLMSWVRQAPGKGLEWVAVISYDGS NKYYADSVKGRFTISRDNSKNTLYLQMDSLRAEDTAVY YCARARRDTNLFDYWGQGTLVTVLEGTGGSGGTGSGT GTSELTQSPSSLSASVGDRVTITCRASQSISSYLNWYQQK PGKAPKLLIYAASSLQSGVPSRFSGSGSGTDFTLTISSLQP EDFATYYCQQSYSTPPTFGQGTKVEIKRAAATTTPAPRPP TPAPTIASQPLSLRPEACRPAAGGAVHTRGLDFACDIYIWAP | 196 |
| | LAGTCGVLLLSLVITLYCKRGRKKLLYIFKQPFMRPVQTTQE EDGCSCRFPEEEEGGCELRVKFSRSADAPAYQQGQNQLY NELNLGRREEYDVLDKRRGRDPEMGGKPQRRKNPQEGL YNELQKDKMAEAYSEIGMKGERRRGKGHDGLYQGLST ATKDTYDALHMQALPPR | |
| CAR: GMSCFR signal peptide (amino acids 1-21) - sc02-025 scFv-CD8a Hinge - CD8a TM - 4-1BB ICD - CD3z | MLLVTSLLLCELPHPAFLLIPAQVQLVQSGAEVKKPGAS VKVSCKASGYTFTSYYMHWVRQAPGQGLEWMGIINPSG GGTSYAQKFQGRVTMTRDTSTSTVYMELSSLRSEDTAV YYCARDYYVTYDSWFDSWGQGTLVTVSRGGGGSGGGG SGGGGSSELTQDPAVSVALGQTVRITCQGDSLRSYYASW YQQKPGQAPVLVIYGKNNRPSGIPDRFSGSSSGNTASLTI TGAQAEDEADYYCNSRDSSGNHVVFGGGTKLTVLGAA ATTTPAPRPPTPAPTIASQPLSLRPEACRPAAGGAVHTRGLD FACDIYIWAPLAGTCGVLLLSLVITLYCKRGRKKLLYIFKQPF MRPVQTTQEEDGCSCRFPEEEEGGCELRVKFSRSADAPA YQQGQNQLYNELNLGRREEYDVLDKRRGRDPEMGGKP QRRKNPQEGLYNELQKDKMAEAYSEIGMKGERRRGKG HDGLYQGLSTATKDTYDALHMQALPPR | 197 |
| CAR: GMCSFR signal peptide (amino acids 1-21) - Myc tag (amino acids 22-32) - Sc02-025 scFv-IgG4 hinge - CD28 TM - 41BB - CD3z | MLLLVTSLLLCELPHPAFLLIP EQKLISEEDLAQVQLVQSGAEVKKPGASVKVSCKASGYTFT SYYMHWVRQAPGQGLEWMGIINPSGGGTSYAQKFQGRVTM TRDTSTSTVYMELSSLRSEDTAVYYCARDYYVTYDSWFDSWG QGTLVTVSRGGGGSGGGGSGGGGSSELTQDPAVSVALGQT VRITCQGDSLRSYYASWYQQKPGQAPVLVIYGKNNRPSGIPD RFSGSSSGNTASLTITGAQAEDEADYYCNSRDSSGNHVVFGG GTKLTVLGAAAESKYGPPCPPCPMFWVLVVVGGVLACYS LLVTVAFIIFWVKRGRKKLLYIFKQPFMRPVQTTQEEDG CSCRFPEEEEGGCELRVKFSRSADAPAYQQGQNQLYNEL NLGRREEYDVLDKRRGRDPEMGGKPRRKNPQEGLYNEL QKDKMAEAYSEIGMKGERRRGKGHDGLYQGLSTATKD TYDALHMQALPPR | 198 |
| CAR: IgK signal peptide (amino acids 1-20) - Myc tag (amino acids 21-30) - Sc02-025 scFv - IgG4 hinge - CD28 TM - 41BB - CD3z | MVLQTQVFISLLLWISGAYG EQKLISEEDLAQVQLVQSGAEVKKPGASVKVSCKASGYTFT SYYMHWVRQAPGQGLEWMGIINPSGGGTSYAQKFQGRVTM TRDTSTSTVYMELSSLRSEDTAVYYCARDYYVTYDSWFDSWG QGTLVTVSRGGGGSGGGGSGGGGSSELTQDPAVSVALGQT VRITCQGDSLRSYYASWYQQKPGQAPVLVIYGKNNRPSGIPD RFSGSSSGNTASLTITGAQAEDEADYYCNSRDSSGNHVVFGG GTKLTVLGAAAESKYGPPCPPCPMFWVLVVVGGVLACYS LLVTVAFIIFWVKRGRKKLLYIFKQPFMRPVQTTQEEDG CSCRFPEEEEGGCELRVKFSRSADAPAYQQGQNQLYNEL NLGRREEYDVLDKRRGRDPEMGGKPRRKNPQEGLYNEL QKDKMAEAYSEIGMKGERRRGKGHDGLYQGLSTATKD TYDALHMQALPPR | 199 |
| CAR: CD8a signal peptide (amino acids 1-18) - Myc tag (amino acids 19-28) - Sc02-025 scFv - IgG4 hinge - CD28 TM - 41BB - CD3z | MALPVTALLLPLALLLHA EQKLISEEDLAQVQLVQSGAEVKKPGASVKVSCKASGYTFT SYYMHWVRQAPGQGLEWMGIINPSGGGTSYAQKFQGRVTM TRDTSTSTVYMELSSLRSEDTAVYYCARDYYVTYDSWFDSWG QGTLVTVSRGGGGSGGGGSGGGGSSELTQDPAVSVALGQT VRITCQGDSLRSYYASWYQQKPGQAPVLVIYGKNNRPSGIPD RFSGSSSGNTASLTITGAQAEDEADYYCNSRDSSGNHVVFGG GTKLTVLGAAAESKYGPPCPPCPMFWVLVVVGGVLACYS LLVTVAFIIFWVKRGRKKLLYIFKQPFMRPVQTTQEEDG | 200 |
| | CSCRFPEEEEGGCELRVKFSRSADAPAYQQGQNQLYNEL NLGRREEYDVLDKRRGRDPEMGGKPRRKNPQEGLYNEL QKDKMAEAYSEIGMKGERRRGKGHDGLYQGLSTATKD TYDALHMQALPPR | |
| CAR: IL2 signal peptide (amino acids 1-20) - Myc tag (amino acids 21-30) - Sc02-025 scFv - IgG4 hinge - CD28 TM - 41BB - CD3z | MYRMQLLSCIALSLALVTNS EQKLISEEDLAQVQLVQSGAEVKKPGASVKVSCKASGYTFT SYYMHWVRQAPGQGLEWMGIINPSGGGTSYAQKFQGRVTM TRDTSTSTVYMELSSLRSEDTAVYYCARDYYVTYDSWFDSWG QGTLVTVSRGGGGSGGGGSGGGGSSELTQDPAVSVALGQT VRITCQGDSLRSYYASWYQQKPGQAPVLVIYGKNNRPSGIPD RFSGSSSGNTASLTITGAQAEDEADYYCNSRDSSGNHVVFGG GTKLTVLGAAAESKYGPPCPPCPMFWVLVVVGGVLACYS LLVTVAFIIFWVKRGRKKLLYIFKQPFMRPVQTTQEEDG CSCRFPEEEEGGCELRVKFSRSADAPAYQQGQNQLYNEL NLGRREEYDVLDKRRGRDPEMGGKPRRKNPQEGLYNEL QKDKMAEAYSEIGMKGERRRGKGHDGLYQGLSTATKD TYDALHMQALPPR | 201 |
| CAR: GMSCFR Signal peptide (amino acids 1-22) - Myc tag - Sc025 scFv - IgG4 hinge - CD28 TM - 41BB - CD3z | MLLLVTSLLLCELPHPAFLLIPEQKLISEEDLAQVQLVQS GAEVKKPGASVKVSCKASGYTFTSYYMHWVRQAPGQG LEWMGIINPSGGGTSYAQKFQGRVTMTRDTSTSTVYME LSSLRSEDTAVYYCARDYYVTYDSWFDSWGQGTLVTVS RGGGGSGGGGSGGGGSSELTQDPAVSVALGQTVRITCQ GDSLRSYYASWYQQKPGQAPVLVIYGKNNRPSGIPDRFS GSSSGNTASLTITGAQAEDEADYYCNSRDSSGNHVVFGG GTKLTVLGAAAESKYGPPCPPCPMFWVLVVVGGVLACY SLLVTVAFIIFWVKRGRKKLLYIFKQPFMRPVQTTQEEDG CSCRFPEEEEGGCELRVKFSRSADAPAYQQGQNQLYNEL NLGRREEYDVLDKRRGRDPEMGGKPRRKNPQEGLYNEL QKDKMAEAYSEIGMKGERRRGKGHDGLYQGLSTATKD TYDALHMQALPPR | 202 |
| pCTX 831 CAR | sc02-002 VL-VH W linker scFv: | 203 |
| | SELDIQMTQSPPTLSLSPGERATLSCRASQSVSSTYLTWY QQRPGQAPRLLIYGASSRATGIPDRFSGSGSGTDFTLTISR LEPEDVAVYYCQQGSAFPPTFGQGTKVEIKRAAAGSTSG SGKPGSGEGSTKGAEVQLVESGGGVVQPGRSLRLSCAAS GFTFSDYTMSWVRQAPGKGLEWVAVISYDGSNKYYAD SVKGRFTISRDNSKNTLYLQMNSLRAEDTAVYYCAKDR GSAQGYPLDYWGQGTLVTV | |
| | IgG4 short hinge: | |
| | ESKYGPPCPPCP | |
| | CD28 transmembrane: | |
| | MFWVLVVVGGVLACYSLLVTVAFIIFWV 4-1BB: | |
| | KRGRKKLLYIFKQPFMRPVQTTQEEDGCSCRFPEEEEGG CEL | |
| | CD3z: | |
| | RVKFSRSADAPAYQQGQNQLYNELNLGRREEYDVLDKR RGRDPEMGGKPRRKNPQEGLYNELQKDKMAEAYSEIG MKGERRRGKGHDGLYQGLSTATKDTYDALHMQALPPR | |
| pCTX 832 CAR | sc02-002 VH-VL W linker scFv: | 204 |
| | AEVQLVESGGGVVQPGRSLRLSCAASGFTFSDYTMSWV RQAPGKGLEWVAVISYDGSNKYYADSVKGRFTISRDNS KNTLYLQMNSLRAEDTAVYYCAKDRGSAQGYPLDYWG QGTLVTVGSTSGSGKPGSGEGSTKGSELDIQMTQSPPTLS LSPGERATLSCRASQSVSSTYLTWYQQRPGQAPRLLIYG | |
| | ASSRATGIPDRFSGSGSGTDFTLTISRLEPEDVAVYYCQQ GSAFPPTFGQGTKVEIKRAAA | |
| | IgG4 short hinge: | |
| | ESKYGPPCPPCP | |
| | CD28 transmembrane: | |
| | MFWVLVVVGGVLACYSLLVTVAFIIFWV | |
| | 4-1BB: | |
| | KRGRKKLLYIFKQPFMRPVQTTQEEDGCSCRFPEEEEGG CEL | |
| | CD3z: | |
| | RVKFSRSADAPAYQQGQNQLYNELNLGRREEYDVLDKR RGRDPEMGGKPRRKNPQEGLYNELQKDKMAEAYSEIG MKGERRRGKGHDGLYQGLSTATKDTYDALHMQALPPR | |
| pCTX 833 CAR | sc02-003 VL-VH W linker scFv: | 205 |
| | SELTQSPSSLSASVGDRVTITCRASQSISSYLNWYQQKPG KAPKLLIYAASSLQSGVPSRFSGSGSGTDFTLTISSLQPED FATYYCQQSYSTPPTFGQGTKVEIKRAAAGSTSGSGKPG SGEGSTKGAEVQLVESGGGLVQPGGSLRLPCAASGFTFS NYVMHWVRQAPGKGLEWVAVISYDGSNKYYADSVKG RFTISRDNSKNTLYLQMNSLRAEDTAVYYCAKSHSNMSF DYWGQGTLVTV | |
| | IgG4 short hinge: | |
| | ESKYGPPCPPCP | |
| | CD28 transmembrane: | |
| | MFWVLVVVGGVLACYSLLVTVAFIIFWV | |
| | 4-1BB: | |
| | KRGRKKLLYIFKQPFMRPVQTTQEEDGCSCRFPEEEEGG CEL | |
| | CD3z: | |
| | RVKFSRSADAPAYQQGQNQLYNELNLGRREEYDVLDKR RGRDPEMGGKPRRKNPQEGLYNELQKDKMAEAYSEIG MKGERRRGKGHDGLYQGLSTATKDTYDALHMQALPPR | |
| pCTX 834 CAR | sc02-003 VH-VL W linker scFv: | 206 |
| | AEVQLVESGGGLVQPGGSLRLPCAASGFTFSNYVMHWV RQAPGKGLEWVAVISYDGSNKYYADSVKGRFTISRDNS KNTLYLQMNSLRAEDTAVYYCAKSHSNMSFDYWGQGT LVTVGSTSGSGKPGSGEGSTKGSELTQSPSSLSASVGDRV TITCRASQSISSYLNWYQQKPGKAPKLLIYAASSLQSGVP SRFSGSGSGTDFTLTISSLQPEDFATYYCQQSYSTPPTFGQ GTKVEIKRAAA | |
| | IgG4 short hinge: | |
| | ESKYGPPCPPCP | |
| | CD28 transmembrane: | |
| | MFWVLVVVGGVLACYSLLVTVAFIIFWV | |
| | 4-1BB: | |
| | KRGRKKLLYIFKQPFMRPVQTTQEEDGCSCRFPEEEEGG CEL | |
| | CD3z: | |
| | RVKFSRSADAPAYQQGQNQLYNELNLGRREEYDVLDKR RGRDPEMGGKPRRKNPQEGLYNELQKDKMAEAYSEIG MKGERRRGKGHDGLYQGLSTATKDTYDALHMQALPPR | |
| pCTX 835 CAR | sc02-004 VL-VH W linker scFv: | 207 |
| | SELTQSPSSLSASVGDRVTITCRASQSISSYLNWYQQKPG KAPKLLIYAASSLQSGVPSRFSGSGSGTDFTLTISSLQPED FATYYCQQSYSTPPTFGQGTKVEIKRAAAGSTSGSGKPG SGEGSTKGAEVQLVESGGGLVQPGGSLRLSCAASGFTFS GYLMSWVRQAPGKGLEWVAVISYDGSNKYYADSVKGR | |
| | FTISRDNSKNTLYLQMDSLRAEDTAVYYCARARRDTNLF DYWGQGTLVTV | |
| | IgG4 short hinge: | |
| | ESKYGPPCPPCP | |
| | CD28 transmembrane: | |
| | MFWVLVVVGGVLACYSLLVTVAFIIFWV | |
| | 4-1BB: | |
| | KRGRKKLLYIFKQPFMRPVQTTQEEDGCSCRFPEEEEGG CEL | |
| | CD3z: | |
| | RVKFSRSADAPAYQQGQNQLYNELNLGRREEYDVLDKR RGRDPEMGGKPRRKNPQEGLYNELQKDKMAEAYSEIG MKGERRRGKGHDGLYQGLSTATKDTYDALHMQALPPR | |
| pCTX 836 CAR | sc02-004 VH-VL W linker scFv: | 208 |
| | AEVQLVESGGGLVQPGGSLRLSCAASGFTFSGYLMSWV RQAPGKGLEWVAVISYDGSNKYYADSVKGRFTISRDNS KNTLYLQMDSLRAEDTAVYYCARARRDTNLFDYWGQG TLVTVGSTSGSGKPGSGEGSTKGSELTQSPSSLSASVGDR VTITCRASQSISSYLNWYQQKPGKAPKLLIYAASSLQSGV PSRFSGSGSGTDFTLTISSLQPEDFATYYCQQSYSTPPTFG QGTKVEIKRAAA | |
| | IgG4 short hinge: | |
| | ESKYGPPCPPCP | |
| | CD28 transmembrane: | |
| | MFWVLVVVGGVLACYSLLVTVAFIIFWV | |
| | 4-1BB: | |
| | KRGRKKLLYIFKQPFMRPVQTTQEEDGCSCRFPEEEEGG CEL | |
| | CD3z: | |
| | RVKFSRSADAPAYQQGQNQLYNELNLGRREEYDVLDKR RGRDPEMGGKPRRKNPQEGLYNELQKDKMAEAYSEIG MKGERRRGKGHDGLYQGLSTATKDTYDALHMQALPPR | |
| pCTX 837 CAR | sc02-024 VL-VH W linker scFv: | 209 |
| | SEIELTQSPSSLSASVGDRVTITCRASQSISSYLNWYQQKP GKAPKLLIYAASSLQSGVPSRFSGSGSGTDFTLTISSLQPE DFATYYCQQSYSTPPTFGQGTKVEIKRAAAGSTSGSGKP GSGEGSTKGAQVQLVQSGPEVKKPGASVKVSCKASGYT FTSYYMHWVRQAPGQGLEWMGIINPSGGSTSYAQKFQG RVTMTRDTSTSTVYMELSSLRSEDAAVYYCARDYVRTS HSGFDYWGQGTLVTV | |
| | IgG4 short hinge: | |
| | ESKYGPPCPPCP | |
| | CD28 transmembrane: | |
| | MFWVLVVVGGVLACYSLLVTVAFIIFWV | |
| | 4-1BB: | |
| | KRGRKKLLYIFKQPFMRPVQTTQEEDGCSCRFPEEEEGG CEL | |
| | CD3z: | |
| | RVKFSRSADAPAYQQGQNQLYNELNLGRREEYDVLDKR RGRDPEMGGKPRRKNPQEGLYNELQKDKMAEAYSEIG MKGERRRGKGHDGLYQGLSTATKDTYDALHMQALPPR | |
| pCTX 838 CAR | sc02-024 VH-VL W linker scFv: | 210 |
| | AQVQLVQSGPEVKKPGASVKVSCKASGYTFTSYYMHW VRQAPGQGLEWMGIINPSGGSTSYAQKFQGRVTMTRDT STSTVYMELSSLRSEDAAVYYCARDYVRTSHSGFDYWG QGTLVTVGSTSGSGKPGSGEGSTKGSEIELTQSPSSLSAS VGDRVTITCRASQSISSYLNWYQQKPGKAPKLLIYAASSL | |
| | QSGVPSRFSGSGSGTDFTLTISSLQPEDFATYYCQQSYSTP PTFGQGTKVEIKRAAA | |
| | IgG4 short hinge: | |
| | ESKYGPPCPPCP | |
| | CD28 transmembrane: | |
| | MFWVLVVVGGVLACYSLLVTVAFIIFWV | |
| | 4-1BB: | |
| | KRGRKKLLYIFKQPFMRPVQTTQEEDGCSCRFPEEEEGG CEL | |
| | CD3z: | |
| | RVKFSRSADAPAYQQGQNQLYNELNLGRREEYDVLDKR RGRDPEMGGKPRRKNPQEGLYNELQKDKMAEAYSEIG MKGERRRGKGHDGLYQGLSTATKDTYDALHMQALPPR | |
| pCTX 839 CAR | sc02-025 VL-VH W linker scFv: | 211 |
| | SELTQDPAVSVALGQTVRITCQGDSLRSYYASWYQQKP GQAPVLVIYGKNNRPSGIPDRFSGSSSGNTASLTITGAQA EDEADYYCNSRDSSGNHVVFGGGTKLTVLGAAAGSTSG SGKPGSGEGSTKGAQVQLVQSGAEVKKPGASVKVSCKA SGYTFTSYYMHWVRQAPGQGLEWMGIINPSGGGTSYAQ KFQGRVTMTRDTSTSTVYMELSSLRSEDTAVYYCARDY YVTYDSWFDSWGQGTLVTVS | |
| | IgG4 short hinge: | |
| | ESKYGPPCPPCP | |
| | CD28 transmembrane: | |
| | MFWVLVVVGGVLACYSLLVTVAFIIFWV | |
| | 4-1BB: | |
| | KRGRKKLLYIFKQPFMRPVQTTQEEDGCSCRFPEEEEGG CEL | |
| | CD3z: | |
| | RVKFSRSADAPAYQQGQNQLYNELNLGRREEYDVLDKR RGRDPEMGGKPRRKNPQEGLYNELQKDKMAEAYSEIG MKGERRRGKGHDGLYQGLSTATKDTYDALHMQALPPR | |
| pCTX 840 CAR | sc02-025 VH-VL W linker scFv: | 212 |
| | AQVQLVQSGAEVKKPGASVKVSCKASGYTFTSYYMHW VRQAPGQGLEWMGIINPSGGGTSYAQKFQGRVTMTRDT STSTVYMELSSLRSEDTAVYYCARDYYVTYDSWFDSWG QGTLVTVSGSTSGSGKPGSGEGSTKGSELTQDPAVSVAL GQTVRITCQGDSLRSYYASWYQQKPGQAPVLVIYGKNN RPSGIPDRFSGSSSGNTASLTITGAQAEDEADYYCNSRDS SGNHVVFGGGTKLTVLGAAA | |
| | IgG4 short hinge: | |
| | ESKYGPPCPPCP | |
| | CD28 transmembrane: | |
| | MFWVLVVVGGVLACYSLLVTVAFIIFWV | |
| | 4-1BB: | |
| | KRGRKKLLYIFKQPFMRPVQTTQEEDGCSCRFPEEEEGG CEL | |
| | CD3z: | |
| | RVKFSRSADAPAYQQGQNQLYNELNLGRREEYDVLDKR RGRDPEMGGKPRRKNPQEGLYNELQKDKMAEAYSEIG MKGERRRGKGHDGLYQGLSTATKDTYDALHMQALPPR | |
| pCTX 841 CAR | sc02-041 VL-VH W linker scFv: | 213 |
| | SELTQSPSSLSASVGDRVTITCRASQSISSYLNWYQQKPG KAPKLLIYAASSLQSGVPSRFSGSGSGTDFTLTISSLQPED FATYYCQQSYSTPPTFGQGTKVEIKRAAAGSTSGSGKPG SGEGSTKGAEVQLVESGGGLVQPGGSLRLPCAASGFTFS NYVMHWVRQAPGKGLEWVAVISYDGSNKYYADSVKG | |
| | RFTISRDNSKNTLYLQMNSLRAEDTAVYYCAKSHSNMA FDYWGQGTLVTV | |
| | IgG4 short hinge: | |
| | ESKYGPPCPPCP | |
| | CD28 transmembrane: | |
| | MFWVLVVVGGVLACYSLLVTVAFIIFWV | |
| | 4-1BB: | |
| | KRGRKKLLYIFKQPFMRPVQTTQEEDGCSCRFPEEEEGG CEL | |
| | CD3z: | |
| | RVKFSRSADAPAYQQGQNQLYNELNLGRREEYDVLDKR RGRDPEMGGKPRRKNPQEGLYNELQKDKMAEAYSEIG MKGERRRGKGHDGLYQGLSTATKDTYDALHMQALPPR | |
| pCTX 842 CAR | sc02-041 VH-VL W linker scFv: | 214 |
| | AEVQLVESGGGLVQPGGSLRLPCAASGFTFSNYVMHWV RQAPGKGLEWVAVISYDGSNKYYADSVKGRFTISRDNS KNTLYLQMNSLRAEDTAVYYCAKSHSNMAFDYWGQGT LVTVGSTSGSGKPGSGEGSTKGSELTQSPSSLSASVGDRV TITCRASQSISSYLNWYQQKPGKAPKLLIYAASSLQSGVP SRFSGSGSGTDFTLTISSLQPEDFATYYCQQSYSTPPTFGQ GTKVEIKRAAA | |
| | IgG4 short hinge: | |
| | ESKYGPPCPPCP | |
| | CD28 transmembrane: | |
| | MFWVLVVVGGVLACYSLLVTVAFIIFWV | |
| | 4-1BB: | |
| | KRGRKKLLYIFKQPFMRPVQTTQEEDGCSCRFPEEEEGG CEL | |
| | CD3z: | |
| | RVKFSRSADAPAYQQGQNQLYNELNLGRREEYDVLDKR RGRDPEMGGKPRRKNPQEGLYNELQKDKMAEAYSEIG MKGERRRGKGHDGLYQGLSTATKDTYDALHMQALPPR | |
| pCTX 843 CAR | sc02-057 VL-VH W linker scFv: | 215 |
| | SEIELTQSPSSLSASVGDRVTITCRASQSISSYLNWYQQKP GKAPKLLIYAASSLQSGVPSRFSGSGSGTDFTLTISSLQPE DFATYYCQQSYSTPPTFGQGTKVEIKRAAAGSTSGSGKP GSGEGSTKGAQVQLVQSGAEVKKPGASVKVSCKASGYT FTSYYMHWVRQAPGQGLEWMGIINPSGGSTSYAQKFQG RVTMTRDTSTSTVYMELSSLRSEDTAVYYCARDYQLSM TSGFDYWGQGTLVTV | |
| | IgG4 short hinge: | |
| | ESKYGPPCPPCP | |
| | CD28 transmembrane: | |
| | MFWVLVVVGGVLACYSLLVTVAFIIFWV | |
| | 4-1BB: | |
| | KRGRKKLLYIFKQPFMRPVQTTQEEDGCSCRFPEEEEGG CEL | |
| | CD3z: | |
| | RVKFSRSADAPAYQQGQNQLYNELNLGRREEYDVLDKR RGRDPEMGGKPRRKNPQEGLYNELQKDKMAEAYSEIG MKGERRRGKGHDGLYQGLSTATKDTYDALHMQALPPR | |
| pCTX 844 CAR | sc02-057 VH-VL W linker scFv: | 216 |
| | AQVQLVQSGAEVKKPGASVKVSCKASGYTFTSYYMHW VRQAPGQGLEWMGIINPSGGSTSYAQKFQGRVTMTRDT STSTVYMELSSLRSEDTAVYYCARDYQLSMTSGFDYWG QGTLVTVGSTSGSGKPGSGEGSTKGSEIELTQSPSSLSAS VGDRVTITCRASQSISSYLNWYQQKPGKAPKLLIYAASSL | |
| | QSGVPSRFSGSGSGTDFTLTISSLQPEDFATYYCQQSYSTP PTFGQGTKVEIKRAAA | |
| | IgG4 short hinge: | |
| | ESKYGPPCPPCP | |
| | CD28 transmembrane: | |
| | MFWVLVVVGGVLACYSLLVTVAFIIFWV | |
| | 4-1BB: | |
| | KRGRKKLLYIFKQPFMRPVQTTQEEDGCSCRFPEEEEGG CEL | |
| | CD3z: | |
| | RVKFSRSADAPAYQQGQNQLYNELNLGRREEYDVLDKR RGRDPEMGGKPRRKNPQEGLYNELQKDKMAEAYSEIG MKGERRRGKGHDGLYQGLSTATKDTYDALHMQALPPR | |
| pCTX 845 CAR | sc02-132 VL-VH W linker scFv: | 217 |
| | SELTQSPSSLSASVGDRVTITCRASQSISSYLNWYQQKPG KAPKLLIYAASSLQSGVPSRFSGSGSGTDFTLTISSLQPED FATYYCQQSYSTPPTFGQGTKVEIKRAAAGSTSGSGKPG SGEGSTKGAEVQLVESGGGLVQPGGSLRLPCAASGFTFS NYVMHWVRQAPGKGLEWVAVISYDGSNKYYADSVKG RFTISRDNSKNTLYLQMNSLRAEDTAVYYCVKSHSNMA FDYWGQGTLVTV | |
| | IgG4 short hinge: | |
| | ESKYGPPCPPCP | |
| | CD28 transmembrane: | |
| | MFWVLVVVGGVLACYSLLVTVAFIIFWV | |
| | 4-1BB: | |
| | KRGRKKLLYIFKQPFMRPVQTTQEEDGCSCRFPEEEEGG CEL | |
| | CD3z: | |
| | RVKFSRSADAPAYQQGQNQLYNELNLGRREEYDVLDKR RGRDPEMGGKPRRKNPQEGLYNELQKDKMAEAYSEIG MKGERRRGKGHDGLYQGLSTATKDTYDALHMQALPPR | |
| pCTX 846 CAR | sc02-132 VH-VL W linker scFv: | 218 |
| | AEVQLVESGGGLVQPGGSLRLPCAASGFTFSNYVMHWV RQAPGKGLEWVAVISYDGSNKYYADSVKGRFTISRDNS KNTLYLQMNSLRAEDTAVYYCVKSHSNMAFDYWGQGT LVTVGSTSGSGKPGSGEGSTKGSELTQSPSSLSASVGDRV TITCRASQSISSYLNWYQQKPGKAPKLLIYAASSLQSGVP SRFSGSGSGTDFTLTISSLQPEDFATYYCQQSYSTPPTFGQ GTKVEIKRAAA | |
| | IgG4 short hinge: | |
| | ESKYGPPCPPCP | |
| | CD28 transmembrane: | |
| | MFWVLVVVGGVLACYSLLVTVAFIIFWV | |
| | 4-1BB: | |
| | KRGRKKLLYIFKQPFMRPVQTTQEEDGCSCRFPEEEEGG CEL | |
| | CD3z: | |
| | RVKFSRSADAPAYQQGQNQLYNELNLGRREEYDVLDKR RGRDPEMGGKPRRKNPQEGLYNELQKDKMAEAYSEIG MKGERRRGKGHDGLYQGLSTATKDTYDALHMQALPPR | |
| pCTX 849 CAR | sc02-002 VL-VH P 002 linker scFv: | 219 |
| | SELDIQMTQSPPTLSLSPGERATLSCRASQSVSSTYLTWY QQRPGQAPRLLIYGASSRATGIPDRFSGSGSGTDFTLTISR LEPEDVAVYYCQQGSAFPPTFGQGTKVEIKRAAALEGTG GSGGTGSGTGT | |
| | AEVQLVESGGGVVQPGRSLRLSCAASGFTFSDYTMSWV RQAPGKGLEWVAVISYDGSNKYYADSVKGRFTISRDNS KNTLYLQMNSLRAEDTAVYYCAKDRGSAQGYPLDYWG QGTLVTV | |
| | IgG4 short hinge: | |
| | ESKYGPPCPPCP | |
| | CD28 transmembrane: | |
| | MFWVLVVVGGVLACYSLLVTVAFIIFWV | |
| | 4-1BB: | |
| | KRGRKKLLYIFKQPFMRPVQTTQEEDGCSCRFPEEEEGG CEL | |
| | CD3z: | |
| | RVKFSRSADAPAYQQGQNQLYNELNLGRREEYDVLDKR RGRDPEMGGKPRRKNPQEGLYNELQKDKMAEAYSEIG MKGERRRGKGHDGLYQGLSTATKDTYDALHMQALPPR | |
| pCTX 850 CAR | sc02-002 VH-VL P 002 linker scFv: | 220 |
| | AEVQLVESGGGVVQPGRSLRLSCAASGFTFSDYTMSWV RQAPGKGLEWVAVISYDGSNKYYADSVKGRFTISRDNS KNTLYLQMNSLRAEDTAVYYCAKDRGSAQGYPLDYWG QGTLVTVLEGTGGSGGTGSGTGTSELDIQMTQSPPTLSLS PGERATLSCRASQSVSSTYLTWYQQRPGQAPRLLIYGAS SRATGIPDRFSGSGSGTDFTLTISRLEPEDVAVYYCQQGS AFPPTFGQGTKVEIKRAAA | |
| | IgG4 short hinge: | |
| | ESKYGPPCPPCP | |
| | CD28 transmembrane: | |
| | MFWVLVVVGGVLACYSLLVTVAFIIFWV | |
| | 4-1BB: | |
| | KRGRKKLLYIFKQPFMRPVQTTQEEDGCSCRFPEEEEGG CEL | |
| | CD3z: | |
| | RVKFSRSADAPAYQQGQNQLYNELNLGRREEYDVLDKR RGRDPEMGGKPRRKNPQEGLYNELQKDKMAEAYSEIG MKGERRRGKGHDGLYQGLSTATKDTYDALHMQALPPR | |
| pCTX 851 CAR | sc02-003 VL-VH P 002 linker scFv: | 221 |
| | SELTQSPSSLSASVGDRVTITCRASQSISSYLNWYQQKPG KAPKLLIYAASSLQSGVPSRFSGSGSGTDFTLTISSLQPED FATYYCQQSYSTPPTFGQGTKVEIKRAAALEGTGGSGGT GSGTGTAEVQLVESGGGLVQPGGSLRLPCAASGFTFSNY VMHWVRQAPGKGLEWVAVISYDGSNKYYADSVKGRFT ISRDNSKNTLYLQMNSLRAEDTAVYYCAKSHSNMSFDY WGQGTLVTV | |
| | IgG4 short hinge: | |
| | ESKYGPPCPPCP | |
| | CD28 transmembrane: | |
| | MFWVLVVVGGVLACYSLLVTVAFIIFWV | |
| | 4-1BB: | |
| | KRGRKKLLYIFKQPFMRPVQTTQEEDGCSCRFPEEEEGG CEL | |
| | CD3z: | |
| | RVKFSRSADAPAYQQGQNQLYNELNLGRREEYDVLDKR RGRDPEMGGKPRRKNPQEGLYNELQKDKMAEAYSEIG MKGERRRGKGHDGLYQGLSTATKDTYDALHMQALPPR | |
| pCTX 852 CAR | sc02-003 VH-VL P 002 linker scFv: | 222 |
| | AEVQLVESGGGLVQPGGSLRLPCAASGFTFSNYVMHWV RQAPGKGLEWVAVISYDGSNKYYADSVKGRFTISRDNS KNTLYLQMNSLRAEDTAVYYCAKSHSNMSFDYWGQGT | |
| | LVTVLEGTGGSGGTGSGTGTSELTQSPSSLSASVGDRVTI TCRASQSISSYLNWYQQKPGKAPKLLIYAASSLQSGVPSR FSGSGSGTDFTLTISSLQPEDFATYYCQQSYSTPPTFGQGT KVEIKRAAA | |
| | IgG4 short hinge: | |
| | ESKYGPPCPPCP | |
| | CD28 transmembrane: | |
| | MFWVLVVVGGVLACYSLLVTVAFIIFWV | |
| | 4-1BB: | |
| | KRGRKKLLYIFKQPFMRPVQTTQEEDGCSCRFPEEEEGG CEL | |
| | CD3z: | |
| | RVKFSRSADAPAYQQGQNQLYNELNLGRREEYDVLDKR RGRDPEMGGKPRRKNPQEGLYNELQKDKMAEAYSEIG MKGERRRGKGHDGLYQGLSTATKDTYDALHMQALPPR | |
| pCTX 853 CAR | sc02-004 VL-VH P 002 linker scFv: | 223 |
| | SELTQSPSSLSASVGDRVTITCRASQSISSYLNWYQQKPG KAPKLLIYAASSLQSGVPSRFSGSGSGTDFTLTISSLQPED FATYYCQQSYSTPPTFGQGTKVEIKRAAALEGTGGSGGT GSGTGTAEVQLVESGGGLVQPGGSLRLSCAASGFTFSGY LMSWVRQAPGKGLEWVAVISYDGSNKYYADSVKGRFTI SRDNSKNTLYLQMDSLRAEDTAVYYCARARRDTNLFDY WGQGTLVTV | |
| | IgG4 short hinge: | |
| | ESKYGPPCPPCP | |
| | CD28 transmembrane: | |
| | MFWVLVVVGGVLACYSLLVTVAFIIFWV | |
| | 4-1BB: | |
| | KRGRKKLLYIFKQPFMRPVQTTQEEDGCSCRFPEEEEGG CEL | |
| | CD3z: | |
| | RVKFSRSADAPAYQQGQNQLYNELNLGRREEYDVLDKR RGRDPEMGGKPRRKNPQEGLYNELQKDKMAEAYSEIG MKGERRRGKGHDGLYQGLSTATKDTYDALHMQALPPR | |
| pCTX 854 CAR | sc02-004 VH-VL P 002 linker scFv: | 224 |
| | AEVQLVESGGGLVQPGGSLRLSCAASGFTFSGYLMSWV RQAPGKGLEWVAVISYDGSNKYYADSVKGRFTISRDNS KNTLYLQMDSLRAEDTAVYYCARARRDTNLFDYWGQG TLVTVLEGTGGSGGTGSGTGTSELTQSPSSLSASVGDRVT ITCRASQSISSYLNWYQQKPGKAPKLLIYAASSLQSGVPS RFSGSGSGTDFTLTISSLQPEDFATYYCQQSYSTPPTFGQG TKVEIKRAAA | |
| | IgG4 short hinge: | |
| | ESKYGPPCPPCP | |
| | CD28 transmembrane: | |
| | MFWVLVVVGGVLACYSLLVTVAFIIFWV | |
| | 4-1BB: | |
| | KRGRKKLLYIFKQPFMRPVQTTQEEDGCSCRFPEEEEGG CEL | |
| | CD3z: | |
| | RVKFSRSADAPAYQQGQNQLYNELNLGRREEYDVLDKR RGRDPEMGGKPRRKNPQEGLYNELQKDKMAEAYSEIG MKGERRRGKGHDGLYQGLSTATKDTYDALHMQALPPR | |
| pCTX 855 CAR | sc02-024 VL-VH P 024 linker scFv: | 225 |
| | SEIELTQSPSSLSASVGDRVTITCRASQSISSYLNWYQQKP GKAPKLLIYAASSLQSGVPSRFSGSGSGTDFTLTISSLQPE DFATYYCQQSYSTPPTFGQGTKVEIKRAAASSGGGGSGG | |
| | GGSGGGGAQVQLVQSGPEVKKPGASVKVSCKASGYTFT SYYMHWVRQAPGQGLEWMGIINPSGGSTSYAQKFQGR VTMTRDTSTSTVYMELSSLRSEDAAVYYCARDYVRTSH SGFDYWGQGTLVTV | |
| | IgG4 short hinge: | |
| | ESKYGPPCPPCP | |
| | CD28 transmembrane: | |
| | MFWVLVVVGGVLACYSLLVTVAFIIFWV | |
| | 4-1BB: | |
| | KRGRKKLLYIFKQPFMRPVQTTQEEDGCSCRFPEEEEGG CEL | |
| | CD3z: | |
| | RVKFSRSADAPAYQQGQNQLYNELNLGRREEYDVLDKR RGRDPEMGGKPRRKNPQEGLYNELQKDKMAEAYSEIG MKGERRRGKGHDGLYQGLSTATKDTYDALHMQALPPR | |
| pCTX 856 CAR | sc02-024 VH-VL P 024 linker scFv: | 226 |
| | AQVQLVQSGPEVKKPGASVKVSCKASGYTFTSYYMHW VRQAPGQGLEWMGIINPSGGSTSYAQKFQGRVTMTRDT STSTVYMELSSLRSEDAAVYYCARDYVRTSHSGFDYWG QGTLVTVSSGGGGSGGGGSGGGGSEIELTQSPSSLSASVG DRVTITCRASQSISSYLNWYQQKPGKAPKLLIYAASSLQS GVPSRFSGSGSGTDFTLTISSLQPEDFATYYCQQSYSTPPT FGQGTKVEIKRAAA | |
| | IgG4 short hinge: | |
| | ESKYGPPCPPCP | |
| | CD28 transmembrane: | |
| | MFWVLVVVGGVLACYSLLVTVAFIIFWV | |
| | 4-1BB: | |
| | KRGRKKLLYIFKQPFMRPVQTTQEEDGCSCRFPEEEEGG CEL | |
| | CD3z: | |
| | RVKFSRSADAPAYQQGQNQLYNELNLGRREEYDVLDKR RGRDPEMGGKPRRKNPQEGLYNELQKDKMAEAYSEIG MKGERRRGKGHDGLYQGLSTATKDTYDALHMQALPPR | |
| pCTX 857 CAR | sc02-025 VL-VH P 025 linker scFv: | 227 |
| | SELTQDPAVSVALGQTVRITCQGDSLRSYYASWYQQKP GQAPVLVIYGKNNRPSGIPDRFSGSSSGNTASLTITGAQA EDEADYYCNSRDSSGNHVVFGGGTKLTVLGAAARGGG GSGGGGSGGGGS AQVQLVQSGAEVKKPGASVKVSCKASGYTFTSYYMHW VRQAPGQGLEWMGIINPSGGGTSYAQKFQGRVTMTRDT STSTVYMELSSLRSEDTAVYYCARDYYVTYDSWFDSWG QGTLVTVS | |
| | IgG4 short hinge: | |
| | ESKYGPPCPPCP | |
| | CD28 transmembrane: | |
| | MFWVLVVVGGVLACYSLLVTVAFIIFWV | |
| | 4-1BB: | |
| | KRGRKKLLYIFKQPFMRPVQTTQEEDGCSCRFPEEEEGG CEL | |
| | CD3z: | |
| | RVKFSRSADAPAYQQGQNQLYNELNLGRREEYDVLDKR RGRDPEMGGKPRRKNPQEGLYNELQKDKMAEAYSEIG MKGERRRGKGHDGLYQGLSTATKDTYDALHMQALPPR | |
| pCTX 858 CAR | sc02-025 VH-VL P 025 linker scFv: | 228 |
| | AQVQLVQSGAEVKKPGASVKVSCKASGYTFTSYYMHW VRQAPGQGLEWMGIINPSGGGTSYAQKFQGRVTMTRDT | |
| | STSTVYMELSSLRSEDTAVYYCARDYYVTYDSWFDSWG QGTLVTVSRGGGGSGGGGSGGGGSSELTQDPAVSVALG QTVRITCQGDSLRSYYASWYQQKPGQAPVLVIYGKNNR PSGIPDRFSGSSSGNTASLTITGAQAEDEADYYCNSRDSS GNHVVFGGGTKLTVLGAAA | |
| | IgG4 short hinge: | |
| | ESKYGPPCPPCP | |
| | CD28 transmembrane: | |
| | MFWVLVVVGGVLACYSLLVTVAFIIFWV | |
| | 4-1BB: | |
| | KRGRKKLLYIFKQPFMRPVQTTQEEDGCSCRFPEEEEGG CEL | |
| | CD3z: | |
| | RVKFSRSADAPAYQQGQNQLYNELNLGRREEYDVLDKR RGRDPEMGGKPRRKNPQEGLYNELQKDKMAEAYSEIG MKGERRRGKGHDGLYQGLSTATKDTYDALHMQALPPR | |
| pCTX 859 CAR | sc02-041 VL-VH P 002 linker scFv: | 229 |
| | SELTQSPSSLSASVGDRVTITCRASQSISSYLNWYQQKPG KAPKLLIYAASSLQSGVPSRFSGSGSGTDFTLTISSLQPED FATYYCQQSYSTPPTFGQGTKVEIKRAAALEGTGGSGGT GSGTGTAEVQLVESGGGLVQPGGSLRLPCAASGFTFSNY VMHWVRQAPGKGLEWVAVISYDGSNKYYADSVKGRFT ISRDNSKNTLYLQMNSLRAEDTAVYYCAKSHSNMAFDY WGQGTLVTV | |
| | IgG4 short hinge: | |
| | ESKYGPPCPPCP | |
| | CD28 transmembrane: | |
| | MFWVLVVVGGVLACYSLLVTVAFIIFWV | |
| | 4-1BB: | |
| | KRGRKKLLYIFKQPFMRPVQTTQEEDGCSCRFPEEEEGG CEL | |
| | CD3z: | |
| | RVKFSRSADAPAYQQGQNQLYNELNLGRREEYDVLDKR RGRDPEMGGKPRRKNPQEGLYNELQKDKMAEAYSEIG MKGERRRGKGHDGLYQGLSTATKDTYDALHMQALPPR | |
| pCTX 860 CAR | sc02-041 VH-VL P 002 linker scFv: | 230 |
| | AEVQLVESGGGLVQPGGSLRLPCAASGFTFSNYVMHWV RQAPGKGLEWVAVISYDGSNKYYADSVKGRFTISRDNS KNTLYLQMNSLRAEDTAVYYCAKSHSNMAFDYWGQGT LVTVLEGTGGSGGTGSGTGTSELTQSPSSLSASVGDRVTI TCRASQSISSYLNWYQQKPGKAPKLLIYAASSLQSGVPSR FSGSGSGTDFTLTISSLQPEDFATYYCQQSYSTPPTFGQGT KVEIKRAAA | |
| | IgG4 short hinge: | |
| | ESKYGPPCPPCP | |
| | CD28 transmembrane: | |
| | MFWVLVVVGGVLACYSLLVTVAFIIFWV | |
| | 4-1BB: | |
| | KRGRKKLLYIFKQPFMRPVQTTQEEDGCSCRFPEEEEGG CEL | |
| | CD3z: | |
| | RVKFSRSADAPAYQQGQNQLYNELNLGRREEYDVLDKR RGRDPEMGGKPRRKNPQEGLYNELQKDKMAEAYSEIG MKGERRRGKGHDGLYQGLSTATKDTYDALHMQALPPR | |
| pCTX 861 CAR | sc02-057 VL-VH P 024 linker scFv: | 231 |
| | SEIELTQSPSSLSASVGDRVTITCRASQSISSYLNWYQQKP GKAPKLLIYAASSLQSGVPSRFSGSGSGTDFTLTISSLQPE | |
| | DFATYYCQQSYSTPPTFGQGTKVEIKRAAASSGGGGSGG GGSGGGGAQVQLVQSGAEVKKPGASVKVSCKASGYTFT SYYMHWVRQAPGQGLEWMGIINPSGGSTSYAQKFQGR VTMTRDTSTSTVYMELSSLRSEDTAVYYCARDYQLSMT SGFDYWGQGTLVTV | |
| | IgG4 short hinge: | |
| | ESKYGPPCPPCP | |
| | CD28 transmembrane: | |
| | MFWVLVVVGGVLACYSLLVTVAFIIFWV | |
| | 4-1BB: | |
| | KRGRKKLLYIFKQPFMRPVQTTQEEDGCSCRFPEEEEGG CEL | |
| | CD3z: | |
| | RVKFSRSADAPAYQQGQNQLYNELNLGRREEYDVLDKR RGRDPEMGGKPRRKNPQEGLYNELQKDKMAEAYSEIG MKGERRRGKGHDGLYQGLSTATKDTYDALHMQALPPR | |
| pCTX 862 CAR | sc02-057 VH-VL P 024 linker scFv: | 232 |
| | AQVQLVQSGAEVKKPGASVKVSCKASGYTFTSYYMHW VRQAPGQGLEWMGIINPSGGSTSYAQKFQGRVTMTRDT STSTVYMELSSLRSEDTAVYYCARDYQLSMTSGFDYWG QGTLVTVSSGGGGSGGGGSGGGGSEIELTQSPSSLSASVG DRVTITCRASQSISSYLNWYQQKPGKAPKLLIYAASSLQS GVPSRFSGSGSGTDFTLTISSLQPEDFATYYCQQSYSTPPT FGQGTKVEIKRAAA | |
| | IgG4 short hinge: | |
| | ESKYGPPCPPCP | |
| | CD28 transmembrane: | |
| | MFWVLVVVGGVLACYSLLVTVAFIIFWV | |
| | 4-1BB: | |
| | KRGRKKLLYIFKQPFMRPVQTTQEEDGCSCRFPEEEEGG CEL | |
| | CD3z: | |
| | RVKFSRSADAPAYQQGQNQLYNELNLGRREEYDVLDKR RGRDPEMGGKPRRKNPQEGLYNELQKDKMAEAYSEIG MKGERRRGKGHDGLYQGLSTATKDTYDALHMQALPPR | |
| pCTX 863 CAR | sc02-132 VL-VH P 002 linker scFv: | 233 |
| | SELTQSPSSLSASVGDRVTITCRASQSISSYLNWYQQKPG KAPKLLIYAASSLQSGVPSRFSGSGSGTDFTLTISSLQPED FATYYCQQSYSTPPTFGQGTKVEIKRAAALEGTGGSGGT GSGTGTAEVQLVESGGGLVQPGGSLRLPCAASGFTFSNY VMHWVRQAPGKGLEWVAVISYDGSNKYYADSVKGRFT ISRDNSKNTLYLQMNSLRAEDTAVYYCVKSHSNMAFDY WGQGTLVTV | |
| | IgG4 short hinge: | |
| | ESKYGPPCPPCP | |
| | CD28 transmembrane: | |
| | MFWVLVVVGGVLACYSLLVTVAFIIFWV | |
| | 4-1BB: | |
| | KRGRKKLLYIFKQPFMRPVQTTQEEDGCSCRFPEEEEGG CEL | |
| | CD3z: | |
| | RVKFSRSADAPAYQQGQNQLYNELNLGRREEYDVLDKR RGRDPEMGGKPRRKNPQEGLYNELQKDKMAEAYSEIG MKGERRRGKGHDGLYQGLSTATKDTYDALHMQALPPR | |
| pCTX 864 CAR | sc02-132 VH-VL P 002 linker scFv: | 234 |
| | AEVQLVESGGGLVQPGGSLRLPCAASGFTFSNYVMHWV RQAPGKGLEWVAVISYDGSNKYYADSVKGRFTISRDNS | |
| | KNTLYLQMNSLRAEDTAVYYCVKSHSNMAFDYWGQGT LVTVLEGTGGSGGTGSGTGTSELTQSPSSLSASVGDRVTI TCRASQSISSYLNWYQQKPGKAPKLLIYAASSLQSGVPSR FSGSGSGTDFTLTISSLQPEDFATYYCQQSYSTPPTFGQGT KVEIKRAAA | |
| | IgG4 short hinge: | |
| | ESKYGPPCPPCP | |
| | CD28 transmembrane: | |
| | MFWVLVVVGGVLACYSLLVTVAFIIFWV | |
| | 4-1BB: | |
| | KRGRKKLLYIFKQPFMRPVQTTQEEDGCSCRFPEEEEGG CEL | |
| | CD3z: | |
| | RVKFSRSADAPAYQQGQNQLYNELNLGRREEYDVLDKR RGRDPEMGGKPRRKNPQEGLYNELQKDKMAEAYSEIG MKGERRRGKGHDGLYQGLSTATKDTYDALHMQALPPR | |
| GMCSFR_Signal Peptide | MLLVTSLLLCELPHPAFLLIP | 235 |
| IgK signal peptide | MVLQTQVFISLLLWISGAYG | 236 |
| CD8a signal peptide | MALPVTALLLPLALLLHA | 237 |
| IL-2 signal peptide | MYRMQLLSCIALSLALVTNS | 238 |
| Myc tag | EQKLISEEDL | 239 |
| GMSCFR Signl peptide | MLLLVTSLLLCELPHPAFLLIP | 240 |

In some embodiments, a CD72 CAR of the present disclosure contains a peptide tag, such as for example a Myc tag (SEQ ID NO:239). In some embodiments, the Myc tag is inserted into the CD72 CAR following the signal peptide but before the first variable region of the scFv binding domain. It is understood that for CAR sequences provided herein that include a Myc tag, the same CAR sequence is contemplated without the Myc tag inserted within the sequence.

Radiation therapy includes external beam radiation therapy (e.g., conventional external beam radiation therapy, stereotactic radiation, 3-dimensional conformal radiation therapy, intensity-modulated radiation therapy, volumetric modulated arc therapy, particle therapy, proton therapy, and auger therapy), brachytherapy, systemic radioisotope therapy, intraoperative radiotherapy, or any combination thereof.

Exemplary antibodies for use in conjunction with the chimeric Tim compositions described herein include rituxmab, pertuzumab, trastuzumab, alemtuzumab, Ibritumomab tiuxetan, Brentuximab vedotin, cetuximab, bevacizumab, abciximab, adalimumab, alefacept, basilizimab, belimumab, bezlotoxumab, canakinumab, certolizumab pegol, daclizumab, denosumab, efalizumab, golimumab, olaratumab, palivizumab, panitumumab, and tocilizumab.

Exemplary inhibitors of immune checkpoint molecules that may be for use in conjunction with the chimeric Tim compositions described herein include checkpoint inhibitors targeting PD-L1, PD-L2, CD80, CD86, B7-H3, B7-H4, HVEM, adenosine, GAL9, VISTA, CEACAM-1, CEACAM-3, CEACAM-5, PVRL2, PD-1, CTLA-4, BTLA, KIR, LAG3, TIM3, A2aR, CD244/2B4, CD160, TIGIT, LAIR-1, PVRIG/CD112R, or any combination thereof. In certain embodiments, an immune checkpoint inhibitor may be an antibody, a peptide, an RNAi agent, or a small molecule. An antibody specific for CTLA-4 may be ipilimumab or tremelimumab. An antibody specific for PD-1 may be pidilizumab, nivolumab, or pembrolizumab. An antibody specific for PD-L1 may be durvalumab, atezolizumab, or avelumab.

Exemplary chemotherapeutics for use in conjunction with the chimeric Tim4 receptor compositions described herein may include an alkylating agent, a platinum based agent, a cytotoxic agent, an inhibitor of chromatin function, a topoisomerase inhibitor, a microtubule inhibiting drug, a DNA damaging agent, an antimetabolite (such as folate antagonists, pyrimidine analogs, purine analogs, and sugar-modified analogs), a DNA synthesis inhibitor, a DNA interactive agent (such as an intercalating agent), and a DNA repair inhibitor.

A chemotherapeutic includes non-specific cytotoxic agents that inhibit mitosis or cell division, as well as molecularly targeted therapy that blocks the growth and spread of cancer cells by targeting specific molecules that are involved in tumor growth, progression, and metastasis (e.g., oncogenes). Exemplary non-specific chemotherapeutics for use in conjunction with the expression cassette compositions described herein may include an alkylating agent, a platinum based agent, a cytotoxic agent, an inhibitor of chromatin function, a topoisomerase inhibitor, a microtubule inhibiting drug, a DNA damaging agent, an antimetabolite (such as folate antagonists, pyrimidine analogs, purine analogs, and sugar-modified analogs), a DNA synthesis inhibitor, a DNA interactive agent (such as an intercalating agent), hypomethylating agent, and a DNA repair inhibitor.

Examples of chemotherapeutic agents considered for use in combination therapies contemplated herein include vemurafenib, dabrafenib, trametinib, cobimetinib, anastrozole (Arimidex^{®}), bicalutamide (Casodex^{®}), bleomycin sulfate (Blenoxane^{®}), busulfan (Myleran^{®}), busulfan injection (Busulfex^{®}), capecitabine (Xeloda^{®}), N4-pentoxycarbonyl-5-deoxy-5-fluorocytidine, carboplatin (Paraplatin^{®}), carmustine (BiCNU^{®}), chlorambucil (Leukeran^{®}), cisplatin (Platinol^{®}), cladribine (Leustatin^{®}), cyclophosphamide (Cytoxan^{®} or Neosar^{®}), cytarabine, cytosine arabinoside (Cytosar-U^{®}), cytarabine liposome injection (DepoCyt^{®}), dacarbazine (DTIC-Dome^{®}), dactinomycin (Actinomycin D, Cosmegan), daunorubicin hydrochloride (Cerubidine^{®}), daunorubicin citrate liposome injection (DaunoXome^{®}), dexamethasone, docetaxel (Taxotere^{®}), doxorubicin hydrochloride (Adriamycin^{®}, Rubex^{®}), etoposide (Vepesid^{®}), fludarabine phosphate (Fludara^{®}), 5-fluorouracil (Adrucil^{®}, Efudex^{®}), flutamide (Eulexin^{®}), tezacitibine, Gemcitabine (difluorodeoxycitidine), hydroxyurea (Hydrea^{®}), Idarubicin (Idamycin^{®}), ifosfamide (IFEX^{®}), irinotecan (Camptosar^{®}), L-asparaginase (ELSPAR^{®}), leucovorin calcium, melphalan (Alkeran^{®}), 6-mercaptopurine (Purinethol^{®}), methotrexate (Folex^{®}), mitoxantrone (Novantrone^{®}), mylotarg, paclitaxel (Taxol^{®}), phoenix (Yttrium90/MX-DTPA), pentostatin, polifeprosan 20 with carmustine implant (Gliadel^{®}),fdabra tamoxifen citrate (Nolvadex^{®}), teniposide (Vumon^{®}), 6-thioguanine, thiotepa, tirapazamine (Tirazone^{®}), topotecan hydrochloride for injection (Hycamptin^{®}), vinblastine (Velban^{®}), vincristine (Oncovin^{®}), ibrutinib, venetoclax, crizotinib, alectinib, brigatinib, ceritinib, and vinorelbine (Navelbine^{®}).

Exemplary alkylating agents for use in combination therapies contemplated herein include nitrogen mustards, ethylenimine derivatives, alkyl sulfonates, nitrosoureas and triazenes): uracil mustard (Aminouracil Mustard^{®}, Chlorethaminacil^{®}, Demethyldopan^{®}, Desmethyldopan^{®}, Haemanthamine^{®}, Nordopan^{®}, Uracil nitrogen Mustard^{®}, Uracillost^{®}, Uracilmostaza^{®}, Uramustin^{®}, Uramustine^{®}), chlormethine (Mustargen^{®}), cyclophosphamide (Cytoxan^{®}, Neosar^{®}, Clafen^{®}, Endoxan^{®}, Procytox^{®}, Revimmune^{™}), ifosfamide (Mitoxana^{®}), melphalan (Alkeran^{®}), Chlorambucil (Leukeran^{®}), pipobroman (Amedel^{®}, Vercyte^{®}), triethylenemelamine (Hemel^{®}, Hexalen^{®}, Hexastat^{®}), triethylenethiophosphoramine, Temozolomide (Temodar^{®}), thiotepa (Thioplex^{®}), busulfan (Busilvex^{®}, Myleran^{®}), carmustine (BiCNU^{®}), lomustine (CeeNU^{®}), streptozocin (Zanosar^{®}), and Dacarbazine (DTIC-Dome^{®}). Additional exemplary alkylating agents for use in combination therapies contemplated herein include, without limitation, Oxaliplatin (Eloxatin^{®}); Temozolomide (Temodar^{®} and Temodal^{®}); Dactinomycin (also known as actinomycin-D, Cosmegen^{®}); Melphalan (also known as L-PAM, L-sarcolysin, and phenylalanine mustard, Alkeran^{®}); Altretamine (also known as hexamethylmelamine (HMM), Hexalen^{®}); Carmustine (BiCNU^{®}); Bendamustine (Treanda^{®}); Busulfan (Busulfex^{®} and Myleran^{®}); Carboplatin (Paraplatin^{®}); Lomustine (also known as CCNU, CeeNU^{®}); Cisplatin (also known as CDDP, Platinol^{®} and Platinol^{®}-AQ); Chlorambucil (Leukeran^{®}); Cyclophosphamide (Cytoxan^{®} and Neosar^{®}); Dacarbazine (also known as DTIC, DIC and imidazole carboxamide, DTIC-Dome^{®}); Altretamine (also known as hexamethylmelamine (HMM), Hexalen^{®}); Ifosfamide (Ifex^{®}); Prednumustine; Procarbazine (Matulane^{®}); Mechlorethamine (also known as nitrogen mustard, mustine and mechloroethamine hydrochloride, Mustargen^{®}); Streptozocin (Zanosar^{®}); Thiotepa (also known as thiophosphoamide, TESPA and TSPA, Thioplex^{®}); Cyclophosphamide (Endoxan^{®}, Cytoxan^{®}, Neosar^{®}, Procytox^{®}, Revimmune^{®}); and Bendamustine HCl (Treanda^{®}).

Exemplary platinum based agents for use in combination therapies contemplated herein include carboplatin, cisplatin, oxaliplatin, nedaplatin, picoplatin, satraplatin, phenanthriplatin, and triplatin tetranitrate.

Exemplary hypomethylating agents for use in combination therapies include azacitidine and decitabine.

Exemplary molecularly targeted inhibitors for use in conjunction with the chimeric Tim4 receptor compositions described herein include small molecules that target molecules involved in cancer cell growth and survival, including for example, receptor tyrosine kinase inhibitors, RAF inhibitors, BCL-2 inhibitors, ABL inhibitors, TRK inhibitors, c-KIT inhibitors, c-MET inhibitors, CDK4/6 inhibitors, FAK inhibitors, FGFR inhibitors, FLT3 inhibitors, IDH1 inhibitors, IDH2 inhibitors, PDGFRA inhibitors, and RET inhibitors

Exemplary molecularly targeted therapy includes hormone antagonists, signal transduction inhibitors, gene expression inhibitors (e.g., translation inhibitors), apoptosis inducers, angiogenesis inhibitors (e.g., a VEGF pathway inhibitor), tyrosine kinase inhibitors (e.g., an EGF/EGFR pathway inhibitor), growth factor inhibitors, GTPase inhibitors, serine/threonine kinase inhibitors, transcription factor inhibitors, inhibitors of driver mutations associated with cancer, B-Raf inhibitors, RAF inhibitors, a MEK inhibitors, mTOR inhibitors, adenosine pathway inhibitors, EGFR inhibitors, PI3K inhibitors, BCL2 inhibitors, VEGFR inhibitors, MET inhibitors, MYC inhibitors, BCR-ABL inhibitors, ABL inhibitors, HER2 inhibitors, H-RAS inhibitors, K-RAS inhibitors, PDGFR inhibitors, ALK inhibitors, ROS1 inhibitors, BTK inhibitors, TRK inhibitors, c-KIT inhibitors, c-MET inhibitors, CDK4/6 inhibitors, FAK inhibitors, FGFR inhibitors, FLT3 inhibitors, IDH1 inhibitors, IDH2 inhibitors, PARP inhibitors, PDGFRA inhibitors, and RET inhibitors. In certain embodiments, use of molecularly targeted therapy comprises administering a molecularly targeted therapy specific for the molecular target to a subject identified as having a tumor that possesses the molecular target (e.g., driver oncogene). In certain embodiments, the molecular target has an activating mutation. In certain embodiments, use of chimeric Tim4 receptor modified cells in combination with a molecularly targeted inhibitor increases the magnitude of anti-tumor response, the durability of anti-tumor response, or both. In certain embodiments, a lower than typical dose of molecularly targeted therapy is used in combination with chimeric Tim4 receptor modified cells.

Exemplary angiogenesis inhibitors include, without limitation A6 (Angstrom Pharmaceuticals), ABT-510 (Abbott Laboratories), ABT-627 (Atrasentan) (Abbott Laboratories/Xinlay), ABT-869 (Abbott Laboratories), Actimid (CC4047, Pomalidomide) (Celgene Corporation), ADGVPEDF.11D (GenVec), ADH-1 (Exherin) (Adherex Technologies), AEE788 (Novartis), AG-013736 (Axitinib) (Pfizer), AG3340 (Prinomastat) (Agouron Pharmaceuticals), AGX1053 (AngioGenex), AGX51 (AngioGenex), ALN-VSP (ALN-VSP O2) (Alnylam Pharmaceuticals), AMG 386 (Amgen), AMG706 (Amgen), Apatinib (YN968D1) (Jiangsu Hengrui Medicine), AP23573 (Ridaforolimus/MK8669) (Ariad Pharmaceuticals), AQ4N (Novavea), ARQ 197 (ArQule), ASA404 (Novartis/Antisoma), Atiprimod (Callisto Pharmaceuticals), ATN-161 (Attenuon), AV-412 (Aveo Pharmaceuticals), AV-951 (Aveo Pharmaceuticals), Avastin (Bevacizumab) (Genentech), AZD2171 (Cediranib/Recentin) (AstraZeneca), BAY 57-9352 (Telatinib) (Bayer), BEZ235 (Novartis), BIBF1120 (Boehringer Ingelheim Pharmaceuticals), BIBW 2992 (Boehringer Ingelheim Pharmaceuticals), BMS-275291 (Bristol-Myers Squibb), BMS-582664 (Brivanib) (Bristol-Myers Squibb), BMS-690514 (Bristol-Myers Squibb), Calcitriol, CCI-779 (Torisel) (Wyeth), CDP-791 (ImClone Systems), Ceflatonin (Homoharringtonine/HHT) (ChemGenex Therapeutics), Celebrex (Celecoxib) (Pfizer), CEP-7055 (Cephalon/Sanofi), CHIR-265 (Chiron Corporation), NGR-TNF, COL-3 (Metastat) (Collagenex Pharmaceuticals), Combretastatin (Oxigene), CP-751,871(Figitumumab) (Pfizer), CP-547,632 (Pfizer), CS-7017 (Daiichi Sankyo Pharma), CT-322 (Angiocept) (Adnexus), Curcumin, Dalteparin (Fragmin) (Pfizer), Disulfiram (Antabuse), E7820 (Eisai Limited), E7080 (Eisai Limited), EMD 121974 (Cilengitide) (EMD Pharmaceuticals), ENMD-1198 (EntreMed), ENMD-2076 (EntreMed), Endostar (Simcere), Erbitux (ImClone/Bristol-Myers Squibb), EZN-2208 (Enzon Pharmaceuticals), EZN-2968 (Enzon Pharmaceuticals), GC1008 (Genzyme), Genistein, GSK1363089 (Foretinib) (GlaxoSmithKline), GW786034 (Pazopanib) (GlaxoSmithKline), GT-111 (Vascular Biogenics Ltd.), IMC-1121B (Ramucirumab) (ImClone Systems), IMC-18F1 (ImClone Systems), IMC-3G3 (ImClone LLC), INCB007839 (Incyte Corporation), INGN 241 (Introgen Therapeutics), Iressa (ZD1839/Gefitinib), LBH589 (Faridak/Panobinostst) (Novartis), Lucentis (Ranibizumab) (Genentech/Novartis), LY317615 (Enzastaurin) (Eli Lilly and Company), Macugen (Pegaptanib) (Pfizer), MEDI522 (Abegrin) (MedImmune), MLN518 (Tandutinib) (Millennium), Neovastat (AE941/Benefin) (Aeterna Zentaris), Nexavar (Bayer/Onyx), NM-3 (Genzyme Corporation), Noscapine (Cougar Biotechnology), NPI-2358 (Nereus Pharmaceuticals), OSI-930 (OSI), Palomid 529 (Paloma Pharmaceuticals, Inc.), Panzem Capsules (2ME2) (EntreMed), Panzem NCD (2ME2) (EntreMed), PF-02341066 (Pfizer), PF-04554878 (Pfizer), PI-88 (Progen Industries/Medigen Biotechnology), PKC412 (Novartis), Polyphenon E (Green Tea Extract) (Polypheno E International, Inc.), PPI-2458 (Praecis Pharmaceuticals), PTC299 (PTC Therapeutics), PTK787 (Vatalanib) (Novartis), PXD101 (Belinostat) (CuraGen Corporation), RAD001 (Everolimus) (Novartis), RAF265 (Novartis), Regorafenib (BAY73-4506) (Bayer), Revlimid (Celgene), Retaane (Alcon Research), SN38 (Liposomal) (Neopharm), SNS-032 (BMS-387032) (Sunesis), SOM230 (Pasireotide) (Novartis), Squalamine (Genaera), Suramin, Sutent (Pfizer), Tarceva (Genentech), TB-403 (Thrombogenics), Tempostatin (Collard Biopharmaceuticals), Tetrathiomolybdate (Sigma-Aldrich), TG100801 (TargeGen), Thalidomide (Celgene Corporation), Tinzaparin Sodium, TKI258 (Novartis), TRC093 (Tracon Pharmaceuticals Inc.), VEGF Trap (Aflibercept) (Regeneron Pharmaceuticals), VEGF Trap-Eye (Regeneron Pharmaceuticals), Veglin (VasGene Therapeutics), Bortezomib (Millennium), XL184 (Exelixis), XL647 (Exelixis), XL784 (Exelixis), XL820 (Exelixis), XL999 (Exelixis), ZD6474 (AstraZeneca), Vorinostat (Merck), and ZSTK474.

Exemplary B-Raf inhibitors include vemurafenib, dabrafenib, and encorafenib.

Exemplary MEK inhibitors include binimetinib, cobimetinib, refametinib, selumetinib, and trametinib.

Exemplary BTK inhibitors include ibrutinib, pirtobrutinib (Loxo-305), tirabrutinib, tolebrutinib, evobrutinib, fenebrutinib (GDC-0853), acalabrutinib, vecabrutinib (SNS-062), ONO-4059, spebrutinib, zanubrutinib (BGB-3111), HM71224, and M7583.

Exemplary TRK inhibitors include entrectinib, larotrectinib, CH7057288, ONO-7579, LOXO-101, lestaurtinib, and LOXO-195.

Exemplary c-KIT inhibitors include imatinb, sunitinb, and ponatinib.

Exemplary c-MET inhibitors include capmatinib, crizotinib, tivantinib, onartuzumab, INCB28060, AMG-458, savolitinib, and tepotinib.

Exemplary CDK4/6 inhibitors include palbociclib, ribociclib, abemaciclib, and trilaciclib.

Exemplary FAK inhibitors include defactinib, GSK2256098, BI853520, and PF-00562271.

Exemplary FGFR inhibitors include erdafitinib, pemigatinib, infigratinib, rogaratinib, AZD4547, BGJ398, FP-1039, and ARQ 087.

Exemplary FLT-3 inhibitors include quizartinib, crenolanib, gilteritinib, midostaurin, and lestaurtinib.

Exemplary IDH1 inhibitors include ivosidenib, BAY-1436032, and AGI-5198.

An exemplary IDH2 inhibitor includes enasidenib.

Exemplary PARP inhibitors include talazoparib, niraparib, rucaparib, olaparib (AZ 2281, KU59436), veliparib (ABT 888), CEP 9722, E7016, AG014699, MK4827, BMN-673, and Pamiparib (BGB-290).Exemplary PDGFRA inhibitors include imatinib, regorafenib, crenolanib, and olaratumab.

Exemplary pan-RAF inhibitors include belvarafenib, LXH254, LY3009120, INU-152, and HM95573.

Exemplary RET inhibitors include lenvatinib, alectinib, vandetanib, cabozantinib, BLU-667, and LOXO-292.

Exemplary ROS1 inhibitors include ceritinib, lorlatinib, entrectinib, crizotinib, TPX-0005, and DS-6051b.

Exemplary Vascular Endothelial Growth Factor (VEGF) receptor inhibitors include, but are not limited to, Bevacizumab (Avastin^{®}), axitinib (Inlyta^{®}); Brivanib alaninate (BMS-582664, (S)-((R)-1-(4-(4-Fluoro-2-methyl-1H-indol-5-yloxy)-5-methylpyrrolo[2,1-f][1,2,4]triazin-6-yloxy)propan-2-yl)2-aminopropanoate); Sorafenib (Nexavar^{®}); Pazopanib (Votrient^{®}); Sunitinib malate (Sutent^{®}); Cediranib (AZD2171, CAS 288383-20-1); Vargatef (BIBF1120, CAS 928326-83-4); Foretinib (GSK1363089); Telatinib (BAY57-9352, CAS 332012-40-5); Apatinib (YN968D1, CAS 811803-05-1); Imatinib (Gleevec^{®}); Ponatinib (AP24534, CAS 943319-70-8); Tivozanib (AV951, CAS 475108-18-0); Regorafenib (BAY73-4506, CAS 755037-03-7); Vatalanib dihydrochloride (PTK787, CAS 212141-51-0); Brivanib (BMS-540215, CAS 649735-46-6); Vandetanib (Caprelsa^{®} or AZD6474); Motesanib diphosphate (AMG706, CAS 857876-30-3, N-(2,3-dihydro-3,3-dimethyl-1H-indol-6-yl)-2-[(4-pyridinylmethyl)amino]-3-pyridinecarboxamide, described in PCT Publication No. WO 02/066470); Dovitinib dilactic acid (TKI258, CAS 852433-84-2); Linfanib (ABT869, CAS 796967-16-3); Cabozantinib (XL184, CAS 849217-68-1); Lestaurtinib (CAS 111358-88-4); N-[5-[[[5-(1,1-Dimethylethyl)-2-oxazolyl]methyl]thio]-2-thiazolyl]-4-piperidinecarboxamide (BMS38703, CAS 345627-80-7); (3R,4R)-4-Amino-1-((4-((3-methoxyphenyl)amino)pyrrolo[2,1-f][1,2,4]triazin-5-yl)methyl)piperidin-3-ol (BMS690514); N-(3,4-Dichloro-2-fluorophenyl)-6-methoxy-7-[[(3aα,5β,6aα)-octahydro-2-methylcyclopenta[c]pyrrol-5-yl]methoxy]-4-quinazolinamine (XL647, CAS 781613-23-8); 4-Methyl-3-[[1-methyl-6-(3-pyridinyl)-1H-pyrazolo[3,4-d]pyrimidin-4-yl]amino]-N-[3-(trifluoromethyl)phenyl]-benzamide (BHG712, CAS 940310-85-0); and Aflibercept (Eylea^{®}).

Exemplary EGF pathway inhibitors include, without limitation tyrphostin 46, EKB-569, erlotinib (Tarceva^{®}), gefitinib (Iressa^{®}), erbitux, nimotuzumab, lapatinib (Tykerb^{®}), cetuximab (anti-EGFR mAb), ¹⁸⁸Re-labeled nimotuzumab (anti-EGFR mAb), and those compounds that are generically and specifically disclosed in WO 97/02266, EP 0 564 409, WO 99/03854, EP 0 520 722, EP 0 566 226, EP 0 787 722, EP 0 837 063, U.S. Pat. No. 5,747,498, WO 98/10767, WO 97/30034, WO 97/49688, WO 97/38983 and WO 96/33980. Exemplary EGFR antibodies include, but are not limited to, Cetuximab (Erbitux^{®}); Panitumumab (Vectibix^{®}); Matuzumab (EMD-72000); Trastuzumab (Herceptin^{®}); Nimotuzumab (hR3); Zalutumumab; TheraCIM h-R3; MDX0447 (CAS 339151-96-1); and ch806 (mAb-806, CAS 946414-09-1). Exemplary Epidermal growth factor receptor (EGFR) inhibitors include, but not limited to, Erlotinib hydrochloride (Tarceva^{®}); ceritinib; brigatinib; osimertinib; dacomitinib; icotinib; Gefitnib (Iressa^{®}); N-[4-[(3-Chloro-4-fluorophenyl)amino]-7-[[(3"S")-tetrahydro-3-furanyl]oxy]-6-quinazolinyl]-4(dimethylamino)-2-butenamide, Tovok^{®}); Vandetanib (Caprelsa^{®}); Lapatinib (Tykerb^{®}); (3R,4R)-4-Amino-1-((4-((3-methoxyphenyl)amino)pyrrolo[2,1-f][1,2,4]triazin-5-yl)methyl)piperidin-3-ol (BMS690514); Canertinib dihydrochloride (CI-1033); 6-[4-[(4-Ethyl-1-piperazinyl)methyl]phenyl]-N-[(1R)-1-phenylethyl]-7H-Pyrrolo[2,3-d]pyrimidin-4-amine (AEE788, CAS 497839-62-0); Mubritinib (TAK165); Pelitinib (EKB569); Afatinib (BIBW2992); Neratinib (HKI-272); N-[4-[[1-[(3-Fluorophenyl)methyl]-1H-indazol-5-yl]amino]-5-methylpyrrolo[2,1-f][1,2,4]triazin-6-yl]-carbamic acid, (3S)-3-morpholinylmethyl ester (BMS599626); N-(3,4-Dichloro-2-fluorophenyl)-6-methoxy-7-[[(3aα,5β,6aα)-octahydro-2-methylcyclopenta[c]pyrrol-5-yl]methoxy]-4-quinazolinamine (XL647, CAS 781613-23-8); 4-[4-[[(1R)-1-Phenylethyl]amino]-7H-pyrrolo[2,3-d]pyrimidin-6-yl]-phenol (PKI166, CAS 187724-61-4); rocelitinib.

Exemplary mTOR inhibitors include, without limitation, rapamycin (Rapamune^{®}), and analogs and derivatives thereof; SDZ-RAD; Temsirolimus (Torisel^{®}; also known as CCI-779); Ridaforolimus (formally known as deferolimus, (1R,2R,4S)-4-[(2R)-2[(1R,9S,12S,15R,16E,18R,19R,21R,23S,24E,26E,28Z,30S,32S,35R)-1,18-dihydroxy-19,30-dimethoxy-15,17,21,23,29,35-hexamethyl-2,3,10,14,20-pentaoxo-11,36-dioxa-4-azatricyclo[30.3.1.0^{4,9}]hexatriaconta-16,24,26,28-tetraen-12-yl]propyl]-2-methoxycyclohexyl dimethylphosphinate, also known as AP23573 and MK8669, and described in PCT Publication No. WO 03/064383); Everolimus (Afinitor^{®} or RAD001); Rapamycin (AY22989, Sirolimus^{®}); Simapimod (CAS 164301-51-3); (5-{2,4-Bis[(3S)-3-methylmorpholin-4-yl]pyrido[2,3-d]pyrimidin-7-yl}-2-methoxyphenyl)methanol (AZD8055); 2-Amino-8-[trans-4-(2-hydroxyethoxy)cyclohexyl]-6-(6-methoxy-3-pyridinyl)-4-methyl-pyrido[2,3-d]pyrimidin-7(8H)-one (PF04691502, CAS 1013101-36-4); and N²-[1,4-dioxo-[[4-(4-oxo-8-phenyl-4H-1-benzopyran-2-yl)morpholinium-4-yl]methoxy]butyl]-L-arginylglycyl-L-α-aspartylL-serine-, inner salt (SF1126, CAS 936487-67-1).

Exemplary Phosphoinositide 3-kinase (PI3K) inhibitors include, but are not limited to, duvelisib, idelalisib, 4-[2-(1H-Indazol-4-yl)-6-[[4-(methylsulfonyl)piperazin-1-yl]methyl]thieno[3,2-d]pyrimidin-4-yl]morpholine (also known as GDC 0941 and described in PCT Publication Nos. WO 09/036082 and WO 09/055730); 2-Methyl-2-[4-[3-methyl-2-oxo-8-(quinolin-3-yl)-2,3-dihydroimidazo[4,5-c]quinolin-1-yl]phenyl]propionitrile (also known as BEZ 235 or NVP-BEZ 235, and described in PCT Publication No. WO 06/122806); 4-(trifluoromethyl)-5-(2,6-dimorpholinopyrimidin-4-yl)pyridin-2-amine (also known as BKM120 or NVP-BKM120, and described in PCT Publication No. WO2007/084786); Tozasertib (VX680 or MK-0457, CAS 639089-54-6); (5Z)-5-[[4-(4-Pyridinyl)-6-quinolinyl]methylene]-2,4-thiazolidinedione (GSK1059615, CAS 958852-01-2); (1E,4S,4aR,5R,6aS,9aR)-5-(Acetyloxy)-1-[(di-2-propenylamino)methylene]-4,4a,5,6,6a,8,9,9a-octahydro-11-hydroxy-4-(methoxymethyl)-4a,6a-dimethyl-cyclopenta[5,6]naphtho[1,2-c]pyran-2,7,10(1H)-trione (PX866, CAS 502632-66-8); and 8-Phenyl-2-(morpholin-4-yl)-chromen-4-one (LY294002, CAS 154447-36-6). Exemplary Protein Kinase B (PKB) or AKT inhibitors include, but are not limited to. 8-[4-(1-Aminocyclobutyl)phenyl]-9-phenyl-1,2,4-triazolo[3,4-f][1,6]naphthyridin-3(2H)-one (MK-2206, CAS 1032349-93-1); Perifosine (KRX0401); 4-Dodecyl-N-1,3,4-thiadiazol-2-yl -benzenesulfonamide (PHT-427, CAS 1191951-57-1); 4-[2-(4-Amino-1,2,5-oxadiazol-3-yl)-1-ethyl-7-[(3S)-3-piperidinylmethoxy]-1H-imidazo[4,5-c]pyridin-4-yl]-2-methyl-3-butyn-2-ol (GSK690693, CAS 937174-76-0); 8-(1-Hydroxyethyl)-2-methoxy-3-[(4-methoxyphenyl)methoxy]-6H-dibenzo[b,d]pyran-6-one (palomid 529, P529, or SG-00529); Tricirbine (6-Amino-4-methyl-8-(β-D-ribofuranosyl)-4H,8H-pyrrolo[4,3,2-de]pyrimido[4,5-c]pyridazine); (αS)-α-[[[5-(3-Methyl-1H-indazol-5-yl)-3-pyridinyl]oxy]methyl]-benzeneethanamine (A674563, CAS 552325-73-2); 4-[(4-Chlorophenyl)methyl]-1-(7H-pyrrolo[2,3-d]pyrimidin-4-yl)-4-piperidinamine (CCT128930, CAS 885499-61-6); 4-(4-Chlorophenyl)-4-[4-(1H pyrazol-4-yl)phenyl]-piperidine (AT7867, CAS 857531-00-1); and Archexin (RX-0201, CAS 663232-27-7).

In certain embodiments, a tyrosine kinase inhibitor used in combination with chimeric Tim4 receptor modified cells is an anaplastic lymphoma kinase (ALK) inhibitor. Exemplary ALK inhibitors include crizotinib, ceritinib, alectinib, brigatinib, dalantercept, entrectinib, and lorlatinib.

In certain embodiments where chimeric Tim4 receptor modified cells are administered in combination with one or more additional therapies, the one or more additional therapies may be administered at a dose that might otherwise be considered subtherapeutic if administered as a monotherapy. In such embodiments, the chimeric Tim4 receptor composition may provide an additive or synergistic effect such that the one or more additional therapies can be administered at a lower dose. Combination therapy includes administration of a chimeric Tim4 receptor compositions as described herein before an additional therapy (*e.g.,* 1 day to 30 days or more before the additional therapy), concurrently with an additional therapy (on the same day), or after an additional therapy (*e.g.,* 1 day - 30 days or more after the additional therapy). In certain embodiments, the chimeric Tim4 receptor modified cells are administered after administration of the one or more additional therapies. In further embodiments, the chimeric Tim4 receptor modified cells are administered 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30 days after administration of the one or more additional therapies. In still further embodiments, the chimeric Tim4 receptor modified cells are administered within 4 weeks, within 3 weeks, within 2 weeks, or within 1 week after administration of the one or more additional therapies. Where the one or more additional therapies involves multiple doses, the chimeric Tim4 receptor modified cells may be administered after the initial dose of the one or more additional therapies, after the final dose of the one or more additional therapies, or in between multiple doses of the one or more additional therapies.

In certain embodiments, therapies of the present disclosure include a depletion step. A depletion step to remove chimeric Tim4 receptors from the subject may occur after a sufficient amount of time for therapeutic benefit in order to mitigate toxicity to a subject. In such embodiments, the chimeric Tim4 receptor vector may include an inducible suicide gene, such as iCASP9, inducible Fas, or HSV-TK. Similarly, a chimeric Tim4 receptor vector may be designed for expression of a known cell surface antigen such as CD20 or truncated EGFR (SEQ ID NO:38) that facilitates depletion of transduced cells through infusion of an associated monoclonal antibody (mAb), for example, Rituximab for CD20 or Cetuximab for EGFR. Alemtuzumab, which targets CD52 present on the surface of mature lymphocytes, may also be used to deplete transduced B cells, T cells, or natural killer cells.

Subjects that can be treated by the compositions of the present disclosure include animals, such as humans, primates, cows, horses, sheep, dogs, cats, mice, rats, rabbits, guinea pigs, or pigs. The subject may be male or female, and can be any suitable age, including infant, juvenile, adolescent, adult, and geriatric subjects.

### EXAMPLES

### EXAMPLE 1: CER-T CELLS ELICIT CYTOTOXIC EFFECTS ON PRIMED-PTD-SER+ TUMOR CELLS

TLR-containing chimeric Tim4 receptor-T cells are engineered to target cells that display elevated levels of the cell membrane stress-signal, phosphatidylserine (Ptd-Ser). Ptd-Ser is a phospholipid normally found on the inner leaflet of the plasma cell membrane. Upon activation of certain downstream signals (e.g., caspase 3/7 activation), Ptd-Ser is externalized to the outer cell membrane. Its engagement by Ptd-Ser specific receptors on professional phagocytes/antigen presenting cells (APCs) triggers multi-component signaling complexes that ultimately culminate in the reorganization of the actin cytoskeleton. Tim-4 (T cell immunoglobulin mucin-4) is one of several receptors that specifically bind to Ptd-Ser. Its expression in resident and peritoneal macrophages is linked to clearance of apoptotic cells during normal tissue homeostasis. In dendritic cells (DCs), Tim-4- Ptd-Ser interactions mediate capture and engulfment of antigen for cross-priming of T cells.

Several strategies were developed to efficiently induce or prime Ptd-Ser on various tumor cell lines. This priming step uses standard of care therapeutics such as targeted small molecule inhibitors to induce cell stress and/or apoptosis. Once induced, Ptd-Ser serves as a target for chimeric Tim4 receptor-T cell engagement, activation and cytolytic function. This two-step 'prime and kill' strategy is shown using a variety of small molecule inhibitor - chimeric Tim4 receptor-T cell product combinations, as well as combinations with engineered CAR T and TCR products.

In ovarian tumors, poly (ADP-ribose) polymerase (PARP) inhibitors, such as Niraparib, are clinically approved drugs which target DNA damage response pathways. Responses are rarely complete, with relapse common after treatment. To induce Ptd-Ser exposure, BRCA-2 mutated Kuramochi cell line was treated with therapeutic doses of the PARP inhibitor Niraparib. Brief exposure to Niraparib elicits changes in membrane phospholipid symmetry, in a dose dependent manner, and is effective in inhibiting growth of Kuramochi cells **(****FIG. 7A****).** The addition of chimeric Tim4 receptor pCTX133 (Tim4 binding domain-TLR2 signaling domain-CD3z signaling domain), at low effector: target ratios (1:1), enhanced the potency of Niraparib *in vitro* compared to transduced-controls, demonstrating the ability of pCTX133 to elicit direct cytotoxic effects on target cells **(****FIG. 7B****).**

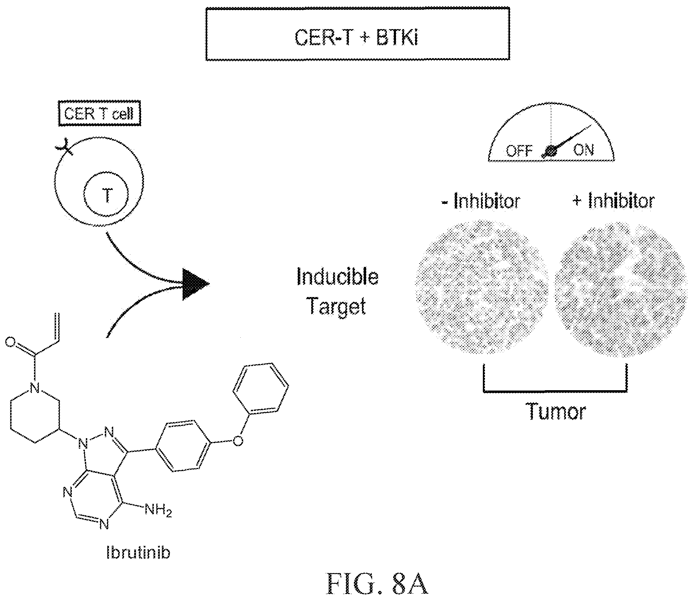

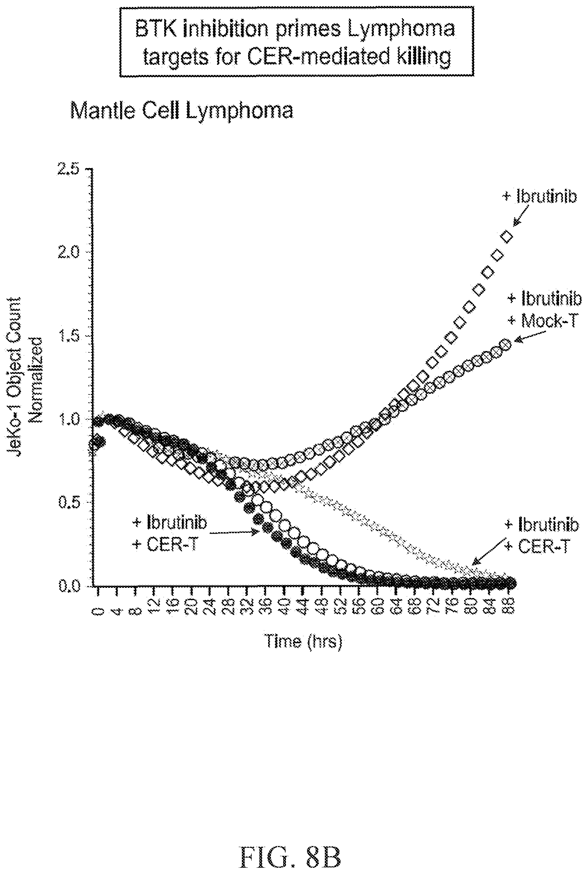

In mantle cell lymphoma, Bruton's Tyrosine Kinase (BTK) inhibitors, such as ibrutinib, are clinically approved drugs that target a pro-survival kinase. Responses are rarely complete, and relapse is common after treatment. Treatment of JeKo-1 MCL with Ibrutinib exposes Ptd-Ser, as determined by immunohistochemistry using a recombinant murine Tim4 protein **(****FIG. 8A****).** JeKo-1 mantle cell lymphoma cell lines were treated with a 25uM Ibrutinib for 24h, followed by drug wash out and co-culture with 0.5uM Ibrutinib with chimeric Tim4 receptor pCTX136 (Tim4 binding domain-CD28 signaling domain-CD3z signaling domain) or control cells at 3:1, 2:1, or 1:1 E:T ratios. Co-culture with chimeric Tim4 receptor cells induced near elimination of JeKo-1 target cells, as compared to Ibrutinib treatment or treatment with control T cells **(****FIG. 8B****).**

Chimeric Tim-4 receptors having TLR2 intracellular signaling domain or TLR8 intracellular signaling domain, and CD28 intracellular signaling domain or CD3zeta intracellular signaling domain were tested for their capacity to promote tumor cell acquisition and elicit cytotoxic and APC-like function. To assess antigen acquisition, target cells were co-cultured with chimeric Tim-4 receptor T cells and evaluated by transmission electron microscopy (TEM) or flow cytometry. Target cells were treated with a small molecule inhibitor to induce Ptd-Ser externalization and evaluated for lysosomal uptake using a pH indicator dye (pHrodo Red).

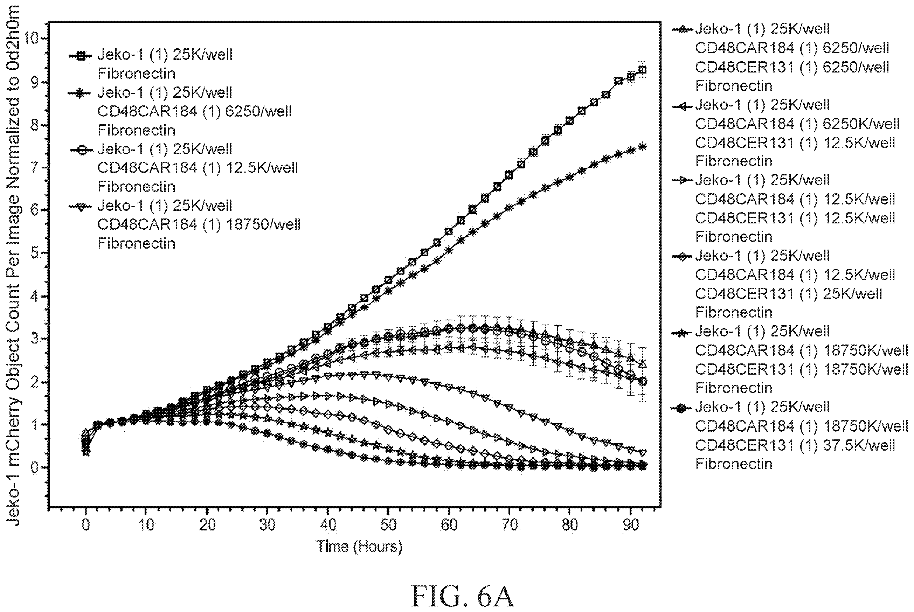

An alternative prime and kill therapeutic strategy combines TLR-containing chimeric Tim4 receptor-T cells with Chimeric Antigen Receptor-T (CAR)-T cells. This combinatorial approach utilizes the CAR to specifically target tumor cells, leading to upregulation of Ptd-Ser. A CD19 CAR-T cell product (anti-CD19 scFv- - CD28 costimulatory signaling domain-CD3ζ signaling domain; "1928z CAR") rapidly induces Ptd-Ser on CD19+ Mantle Cell Lymphoma (MCL) cells in a dose dependent manner **(****FIG. 5A****).** In co-culture studies, measured by incucyte and by FACS, a combination of 1928z CAR-T cells (pCTX184) and pCTX131 (Tim4-TLR8-CD3z) showed enhanced potency. 1928z CAR T cells were combined at low effector:target ratios at multiple CAR:CER ratios using pCTX131 (Tim4-TLR8-CD3z) cells **(****FIG. 5B****,** **FIG. 6A****).** pCTX156 is truncated EGFR (EGFRt) control. Increases in inflammatory cytokines such as IFN-γ were observed from supernatants, in line with the observed increase in cytolytic function **(****FIG. 5C****).** In addition, in co-culture studies, measured by incucyte, a combination of 1928z CAR-T cells and pCTX131 (Tim4-TLR8-CD3z) showed enhanced induction of cleaved caspase in target cells. 1928z CAR T cells were combined at low effector:target ratios at multiple CAR:CER ratio using pCTX131 (Tim4-TLR8-CD3z) cells **(****FIG. 6B****).**

Thus, this example highlights that pCTX133 and pCTX131 (TLR2 and TLR8)-containing chimeric Tim4 receptor-T cells can elicit cytolytic activity towards primed solid tumor and hematologic target cell lines expressing cell surface Ptd-Ser and augment small molecule and CAR-based therapeutic approaches.

### EXAMPLE 2: CHIMERIC TIM4 RECEPTOR-T CELLS MEDIATE ANTIGEN CAPTURE AND PRESENTATION

Activated T cells have been shown to be capable of Ag processing and presentation, since they express class II molecules, display antigen on the cell surface, and can deliver costimulatory signals to other T cells¹⁰. Unlike professional APCs, though, T cells are limited by their inefficient capture of soluble antigens⁹. APCs, in contrast, utilize constitutively expressed Ag uptake receptors to capture and engulf antigen for subsequent degradation and MHC loading¹²¹³. The capture of a soluble antigen can be up to 10³ times more efficient in the presence of surface receptors that bind to Ag with high affinity¹⁴.

In dendritic cells (DCs), Tim-4- Ptd-Ser interactions mediate capture, engulfment, and concentration of antigen, allowing DCs to present antigen to T cells with high efficiency. Indeed, Tim-4 receptor blockade in preclinical NSCLC models impairs activation of tumor specific CD8+ T cells and promotes tumor progression¹². Furthermore, gene expression profiling shows downregulation of Tim-4 expression in advanced tumor cells, concordant with decreased antigen uptake, presentation, and T cell activation.

Experimental results presented here indicates that T cells can be redirected as antigen-presenting T cells for immunotherapy by enhancing their antigen uptake, capture and co-stimulatory capabilities. The fusion of a human Tim-4 phagocytic uptake receptor to intracellular signaling sequences that drive antigen uptake as well as antigen processing and presentation adds enhanced APC capabilities to T cells. The modular design of chimeric Tim4 receptors incorporates intracellular domains that drive multicomponent signaling complexes, such as CD3ζ, CD28, 4-1BB, ITAM and TLR signaling, to induce cellular activation, cytolytic function, secretion of cytokines and chemokines, upregulation of adhesion and costimulatory molecules, and antigen degradation- processes required for mediating efficient T cell activation¹⁵.

Chimeric Tim4 receptor-T cells were tested to determine whether they can capture and present soluble antigens and trigger activation and proliferation of recombinant E7-restricted T cell clones in a co-culture system. First expression of Tim4 was confirmed on transduced CER T cells by flow cytometry. Tim4 binding domain-CD28 transmembrane-CD28 signaling domain-CD3z signaling domain (CTX247, also referred to as CER1161) and Tim4 binding domain-CD28 signaling domain-CD3z signaling domain-TLR2 signaling domain (CTX1107, as referred to as CER1107 or CER1236 - SEQ ID NO:19) were stained for Tim4 and EGFR, a transduction marker encoded on each vector. CER1107 and CER1236 have the same amino acid sequence but different vector backbones. Tim4 expression was observed on CTX247 or CTX1107 transduced cells, but not mock transduced controls **(****FIG. 4****)** E7-restricted TCRs target the E7 protein from HPV16 and have the TCRα chain and TCRβ chain sequence provided in SEQ ID NO:241. E7 TCRs proliferate in response to APCs pulsed with E7 peptide through MHC class I. For autologous APCs, CD4+ and CD8+ chimeric Tim4 receptor T cell products transduced with different Tim-4 chimeric receptors were pulsed with a pool of 15-mer peptides containing 11-mer overlaps derived from the E7 protein from HPV16 or vehicle. Chimeric Tim4 receptor T cells were pulsed at 37°C for 4 hrs with E7 peptides and tested for their capacity to trigger E7-specific activation and proliferation. E7-TCR cell surface activation marker responses were evaluated by flow cytometry 24 hours following co-culture with chimeric Tim4 receptor-T cell products. After an additional 5 days of co-culture, proliferative responses were assessed by Cell Trace (CT) Violet dilution.

**FIGS. 1B-1D** show that pCTX1107 (Tim4 binding domain-CD28 intracellular signaling domain-CD3ζ intracellular signaling domain-TLR2 intracellular signaling domain; SEQ ID NO: 19) CER T cells were indeed stimulatory for E7-specific T cells, whereas pCTX247 (Tim4 binding domain-CD28 intracellular signaling domain-CD3ζ intracellular signaling domain) or untransduced T cells were not stimulatory, even when pulsed with high concentrations of E7 peptide. In these experiments, the only difference in construct design between pCTX247 and pCTX1107 was the addition of a TLR-2 intracellular sequence **(****FIG. 1A****),** implicating TLR signaling in amplifying T cell presentation of soluble antigen and triggering of E7 TCRs. CER T cell-induced proliferation is HLA-dependent, confirming the role of antigen presentation in the CER-mediated activity.

Evidence for pCTX1107 being a strong stimulatory of E7-specific activation was also seen in CD25 and CD69 upregulation measured by flow cytometry **(****FIG. 2****).** A higher frequency of E7-TCR containing T cells expressed both activation markers 24 hours after co-culture **(****FIG. 2****).** The E7 TCR-T cell surface activation markers CD25 and CD69 were upregulated 41.2% vs. 23.1% relative to controls 24 hrs. following co-culture with chimeric Tim4 receptor-T and the percentage of dividing E7-TCR-T cells by 6 days was 44% for TIM4/CD28/CD3z/TLR2 chimeric Tim4 receptor T cells vs 8% relative to controls.

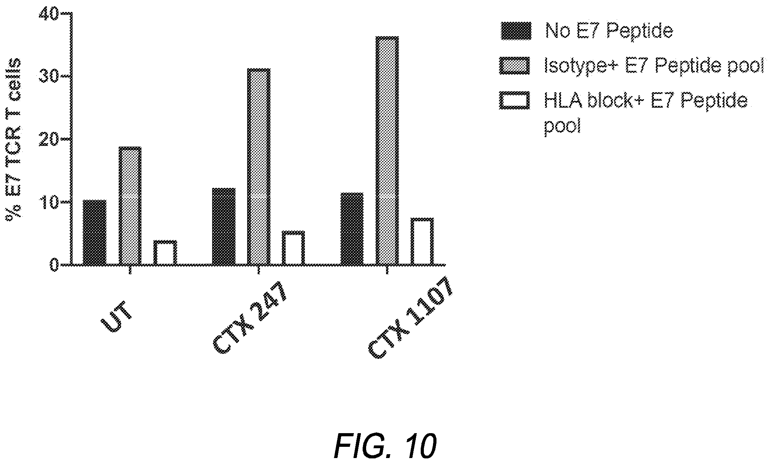

Cell Trace Violet labeled E7-specific TCR T cells were cultured for 4 days with untransduced T cells (UT), T cells transduced with a Tim4-CD28-CD3z construct (CTX247) or a Tim4-CD28-CD3z-TLR2 construct (CTX1107) and JeKo-1 cells at a 1:2:2 ratio in the presence of a pool of 15 mer peptides with 11aa overlap (100ng each peptide) derived from the HPV16 E7 protein. Duplicate cultures were incubated with HLA A, B, C blocking antibodies (clone W6/32) or matched mouse IgG2a antibodies for the duration of the culture. **FIG. 10** shows percent E7 TCR cells in culture among live cells as determined by flow cytometry based on staining of mouse TCRb+ cells. Activation of E7-specific TCR T cells mediated by chimeric Tim4 receptor antigen presentation is blocked by anti-HLA-I antibodies.

### EXAMPLE 3: TRANSFECTION OF T CELLS WITH CHIMERIC TIM4 RECEPTORS

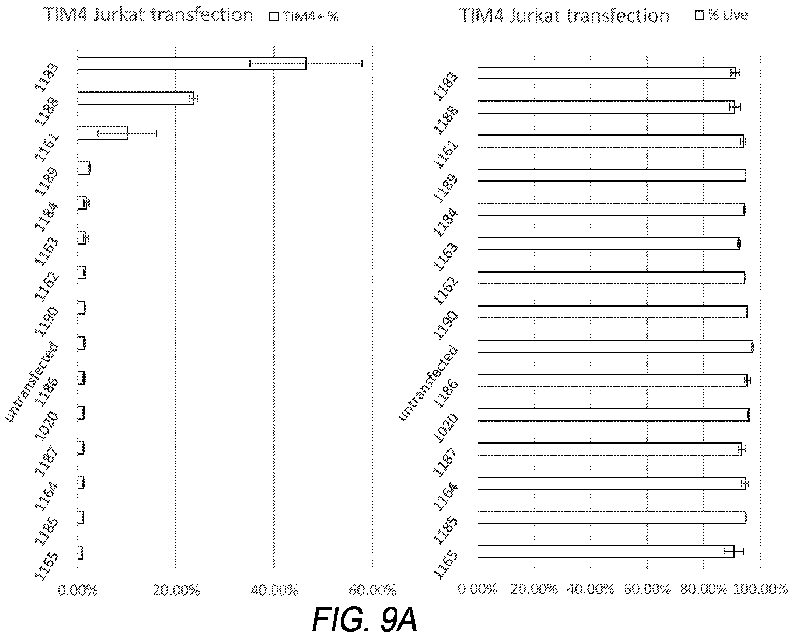

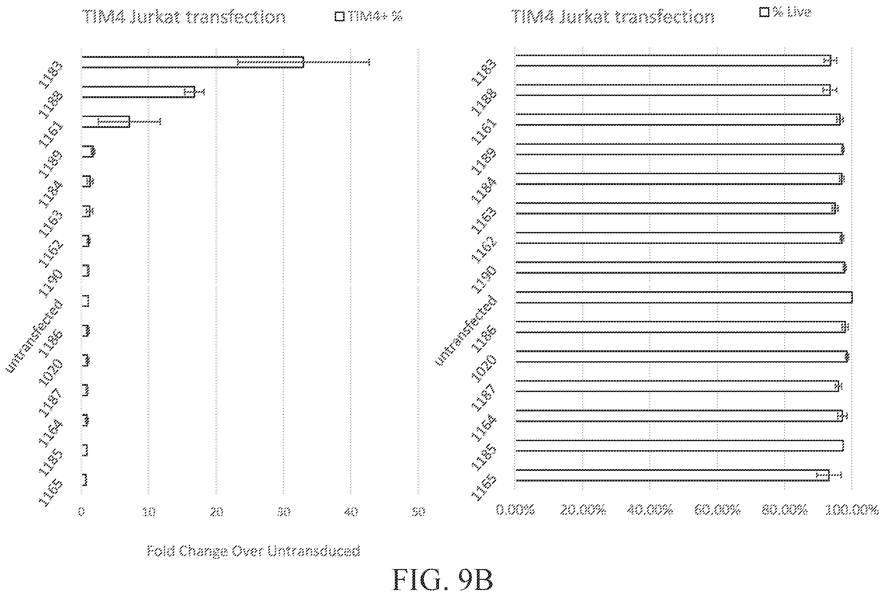

Chimeric Tim4 receptors pCTX1183, pCTX1161, pCTX1189, pCTX1184, pCTX1163, pCTX1162, pCTX1190, pCTX1186, pCTX1187, pCTX1164, pCTX1185, and pCTX1165 (see, Table 8) were transfected into Jurkat T lymphocyte cell line **(****FIGS. 9A-9B****).**

### EXAMPLE 4: ENHANCED ANTIGEN CAPTURE, ANTIGEN-PRESENTING CELL-LIKE FUNCTION, AND CYTOTOXIC RESPONSES WITH CHIMERIC TIM4 RECEPTOR T CELLS

### Methods:

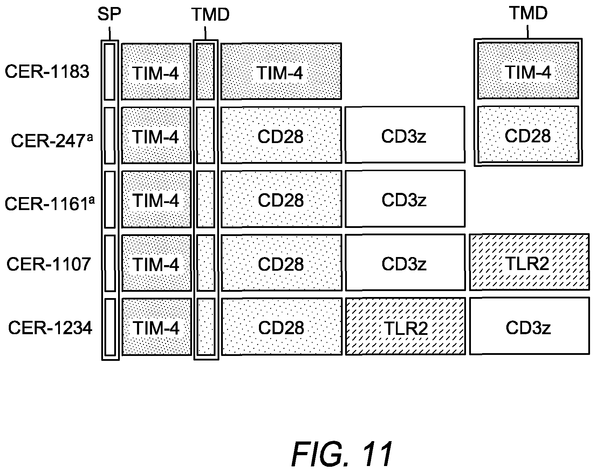

Chimeric Tim4 Receptor T cells (also referred to as CER T cells) containing the Tim-4 receptor fused to various transmembrane and intracellular signaling domains were generated using healthy donor T cells **(****FIG. 11****).** CER1234 has an amino acid sequence set forth in SEQ ID NO:18. CER1107 (also referred to herein as CTX1107 or CER1236) has the amino acid sequence set forth in SEQ ID NO:19. CER1107 and CER1236 have the same amino acid sequence but differ by their lentiviral backbone. CER247 and CER1161 (Tim4 binding domain-CD28tm-CD28icd-CD3zICD, SEQ ID NO:21) have the same amino acid sequence but differ by their lentiviral backbone and promoter systems.

CER T cell products were characterized on day 6 following transduction for expression of Tim-4, CD45RA, CCR7, and CD4/CD8 ratios, all measured by flow cytometry (FC) using commercially available fluorochrome-conjugated antibodies.

The induction of surface phosphatidylserine expression of REC-1 and JeKo-1 mantle cell lymphoma (MCL) cell lines treated with ibrutinib was measured by FC using histidine-tagged recombinant murine Tim-4; secondary detection was performed using an anti-histidine antibody.

Phagocytosis was assessed in in co-culture assays of REC-1 cells with CER T cells. REC-1 cells were pretreated for 24 hours with 10 µM ibrutinib, labeled with pHrodo red, and cultured with untransduced T cells or CER T cells at 0.1 to 1 effector to target (E:T) ratios. The percentage of pHrodo red-positive cells was determined by flow cytometry after 16 or 40 hours of co-culture. The localization of tumor fragments in CER T-cell lysosomal compartments was assessed in a separate experiment with REC-1 cells labeled with pHrodo green; following co-culture, all cells were labeled with Lyso-tracker red. Visualization of engulfment was performed by fluorescence microscopy. 1-way ANOVA with Dunnett's post hoc test was performed compared with untransduced T cells.

CER T cell cytotoxic function was evaluated using the Incucyte^{®} live-cell analysis system in co-culture assays with REC-1 cells. REC-1 cells engineered to constitutively express mCherry (REC-1 mCherry) were pretreated with 10 µM ibrutinib or vehicle (20% 2-OH β-cyclodextrin) for 24 hours. Ibrutinib was washed off prior to co-culture with CER T cells. CER T cells (6-day expansion post-transduction) were cocultured with ibrutinib-pretreated REC-1 cells at an E:T ratio of 1:1 in the presence of 0.5 µM ibrutinib or vehicle. Total fluorescence of REC-1 mCherry cells decreased as cytotoxicity occurred.

T-cell activation markers programmed death-1 (PD-1) and 4-1BB were evaluated on viable CD3+ T cells by flow cytometry using commercially available fluorochrome-conjugated antibodies. REC-1 mCherry cells were pretreated for 24 hours with 10 µM ibrutinib or vehicle; after wash out, co-cultures were initiated with 0.5 µM ibrutinib or vehicle. Assays were conducted 96 hours after initiation of co-culture of CER T cells with REC-1 cells.

Cytokine induction was assessed by multiparametric ELISA using conditioned supernatants harvested after 96 hours in the cytotoxicity assay described above.

Antigen-presenting cell (APC)-like function of CER T cells was assessed as follows (FIG. 1A). Cell trace violet (CTV)-labeled E7 T-cell receptor (TCR) T cells were cultured for 6 days with untransduced T cells or CER T cells and JeKo-1 cells at a 1:2:2 ratio in the presence of a pool of 15-mer peptides with 11 amino acid overlap (100 ng of each peptide) derived from the HPV16 E7 protein. To demonstrate the role of major histocompatibility complex (MHC) class I antigen presentation, cultures as described above were incubated with a mouse anti-human leukocyte antigen (HLA) class I (A, B, and C) blocking antibody (clone W6/32) or matched mouse IgG2a antibodies for 4 days before analysis. Proliferation was measured by dye dilution of CTV.

### Results:

CERs were expressed by T cells **(Table 4).** High proportions of CER T cells express the engineered Tim-4 receptor. CD4/CD8 ratios were similar after transduction and expansion of CER T cells versus untransduced T cells. CER T cells exhibit a predominantly central memory and effector memory phenotype as measured by CCR7 and CD45RA.

**Table 4: CER T Cell Characteristics (as measured on day 6 followin transduction)**

| **Parameter** | **Untransduced** | **CER1183 (SEQ ID NO:20)** | **CER1234 (SEQ ID NO:18)** |
|---|---|---|---|
| **Tim4+, %** | Undetected | 69.4 | 75.0 |
| **T cell CD4/CD8 ratio** | 0.6 | 0.6 | 0.6 |
| **T cell subsets, %** | | | |
| **CD45RA+CCR7+ (naive)** | 11.8 | 13.8 | 14.6 |
| **CD45RA-/CCR7+ (central memory)** | 47.6 | 41.2 | 42.2 |
| **CD45RA-/CCR7-(effector memory)** | 33.8 | 33.9 | 32.2 |
| **CD45RA+/CCR7-(effector)** | 6.9 | 11.2 | 10.9 |

### Ibrutinib Induces Phosphatidylserine Exposure in REC-1 and JEKO-1 Cells

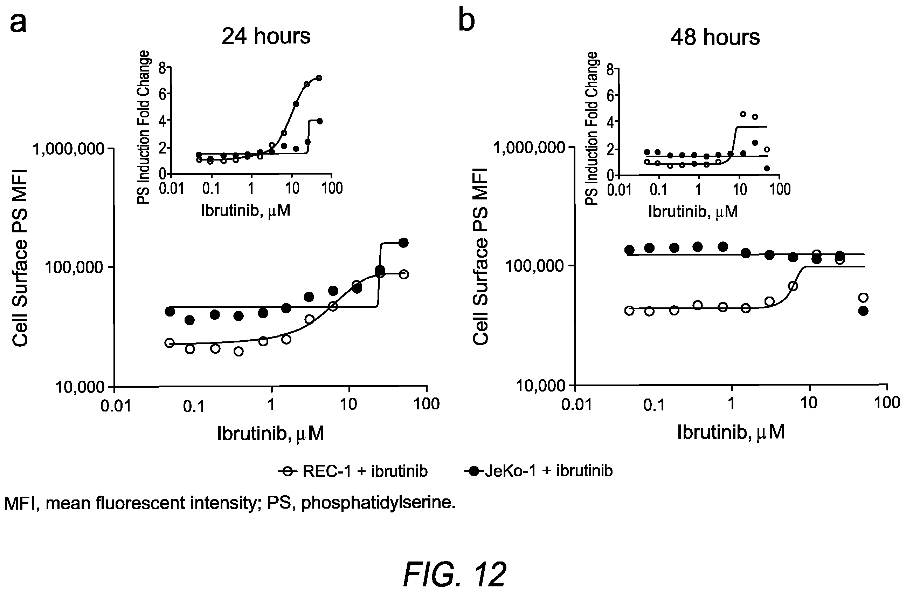

Ibrutinib (0.05 to 50 µM for 24 to 48 hours) induced PS exposure measured by flow cytometry **(****FIGS. 12A-12B****).** REC-1 cells were more sensitive than JeKo-1 cells to ibrutinib-induced phosphatidylserine exposure.

### CER1161 and CER1234 T Cells Demonstrate Phagocytic Function

CER-1161 and CER-1234 T cells increased engulfment of pHrodo red+ target cells relative to untransduced T cells after 16 and 40 hours of co-culture as measured by flow cytometry and fluorescence microscopy (FIGS. 13A to 13D). CER constructs containing intracellular signaling domains (ie, CD28, CD3ζ, TLR2) may enhance cytolytic function, leading to enhanced phagocytosis.

### CER1234 T Cells Demonstrate Cytotoxic Activity with Ibrutinib

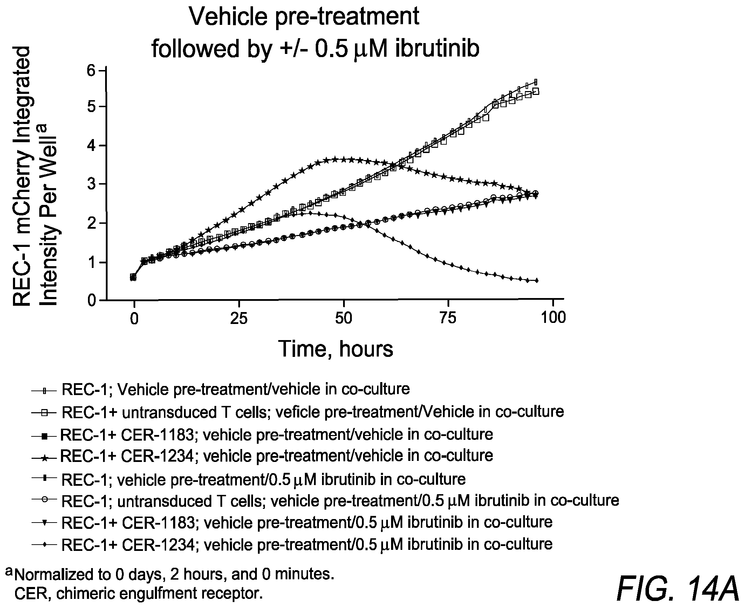

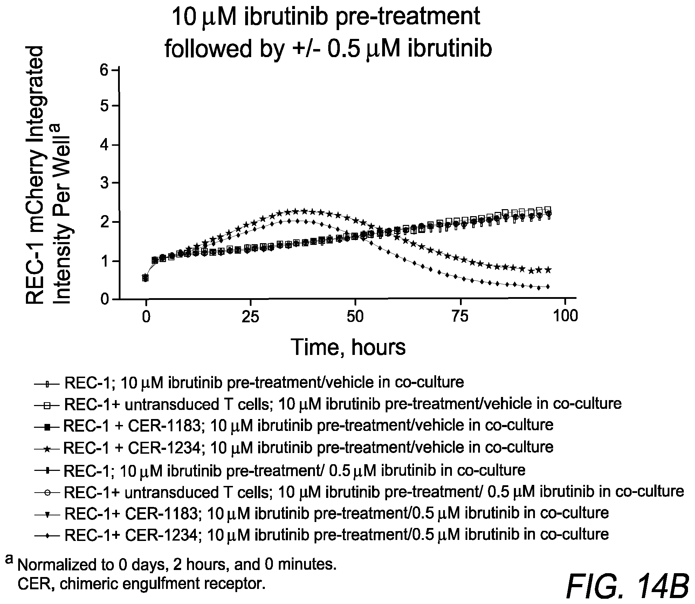

Tumor cell killing was enhanced by the combination of CER-1234 T cells and ibrutinib (pre-treatment, in co-culture, or both; FIGS. 14A and 14B). Co-culture with 0.5 µM ibrutinib reflects the concentration (Cmax) following standard dosing (560 mg once daily) and had a similar cytotoxic effect as pre-treatment alone or both pre-treatment and co-culture with ibrutinib. CER-1183 T cells (no intracellular signaling domain) show no additive effect with ibrutinib. CER-1234 T cells alone exhibited limited cytotoxic activity.

### CER1234 T Cells Express Cell-Surface Markers of T Cell Activation When Co-Cultured with Ibrutinib-Treated REC-1 Cells

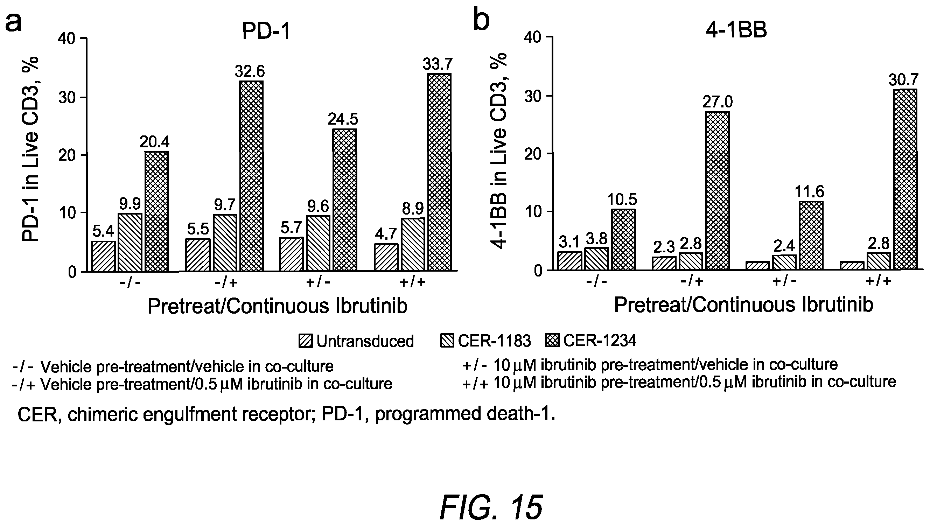

Co-cultures of CER-1234 T cells and target REC-1 cells in the presence of 0.5 µM ibrutinib increased PD-1 and 4-1BB expression on the T-cell surface (FIGS. 15A and B). PD-1 and 4-1BB were not increased in co-cultures containing CER-1183 T cells, which lack intracellular signaling domains.

### Cytokine Expression was Induced in Co-Cultures of CER1234 T Cells and Ibrutinib-Treated REC1 Cells

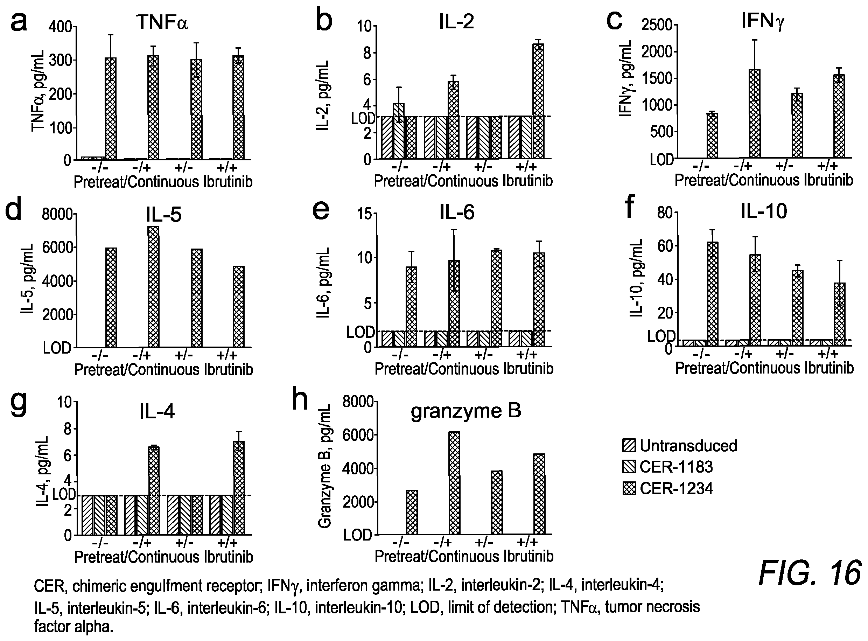

The following categories of cytokines were induced in co-cultures of CER-1234 T cells and target REC-1 cells in the presence of 0.5 µM ibrutinib (FIGS 16A to 16H): T1 - tumor necrosis factor alpha (TNF-α), interferon gamma (IFN-γ), and interleukin (IL)-10; T2 - IL-5; Effector - granzyme B. Cytokine expression was not induced in co-cultures containing CER-1183 T cells, which contain Tim-4 but lack intracellular signaling domains. IL-2 (homeostatic), IL-4 (T2), and IL-6 (inflammatory) were nominally increased. IL-12p70 (APC), IL-17A (inflammatory), and IL-1β (inflammatory) were at the limit of detection for all co-culture conditions.

### EXAMPLE 5: CHIMERIC TIM4 RECEPTOR T CELL THERAPY ELICITS PHOSPHATIDYLSERINE DEPENDENT CYTOTOXIC AND ANTIGEN-PRESENTING CELL-LIKE FUNCTION AND SYNERGIZES WITH APPROVED BTK INHIBITORS AGAINST HEMATOLOGIC MALIGNANCIES

Tumor microenvironments largely suppress immune responses, in part due to dysfunction of antigen presentation. While activated conventional αβ T cells are nominally capable of processing and displaying antigens, they are limited in antigen-presenting capacity due to inefficient antigen capture. Chimeric Tim4 receptors facilitate antigen-presenting cell (APC)-like function and impart target-dependent cytotoxic responses. Phosphatidylserine (PS) receptor Tim-4, which plays a central role in cross-presentation in subsets of dendritic cells, was fused with innate signaling domains to drive tumor antigen uptake and enhance APC-like function. Using Chimeric Tim4 receptor T cells engineered with Tim-4 binding domain fused with a toll/interleukin-1 (TIR) domain and the T cell-derived signaling domains CD28 and CD3ζ, APC-like and cytotoxic responses were tested against Mantle Cell Lymphoma (MCL) cell lines.

Chimeric Tim-4 receptor constructs were designed to enhance target-dependent phagocytosis and cytotoxicity. Using an engineered TMEM30a phospholipid flippase knockout JeKo-1 lymphoma cell line with constitutive phosphatidylserine exposure (JeKo-1 PS⁺), tumor fragment uptake, cytotoxicity, cytokine secretion (GrB, IFN-γ, and TNF-α), and APC-like activity were evaluated in vitro. JeKo-1 PS⁺ cells were labeled with the pH-sensitive pHrodo^{™} dye to quantify the uptake of tumor cells. Chimeric Tim4 receptor-T cells were tested for antigen-display capacity by evaluating autologous HPV E7 TCR T cell activation and proliferation following Chimeric Tim4 receptor-T cell co-culture with JeKo PS⁺ cells and HPV-derived peptides. Finally, Chimeric Tim4 receptor T cell anti-tumor activity was examined in xenografted MCL models. Statistical analysis was performed using 2-way ANOVA.

CER-1234 (SEQ ID NO: 18) and CER-1236 (SEQ ID NO: 19) expressing T cells were engineered that readily express Tim-4 on the cell surface and differ in their orientation of the TLR2 TIR signaling domain relative to the T-cell derived signaling domains CD28 and CD3ζ. Both CER1234 and CER1236 T cells showed increased uptake of JeKo-1 PS⁺ tumor cell fragments (p < 0.001) upon co-culture in vitro. Internalization of pHrodo⁺JeKo-1 fragments was dependent on phosphatidylserine (PS) binding, and treatment with Cytochalasin D, an inhibitor of actin polymerization, or Bafilomycin A, a lysosome inhibitor, blocked tumor cell fragment uptake (p < 0.0001 and p < 0.0001). In BTK inhibitor (BTKi)-primed REC-1 cells, CER-1234 internalized fragments were localized to lysosomal compartments.

Upon in vitro co-culture with JeKo-1 PS⁺ cells, both CER T cell constructs displayed cytotoxic function, with CER-1234 eliminating 90% of targets at 96 hrs at low effector:target (E:T) ratios. BTKi treatment in combination with CER-1234 exhibited > 90% cytotoxicity, with production of Th1 cytokines (GrB, IFN-γ, and TNF-α). Lastly, CER T cells were evaluated for APC-like activity in vitro. HPV E7 TCR T cells showed a significant increase in proliferation (p < 0.05) at 72h relative to controls, indicating Tim-4 CER T cells are capable of processing, displaying, and triggering proliferation of antigen-specific T cells. In vivo, CER T cells reduced tumor burden relative to controls in xenograft models of MCL, with no overt morbidity observed.

CER1234 and/or CER1236 T cells overcome antigen presentation defects in tumor microenvironments by enhancing antigen acquisition, APC-like function, and cytotoxic responses. These CER T cells exhibit these combined functions in vitro, synergize with BTK inhibition, and mediate significant anti-tumor effects in vivo against a clinically relevant model of mantle cell lymphoma.

T cell antigen capture and presentation capabilities, combined with inducible and target-specific cytotoxic function in single T cells, suggest the potential for secondary immune responses via activation and enhancement of endogenous anti-tumor immunity.

### Methods

CER T cells containing the T-cell immunoglobulin and mucin domain containing-4 (TIM-4) receptor fused to various transmembrane and intracellular signaling domains were generated using healthy donor T cells (see Table 5 below).

**Table 5: CER Construct Designs**

| **Construct Name/SEQ ID NO:#** | **Binding Domain** | **Transmembrane Domain** | **Intracellular Signaling Domain** | **Intracellular Signaling Domain** | **Intracellular Signaling Domain** |
|---|---|---|---|---|---|
| wtTim-4/ SEQ ID NO:20 | Tim-4 | Tim-4 | Tim4 | | |
| CER1234/ SEQ ID NO:18 | Tim-4 | CD28 | CD28 | TLR2 | CD3ζ |
| CER1236/ SEQ ID NO:19 | Tim-4 | CD28 | CD28 | CD3ζ | TLR2 |
| Anti-CD19CAR/ SEQ ID NO:26 | Anti-CD19 scFv | CD28 | CD28 | CD3ζ | |

CER T-cell activation was assessed by measuring induction of IFN-gamma by plate- and cell-based assays. Single induction plate assay: varying concentrations of phosphatidylserine (PS) or phosphatidylethanolamine (PE), a control membrane phospholipid not recognized by wild type TIM-4 (wtTIM-4), were coated onto high-binding tissue culture plates. CER T cells were added and IFN-γ levels were measured 24 hours after ligand exposure by ELISA. EC50 concentrations were determined by nonlinear regression (agonist vs. response - variable slope (4 parameters)). Induction by recursive stimulation plate assay: CER T cells were added to phosphatidylserine coated plates (2.5-20 µg/mL PS coating). Five days later, CER T cells were transferred to uncoated plates with fresh T cell optimizer media for 24 hours. Then, CER T cells were transferred again to a fresh PS coated plate. Supernatant was collected to assess IFN-γ induction by ELISA at various timepoints during re-stimulation (24, 72, and 120 hours). Cell-based assay: cytokine induction was assessed by multiparametric ELISA using conditioned supernatants harvested after 120 hours form the cytotoxicity assay described below.

CER T-cell cytotoxic function was evaluated using the Incucyte^{®} live-cell analysis system in co-culture assays with Jeko1 TMEM30A^{-/-} cells (engineered by knockout of the TMEM30A gene (a flippase chaperone required for phosphatidylserine (PS) internalization)) or REC-1 MCL cells. REC-1 MCL cells engineered to constitutively express mCherry were pretreated with 100 nM ibrutinib or no drug for 96 hours. CER T cells (expanded for 6 days after transduction) in T-cell optimizer media containing IL-2, IL-7, and IL-15 were co-cultured with A2780 cells at an effector:target (E:T) ratio of 0.25:1 in the presence of the same concentration of ibrutinib.

Expression of TIM-4, CD45RA, and CCR7 as well as CD4/CD8 ratios were evaluated by flow cytometry using commercially available fluorochrome-conjugated antibodies. Proliferation was measured by flow cytometry using precision count beads to determine the absolute counts of viable T cells at the end of the co-culture. T-cell proliferation is reported as the fold expansion of T cell counts at 120 hours relative to 0 hours.

Antigen-presenting cell (APC)-like function of CER T cells was assessed after a 48-hour co-culture with phosphatidylserine-positive E7 oncoprotein-positive cells. T cells were separated and co-cultured for 4 days with cell trace violet-labeled E7 TCR T cells. The activation marker HLA-DR on E7 TCR T cells was analyzed using flow cytometry. Phosphatidylserine-positive SCC152 squamous cell carcinoma cells were engineered by knockout of the TMEM30A gene (a flippase chaperone required for PS internalization). The role of HLA-I in APC-like function was determined by performing the same experiment in the presence of HLA-I blocking or the corresponding isotype antibodies.

### Results:

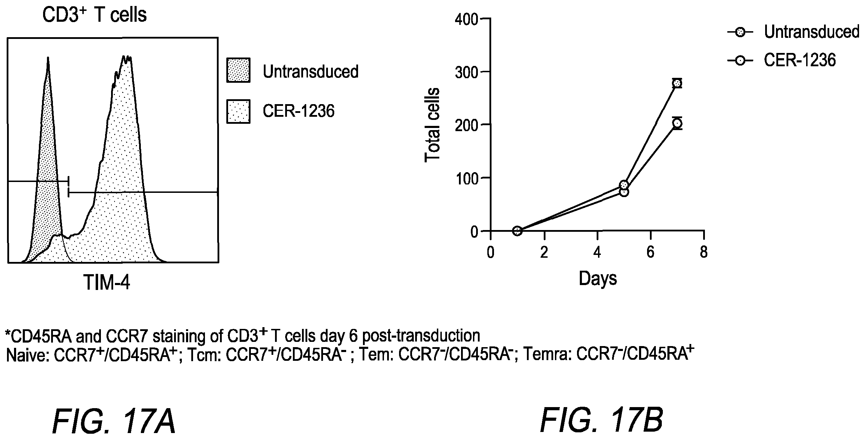

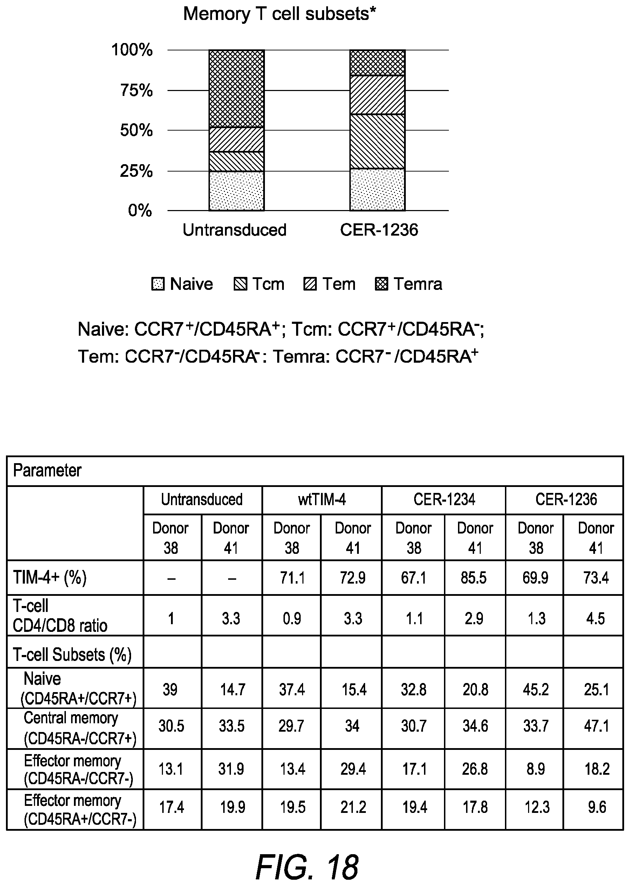

CERs (CER1234 and CER1236) according to Table 5 were constructed and expressed in T cells. CER T cells readily express TIM-4 on transduced T cells 6 days post transduction (FIG. 17A). CD4:CD8 ratios were similar after transduction and expansion of CER T cells vs. untransduced T cells. CER1234 or CER1236 T cells exhibited a predominantly naive and central memory phenotype as measured by CCR7 and CD45RA expression **(****FIG. 18****).**

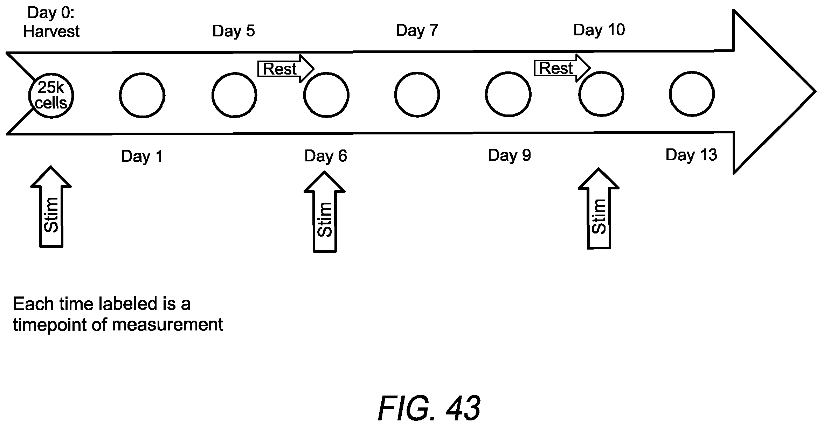

CER1234 or CER1236 T cells specifically produce IFN-γ in a dose-dependent manner in response to binding to phosphatidylserine (PS), but not to phosphatidylethanolamine (PE) **(****FIGS. 19A-19B****).** wtTIM-4, which lacks intracellular T-cell signaling moieties, did not induce IFN-γ upon phosphatidylserine stimulation. CER-1236 T cells elicited repeated IFN-γ response upon 3 serial rounds of exposure to phosphatidylserine **(****FIG. 19C****).** Assay design is shown in **FIG. 43****.** The ability of CER-1236 T cells to become reactivated upon additional antigen exposure in vitro suggests a durable effect in vivo.

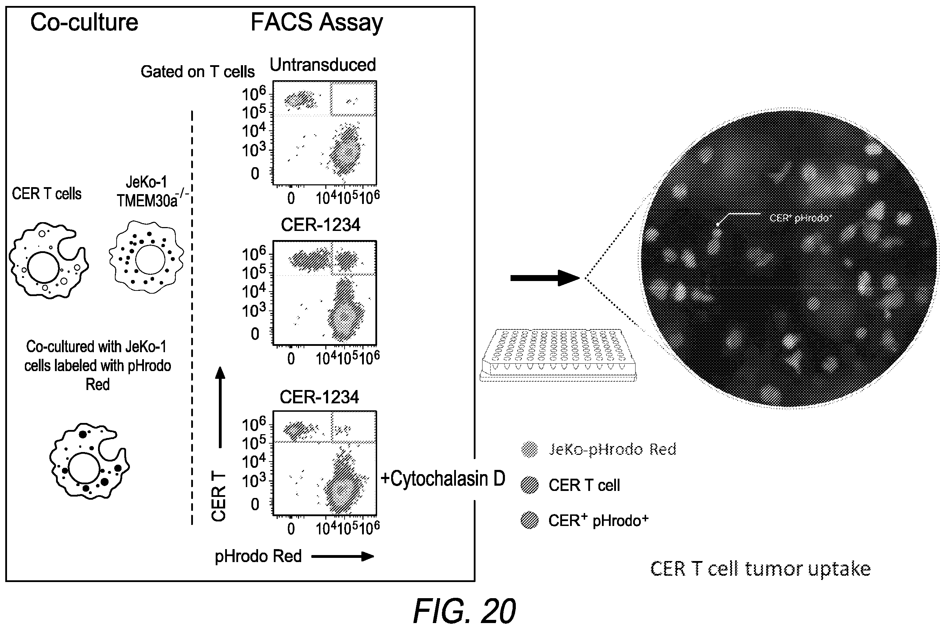

FACS and imaging based detection of pHrodo dye-labeled tumor cell (Jeko1 TMEM30A^{-/-} cells) phagocytosis and endocytosis by CER1234 T cell is shown in **FIG. 20****.**

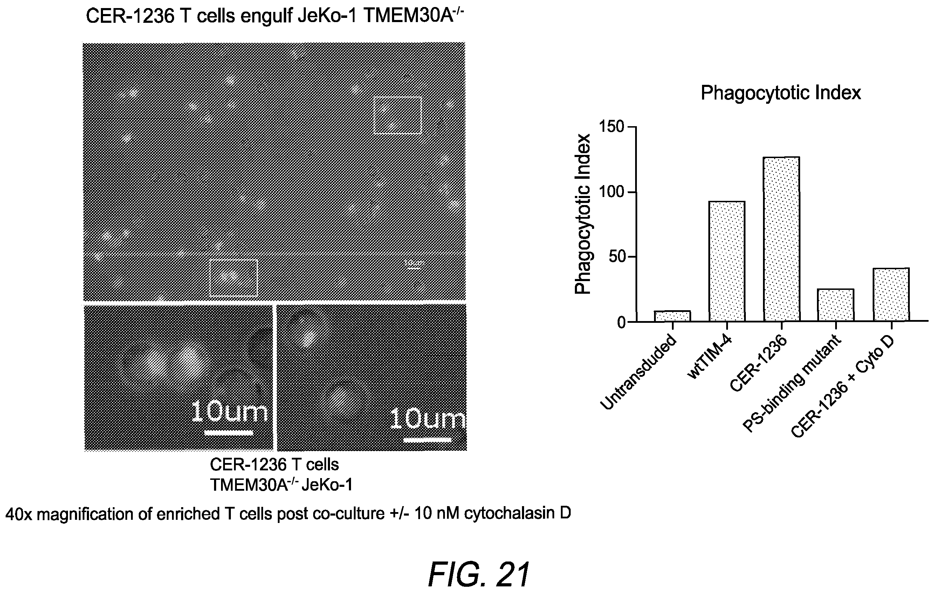

CER1236 T cells exhibited increased engulfment of Jeko1 TMEM30A^{-/-} cells **(****FIG. 21****).** Engulfment is enhanced for CER1236 T cells versus wtTIM-4 T cells. Engulfment is blocked by mutation of the TIM-4 phosphatidylserine-binding domain (CER1250) or an actin polymerization inhibitor. CER-1250 (Tim-4 binding mutant/CD28/TLR2/CD3z; SEQ ID NO:24) has the same intracellular signaling domains as CER1236 but has a mutated IgV domain with AAAA substitution to abrogate phosphatidylserine binding. Higher frequency and magnitude of uptake (phagocytic index) was observed by CER-1236 T cells compared to wtTim-4, CER1250 and CER1236 + actin polymerization inhibitor.

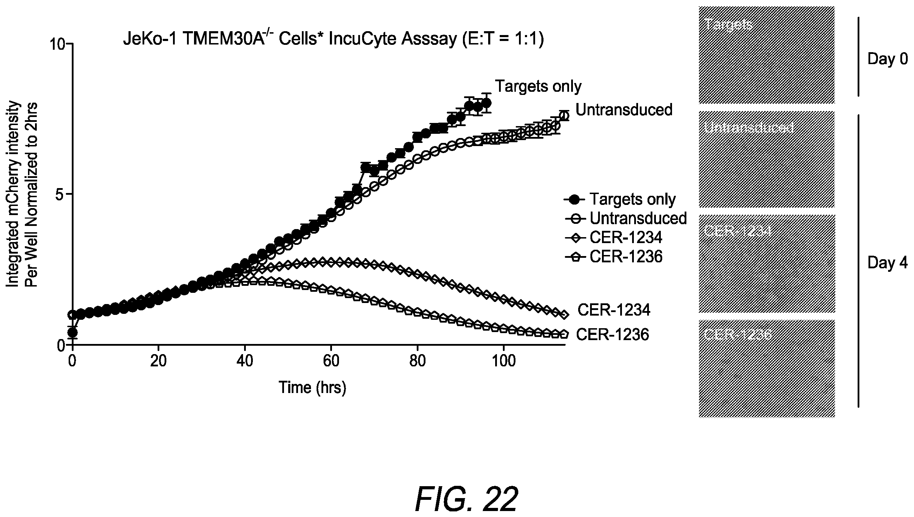

CER1234 or CER1236 T cells efficiently eliminate Jeko1 TMEM30A^{-/-} cells in co-culture assays **(****FIG. 22****).**

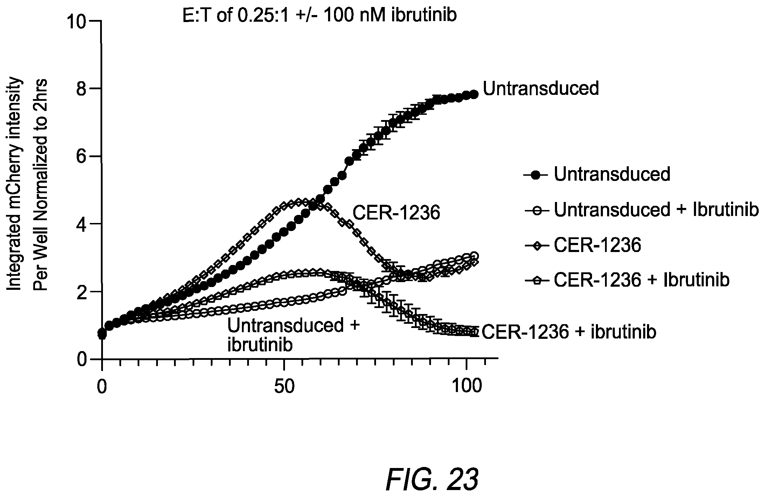

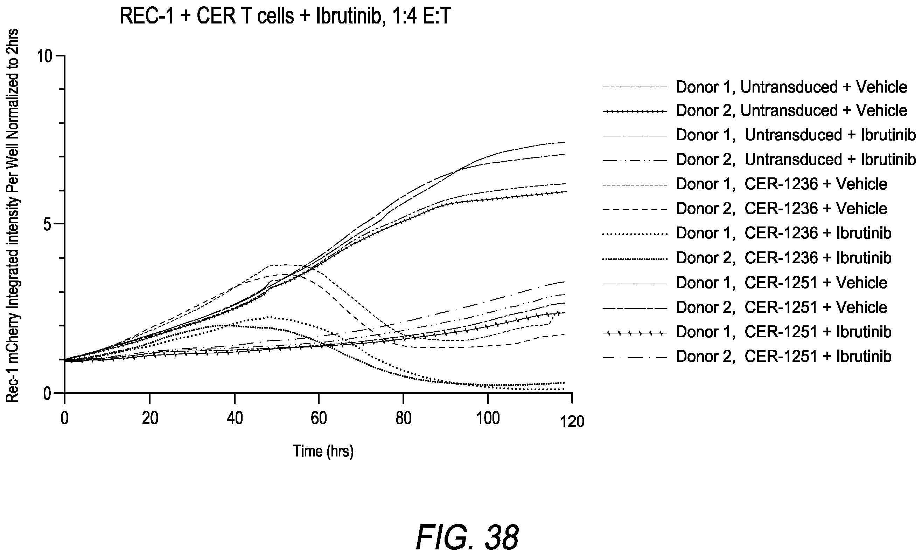

CER1236 T cells synergize with ibrutinib to enhance killing of REC-1 mantle cell lymphoma cells. BTK pathway inhibition elicits cell stress and membrane phosphatidylserine exposure. Once primed, CER1236 T cells eliminate tumor cells **(****FIG. 23****).** CER1236 T cells have enhanced and more complete cytotoxic effect against ibrutinib-treated REC-1 MCL cells but not untreated REC-1 MCL cells **(****FIG. 38****).** Some CER1236 T cytotoxicity against untreated REC-1 cells is observed, consistent with the constitutive phosphatidylserine exposure measured on this cell line. CER1251 (Tim-4 binding mutant/CD28/CD3z/TLR2; SEQ ID NO:25) has the same intracellular signaling domains and orientation as CER1236 but has a mutated IgV domain with AAAA substitution to abrogate phosphatidylserine binding.

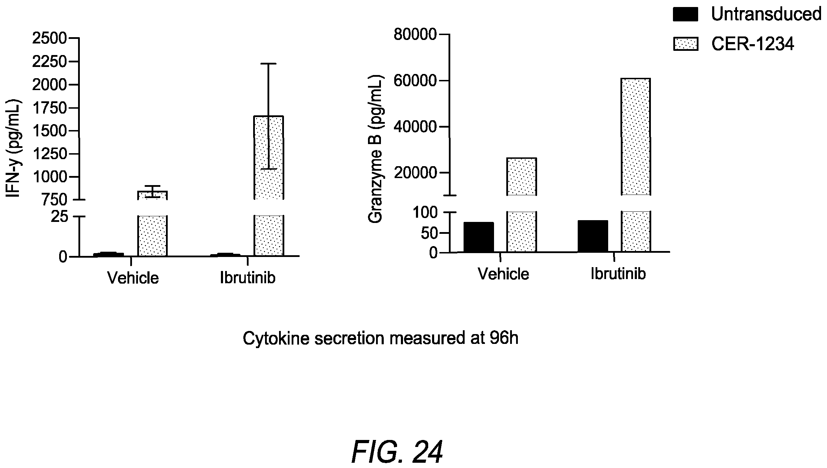

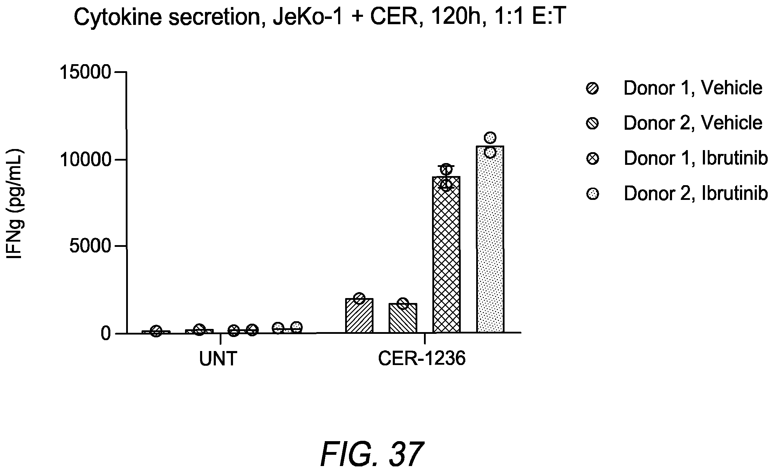

In line with cytotoxic synergy, CER1234 cells or CER1236 cells plus BTK inhibition with ibrutinib demonstrate inducible cytokine production **(****FIG. 24** **and** **FIG. 37****).**

CER1236 T cells demonstrate antigen-presenting cell-like function in an in vitro system. In an E7 HLA2-restricted TCR system, CER1236-T cells supported greater activation of autologous E7 TCR T cells, indicative of enhanced E7 antigen presentation by CER-1236 T cells, when compared to untransduced T cells or anti-CD19 CAR T cells **(****FIG. 25A****).** CER1236 T-cell-induced E7 TCR activation is dependent on antigen presentation via the HLA class I system **(****FIG. 25B****).** CER1236 T cells can acquire antigen, a function that was considered to be a barrier in their ability to process and present antigen to naive T cells. The engineered antigen presenting cell (APC) function of CER1236 T cells may lead to initiation of secondary immune responses in vivo.

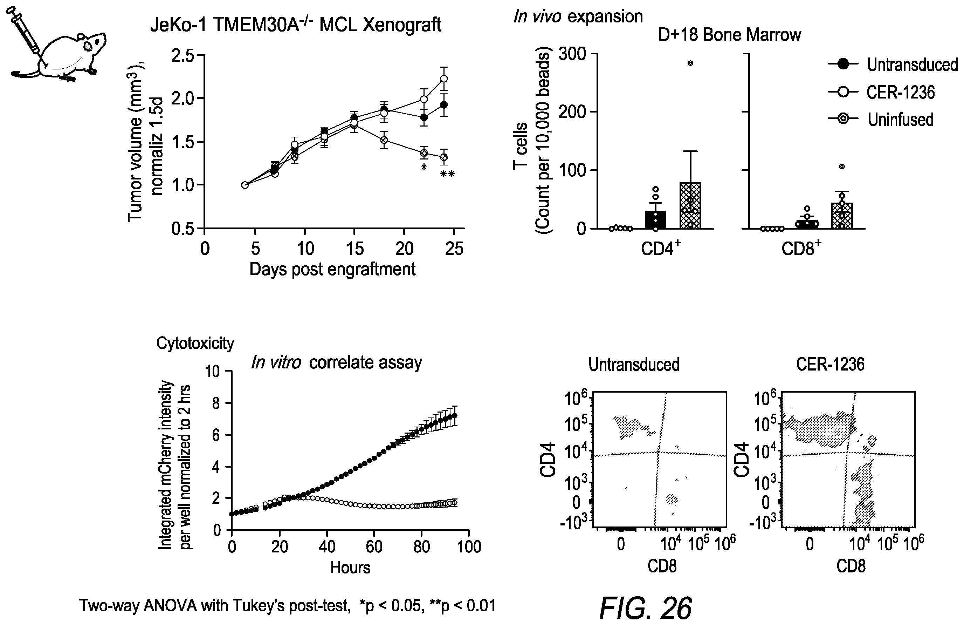

CER1236 T cells showed anti-tumor effect against Jeko1 TMEM30A^{-/-} cells subcutaneous tumor engraftment in mantle cell lymphoma mouse model **(****FIG. 26****).**

### EXAMPLE 6: T CELL PHENOTYPING ON CHIMERIC TIM4 RECEPTOR TRANSDUCED T CELLS CO-CULTURED WITH PARP INHIBITOR AND TARGET OVARIAN CANCER CELLS

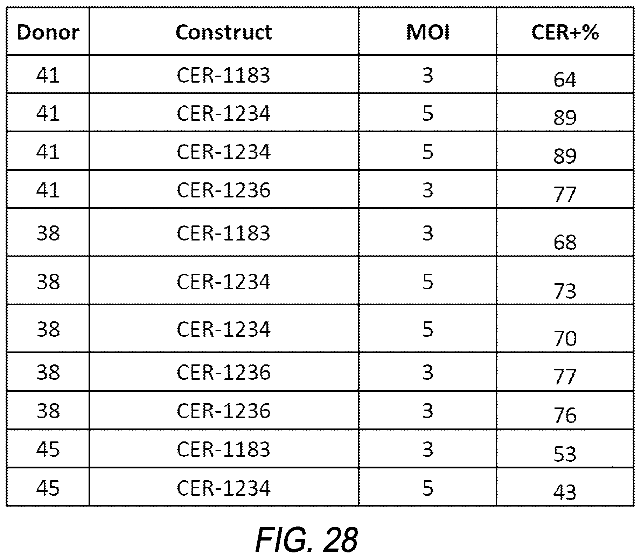

For assays described herein, A2780 (human ovarian cancer cell line) cells were plated, allowed to adhere and at day -2, treated wotj PARP inhibitor (PARPi) at effective concentrations that killed 25% cells (EC25), niraparib at 0.5 µM or olaparib at 1.5 µM. At day 0, media was exchanged and donor T cells from three subjects **(****FIG. 28****)** transduced with CER-1234, CER-1236, or control CER-1183 **(****FIG. 27A****)** were added at various effector:target ratios to A2780 cells treated with PARPi to assess chimeric Tim4 receptor-T cell activation, cytokine production, proliferation, and cytotoxic responses. Chimeric Tim4 receptor-T cells immunophenotyping and bulk supernatant analysis was performed on day 2 and day 5 following initiation of co-culture.

CCR7 expression was measured following 120 hours co-culture by measuring % CCR7 + cells in Tim4+ T cells **(****FIGS. 29A-29C****).** Higher CCR7 expression was observed on T cells expressing CER-1234 and CER-1236 co-cultured with olaparib-treated A2780 cells. CCR7 is expressed at high levels on naive and central memory T cells and enables homestasis T cell subsets to recirculate and home to T cell areas in lymphoid organs, such as the white pulp areas of the spleen and lymph nodes. CCR7 expressing T cells may reflect a population of T cells of early differentiation status.

CD4/CD8 ratio was measuring following 120 hours co-culture with A280 cells treated with PARPi **(****FIGS. 30A-30C****).** CER-1236 transduced T cells had higher CD4/CD8 ratios compared to T cells transduced with CER-1234 or control CER-1183 in both donor 38 and donor 41 samples.

### EXAMPLE 7: TIM4 CHIMERIC RECEPTOR-T CELLS ELICIT PHOSPHATIDYLSERINE-DEPENDENT CYTOTOXIC AND INNATE-LIKE FUNCTION AND SYNERGISZES WITH APPROVED PARP INHIBITORS IN AN OVARIAN CANCER MODEL

CER T cells were assessed in combination with approved small molecule inhibitors of poly (ADP-ribose) polymerase (PARP) enzymes in a PARP-sensitive ovarian cancer model.

Phosphatidylserine exposure on A2780 ovarian cancer cells upon treatment with the small molecule PARP inhibitors niraparib or olaparib was evaluated by annexin v staining to determine the appropriate drug dose. Phosphatidylserine targeting Tim-4 CER constructs designed to enhance cytotoxicity and target-dependent tumor cell fragment uptake were engineered. CER T cell designs were as follows: CER-1234 (Tim-4/CD28/TLR2/CD3z; SEQ ID NO:18); CER-1236 (Tim-4/CD28/CD3z/TLR2; SEQ ID NO:19); and CER-1250 (Tim-4 binding mutant/CD28/TLR2/CD3z; SEQ ID NO:24). CER-1250 has the same intracellular signaling domains as CER1236 but has a mutated IgV domain with AAAA substitution to abrogate phosphatidylserine binding. Niraparib (0.5µM) and olaparib (1.5µM) were used to expose phosphatidylserine on the surface of A2780 cells for CER T cell recognition and cytotoxicity, T-cell activation, and cytokine responses were evaluated. Uptake of antigen by CER-1236 T cells was modeled with human papilloma virus E7 antigen-expressing squamous carcinoma (SCC152) TMEM30A phospholipid flippase knockout cells. These cells constitutively exposed outer surface phosphatidylserine. E7 antigen presentation by CER-1236 T cells was assayed by surface expression of HLA-DR, a marker of T cell activation, on autologous HLA-A2-restricted E7 TCR T cells.

PARP inhibitors induced phosphatidylserine exposure on the outer cell surface at subtherapeutic doses in viable A2780 cells after 96 hours of treatment. Compared to untreated, 23% of A2780 cells were eliminated after addition of niraparib and 34% were eliminated after olaparib addition. CER-1234 or CER-1236 eliminated A2780 cells by 39% or 19%, respectively. Niraparib synergized with CER-1234 or CER-1236 to eliminate A2780 cells by 58% or 81% after 5 days of co-culture, while olaparib eliminated 66% of targets with CER-1234 or 73% with CER-1236. Interferon-γ increased by 7-fold and granzyme B increased by 5-7-fold for either drug combined with CER-1234 at 96 hours compared to CER-1234 with no drug. Relative to CER-1236 T cells alone, CER-1236 T cells exposed to TMEM30A⁻ SCC152 cancer cells increased the activation status of E7 TCR T cells 5-fold, demonstrating partial engulfment, antigen processing and presentation of HLA-A2 cognate peptide.

CER1234 and CER1236 T cells exhibit adaptive and innate functionality. CER1234 and CER1236 T cells have demonstrated cytolytic activity against A2780 ovarian cancer cells treated with subtherapeutic doses of PARP inhibitors. CER1236 demonstrated enhanced APC-like functionality demonstrated in the HPV E7 model. The combined functionality of CER T cell products provides a promising clinical application as a treatment option for patients with ovarian cancer receiving approved PARP inhibitors.

### Methods

The induction of surface phosphatidylserine expression on A2780 ovarian cells treated with the PARP inhibitors olaparib and niraparib was measured by flow cytometry (FC) using annexin V.

CER T cells containing the T-cell immunoglobulin and mucin domain containing-4 (TIM-4) receptor fused to various transmembrane and intracellular signaling domains were generated using healthy donor T cells (see Table 5).

CER T-cell activation was assessed by measuring induction of IFN-gamma by plate- and cell-based assays. Single induction plate assay: varying concentrations of phosphatidylserine (PS) or phosphatidylethanolamine (PE), a control membrane phospholipid not recognized by wild type TIM-4 (wtTIM-4), were coated onto high-binding tissue culture plates. CER T cells were added and IFN-γ levels were measured 24 hours after ligand exposure by ELISA. EC50 concentrations were determined by nonlinear regression (agonist vs. response - variable slope (4 parameters)). Induction by recursive stimulation plate assay: CER T cells were added to phosphatidylserine coated plates (2.5-20 µg/mL PS coating). Five days later, CER T cells were transferred to uncoated plates with fresh T cell optimizer media for 24 hours. Then, CER T cells were transferred again to a fresh PS coated plate. Supernatant was collected to assess IFN-γ induction by ELISA at various timepoints during re-stimulation (24, 72, and 120 hours). Cell-based assay: cytokine induction was assessed by multiparametric ELISA using conditioned supernatants harvested after 120 hours from the cytotoxicity assay described below.

CER T-cell cytotoxic function was evaluated using the Incucyte^{®} live-cell analysis system in co-culture assays with A2780 cells. A2780 cells engineered to constitutively express mCherry were pretreated with 1.5 µM olaparib, 0.5 µM niraparib or no drug for 96 hours. CER T cells (expanded for 6 days after transduction) in T-cell optimizer media containing IL-2, IL-7, and IL-15 were co-cultured with A2780 cells at an effector:target (E:T) ratio of 1:2 in the presence of the same concentrations of olaparib or niraparib.

Expression of TIM-4, CD45RA, and CCR7 as well as CD4/CD8 ratios were evaluated by flow cytometry using commercially available fluorochrome-conjugated antibodies. Proliferation was measured by flow cytometry using precision count beads to determine the absolute counts of viable T cells at the end of the co-culture. T-cell proliferation is reported as the fold expansion of T cell counts at 120 hours relative to 0 hours.

Antigen-presenting cell (APC)-like function of CER T cells was assessed after a 48-hour co-culture with phosphatidylserine-positive E7 oncoprotein-positive cells. T cells were separated and co-cultured for 4 days with cell trace violet-labeled E7 TCR T cells. The activation marker HLA-DR on E7 TCR T cells was analyzed using flow cytometry. Phosphatidylserine-positive SCC152 squamous cell carcinoma cells were engineered by knockout of the TMEM30A gene (a flippase chaperone required for PS internalization). The role of HLA-I in APC-like function was determined by performing the same experiment in the presence of HLA-I blocking or the corresponding isotype antibodies.

### Results

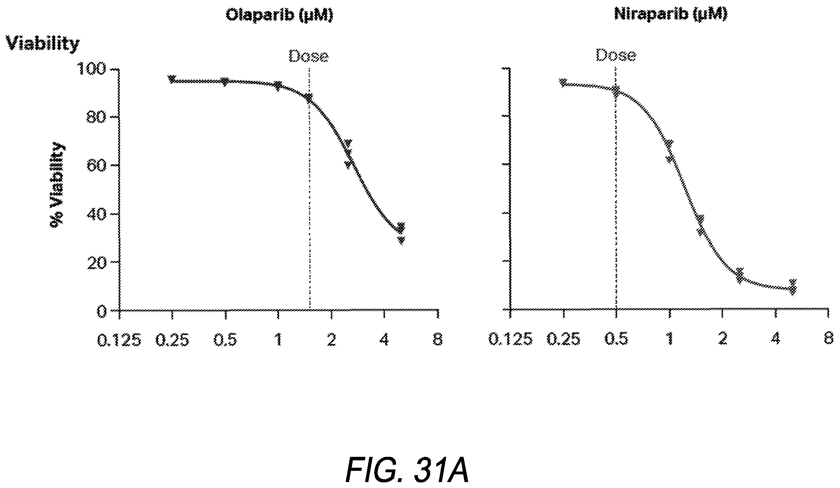

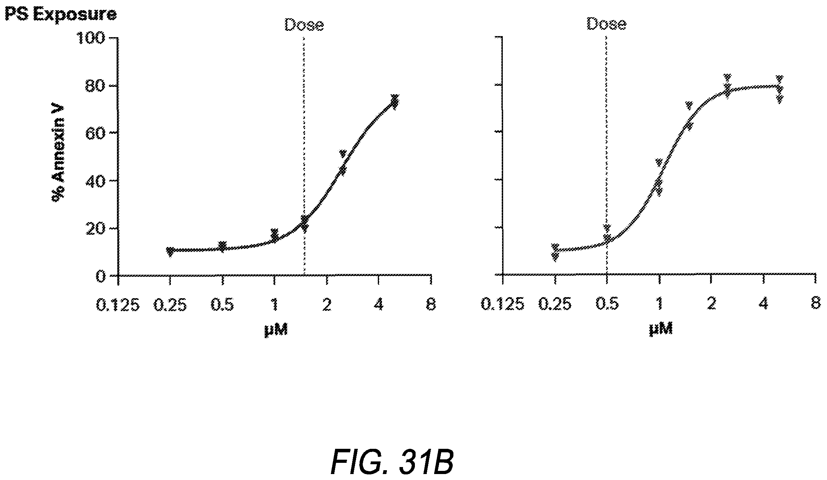

Subtherapeutic doses of niraparib and olaprib induce phosphatidylserine exposure on viable A2780 cells (FIGS. 31A-31B). Based upon these curves, a dose of 1.5 µM was selected for olaparib and 0.5 µM was selected for niraparib for combination therapy with CER T cells as noted by the dotted line.

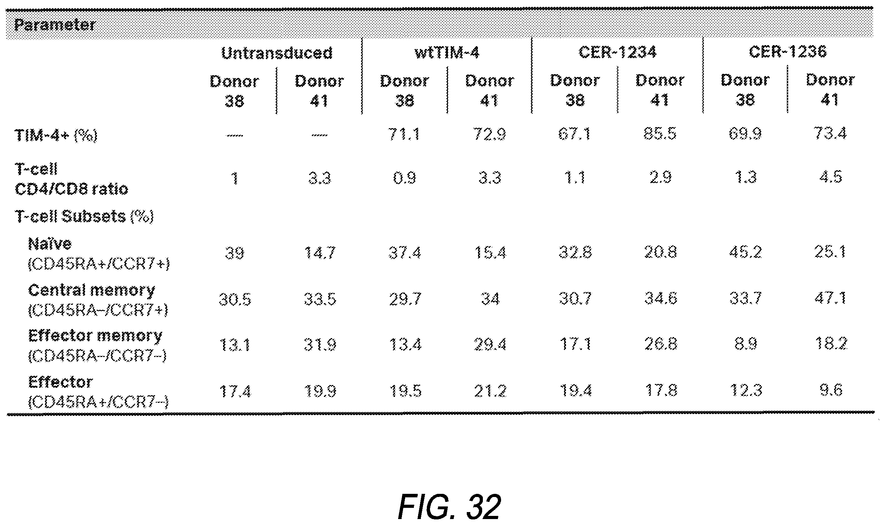

CER constructs were designed as single chain chimeric proteins according to Table 5. Phenotypes of CER T cells were generally consistent across 2 donors (FIG. 32). High proportions of CER T cells express the engineered Tim-4 receptor. CD4:CD8 ratios were similar after transduction and expansion of CER T cells vs. untransduced T cells. CER1234 or CER1236 T cells exhibited a predominantly naïve and central memory phenotype as measured by CCR7 and CD45RA expression.

CER1234 or CER1236 T cells produced concentration-dependent IFN-γ responses upon stimulation with phosphatidylserine (FIGS. 33A-33B). CER T cells show target-specific cytokine induction to phosphatidylserine, but not to phosphatidylethanolamine. Saturation of IFN-γ induction by CER T cells occured at 5 to 10 µg/mL of phosphatidylserine. wtTIM-4, which lacks intracellular T-cell signaling moieties, did not induce IFN-γ upon phosphatidylserine stimulation. CER-1236 T cells elicited repeated IFN-γ response upon 3 serial rounds of exposure to phosphatidylserine (FIG. 33C). The ability of CER-1236 T cells to become reactivated upon additional antigen exposure in vitro suggests a durable effect in vivo.

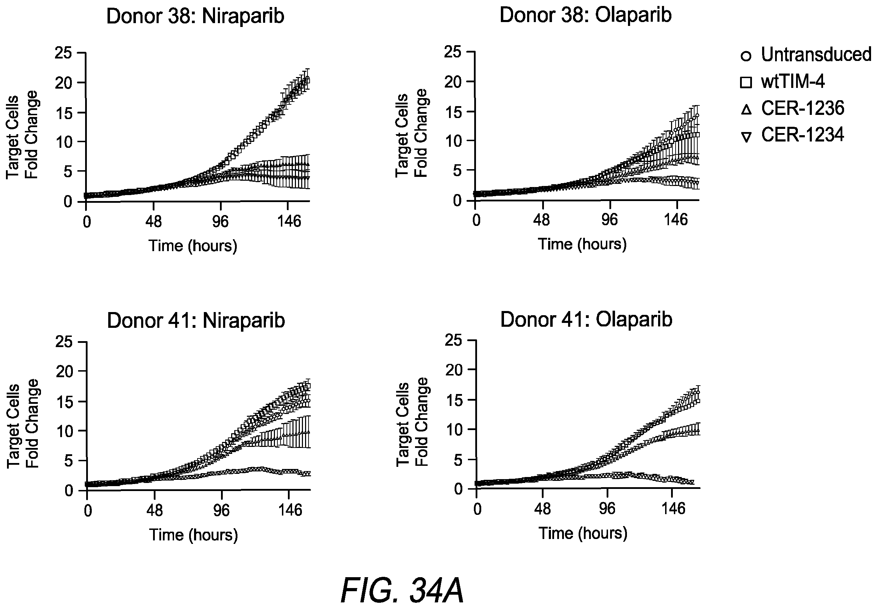

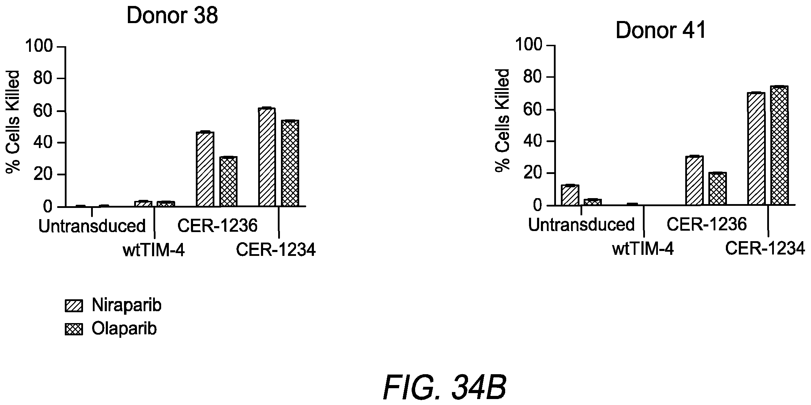

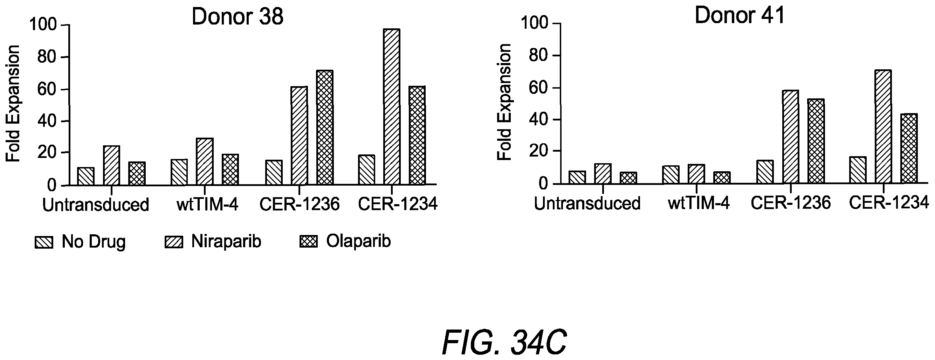

CER1234 or CER1236 T cells demonstrated synergistic cytotoxic activity with niraparib and olaparib aginst A2780 cells. Tumor cell killing was enhanced by the combination of CER-1236 or CER-1234 T cells with niraparib or olaparib (FIGS. 34A-34B). wtTIM-4 T cells (no intracellular signaling domain) show no additive effect with niraparib or olaparib. CER-1236 and CER-1234 T cells alone exhibited limited cytotoxic activity. CER-1234 and CER-1236 prolilferated in cocultures treated with PARPi compared to co-culture with wtTIM-4 expressing T cells or no drug **(****FIG. 34C****).** Normalized to 0 days, 0 hours, and 0 minutes of co-culture. A2780 cells express mCherry. Effector:Target (E:T) ratio is 1:2 for **FIGS. 34A** and **4B.** E:T ratio is 1:1 for **FIG. 34C****.** Niraparib = 0.5 µM, Olaparib = 1.5 µM.

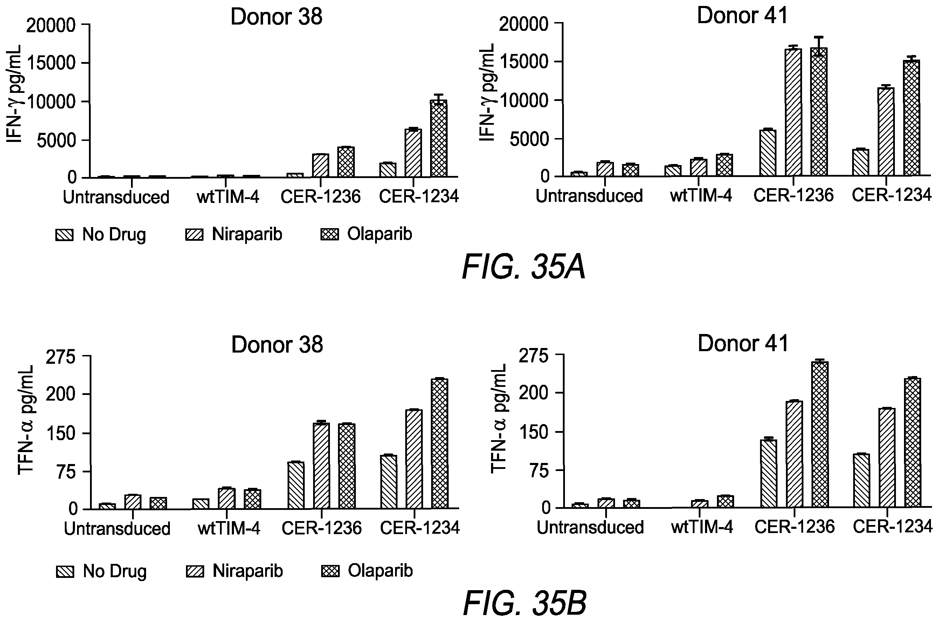

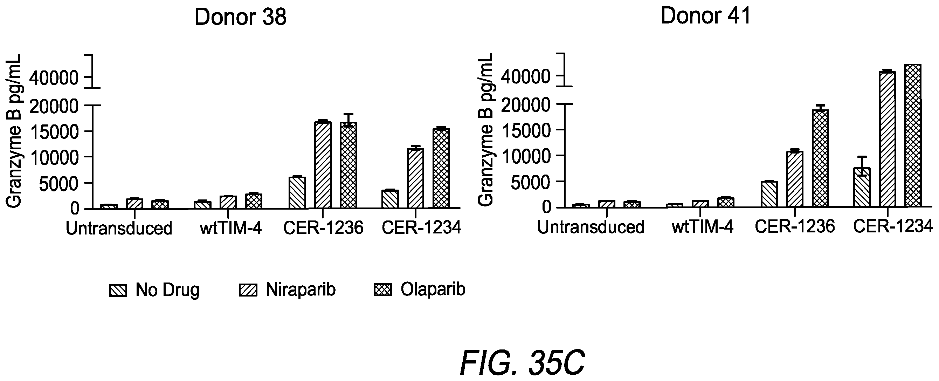

CER1234 or CER1236 T cells are activated and produce IFN-γ in response to Co-culture with PARP inhibitor-treated A2780 Ovarian Cancer Cells. TNF-α **(****FIG. 35A****),** IFN-γ **(****FIG. 35B****),** granzyme B **(****FIG. 35C****)** were induced by CER1234 or CER1236 T cells but not by untransduced or wtTIM-4 T cells. TNF-α, IFN-γ, and granzyme B were induced in the presence of PARP inhibitor but not by vehicle. Effector:Target ratio is 1:1; niraparib. Niraparib = 0.5 µM, Olaparib = 1.5 µM.

CER1236 T cells demonstrate antigen-presenting cell-like function in an in vitro system. In an E7 HLA2-restricted TCR system, CER1236-T cells supported greater activation of autologous E7 TCR T cells, indicative of enhanced E7 antigen presentation by CER-1236 T cells, when compared to untransduced T cells or anti-CD19 CAR T cells **(****FIG. 36A****).** CER1236 T-cell-induced E7 TCR activation is dependent on antigen presentation via the HLA class I system **(****FIG. 36B****).** CER1236 T cells can acquire antigen, a function that was considered to be a barrier in their ability to process and present antigen to naive T cells. The engineered antigen presenting cell (APC) function of CER1236 T cells may lead to initiation of secondary immune responses in vivo.

### EXAMPLE 8: IN VITRO STUDIES OF CER1236 T CELLS IN COMBINATION WITH OSIMERTINIB

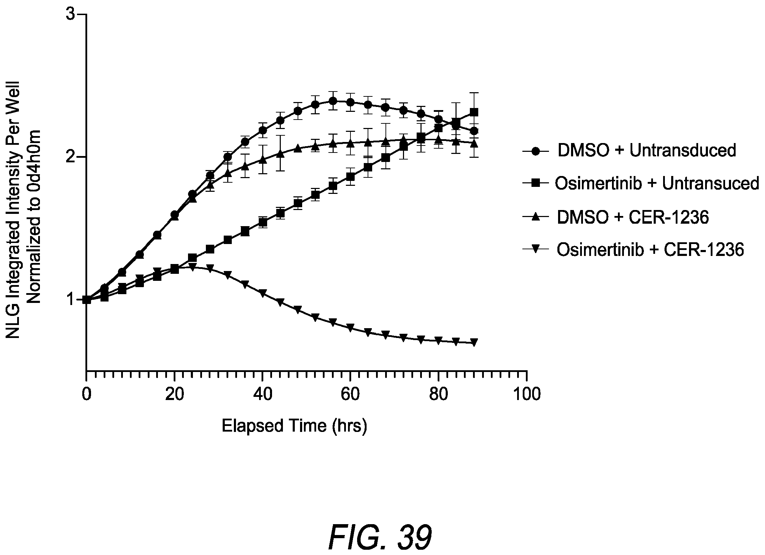

CER1236 T cells were tested in combination with EGFR tyrosine kinase inhibitor osimertinib on a non-small cell lung cancer (NSCLC) cell line. Ten-thousand NSCLC H1975 cells were cultured for 24 h with 100 nM osimertinib or vehicle (DMSO) and then co-cultured with 2500 CER1236 T cells at an Effector:Target cell ratio of 0.25:1 in the presence or absence of 4.88 nM osimertinib or vehicle. Tumor cell growth was monitored with indicated for 120 h in an Incucyte assay. As shown in **FIG. 39****,** the combination of osimertinib and CER1236 T cells synergizes to inhibit H1975 NSCLC cells in vitro.

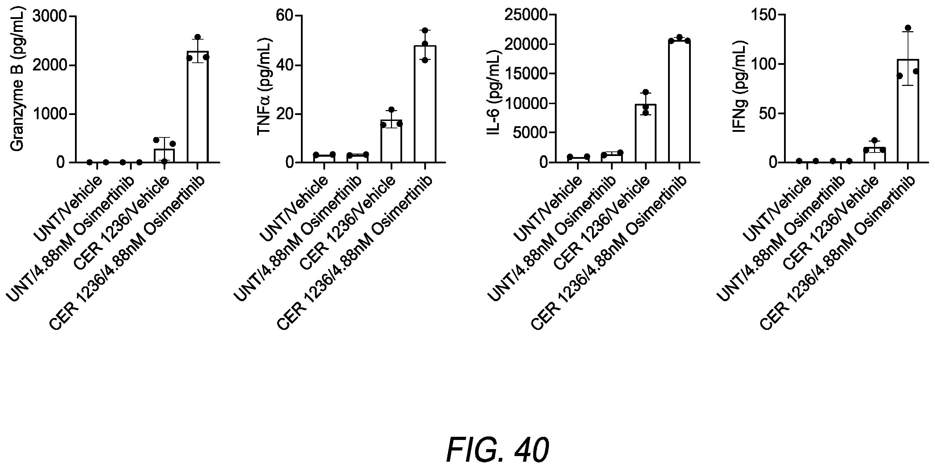

Cytokine production was also measured in CER1236 T cells co-cultured with H1975 NSCLC cells pre-treated with osimertinib or vehicle. Ten-thousand NSCLC H1975 cells were cultured for 24 h with 100 nM Osimertinib or vehicle (DMSO) and then co-cultured with CER T cells for 96h at an E:T of 1:4 in the presence or absence of 4.88nM Osimertinib or vehicle. Effector:Target ratio was 1:4, and H1975 cells were plated at 10,000 cells/well. Cytokine production was determined by Ella assay. As shown in FIG. 40, increased cytokine production (granzyme B, TNFα, IL-6 and IFNγ) was observed CER1236 T cells + osimertinib compared with CER1236 T cells + vehicle and untransduced T cells with or without osimertinib.

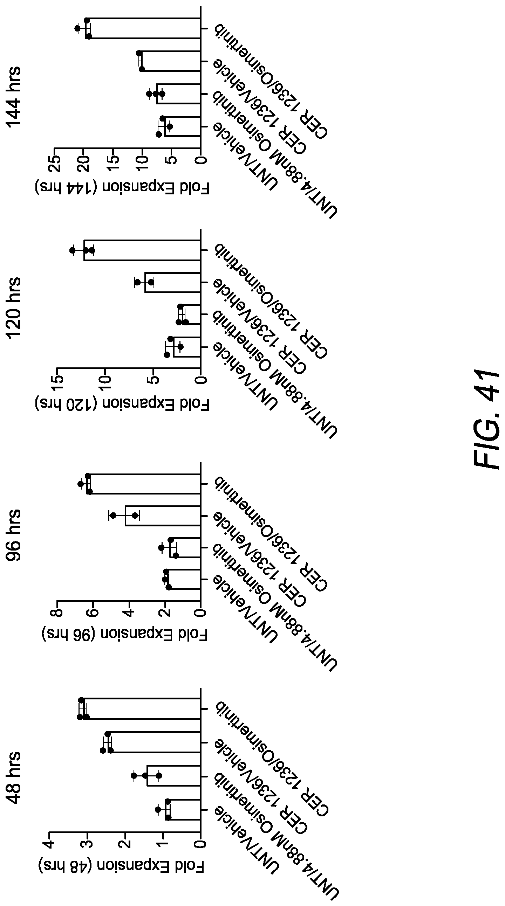

Proliferation of CER1236 T cells co-cultured with H1975 NSCLC cells pre-treated with osimertinib or vehicle was also measured. NSCLC H1975 cells were pretreated for 24h with 100nM of Osimertinib or DMSO followed by a continuous lower dose of 4.88nM Osimertinib/DMSO treatment in the presence of untransduced T cells or CER-1236 T cells at an Effector:Target ratio of 1:4. Total CD3+ T cells fold expansion was determined by quantitative flow cytometry using quantitation beads over a time course (48hrs, 96hrs, 120hrs, and 144hrs). Experiment was performed twice, once with 5,000 target H1975 cells and once with 10,000 target H1975 cells. As shown in **FIG. 41****,** osimertinib drives CER1236 T cell proliferation compared with CER1236 T cells + vehicle and untransduced T cells with or without osimertinib.

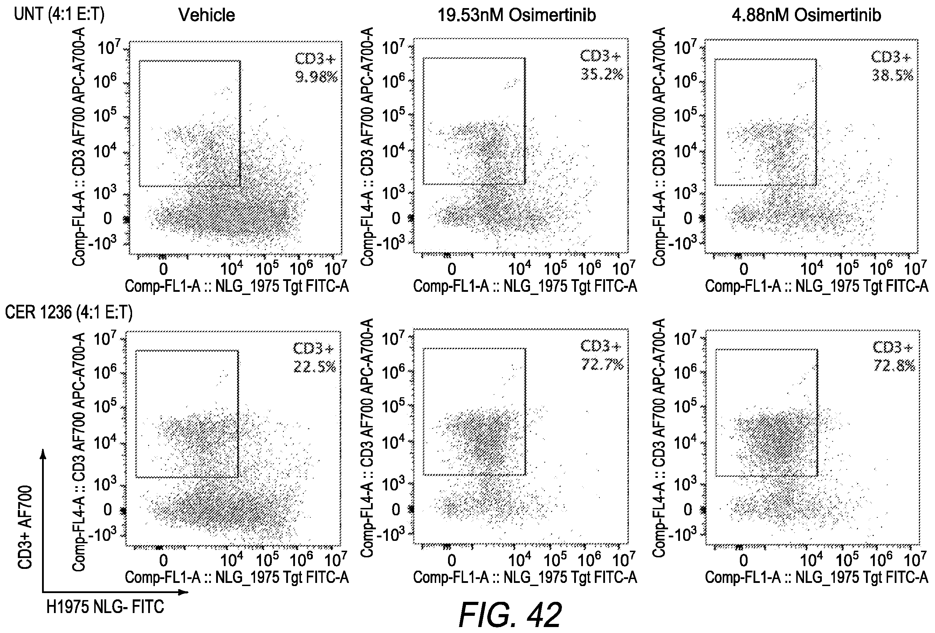

**FIG. 42** shows expansion of CD3+ T cells and reduction of H1975 target cells in experiments with CER1236 T cells co-cultured for 120 hours with H1975 NSCLC cells (4:1 effector:target ratio) pre-treated with osimertinib or vehicle as measured by flow cytometry. As shown in **FIG. 42****,** bottom row, CD3+ T cell proliferation was highest and H1975 cell numbers lowest in samples with CER1236 T cells co-culturewith H1975 cells treated with osimertinib.

### EXAMPLE 9: COMPARISON OF CER1234 T CELLS VS. CER1236 T CELLS

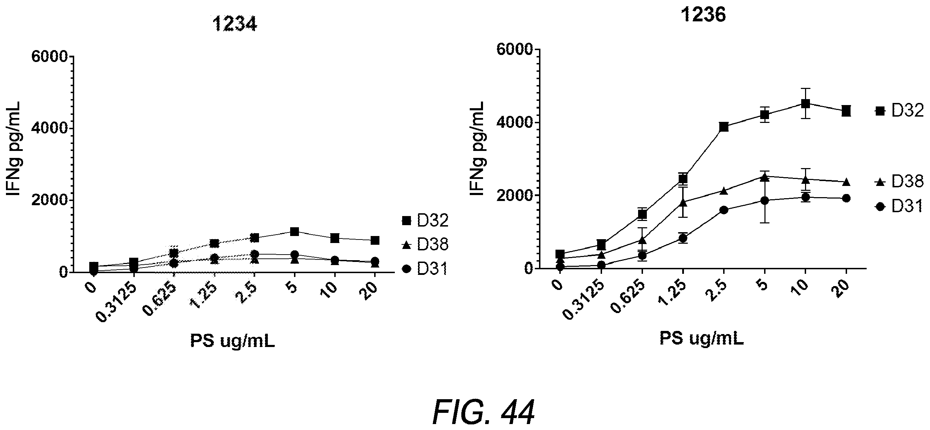

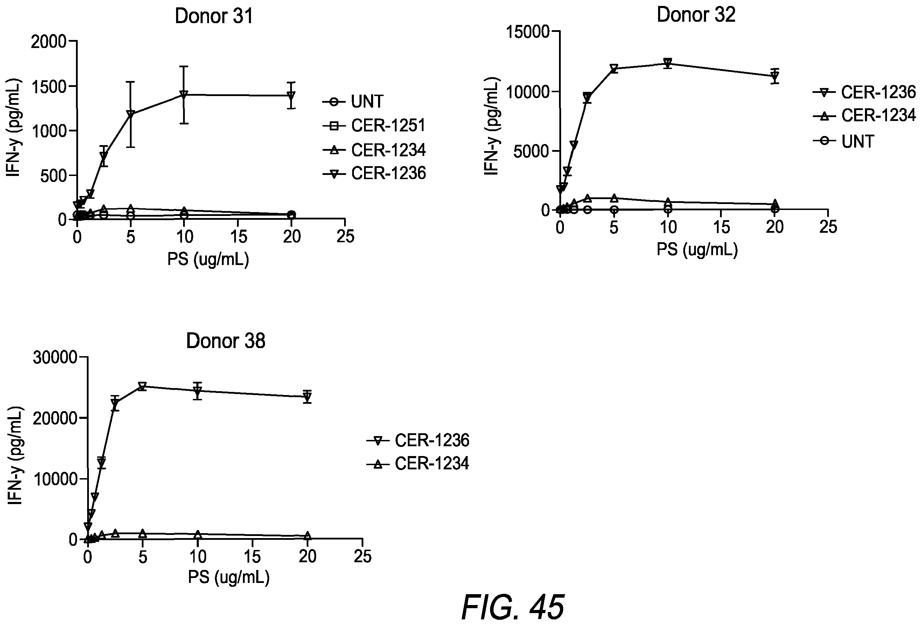

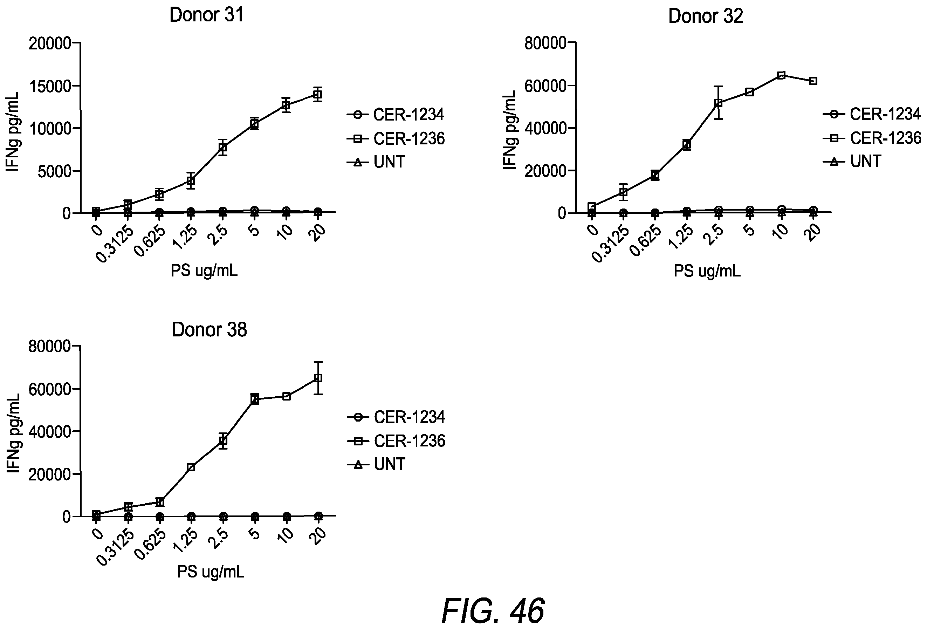

T cells obtained from three different donors were transduced with CER1234 or CER1236 and incubated on titrating doses of plate-bound phosphatidylserine. As shown in **FIG. 44****,** plate-bound phosphatidylserine can induce IFNγ in all donor T cells transduced with CER1236 and this induced response appears to be more consistent in CER1236 T cells than CER1234 T cells.**FIG. 45** shows IFNγ production in CER1234 T cells or CER1236 T cells by donor in response to increasing doses of plate-bound phosphatidylserine. CER1236 T cells from all donors shows strong activation. CER1234 T cells shows lower activation for donors 32 and 38, but CER1234 T cells from donor 31 did not pass. **FIG. 46** shows shows IFNγ production in CER1234 T cells or CER1236 T cells by donor in response to increasing doses of plate-bound phosphatidylserine at day 14 post-thaw. Again, CER1236 T cells produces more IFNγ across all donors compared to CER1234 T cells.

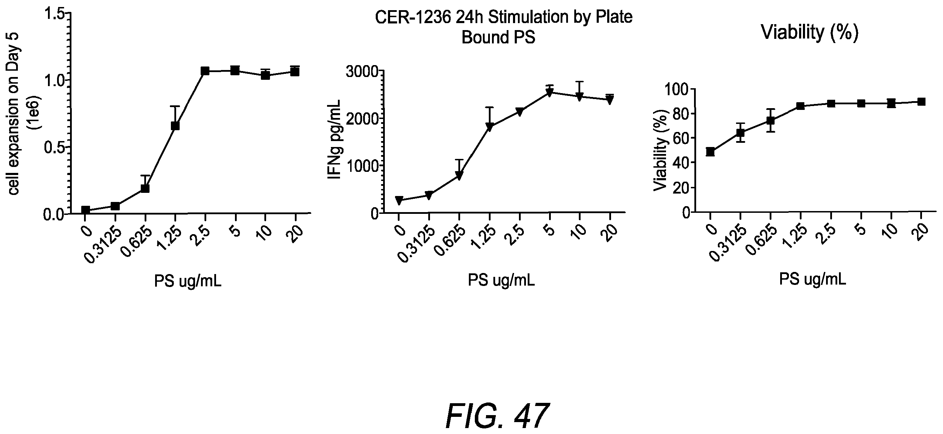

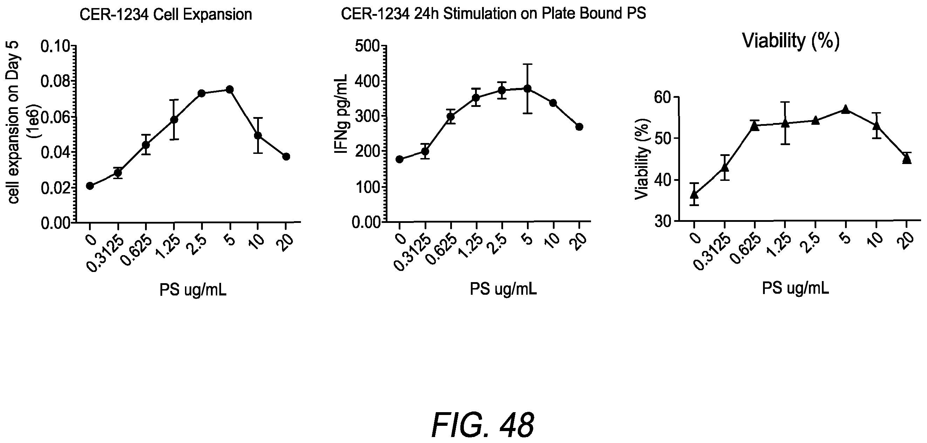

**FIG. 47** shows T cell expansion (left), IFNγ production (center), and cell viability (right) in CER1236 T cells in response to increasing doses of plate-bound phosphatidylserine. 24 hour IFNγ stimulation by plate-bound phosphatidylserine aligns with day 5 post-activation expansion for CER1236 T cells. **FIG. 48** shows T cell expansion (left), IFNγ production (center), and cell viability (right) in CER1234 T cells in response to increasing doses of plate-bound phosphatidylserine. 24 hour IFNγ stimulation by plate-bound phosphatidylserine aligns with day 5 post-activation expansion for CER1234 T cells.

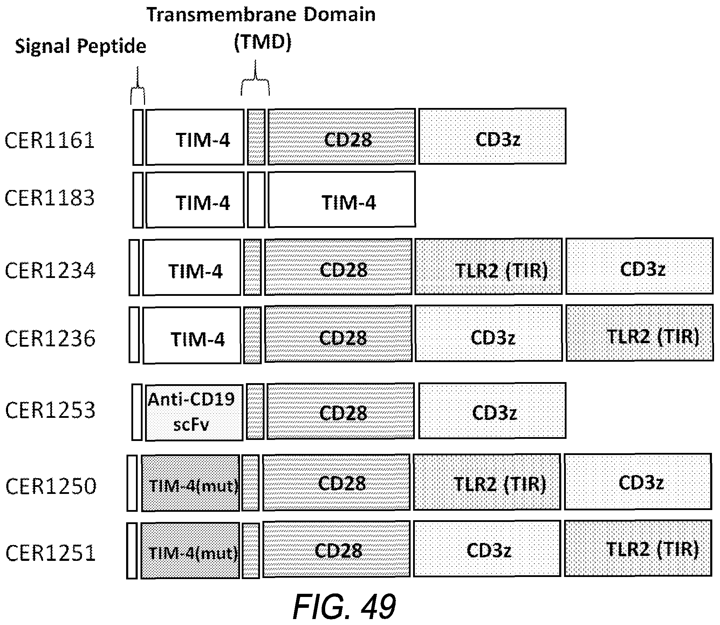

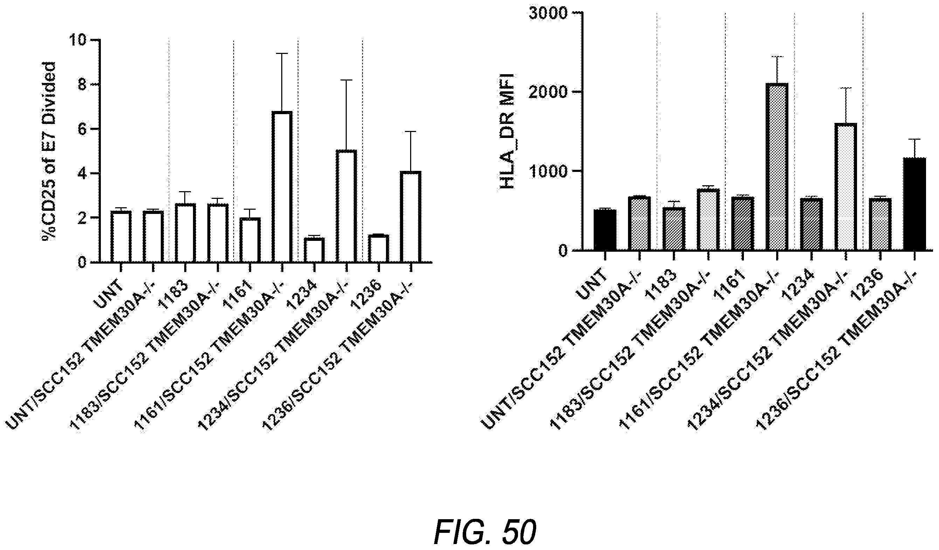

Antigen-presenting cell (APC)-like function of untransduced T cells or CER1183, CER1161, CER1234 or CER1236 T cells **(****FIG. 49** for schematic of constructs) was assessed after culture alone or co-culture with phosphatidylserine-positive HPV E7 oncoprotein-positive cells (TMEM30A-/- SCC152) for 48 hours, followed by positive selection of T cells and co-culture for a period of 4-6 days with cell trace violet labeled-HPV E7 TCR T cells. The activation markers HLA-DR and CD25 on E7 TCR T cells were analyzed using flow cytometry in repeated experiments (FIGs. 50-54).

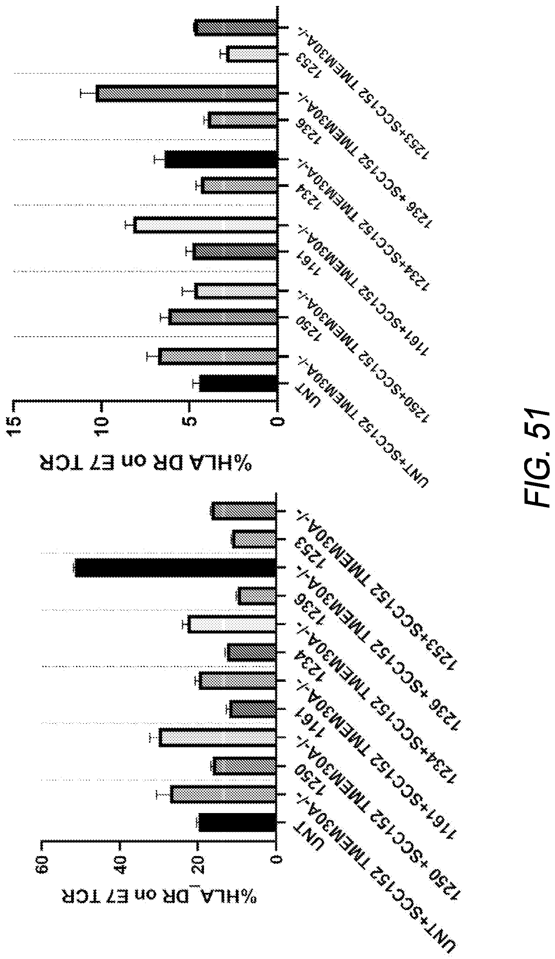

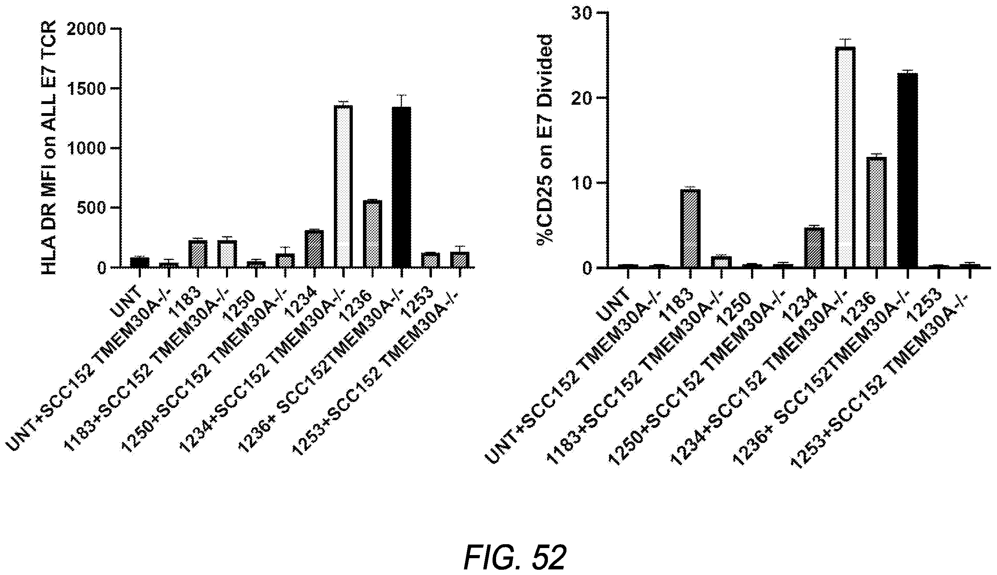

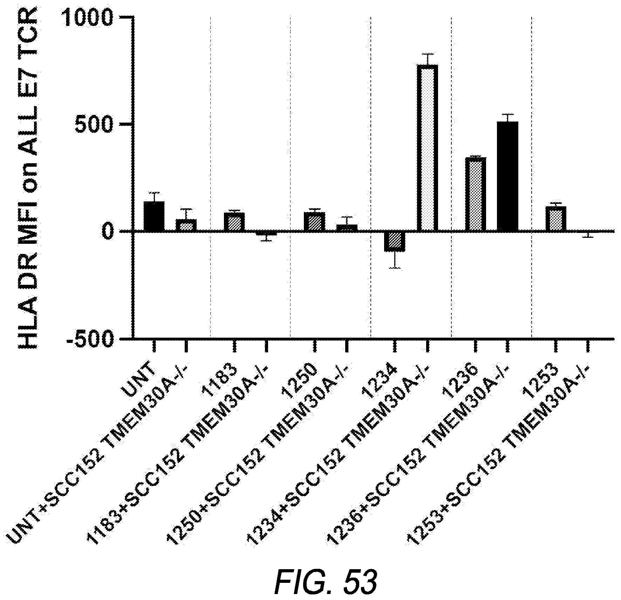

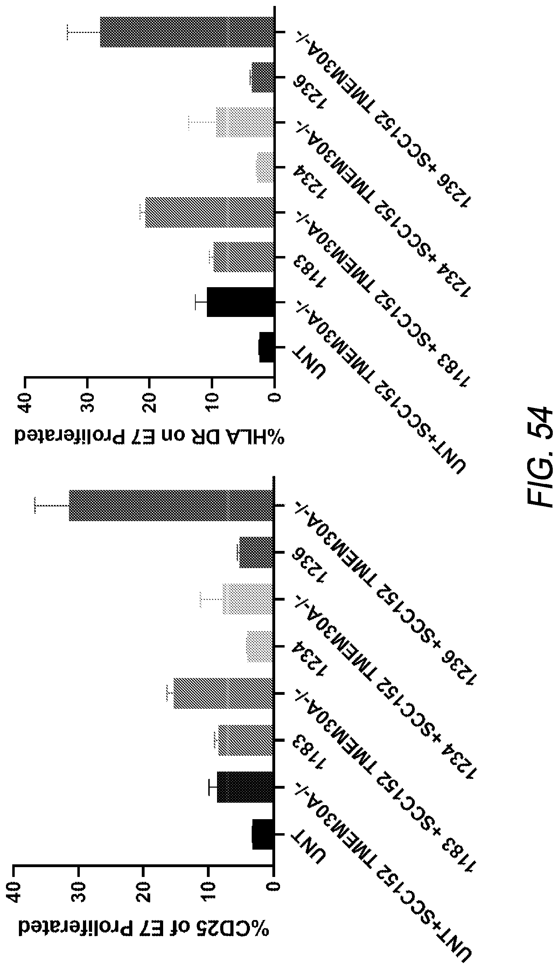

In **FIG. 50****,** significant difference in HLA-DR MFI was observed between untransduced T cells and CER1236 T cells. No significant difference was observed between CER1234 and CER1236 T cells. In **FIG. 51****,** significant difference in HLA-DR MFI was observed between untransduced T cells and CER1236 T cells, and CER1236 appears to have better antigen presentation function over CER1234 in two different experiments. In **FIG. 52****,** both CER1234 and CER1236 T cells display better antigen presentation function than untransduced T cells. No significant difference was observed between CER1234 and CER1236. In **FIG. 53****,** both CER1234 and CER1236 T cells display better antigen presentation function than untransduced T cells. No significant difference was observed between CER1234 and CER1236. In **FIG. 54****,** significant difference in HLA-DR MFI was observed between untransduced T cells and CER1236 T cells. CER1236 T cells also displayed improved antigen presentation function as measured by CD25 and HLA-DR compared to CER1234 T cells.

Overall, these results show that CER1236 T cells consistently had better antigen presentation function compared to untransduced T cells or T cells having constructs that were unable to bind phoshatidylserine. CER1234 did not show any antigen presentation function over untransduced T cells in some experiments. Compared to CER1234 T cells, CER1236 T cells consistently showed antigen presentation function.

As previously described in Example 5, CER-1236 T cells demonstrated repeated IFN-γ response upon 3 serial rounds of exposure to phosphatidylserine **(****FIG. 19C****).** The ability of CER-1236 T cells to become reactivated upon additional antigen exposure in vitro suggests a durable effect in vivo

### REFERNCES

1. Aderem, A. & Underhill, D. M. Mechanisms of phagocytosis in macrophages. Annu. Rev. Immunol. 17, 593-623 (1999).
2. Fu, C. & Jiang, A. Dendritic Cells and CD8 T Cell Immunity in Tumor Microenvironment. Front. Immunol. 9, 3059 (2018).
3. Steinhagen, F., Kinjo, T., Bode, C. & Klinman, D. M. TLR-based immune adjuvants. Vaccine 29, 3341-3355 (2011).
4. Meås, H. Z. et al. Sensing of HIV-1 by TLR8 activates human T cells and reverses latency. Nat. Commun. 11, 147 (2020).
5. Wille-Reece, U. et al. HIV Gag protein conjugated to a Toll-like receptor 7/8 agonist improves the magnitude and quality of Th1 and CD8+ T cell responses in nonhuman primates. Proc. Natl. Acad. Sci. U. S. A. 102, 15190-15194 (2005).
6. Deres, K., Schild, H., Wiesmüller, K. H., Jung, G. & Rammensee, H. G. In vivo priming of virus-specific cytotoxic T lymphocytes with synthetic lipopeptide vaccine. Nature 342, 561-564 (1989).
7. Jackson, D. C. et al. A totally synthetic vaccine of generic structure that targets Toll-like receptor 2 on dendritic cells and promotes antibody or cytotoxic T cell responses. Proc. Natl. Acad. Sci. U. S. A. 101, 15440-15445 (2004).
8. Zhu, X., Ramos, T. V., Gras-Masse, H., Kaplan, B. E. & BenMohamed, L. Lipopeptide epitopes extended by an Nepsilon-palmitoyl-lysine moiety increase uptake and maturation of dendritic cells through a Toll-like receptor-2 pathway and trigger a Th1-dependent protective immunity. Eur. J. Immunol. 34, 3102-3114 (2004).
9. Lanzavecchia, A., Roosnek, E., Gregory, T., Berman, P. & Abrignani, S. T cells can present antigens such as HIV gp120 targeted to their own surface molecules. Nature 334, 530-532 (1988).
10. Barnaba, V., Watts, C., de Boer, M., Lane, P. & Lanzavecchia, A. Professional presentation of antigen by activated human T cells. Eur. J. Immunol. 24, 71-75 (1994).
11. Bandola-Simon, J. & Roche, P. A. Dysfunction of antigen processing and presentation by dendritic cells in cancer. Mol. Immunol. 113, 31-37 (2019).
12. Caronni, N. et al. TIM4 expression by dendritic cells mediates uptake of tumor-associated antigens and anti-tumor responses. Nat. Commun. 12, 2237 (2021).
13. Engering, A. et al. The dendritic cell-specific adhesion receptor DC-SIGN internalizes antigen for presentation to T cells. J. Immunol. Baltim. Md 1950 168, 2118-2126 (2002).
14. Lanzavecchia, A. Antigen-specific interaction between T and B cells. Nature 314, 537-539 (1985).
15. Boross, P. et al. FcRγ-chain ITAM signaling is critically required for cross-presentation of soluble antibody-antigen complexes by dendritic cells. J. Immunol. Baltim. Md 1950 193, 5506-5514 (2014).
16. Greenberg, S., Chang, P., Wang, D. C., Xavier, R. & Seed, B. Clustered syk tyrosine kinase domains trigger phagocytosis. Proc. Natl. Acad. Sci. U. S. A. 93, 1103-1107 (1996).
17. Lennartz, M. & Drake, J. Molecular mechanisms of macrophage Toll-like receptor-Fc receptor synergy. F1000Research 7, 21 (2018).

## Claims

1. A chimeric T-cell immunoglobulin and mucin 4 (Tim4) receptor comprising a single chain chimeric protein, the single chain chimeric protein comprising:
(a) an extracellular domain comprising a Tim4 binding domain comprising the amino acid sequence of SEQ ID NO:6:
(b) an intracellular signaling domain, wherein the intracellular signaling domain comprises:
(i) a primary CD28 intracellular signaling domain comprising the amino acid sequence of SEQ ID NO: 12, a secondary TLR2 TIR intracellular signaling domain comprising the amino acid sequence of SEQ ID NO: 17, and a tertiary CD3ζ intracellular signaling domain comprising the amino acid sequence of SEQ ID NO:14; or
(ii) a primary CD28 intracellular signaling domain comprising the amino acid sequence of SEQ ID NO:12, a secondary CD3ζ intracellular signaling domain comprising the amino acid sequence of SEQ ID NO: 14, and a tertiary TLR2 TIR intracellular signaling domain comprising the amino acid sequence of SEQ ID NO:17; and
(c) a CD28 transmembrane domain comprising the amino acid sequence of SEQ ID NO:11 positioned between and connecting the extracellular domain and the CD28 intracellular signaling domain;
optionally, wherein:
- the chimeric Tim4 receptor further comprises a signal peptide at the N-terminus, further optionally wherein the signal peptide is a Tim4 signal peptide, further optionally wherein the Tim4 signal peptide comprises the amino acid sequence of SEQ ID NO:7;
- the chimeric Tim4 receptor comprises the amino acid sequence of SEQ ID NO:18 or SEQ ID NO:18 absent amino acids 1-24; or
- the chimeric Tim4 receptor comprises the amino acid sequence of SEQ ID NO:19 or SEQ ID NO:19 absent amino acids 1-24.

2. A polynucleotide encoding the chimeric Tim4 receptor of claim 1.

3. An expression vector comprising the polynucleotide of claim 2, optionally wherein the expression vector is a viral vector, further optionally wherein the viral vector is a lentiviral vector.

4. An engineered T cell comprising the chimeric Tim4 receptor of claim 1, the polynucleotide of claim 2, or the expression vector of claim 3; optionally wherein:
(i) the T cell is a CD4+ T cell, a CD8+ T cell, or a CD4+/CD8+ T cell; and/or
(ii) the T cell is a human T cell.

5. A pharmaceutical composition comprising the polynucleotide of claim 2, the expression vector of claim 3, or the engineered T cell of claim 4, and a pharmaceutically acceptable excipient.

6. The engineered T cell of claim 4, or the pharmaceutical composition of claim 5 comprising the engineered T cell, for use as a medicament.

7. The engineered T cell of claim 4, or the pharmaceutical composition of claim 5 comprising the engineered T cell, for use in a method of treating cancer,
optionally wherein the cancer is breast cancer; acute myeloid leukemia (AML); prostate cancer; ovarian cancer; cervical cancer; skin cancer; pancreatic cancer; colorectal cancer; renal cancer; liver cancer; brain cancer; lymphoma; leukemia; lung cancer; adenocarcinoma of the breast; adenocarcinoma of the prostate; adenocarcinoma of the colon; a form of bronchogenic carcinoma of the lung; myeloid leukemia; melanoma; hepatoma; neuroblastoma; papilloma; apudoma; choristoma; branchioma; malignant carcinoid syndrome; carcinoid heart disease; Walker carcinoma; basal cell carcinoma; basosquamous carcinoma; Brown-Pearce carcinoma; ductal carcinoma; Ehrlich tumor carcinoma; Krebs 2 carcinoma; Merkel cell carcinoma; mucinous carcinoma; non-small cell lung carcinoma; oat cell carcinoma; papillary carcinoma; scirrhous carcinoma; bronchiolar carcinoma; bronchogenic carcinoma; squamous cell carcinoma; transitional cell carcinoma; a histiocytic disorder; malignant histiocytosis; Hodgkin's disease; non-Hodgkin's lymphoma; plasmacytoma; multiple myeloma; chronic myeloid leukemia (CML); reticuloendotheliosis; chondroblastoma; chondroma; chondrosarcoma; fibroma; fibrosarcoma; giant cell tumor; histiocytoma; lipoma; liposarcoma; mesothelioma; myxoma; myxosarcoma; osteoma; osteosarcoma; chordoma; craniopharyngioma; dysgerminoma; hamartoma; mesenchymoma; mesonephroma; myosarcoma; ameloblastoma; cementoma; odontoma; teratoma; thymoma; trophoblastic tumor; adenoma; cholangioma; cholesteatoma; cyclindroma; cystadenocarcinoma; cystadenoma; granulosa cell tumor; gynandroblastoma; hidradenoma; islet cell tumor; Leydig cell tumor; sertoli cell tumor; theca cell tumor; leimyoma; leiomyosarcoma; myoblastoma; myomma; rhabdomyoma; rhabdomyosarcoma; ependymoma; ganglioneuroma; glioma; medulloblastoma; meningioma; neurilemmoma; neuroepithelioma; neurofibroma; neuroma; paraganglioma; paraganglioma nonchromaffin; angiokeratoma; angiolymphoid hyperplasia with eosinophilia; angioma sclerosing; angiomatosis; glomangioma; hemangioendothelioma; hemangioma; hemangiopericytoma; hemangiosarcoma; lymphangioma; lymphangiomyoma; lymphangiosarcoma; pinealoma; carcinosarcoma; chondrosarcoma; cystosarcoma phyllodes; leukosarcoma; sarcoma; neoplasm; neurofibromatosis; cervical dysplasia; peritoneal cancer; B-cell cancer; B-cell lymphoma; central nervous system lymphoma; acute lymphoblastic leukemia (ALL); chronic lymphocytic leukemia (CLL); Hairy cell leukemia; B cell blast transformation of chronic myeloid leukemia; myeloma; small lymphocytic lymphoma; small lymphocytic leukemia; Waldenström macroglobulinemia; B-cell prolymphocytic leukemia; lymphoplasmacytic lymphoma; splenic marginal zone lymphoma; plasma cell myeloma; solitary plasmacytoma of bone; extraosseous plasmacytoma; extra-nodal marginal zone B-cell lymphoma of mucosa-associated (MALT) lymphoid tissue; nodal marginal zone B-cell lymphoma; follicular lymphoma; mantle cell lymphoma; diffuse large B-cell lymphoma; mediastinal (thymic) large B-cell lymphoma; intravascular large B-cell lymphoma; primary effusion lymphoma; Burkitt's lymphoma/leukemia; B-cell proliferation of uncertain malignant potential;, lymphomatoid granulomatosis; or post-transplant lymphoproliferative disorder;
further optionally wherein the B cell cancer is a B cell lymphoma, further optionally wherein the B cell lymphoma is mantle cell lymphoma.

8. The engineered T cell or pharmaceutical composition for use of claim 7, wherein the method further comprises administration of an additional therapeutic agent,
optionally wherein the additional therapeutic agent comprises radiation, cellular immunotherapy, antibody, immune checkpoint molecule inhibitor, chemotherapy, hormone therapy, peptide, antibiotic, anti-viral agent, anti-fungal agent, anti-inflammatory agent, UV light therapy, electric pulse therapy, high intensity focused ultrasound therapy, oncolytic virus therapy, a small molecule therapy, or any combination thereof,
further optionally wherein:
(i) the cellular immunotherapy is a chimeric antigen receptor; or
(ii) the additional therapeutic agent comprises an angiogenesis inhibitor, optionally, wherein the angiogenesis inhibitor is a VEGF pathway inhibitor, tyrosine kinase inhibitor, optionally, wherein the tyrosine kinase inhibitor is an EGF pathway inhibitor, receptor tyrosine kinase inhibitor, growth factor inhibitor, GTPase inhibitor, serine/threonine kinase inhibitor, transcription factor inhibitor, B-Raf inhibitor, RAF inhibitor, MEK inhibitor, mTOR inhibitor, EGFR inhibitor, ALK inhibitor, PARP inhibitor, ROS1 inhibitor, BCL-2 inhibitor, PI3K inhibitor, VEGFR inhibitor, BCR-ABL inhibitor, MET inhibitor, MYC inhibitor, ABL inhibitor, HER2 inhibitor, BTK inhibitor, H-RAS inhibitor, K-RAS inhibitor, PDGFR inhibitor, TRK inhibitor, c-KIT inhibitor, c-MET inhibitor, CDK4/6 inhibitor, FAK inhibitor, FGFR inhibitor, FLT3 inhibitor, IDH1 inhibitor, IDH2 inhibitor, PDGFRA inhibitor, or RET inhibitor.

9. The engineered T cell or pharmaceutical composition for use of claim 8, wherein:
(i) the additional therapeutic agent comprises a BTK inhibitor, optionally wherein:
- the BTK inhibitor is ibrutinib, pirtobrutinib (Loxo-305), tirabrutinib (ONO-4059), tolebrutinib, evobrutinib, fenebrutinib (GDC-0853), acalabrutinib, spebrutinib, zanubrutinib (BGB-3111), HM71224, or M7583; and/or
- the cancer is mantle cell lymphoma. chronic lymphocytic leukemia, small lymphocytic lymphoma, small lymphocytic leukemia, Waldenström macroglobulinemia, or marginal zone lymphoma;
(ii) the additional therapeutic agent comprises an EGFR inhibitor, optionally wherein:
- the EGFR inhibitor is osimertinib, erlotinib, gefitnib, afatinib, or dacomitinib; and/or
- the cancer is non-small cell lung cancer; or
(iii) the additional therapeutic agent comprises a Poly ADP-ribose polymerase (PARP) inhibitor, optionally wherein:
- the PARP inhibitor is talazoparib, niraparib, rucaparib, olaparib, veliparib, CEP 9722, E7016, AG014699, MK4827, BMN-673, or pamiparib; and/or
- the cancer is breast cancer, ovarian cancer, colorectal cancer, fallopian cancer, peritoneal cancer, prostate cancer, lung cancer, or melanoma.

10. The engineered T cell or pharmaceutical composition for use of claim 8 or 9, wherein the additional therapeutic agent is administered at a subtherapeutic dose and/or the additional therapeutic agent is administered to the subject sequentially or concurrently with the engineered T cell or pharmaceutical composition.

11. The engineered T cell or pharmaceutical composition for use of claim 7, wherein the method enhances an effector response or anti-tumor efficacy in the subject.

12. The engineered T cell or pharmaceutical composition for use of claim 7, wherein the method enhances CCR7+ expressing T cells in the subject,
optionally wherein the method further comprises administering a Poly ADP-ribose polymerase (PARP) inhibitor, further optionally wherein the PARP inhibitor is talazoparib, niraparib, rucaparib, olaparib, veliparib, CEP 9722, E7016, AG014699, MK4827, BMN-673, pamiparib, or a combination thereof.

13. The engineered T cell or pharmaceutical composition for use of claim 7, wherein the method enhances CD4/CD8 T cell ratio in the subject,
optionally wherein the method further comprises administering a Poly ADP-ribose polymerase (PARP) inhibitor, further optionally wherein the PARP inhibitor is talazoparib, niraparib, rucaparib, olaparib, veliparib, CEP 9722, E7016, AG014699, MK4827, BMN-673, pamiparib, or a combination thereof.

14. The engineered T cell or pharmaceutical composition for use of claim 12 or 13, wherein the cancer is breast cancer, ovarian cancer, colorectal cancer, fallopian cancer, peritoneal cancer, prostate cancer, lung cancer, or melanoma.

15. The engineered T cell or pharmaceutical composition for use of any one of claims 12-14, wherein the method further comprises administering an additional therapeutic agent,
optionally wherein the additional therapeutic agent comprises radiation, cellular immunotherapy, antibody, immune checkpoint molecule inhibitor, chemotherapy, hormone therapy, peptide, antibiotic, anti-viral agent, anti-fungal agent, anti-inflammatory agent, UV light therapy, electric pulse therapy, high intensity focused ultrasound therapy, oncolytic virus therapy, a small molecule therapy, or a combination thereof,
further optionally wherein:
(i) the cellular immunotherapy is a chimeric antigen receptor or T cell receptor; or
(ii) the additional therapeutic agent comprises an angiogenesis inhibitor, optionally, wherein the angiogenesis inhibitor is a VEGF pathway inhibitor, tyrosine kinase inhibitor, optionally, wherein the tyrosine kinase inhibitor is an EGF pathway inhibitor, receptor tyrosine kinase inhibitor, growth factor inhibitor, GTPase inhibitor, serine/threonine kinase inhibitor, transcription factor inhibitor, B-Raf inhibitor, RAF inhibitor, MEK inhibitor, mTOR inhibitor, EGFR inhibitor, ALK inhibitor, ROS 1 inhibitor, BCL-2 inhibitor, PI3K inhibitor, VEGFR inhibitor, BCR-ABL inhibitor, MET inhibitor, MYC inhibitor, ABL inhibitor, HER2 inhibitor, BTK inhibitor, H-RAS inhibitor, K-RAS inhibitor, PDGFR inhibitor, TRK inhibitor, c-KIT inhibitor, c-MET inhibitor, CDK4/6 inhibitor, FAK inhibitor, FGFR inhibitor, FLT3 inhibitor, IDH1 inhibitor, IDH2 inhibitor, PDGFRA inhibitor, or RET inhibitor.

## Patentansprüche

1. Chimärer T-Zell-Immunglobulin-und-Mucin 4 (Tim4)-Rezeptor, umfassend ein einzelkettiges chimäres Protein, wobei das einzelkettige chimäre Protein umfasst:
(a) eine extrazelluläre Domäne, umfassend eine Tim4-Bindungsdomäne, welche die Aminosäuresequenz von SEQ ID NO: 6 umfasst:
(b) eine intrazelluläre Signaldomäne, wobei die intrazelluläre Signaldomäne umfasst:
(i) eine primäre intrazelluläre CD28-Signaldomäne, umfassend die Aminosäuresequenz von SEQ ID NO: 12, eine sekundäre intrazelluläre TLR2 TIR-Signaldomäne, umfassend die Aminosäuresequenz von SEQ ID NO: 17, und eine tertiäre intrazelluläre CD3ζ-Signaldomäne, umfassend die Aminosäuresequenz von SEQ ID NO: 14; oder
(ii) eine primäre intrazelluläre CD28-Signaldomäne, umfassend die Aminosäuresequenz von SEQ ID NO: 12, eine sekundäre intrazelluläre CD3ζ-Signaldomäne, umfassend die Aminosäuresequenz von SEQ ID NO: 14, und eine tertiäre intrazelluläre TLR2 TIR-Signaldomäne, umfassend die Aminosäuresequenz von SEQ ID NO: 17; und
(c) eine CD28-Transmembrandomäne, umfassend die Aminosäuresequenz von SEQ ID NO: 11, die zwischen der extrazellulären Domäne und der intrazellulären CD28-Signaldomäne positioniert ist und diese verbindet;
wobei optional:
- der chimäre Tim4-Rezeptor ferner ein Signalpeptid am N-Terminus umfasst, wobei ferner optional das Signalpeptid ein Tim4-Signalpeptid ist, wobei ferner optional das Tim4-Signalpeptid die Aminosäuresequenz von SEQ ID NO: 7 umfasst;
- der chimäre Tim4-Rezeptor die Aminosäuresequenz von SEQ ID NO: 18 oder SEQ ID NO: 18 ohne die Aminosäuren 1-24 umfasst; oder
- der chimäre Tim4-Rezeptor die Aminosäuresequenz von SEQ ID NO: 19 oder SEQ ID NO: 19 ohne die Aminosäuren 1-24 umfasst.

2. Polynukleotid, welches den chimären Tim4-Rezeptor nach Anspruch 1 codiert.

3. Expressionsvektor, welcher das Polynukleotid nach Anspruch 2 umfasst, wobei der Expressionsvektor optional ein viraler Vektor ist, wobei ferner optional der virale Vektor ein lentiviraler Vektor ist.

4. Manipulierte T-Zelle, umfassend den chimären Tim4-Rezeptor nach Anspruch 1, das Polynukleotid nach Anspruch 2 oder den Expressionsvektor nach Anspruch 3; wobei optional:
(i) die T-Zelle eine CD4+-T-Zelle, eine CD8+-T-Zelle oder eine CD4+/CD8+-T-Zelle ist; und/oder
(ii) die T-Zelle eine menschliche T-Zelle ist.

5. Pharmazeutische Zusammensetzung, umfassend das Polynukleotid nach Anspruch 2, den Expressionsvektor nach Anspruch 3 oder die manipulierte T-Zelle nach Anspruch 4 und einen pharmazeutisch akzeptablen Hilfsstoff.

6. Manipulierte T-Zelle nach Anspruch 4 oder pharmazeutische Zusammensetzung nach Anspruch 5, umfassend die manipulierte T-Zelle, zur Verwendung als Medikament.

7. Manipulierte T-Zelle nach Anspruch 4 oder pharmazeutische Zusammensetzung nach Anspruch 5, umfassend die manipulierte T-Zelle, zur Verwendung bei einem Verfahren zur Behandlung von Krebs,
wobei der Krebs optional Brustkrebs; akute myeloische Leukämie (AML); Prostatakrebs; Eierstockkrebs; Zervixkrebs; Hautkrebs; Pankreaskrebs; Kolorektalkrebs; Nierenkrebs; Leberkrebs; Hirntumor; Lymphom; Leukämie; Lungenkrebs; Adenokarzinom der Brust; Adenokarzinom der Prostata; Adenokarzinom des Kolons; eine Form eines bronchogenen Karzinoms der Lunge; myeloische Leukämie; Melanom; Hepatom; Neuroblastom; Papillom; Apudom; Choristom; Branchiom; malignes karzinoides Syndrom; karzinoide Herzkrankheit; Walker-Karzinom; Basalzellkarzinom; basosquamöses Karzinom; Brown-Pearce-Karzinom; duktales Karzinom; Ehrlich-Tumor-Karzinom; Krebs 2-Karzinom; Merkelzellkarzinom; muzinöses Karzinom; nicht-kleinzelliges Lungenkarzinom; Oatzellkarzinom; papilläres Karzinom; szirrhöses Karzinom; bronchioläres Karzinom; bronchogenes Karzinom; Plattenepithelkarzinom; Übergangszellkarzinom; eine histiozytische Störung; maligne Histiozytose; Hodgkin-Krankheit; Non-Hodgkin-Lymphom; Plasmazytom; multiples Myelom; chronische myeloische Leukämie (CML); Reticuloendotheliose; Chondroblastom; Chondrom; Chondrosarkom; Fibrom; Fibrosarkom; Riesenzelltumor; Histiozytom; Lipom; Liposarkom; Mesotheliom; Myxom; Myxosarkom; Osteom; Osteosarkom; Chordom; Craniopharyngiom; Dysgerminom; Hamartom; Mesenchymom; Mesonephrom; Myosarkom; Ameloblastom; Cementom; Odontom; Teratom; Thymom; trophoblastischer Tumor; Adenom; Cholangiom; Cholesteatom; Cylindrom; Cystadenokarzinom; Cystadenom; Granulosazelltumor; Gynandroblastom; Hidradenom; Inselzelltumor; Leydigzelltumor; Sertolizelltumor; Thecazelltumor; Leimyom; Leiomyosarkom; Myoblastom; Myom; Rhabdomyom; Rhabdomyosarkom; Ependymom; Ganglioneurom; Gliom; Medulloblastom; Meningiom; Neurilemmom; Neuroepitheliom; Neurofibrom; Neurom; Paragangliom; Paragangliom nonchromaffin; Angiokeratom; angiolymphoide Hyperplasie mit Eosinophilie; sklerosierendes Angiom; Angiomatose; Glomangiom; Hämangioendotheliom; Hämangiom; Hämangiopericytom; Hämangiosarkom; Lymphangiom; Lymphangiomyom; Lymphangiosarkom; Pinealom; Carcinosarkom; Chondrosarkom; Cystosarcoma phyllodes; Leukosarkom; Sarkom; Neoplasma; Neurofibromatose; zervikale Dysplasie; Peritonealkrebs; B-Zell-Krebs; B-Zell-Lymphom; zentralnervöses Lymphom; akute lymphoblastische Leukämie (ALL); chronische lymphozytische Leukämie (CLL); Haarzellleukämie; B-Zell-Blastentransformation chronisch myeloischer Leukämie; Myelom; kleinzelliges lymphozytisches Lymphom; kleinzellige lymphozytische Leukämie; Waldenström-Makroglobulinämie; prolymphozytische B-Zell-Leukämie; lymphoplasmazytisches Lymphom; Marginalzonenlymphom der Milz; Plasmazellmyelom; solitäres Plasmazytom der Knochen; extraossäres Plasmazytom; extranodales Marginalzonen-B-Zell-Lymphom des Mucosa-assoziierten (MALT) Lymphoidgewebes; nodales Marginalzonen-B-Zell-Lymphom; follikuläres Lymphom; Mantelzelllymphom; diffuses großzelliges B-Zell-Lymphom; mediastinales (thymisches) großzelliges B-Zell-Lymphom; intravaskuläres großzelliges B-Zell-Lymphom; primäres Effusionslymphom; Burkitt-Lymphom/Leukämie; B-Zell-Proliferation ungewissen malignen Potentials; lymphomatoide Granulomatose; oder lymphoproliferative Störung post Transplantation;
wobei ferner optional der B-Zell-Krebs ein B-Zell-Lymphom ist, wobei ferner optional das B-Zell-Lymphom Mantelzelllymphom ist.

8. Manipulierte T-Zelle oder pharmazeutische Zusammensetzung zur Verwendung nach Anspruch 7, wobei das Verfahren ferner das Verabreichen eines zusätzlichen therapeutischen Mittels umfasst;
wobei das zusätzliche therapeutische Mittel optional Bestrahlung, zelluläre Immuntherapie, Antikörper, Immun-Checkpoint-Molekül-Inhibitor, Chemotherapie, Hormontherapie, Peptid, Antibiotikum, antivirales Mittel, antifungales Mittel, antiinflammatorisches Mittel, UV-Lichttherapie, Elektropulstherapie, Therapie mit hochintensivem fokussiertem Ultraschall, onkolytische Virustherapie, eine Therapie mit kleinen Molekülen (small molecule therapy) oder irgendeine Kombination davon umfasst,
wobei ferner optional:
(i) die zelluläre Immuntherapie ein chimärer Antigenrezeptor ist; oder
(ii) das zusätzliche therapeutische Mittel einen Angiogeneseinhibitor umfasst, wobei der Angiogeneseinhibitor optional ein VEGF-Pathway-Inhibitor, Tyrosinkinaseinhibitor ist, wobei der Tyrosinkinaseinhibitor optional ein EGF-Pathway-Inhibitor, Rezeptor-Tyrosinkinaseinhibitor, Wachstumsfaktorinhibitor, GTPase-Inhibitor, Serin/Threoninkinaseinhibitor, Transkriptionsfaktorinhibitor, B-Raf-Inhibitor, RAF-Inhibitor, MEK-Inhibitor, mTOR-Inhibitor, EGFR-Inhibitor, ALK-Inhibitor, PARP-Inhibitor, ROS1-Inhibitor, BCL-2-Inhibitor, PI3K-Inhibitor, VEGFR-Inhibitor, BCR-ABL-Inhibitor, MET-Inhibitor, MYC-Inhibitor, ABL-Inhibitor, HER2-Inhibitor, BTK-Inhibitor, H-RAS-Inhibitor, K-RAS-Inhibitor, PDGFR-Inhibitor, TRK-Inhibitor, c-KIT-Inhibitor, c-MET-Inhibitor, CDK4/6-Inhibitor, FAK-Inhibitor, FGFR-Inhibitor, FLT3-Inhibitor, IDH1-Inhibitor, IDH2-Inhibitor, PDGFRA-Inhibitor oder RET-Inhibitor ist.

9. Manipulierte T-Zelle oder pharmazeutische Zusammensetzung zur Verwendung nach Anspruch 8, wobei:
(i) das zusätzliche therapeutische Mittel einen BTK-Inhibitor umfasst, wobei optional:
- der BTK-Inhibitor Ibrutinib, Pirtobrutinib (Loxo-305), Tirabrutinib (ONO-4059), Tolebrutinib, Evobrutinib, Fenebrutinib (GDC-0853), Acalabrutinib, Spebrutinib, Zanubrutinib (BGB-3111), HM71224 oder M7583 ist; und/oder
- der Krebs Mantelzelllymphom, chronische lymphozytische Leukämie, kleinzelliges lymphozytisches Lymphom; kleinzellige lymphozytische Leukämie, Waldenström-Makroglobulinämie oder Marginalzonenlymphom ist;
(ii) das zusätzliche therapeutische Mittel einen EGFR-Inhibitor umfasst, wobei optional:
- der EGFR-Inhibitor Osimertinib, Erlotinib, Gefitnib, Afatinib oder Dacomitinib ist; und/oder
- der Krebs nicht-kleinzelliger Lungenkrebs ist; oder
(iii) das zusätzliche therapeutische Mittel einen Poly ADP-Ribosepolymerase (PARP)-Inhibitor umfasst, wobei optional:
- der PARP-Inhibitor Talazoparib, Niraparib, Rucaparib, Olaparib, Veliparib, CEP 9722, E7016, AG014699, MK4827, BMN-673 oder Pamiparib ist; und/oder
- der Krebs Brustkrebs, Eierstockkrebs, Kolorektalkrebs, Eileiterkrebs, Peritonealkrebs, Prostatakrebs, Lungenkrebs oder Melanom ist.

10. Manipulierte T-Zelle oder pharmazeutische Zusammensetzung zur Verwendung nach Anspruch 8 oder 9, wobei das zusätzliche therapeutische Mittel bei einer subtherapeutischen Dosis verabreicht wird und/oder das zusätzliche therapeutische Mittel dem Subjekt sequentiell oder gleichzeitig mit der manipulierten T-Zelle oder der pharmazeutischen Zusammensetzung verabreicht wird.

11. Manipulierte T-Zelle oder pharmazeutische Zusammensetzung zur Verwendung nach Anspruch 7, wobei das Verfahren eine Effektorantwort oder Anti-Tumor-Wirksamkeit in dem Subjekt verstärkt.

12. Manipulierte T-Zelle oder pharmazeutische Zusammensetzung zur Verwendung nach Anspruch 7, wobei das Verfahren CCR7+-exprimierende T-Zellen in dem Subjekt erhöht,
wobei das Verfahren optional ferner das Verabreichen eines Poly ADP-Ribosepolymerase (PARP)-Inhibitors umfasst, wobei ferner optional der PARP-Inhibitor Talazoparib, Niraparib, Rucaparib, Olaparib, Veliparib, CEP 9722, E7016, AG014699, MK4827, BMN-673, Pamiparib oder eine Kombination davon ist.

13. Manipulierte T-Zelle oder pharmazeutische Zusammensetzung zur Verwendung nach Anspruch 7, wobei das Verfahren das CD4/CD8-T-Zell-Verhältnis in dem Subjekt erhöht,
wobei das Verfahren optional ferner das Verabreichen eines Poly ADP-Ribosepolymerase (PARP)-Inhibitors umfasst, wobei ferner optional der PARP-Inhibitor Talazoparib, Niraparib, Rucaparib, Olaparib, Veliparib, CEP 9722, E7016, AG014699, MK4827, BMN-673, Pamiparib oder eine Kombination davon ist.

14. Manipulierte T-Zelle oder pharmazeutische Zusammensetzung zur Verwendung nach Anspruch 12 oder 13, wobei der Krebs Brustkrebs, Eierstockkrebs, Kolorektalkrebs, Eileiterkrebs, Peritonealkrebs, Prostatakrebs, Lungenkrebs oder Melanom ist.

15. Manipulierte T-Zelle oder pharmazeutische Zusammensetzung zur Verwendung nach irgendeinem der Ansprüche 12-14, wobei das Verfahren ferner das Verabreichen eines zusätzlichen therapeutischen Mittels umfasst,
wobei das zusätzliche therapeutische Mittel optional Bestrahlung, zelluläre Immuntherapie, Antikörper, Immun-Checkpoint-Molekül-Inhibitor, Chemotherapie, Hormontherapie, Peptid, Antibiotikum, antivirales Mittel, antifungales Mittel, antiinflammatorisches Mittel, UV-Lichttherapie, Elektropulstherapie, Therapie mit hochintensivem fokussiertem Ultraschall, onkolytische Virustherapie, eine Therapie mit kleinzelligen Molekülen oder eine Kombination davon umfasst,
wobei ferner optional:
(i) die zelluläre Immuntherapie ein chimärer Antigenrezeptor oder T-Zell-Rezeptor ist; oder
(ii) das zusätzliche therapeutische Mittel einen Angiogeneseinhibitor umfasst, wobei optional der Angiogeneseinhibitor ein VEGF-Pathway-Inhibitor, Tyrosinkinaseinhibitor ist, wobei optional der Tyrosinkinaseinhibitor ein EGF-Pathway-Inhibitor, Rezeptor-Tyrosinkinase-Inhibitor, Wachstumsfaktorinhibitor, GTPase-Inhibitor, Serin/Threoninkinaseinhibitor, Transkriptionsfaktorinhibitor, B-Raf-Inhibitor, RAF-Inhibitor, MEK-Inhibitor, mTOR-Inhibitor, EGFR-Inhibitor, ALK-Inhibitor, ROSI-Inhibitor, BCL-2-Inhibitor, PI3K-Inhibitor, VEGFR-Inhibitor, BCR-ABL-Inhibitor, MET-Inhibitor, MYC-Inhibitor, ABL-Inhibitor, HER2-Inhibitor, BTK-Inhibitor, H-RAS-Inhibitor, K-RAS-Inhibitor, PDGFR-Inhibitor, TRK-Inhibitor, c-KIT-Inhibitor, c-MET-Inhibitor, CDK4/6-Inhibitor, FAK-Inhibitor, FGFR-Inhibitor, FLT3-Inhibitor, IDHI-Inhibitor, IDH2-Inhibitor, PDGFRA-Inhibitor oder RET-Inhibitor ist.

## Revendications

1. Un récepteur chimérique de cellules T immunoglobuline et mucine 4 (Tim4) comprenant une protéine chimérique à chaîne unique, la protéine chimérique à chaîne unique comprenant :
(a) un domaine extracellulaire comprenant un domaine de liaison Tim4 comprenant la séquence d'acides aminés de SEQ ID NO:6 :
(b) un domaine de signalisation intracellulaire, dans lequel le domaine de signalisation intracellulaire comprend :
(i) un domaine de signalisation intracellulaire CD28 primaire comprenant la séquence d'acides aminés de SEQ ID NO:12, un domaine de signalisation intracellulaire TLR2 TIR secondaire comprenant la séquence d'acides aminés de SEQ ID NO:17, et un domaine de signalisation intracellulaire CD3ζ tertiaire comprenant la séquence d'acides aminés de SEQ ID NO:14 ; ou
(ii) un domaine de signalisation intracellulaire CD28 primaire comprenant la séquence d'acides aminés de SEQ ID NO:12, un domaine de signalisation intracellulaire CD3ζ secondaire comprenant la séquence d'acides aminés de SEQ ID NO:14, et un domaine de signalisation intracellulaire TLR2 TIR tertiaire comprenant la séquence d'acides aminés de SEQ ID NO:17 ; et
(c) un domaine transmembranaire CD28 comprenant la séquence d'acides aminés de SEQ ID NO:11 positionnée entre et reliant le domaine extracellulaire et le domaine de signalisation intracellulaire CD28 ; optionnellement, dans lequel :
- le récepteur chimérique Tim4 comprend en outre un peptide signal à l'extrémité N-terminale, optionnellement dans lequel le peptide signal est un peptide signal Tim4, optionnellement dans lequel le peptide signal Tim4 comprend la séquence d'acides aminés de SEQ ID NO:7 ;
- le récepteur chimérique Tim4 comprend la séquence d'acides aminés de SEQ ID NO:18 ou SEQ ID NO:18 sans les acides aminés 1-24 ; ou
- le récepteur chimérique Tim4 comprend la séquence d'acides aminés de SEQ ID NO:19 ou SEQ ID NO:19 sans les acides aminés 1-24.

2. Un polynucléotide codant pour le récepteur chimérique Tim4 selon la revendication 1.

3. Un vecteur d'expression comprenant le polynucléotide selon la revendication 2, dans lequel, en option, le vecteur d'expression est un vecteur viral, dans lequel, en option, le vecteur viral est un vecteur lentiviral.

4. Une cellule T modifiée comprenant le récepteur chimérique Tim4 selon la revendication 1, le polynucléotide selon la revendication 2 ou le vecteur d'expression selon la revendication 3 ; optionnellement dans lequel :
(i) la cellule T est une cellule T CD4+, une cellule T CD8+ ou une cellule T CD4+/CD8+ ; et/ou
(ii) la cellule T est une cellule T humaine.

5. Une composition pharmaceutique comprenant le polynucléotide de la revendication 2, le vecteur d'expression de la revendication 3 ou la cellule T modifiée de la revendication 4, et un excipient pharmaceutiquement acceptable.

6. La cellule T modifiée selon la revendication 4, ou la composition pharmaceutique selon la revendication 5 comprenant la cellule T modifiée, destinée à être utilisée comme médicament.

7. La cellule T modifiée selon la revendication 4, ou la composition pharmaceutique selon la revendication 5 comprenant la cellule T modifiée, destinée à être utilisée dans un procédé de traitement du cancer, dans lequel, en option, le cancer est un cancer du sein ; une leucémie myéloïde aiguë (LMA) ; un cancer de la prostate ; un cancer de l'ovaire ; un cancer du col de l'utérus ; un cancer de la peau ; un cancer du pancréas ; un cancer colorectal ; un cancer du rein ; un cancer du foie ; un cancer du cerveau ; un lymphome ; une leucémie ; le cancer du poumon ; l'adénocarcinome du sein ; l'adénocarcinome de la prostate ; l'adénocarcinome du côlon ; une forme de carcinome bronchogénique du poumon ; la leucémie myéloïde ; le mélanome ; l'hépatome ; le neuroblastome ; le papillome ; l'apudome ; le choristome ; le branchiome ; le syndrome carcinoïde malin ; la cardiopathie carcinoïde ; carcinome de Walker ; carcinome basocellulaire ; carcinome basosquameux ; carcinome de Brown-Pearce ; carcinome canalaire ; carcinome de Ehrlich ; carcinome de Krebs 2 ; carcinome à cellules de Merkel ; carcinome mucineux ; carcinome pulmonaire non à petites cellules ; carcinome à cellules d'avoine ; carcinome papillaire ; carcinome scirreux ; carcinome bronchiolaire ; carcinome bronchogénique ; carcinome épidermoïde ; carcinome à cellules transitionnelles ; trouble histiocytaire ; histiocytose maligne ; maladie de Hodgkin ; lymphome non hodgkinien ; plasmocytome ; myélome multiple ; leucémie myéloïde chronique (LMC) ; réticuloendothéliose ; chondroblastome ; chondrome ; chondrosarcome ; fibrome ; fibrosarcome ; tumeur à cellules géantes ; histiocytome ; lipome ; liposarcome ; mésothéliome ; myxome ; myxosarcome ; ostéome ; ostéosarcome ; chordome ; craniopharyngiome ; dysgerminome ; hamartome ; mésenchymome ; mésonéphrome ; myosarcome ; améloblastome ; cémentome ; odontome ; tératome ; thymome ; tumeur trophoblastique ; adénome ; cholangiome ; cholestéatome ; cyclindrome ; cystadénocarcinome ; cystadénome ; tumeur à cellules granuleuses ; gynandroblastome ; hidradénome ; tumeur des cellules des îlots pancréatiques ; tumeur des cellules de Leydig ; tumeur des cellules de Sertoli ; tumeur des cellules thécales ; léiomyome ; léiomyosarcome ; myoblastome ; myome ; rhabdomyome ; rhabdomyosarcome ; épendymome ; ganglioneurome ; gliome ; médulloblastome ; méningiome ; neurilemmome ; neuroépithéliome ; neurofibrome ; névrome ; paragangliome ; paragangliome non chromaffine ; angiokératome ; hyperplasie angiolymphoïde avec éosinophilie ; angiome sclérosant ; angiomatose ; glomangiome ; hémangioendothéliome ; hémangiome ; hémangiopéricytome ; hémangiosarcome ; lymphangiome ; lymphangiomyome ; lymphangiose sarcome ; pinéalome ; carcinosarcome ; chondrosarcome ; cystosarcome phyllode ; leucosarcome ; sarcome ; néoplasme ; neurofibromatose ; dysplasie cervicale ; cancer péritonéal ; cancer à cellules B ; lymphome à cellules B ; lymphome du système nerveux central ; leucémie lymphoblastique aiguë (LLA) ; leucémie lymphoïde chronique (LLC) ; leucémie à tricholeucocytes ; transformation blastique à cellules B de la leucémie myéloïde chronique ; myélome ; lymphome lymphocytaire petit ; leucémie lymphocytaire petite ; macroglobulinémie de Waldenström ; leucémie prolymphocytaire à cellules B ; lymphome lymphoplasmocytaire ; lymphome de la zone marginale splénique ; myélome plasmocytaire ; plasmocytome solitaire de l'os ; plasmocytome extra-osseux ; lymphome à cellules B de la zone marginale extranodale du tissu lymphoïde associé aux muqueuses (MALT) ; lymphome B de la zone marginale ganglionnaire ; lymphome folliculaire ; lymphome à cellules du manteau ; lymphome diffus à grandes cellules B ; lymphome médiastinal (thymique) à grandes cellules B ; lymphome intravasculaire à grandes cellules B ; lymphome primitif d'effusion ; lymphome/leucémie de Burkitt ; prolifération de cellules B à potentiel malin incertain ; granulomatose lymphomatoïde ; ou trouble lymphoprolifératif post-transplantation ; en outre, en option, dans lequel le cancer des cellules B est un lymphome à cellules B, en outre, en option, dans lequel le lymphome à cellules B est un lymphome à cellules du manteau.

8. La cellule T modifiée ou la composition pharmaceutique destinée à être utilisée selon la revendication 7, dans laquelle le procédé comprend en outre l'administration d'un agent thérapeutique supplémentaire, en option, dans lequel l'agent thérapeutique supplémentaire comprend une radiothérapie, une immunothérapie cellulaire, un anticorps, un inhibiteur de molécule de point de contrôle immunitaire, une chimiothérapie, une hormonothérapie, un peptide, un antibiotique, un agent antiviral, un agent antifongique, un agent anti-inflammatoire, une thérapie par rayons UV, une thérapie par impulsions électriques, une thérapie par ultrasons focalisés de haute intensité, une thérapie par virus oncolytique, une thérapie par petites molécules, ou toute combinaison de ceux-ci, en option, dans lequel :
(i) l'immunothérapie cellulaire est un récepteur d'antigène chimérique ; ou
(ii) l'agent thérapeutique supplémentaire comprend un inhibiteur de l'angiogenèse, optionnellement, dans lequel l'inhibiteur de l'angiogenèse est un inhibiteur de la voie VEGF, un inhibiteur de la tyrosine kinase, optionnellement, dans lequel l'inhibiteur de la tyrosine kinase est un inhibiteur de la voie EGF, un inhibiteur de la tyrosine kinase réceptrice, un inhibiteur du facteur de croissance, un inhibiteur de la GTPase, un inhibiteur de la sérine/thréonine kinase, un inhibiteur du facteur de transcription, un inhibiteur de B-Raf, un inhibiteur de RAF, un inhibiteur de MEK, un inhibiteur de mTOR, un inhibiteur d'EGFR, un inhibiteur d'ALK, un inhibiteur de PARP, un inhibiteur de ROS1, un inhibiteur de BCL-2, un inhibiteur de PI3K, un inhibiteur de VEGFR, un inhibiteur de BCR-ABL, un inhibiteur de MET, un inhibiteur de MYC, un inhibiteur d'ABL, un inhibiteur de HER2, un inhibiteur de BTK, un inhibiteur de H-RAS, un inhibiteur de K-RAS, un inhibiteur de PDGFR, un inhibiteur de TRK, inhibiteur de c-KIT, inhibiteur de c-MET, inhibiteur de CDK4/6, inhibiteur de FAK, inhibiteur de FGFR, inhibiteur de FLT3, inhibiteur d'IDH1, inhibiteur d'IDH2, inhibiteur de PDGFRA ou inhibiteur de RET.

9. La cellule T modifiée ou la composition pharmaceutique destinée à être utilisée selon la revendication 8, dans laquelle :
(i) l'agent thérapeutique supplémentaire comprend un inhibiteur de BTK, optionnellement dans lequel :
- l'inhibiteur BTK est l'ibrutinib, le pirtobrutinib (Loxo-305), le tirabrutinib (ONO-4059), le tolebrutinib, l'évobrutinib, le fénébrotinib (GDC-0853), l'acalabrutinib, le spébrotinib, le zanubrutinib (BGB-3111), HM71224 ou M7583 ; et/ou
- le cancer est un lymphome à cellules du manteau, une leucémie lymphoïde chronique, un lymphome lymphocytaire petit, une leucémie lymphocytaire petite, une macroglobulinémie de Waldenström ou un lymphome de la zone marginale ;
(ii) l'agent thérapeutique supplémentaire comprend un inhibiteur de l'EGFR, dans lequel, optionnellement :
- l'inhibiteur de l'EGFR est l'osimertinib, l'erlotinib, le géfitinib, l'afatinib ou le dacomitinib ; et/ou
- le cancer est un cancer du poumon non à petites cellules ; ou
(iii) l'agent thérapeutique supplémentaire comprend un inhibiteur de la poly ADP-ribose polymérase (PARP), dans lequel, optionnellement :
- l'inhibiteur de PARP est le talazoparib, le niraparib, le rucaparib, l'olaparib, le veliparib, le CEP 9722, l'E7016, l'AG014699, le MK4827, le BMN-673 ou le pamiparib ; et/ou
- le cancer est un cancer du sein, un cancer de l'ovaire, un cancer colorectal, un cancer des trompes de Fallope, un cancer péritonéal, un cancer de la prostate, un cancer du poumon ou un mélanome.

10. La cellule T modifiée ou la composition pharmaceutique destinée à être utilisée selon la revendication 8 ou 9, dans laquelle l'agent thérapeutique supplémentaire est administré à une dose sous-thérapeutique et/ou l'agent thérapeutique supplémentaire est administré au sujet de manière séquentielle ou simultanément avec la cellule T modifiée ou la composition pharmaceutique.

11. La cellule T modifiée ou la composition pharmaceutique destinée à être utilisée selon la revendication 7, dans laquelle le procédé améliore une réponse effectrice ou une efficacité antitumorale chez le sujet.

12. La cellule T modifiée ou la composition pharmaceutique destinée à être utilisée selon la revendication 7, dans laquelle le procédé renforce les cellules T exprimant CCR7+ chez le sujet,
optionnellement dans laquelle le procédé comprend en outre l'administration d'un inhibiteur de la poly ADP-ribose polymérase (PARP), optionnellement dans laquelle l'inhibiteur de PARP est le talazoparib, le niraparib, le rucaparib, l'olaparib, le veliparib, le CEP 9722, E7016, AG014699, MK4827, BMN-673, pamiparib, ou une combinaison de ceux-ci.

13. La cellule T modifiée ou la composition pharmaceutique selon la revendication 7, dans laquelle le procédé améliore le rapport des cellules T CD4/CD8 chez le sujet,
optionnellement dans lequel le procédé comprend en outre l'administration d'un inhibiteur de la poly ADP-ribose polymérase (PARP), optionnellement dans lequel l'inhibiteur de PARP est le talazoparib, le niraparib, le rucaparib, l'olaparib, le veliparib, le CEP 9722, E7016, AG014699, MK4827, BMN-673, pamiparib, ou une combinaison de ceux-ci.

14. La cellule T modifiée ou la composition pharmaceutique destinée à être utilisée selon la revendication 12 ou 13, dans laquelle le cancer est un cancer du sein, un cancer de l'ovaire, un cancer colorectal, un cancer des trompes de Fallope, un cancer péritonéal, un cancer de la prostate, un cancer du poumon ou un mélanome.

15. La cellule T modifiée ou la composition pharmaceutique destinée à être utilisée selon l'une quelconque des revendications 12 à 14, dans laquelle le procédé comprend en outre l'administration d'un agent thérapeutique supplémentaire, optionnellement dans lequel l'agent thérapeutique supplémentaire comprend une radiothérapie, une immunothérapie cellulaire, un anticorps, un inhibiteur de molécule de point de contrôle immunitaire, une chimiothérapie, une hormonothérapie, un peptide, un antibiotique, un agent antiviral, un agent antifongique, un agent anti-inflammatoire, une thérapie par rayons UV, une thérapie par impulsions électriques, une thérapie par ultrasons focalisés de haute intensité, une thérapie par virus oncolytique, une thérapie par petites molécules ou une combinaison de celles-ci, en outre, optionnellement, dans lequel :
(i) l'immunothérapie cellulaire est un récepteur d'antigène chimérique ou un récepteur de cellules T ; ou
(ii) l'agent thérapeutique supplémentaire comprend un inhibiteur de l'angiogenèse, optionnellement, dans lequel l'inhibiteur de l'angiogenèse est un inhibiteur de la voie VEGF, un inhibiteur de la tyrosine kinase, optionnellement, dans lequel l'inhibiteur de la tyrosine kinase est un inhibiteur de la voie EGF, un inhibiteur du récepteur de la tyrosine kinase, un inhibiteur du facteur de croissance, un inhibiteur de la GTPase, un inhibiteur de la sérine/thréonine kinase, un inhibiteur du facteur de transcription, un inhibiteur de B-Raf, un inhibiteur de RAF, un inhibiteur de MEK, un inhibiteur de mTOR, un inhibiteur d'EGFR, un inhibiteur d'ALK, un inhibiteur de ROS1, un inhibiteur de BCL-2, un inhibiteur de PI3K, un inhibiteur de VEGFR, un inhibiteur de BCR-ABL, un inhibiteur de MET, un inhibiteur de MYC, un inhibiteur d'ABL, un inhibiteur de HER2, un inhibiteur de BTK, un inhibiteur de H-RAS, un inhibiteur de K-RAS, un inhibiteur de PDGFR, un inhibiteur de TRK, inhibiteur de c-KIT, inhibiteur de c-MET, inhibiteur de CDK4/6, inhibiteur de FAK, inhibiteur de FGFR, inhibiteur de FLT3, inhibiteur d'IDH1, inhibiteur d'IDH2, inhibiteur de PDGFRA ou inhibiteur de RET.
